(19) Europäisches Patentamt
European Patent Office
Office européen des brevets

(11) EP 3 110 950 B1

(12) **EUROPEAN PATENT SPECIFICATION**

(45) Date of publication and mention of the grant of the patent:
**21.09.2022 Bulletin 2022/38**

(21) Application number: **15707625.8**

(22) Date of filing: **02.03.2015**

(51) International Patent Classification (IPC):
***C12N 15/09*** *(2006.01)*

(52) Cooperative Patent Classification (CPC):
**C07K 14/705; C07K 16/00; C12N 15/1037;**
C07K 2317/31; C07K 2317/52; C07K 2317/622

(86) International application number:
**PCT/EP2015/054331**

(87) International publication number:
**WO 2015/128509 (03.09.2015 Gazette 2015/35)**

(54) **EXPRESSION CONSTRUCTS AND METHODS FOR SELECTING HOST CELLS EXPRESSING POLYPEPTIDES**

EXPRESSIONSKONSTRUKTE UND VERFAHREN ZUR AUSWAHL VON POLYPEPTIDEXPRIMIERENDEN WIRTSZELLEN

CONSTRUCTIONS D'EXPRESSION ET PROCÉDÉS PERMETTANT DE SÉLECTIONNER DES CELLULES HÔTES EXPRIMANT DES POLYPEPTIDES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **28.02.2014 EP 14157324**

(43) Date of publication of application:
**04.01.2017 Bulletin 2017/01**

(73) Proprietor: **Ichnos Sciences SA**
**2300 La Chaux-de-Fonds (CH)**

(72) Inventors:
- **AEBISCHER-GUMY, Christel**
**CH-2300 La Chaux-de-Fonds (CH)**
- **MORETTI, Pierre**
**CH-2300 La Chaux-de-Fonds (CH)**
- **BERTSCHINGER, Martin**
**CH-2300 La Chaux-de-Fonds (CH)**

(74) Representative: **Thomas, Dean et al**
**Glenmark Pharmaceuticals S.A.**
**Chemin de la Combeta 5**
**2300 La Chaux de Fonds (CH)**

(56) References cited:
**WO-A1-2007/131774 WO-A1-2009/151605**

- **BENHAR ITAI: "Design of synthetic antibody libraries", EXPERT OPINION ON BIOLOGICAL THERAPY, INFORMA HEALTHCARE, UK, vol. 7, no. 5, 1 May 2007 (2007-05-01), pages 763-779, XP009113525, ISSN: 1744-7682, DOI: 10.1517/14712598.7.5.763**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Processed by Luminess, 75001 PARIS (FR)

**Description**

**Field of the Invention**

The present invention relates to expression constructs and methods for expressing a fraction of a secreted protein of interest on the surface of eukaryotic cells using alternate splicing. The present invention also relates to methods of selecting cell(s) which express a secreted protein of interest at a desired level by detecting the membrane expression level of the protein of interest. The present invention further relates to the selection of cell(s) expressing secreted heteromultimeric proteins of interest by detecting the membrane expression level of the heteromultimeric protein of interest.

**Background of the Invention**

In order to produce a protein in a eukaryotic cell, the DNA coding for this protein has to be transcribed into a messenger RNA (mRNA) which will in turn be translated into a protein. The mRNA is first transcribed in the nucleus as pre-mRNA, containing introns and exons. During the maturation of the pre-mRNA into mature mRNA, the introns are cut out ("spliced") by cellular machinery called the spliceosome. The exons are fused together and the mRNA is modified by the addition of a so called CAP at its 5'end and a poly adenylation (poly(A)) tail at its 3' end. The mature mRNA is exported to the cytoplasm and serves as template for the translation of proteins.

Alternate splicing is a term describing that the same transcript might be spliced in different fashions leading to different mature mRNAs and in some cases to different proteins. This mechanism is used in nature to change the expression level of proteins or in order to modify the activity of certain proteins during development (Cooper TA & Ordahl CP (1985), J Biol Chem, 260(20): 11140-8). Alternate splicing is usually controlled by complex interactions of many factors (Orengo JP et al., (2006) Nucleic Acids Res, 34(22): e148).

Alternate splicing enables the production of two (or more) different RNA transcripts from the same DNA template. This can be used to produce two (or more) isoforms of the same protein or polypeptide. Throughout the specification the terms protein and polypeptide will be used interchangeably.

In nature, this is a highly controlled process, used for example during the process of antibody production by activated B cells in the human immune system. Most of the antibody is secreted into the extracellular space, but a fraction of the produced antibody is redirected from the secretory pathway to the outer cellular membrane in the form of membrane bound isoforms.

The membrane bound isoforms have the same amino acid sequence and structure as the antibody secreted into the extracellular space. The difference is a C-terminal extension of the secreted antibody's heavy chain with a transmembrane region comprising a transmembrane domain. In B cells this domain can have a length of between approximately 40 to 75 amino acids (Major JG et al., (1996) Mol. Immunol. 33: 179-87).

Expression systems for the production of recombinant polypeptides are well-known in the art. For the production of polypeptides and proteins used in pharmaceutical preparations mammalian host cells such as CHO, BHK, NS0, Sp2/0, COS, HEK and PER.C6 are generally used. For large scale production of therapeutic proteins a high producer cell line has to be generated. After transfection of the host cell line with a gene encoding the polypeptide of interest, a number of clones with different characteristics are obtained and selected for. This is routinely carried out by using, for example, a selectable marker, gene amplification and/or reporter molecules. The selection of an appropriate clone with desired properties e.g. a high producer clone, is a time consuming, often non-routine and therefore expensive process.

For the expression of heteromultimeric proteins, such as bispecific antibodies, the generation of a suitable host cell line becomes even more complicated. The protein subunits that make up the multimer can be expressed in separate cell lines and then brought together for association into the multimeric protein or alternatively the different subunits can be expressed in the same cell line. Expression of the subunits in the same cell line is associated with disadvantages wherein not all protein subunits will associate into the correct form, resulting in a mixture of different species. For the generation of bispecifc antibodies it is common for significant levels of homodimers to be produced rather than the desired heterodimer and this greatly impacts bispecific antibody production yields. Whilst the unwanted homodimers can be removed by various purification techniques, it is desirable to have a host cell line in which heterodimers are expressed at higher levels than homodimers to reduce the time and costs wasted on downstream processing.

**An expression system capable of expressing a polypeptide in both membrane-bound form and secreted form via alternative splicing has been described in** WO20019/151605**, nevertheless a system that allows the modulation of the splicing ration still remains a challenge.**

Hence there is a need for an expression system that can be used to select for cell clone(s) that express a product of interest at a high level i.e. quantitative selection. There is also a need to be able to select for cell clone(s) that express a product of interest of a desired quality i.e. qualitative selection, for instance the expression of a heteromultimeric protein of interest.

## Summary of the Invention

The present invention relates to an expression construct or set of constructs for the expression by alternate splicing of a soluble polypeptide of interest, wherein a portion of the soluble peptide is secreted into the extra cellular space and a portion is the displayed on the outer membrane of a cell(s).

The present invention also comprises methods for the selection of host cells comprising one or more constructs according to the present invention, which display on their outer membrane a polypeptide of interest at a desired level.

The present invention further relates to the selection of cell(s) expressing secreted heterodimeric proteins of interest by detecting the membrane display of the heterodimeric protein of interest on a cell(s) comprising one or more constructs according to the present invention.

**The invention is set out in the appended claim set.**

The inventors have modified a diverse group of transmembrane regions, so as to alter their properties and in particular modulate their integration into the cell membrane and hence the display of the polypeptide of interest. In particular the amino acid composition of the transmembrane region may be altered so as to reduce or increase the number of non-hydrophobic residues therein by one or more residues, as well as increase or decrease the size of the transmembrane region.

The inventors of the present invention have surprisingly found that use of a non-immunoglobulin transmembrane region for instance a murine B7-1 transmembrane region (SEQ ID NO: 173) leads to a high level of cell membrane expression compared to the levels observed when using the IgG1 transmembrane region.

The inventors have also tested other transmembrane regions from ACLV1 (NP_001070869.1) SEQ ID NO: 174, ANTR2 (NP_477520.2) SEQ ID NO: 175, CD4 (NP_000607.1) SEQ ID NO: 176, PTPRM (NP_002836.3) SEQ ID NO: 177, TNR5 (NP_001241.1) SEQ ID NO: 178, ITB1 (NP_596867.1) SEQ ID NO: 179, IGF1R (NP_000866.1) SEQ ID NO: 181, 1B07 (NP_005505.2) SEQ ID NO: 180, TRMB (NP_000352.1) SEQ ID NO: 182, IL4RA (NP_000409.1) SEQ ID NO: 183, LRP6 (NP_002327.2) SEQ ID NO: 184, GpA (NP_002090) SEQ ID NO: 185, PTCRA (NP_001230097.1) SEQ ID NO: 186 as well as modified versions of these transmembrane regions, and shown these to be suitable for use in the construct(s) according to the present invention.

In accordance with another aspect of the present invention the first stop codon is located 3' of the splice donor site of the intron.

In one embodiment the present invention also provides methods for altering the splice ratio observed for a given construct, so that a sufficient amount of polypeptide is displayed on the cell membrane to enable cell selection, whilst most of the polypeptide is expressed solubly.

The inventors have tested the different components of the claimed expression constructs allowing them to modulate the membrane display of the protein of interest.

According to the present invention, the level of soluble polypeptide expression can be increased by including a poly(A) site in the intron of the expression construct as described herein.

According to an aspect of the present invention the construct may comprise a constitutive intron positioned between the promoter and the first exon.

In accordance with the present invention the strength of the splice acceptor and the splice donor may be modified so as to increase or decrease the amount of the protein of interest displayed on the cell membrane.

In particular the consensus sequence of the splice donor site may be modified, by decreasing or increasing the % identity or similarity of the sequence of the splice donor site present in the construct to the consensus splice donor site sequence (C/A)AGGT(A/G)AGT (SEQ ID NO: 345).

In a further embodiment, the consensus sequence of the splice donor site is modified in order to decrease the splice donor strength. The consensus sequence of the splice donor site can be modified by alternative codon usage, e.g. by replacing AAA coding for lysine with an AAG coding for lysine. This will reduce the level of membrane expression. Conversely by increasing the % identity or similarity of the splice donor site to the consensus splice donor site sequence (C/A)AGGT(A/G)AGT (SEQ ID NO: 345), this will increase the strength of the splice donor site in the construct and increase the level of membrane expression.

According to an aspect of the present invention, the splice donor site overlaps the 3' end of the first exon and the 5' end of the intron and the splice acceptor site of the intron is located at the 3' end of the intron.

According to an aspect of the present invention the first stop codon is located 3' of the splice donor site.

**The** intron of the expression construct comprises a poly(Y) tract included in the splice acceptor site. The Y content of the poly(Y) tract included in the splice acceptor site of the intron of the expression construct can be modified. Altering the number of pyrimidine bases (Ys) in the poly(Y) tract can be used to decrease or increase cell membrane expression of the polypeptide of interest. For a polypeptide of interest with high level of membrane expression by the cell, reducing the number of Ys in the poly(Y) tract will reduce the level of membrane expression. For a polypeptide with low level of membrane expression by the cell, increasing the number of Ys in the poly(Y) tract will increase the level of membrane expression.

In particular the poly(Y) content of the splice acceptor may be decreased so as to decrease the strength of the splice acceptor site, by altering the poly(Y) content of the splice acceptor site, reducing the membrane display of the polypeptide of interest.

Alternatively the poly(Y) content of the splice acceptor may be increased so as to increase the strength of the splice acceptor site, by altering the poly(Y) content of the splice acceptor site, so increasing the membrane display of the polypeptide of interest.

In addition to the modifications outlined above, the alternate splicing event leading to membrane displayed polypeptide can also be influenced by modifying the DNA (and hence the RNA) sequence of the branch point region. A nucleotide of the branch point region initiates the splice event by attacking the first nucleotide of the intron at the 5' splice site, thus forming a lariat intermediate. Changing the sequence of the branch point region relative to the consensus sequence (CTRAYY SEQ ID NO: 347) can have an impact on the efficacy of the initiation of the splice event and thus on the ratio of secreted polypeptide versus membrane displayed polypeptide.

Further, the length of the intron can impact the splice ratio. It has been demonstrated that the likelihood of including an alternate exon in CD44 increases when the intron directly upstream of the exon was shortened (Bell, M. , Cowper, A., Lefranc, M., Bell, J. and Screaton, G. (1998). Influence of Intron Length on Alternative Splicing of CD44 Mol Cell Biol. 18(10): 5930-5941.). Hence a further means to modify the constructs is to shorten the length of the intron in the alternate splicing constructs so as to to increase the fraction of membrane displayed polypeptide or vice versa.

An additional mechanism to influence the splice event is the co-expression of RNA-binding proteins leading to exon inclusion or skipping. For example the proteins CUG-BP (Uniprot Acc.-No.: Q5F3T7) and muscle-blind like 3 (MBNL) (Uniprot Acc.-No.: Q5ZKW9) have been shown to influence the splice ratio in a construct expressing EGFP and dsRED (Orengo et al., 2006).

The present invention therefore also provides a further modified construct comprising expressible ORFs encoding RNA-binding proteins which cause exon inclusion or skipping. As well as methods involving the co-transfection of constructs according to the present invention, comprising expressible ORFs encoding RNA-binding proteins which cause exon inclusion or skipping and/or separate constructs comprising such ORFs.

In addition most membrane proteins pass through the endoplasmic reticulum (ER) and Golgi apparatus before reaching the cell surface. Export from the ER is a selective process that is mediated by coatomer complex II (COPII) transport vesicles that bud from sites of ER exit. Interactions between components of the COPII transport vesicles and short amino acid sequences with linear di-acid, hydrophobic and aromatic motifs or structural motifs in the cytoplasmic domain of membrane-anchored proteins concentrate cargo proteins at ER exit sites and enhance cargo recruitment into COPII vesicles. These short linear or structural amino acid sequence motifs are called ER exportation signal.

Another approach to adjust the membrane display of a protein of interest therefore is to include an ER exportation signal or not, so as to modify ER passage of the polypeptide of interest fused with the transmembrane region. Modification of the ER exportation signal comprised within the construct so as to increase ER exportation is useful for increasing the membrane display of proteins which have low half-life or other stability or degradation issues and vice versa.

The inventors of the present invention have found that the amount of polypeptide of interest displayed on the cell membrane is directly proportional to the level of expression of soluble polypeptide. Therefore host cells expressing the polypeptide of interest at high titre display more polypeptide on the membrane than host cells expressing the polypeptide at low titre. This enables the straightforward identification and isolation of high producing recombinant host cells.

In an aspect of the present invention, the polypeptide encoded by the expression construct can be part of a protein multimer for instance a heteromultimeric polypeptide such as recombinant antibody or fragments thereof. The antibody fragments may be selected from the list consisting of: Fab, Fd, Fv, dAb, $F(ab')_2$ and scFv. In a preferred embodiment, the polypeptide expressed by the expression construct can be an antibody heavy chain or fragments thereof.

In a further aspect of the present invention, the expression construct can be used for the expression of a bispecific antibody in a host cell. In one embodiment, the polypeptide expressed is an antibody heavy chain. Alternatively, the polypeptide expressed is a fragment of antibody linked to an antibody Fc region. The antibody fragment may be selected from the list consisting of: Fab, Fd, Fv, dAb, $F(ab')_2$ and scFv. Preferably the antibody fragment is a Fab or a scFv. More preferably the antibody fragment is a scFv. To effect expression of a bispecific antibody, a separate expression construct may also be provided for the expression of an antibody light chain. Co-expression of the expression constructs coding for an antibody heavy chain and an antibody fragment-Fc with an expression construct coding for an antibody light chain in host cells, results in the expression of bispecific antibody. As discussed above, expression of a bispecific antibody in host cells results in a number of unwanted homodimeric species besides the desired heterodimer. In a preferred embodiment of the invention, cell membrane display of these bispecific antibody components enables the straightforward selection of a host cell expressing predominantly heterodimeric antibodies.

The present invention also provides a method for altering the splice ratio so that a sufficient amount of polypeptide is displayed on the cell membrane to enable cell selection, whilst most of the polypeptide is expressed solubly.

In accordance with this aspect of the present invention the method involves measuring the membrane display of the polypeptide of interest and then modifying the components of the construct based upon the observed membrane display of the polypeptide of interest so as to increase or decrease membrane display so as to allow the better selection of cell(s) expressing the polypeptide of interest.

The present invention also provides a method to select a cell(s) comprising at least one construct according to the present invention, involving the selection of cell(s) by detecting the membrane expression level of the protein of interest or a heteromultimeric protein comprising the protein of interest.

**Brief Description of the Figures**

**Figure 1:** Schematic drawing of an alternate splicing construct of the present invention. Alternate splicing of the polypeptide gives rise to two splice products. For splice product 1, the codons coding for the two last amino acids glycin (G) and lysine (K) are incorporated in the mRNA and the polypeptide is secreted from the cell. For splice product 2, the polypeptide does not include the two amino acids G and K, but is extended by a transmembrane region, composed of an optional connecting region, a transmembrane domain and an optional small intracellular domain and the resulting polypeptide is not secreted, but presented on the outer membrane of the cell.

**Figure 2:** Staining profiles of CHO-S cells transfected with alternate splicing constructs for the display and secretion of an IgG1 antibody using different transmembrane regions. The analysis was performed the day after transfection. As a negative control, CHO-S cells were transfected with non-splicing constructs coding for the secreted IgG1 antibody (ctrl). The staining performed for the cells transfected with different alternate splicing constructs is presented as a filled histogram and the staining of the control cells transfected with non-splicing constructs coding for secreted IgG1 antibody is included as overlaying black line (A-D). The histograms were used in order to determine the percentage of stained cells (E) and the mean fluorescence of staining (G) of the alternate splicing constructs and the control. Titers of secreted antibody were determined 4 days after transfection using the Octet device (H).

**Figure 3:** Staining profiles of CHO-S cells transfected with alternate splicing constructs for the display and secretion of an IgG4 antibody. The analysis was performed the day after transfection. As a negative control, CHO-S cells were transfected with non-splicing constructs coding for the secreted IgG4 antibody (IgG4). The staining performed for the cells transfected with the alternate splicing construct is presented as a filled histogram and the staining of the control cells transfected with non-splicing construct coding for secreted IgG4 antibody is included as overlaying black line (A). The histograms were used in order to determine the percentage of stained cells (B) and the mean fluorescence of staining (D) of the alternate splicing construct and the control. Titers of secreted antibody were determined 4 days after transfection using the Octet device (C).

**Figure 4:** Specific detection of a bispecific antibody displayed on the cell membrane using alternate splicing technology. Cells were stained 1 day after transfection. Fc fragments (scFv-Fc and HC) were detected using a PE conjugated goat anti-human Fc gamma specific antibody (A-C, J-L). Kappa light chains were detected using a mouse anti-human kappa LC APC labelled antibody (D-F, M-O). The staining of the scFv-Fc was performed using a FITC-labelled Protein A. The staining performed for the transfected cells was presented as a filled histogram. For the first transfection cocktail (A, D, G) the profile obtained for non-transfected cells was presented as an overlay (black line). For all other experiments (B, C, E, F and H-R) the negative controls (profiles obtained for transfected cells without transmembrane domain) were presented as an overlay (dashed lines).

**Figure 5:** Titers of secreted BEAT® and scFv-Fc homodimer molecule 6 days after transient transfection.

**Figure 6:** Staining profiles of CHO-S cells transfected with alternate splicing constructs for display and secretion of an IgG1 antibody including different transmembrane regions and different modifications of the expression construct. The analysis was performed the day after transfection. As a negative control, CHO-S cells were transfected with non-splicing constructs coding for secreted IgG1 antibody (Control).

**Figure 7:** Normalized expression levels of different alternate splicing constructs for display and secretion of an IgG1 antibody transfected in CHO-S cells. The supernatant was analysed using the Octet QK device and protein A bioprobes (standard HC: Control without alternate splicing).

**Figure 8:** Staining profiles of CHO-S cells transfected with alternate splicing constructs for the display and secretion of an IgG1 antibody using the B7-1 transmembrane region and different Y content of the poly(Y) tract of the splice acceptor. The analysis was performed the day after transfection. As a negative control, CHO-S cells were transfected with non-splicing constructs coding for secreted IgG1 antibody (ctrl). The staining performed for the cells transfected with different alternate splicing constructs is presented as a filled histogram, the staining of cells transfected with the non-splicing construct coding for secreted IgG1 antibody is included as overlaying black line (A-G). The histograms were used in order to determine the percentage of stained cells (H) and the mean fluorescence of staining (J) of the alternate splicing constructs and the control. Titers of secreted antibody were determined 4 days after transfection using the Octet device (I).

**Figure 9:** Staining profiles of CHO-S cells transfected with alternate splicing constructs for the display and secretion of an IgG1 antibody using the B7-1 transmembrane region and modification of the splice donor consensus sequence. The analysis was performed the day after transfection. As a negative control, CHO-S cells were transfected with non-splicing constructs coding for secreted IgG1 (ctrl). The staining performed for the cells transfected with different alternate splicing constructs is presented as a filled histogram, the staining of cells transfected with the non-splicing constructs coding for secreted IgG1 antibody is included as overlaying black line (A-C). The histograms were used in order to determine the percentage of stained cells (D) and the mean fluorescence of staining (F) of the alternate splicing constructs and the control. Titers of secreted antibody were determined 4 days after transfection using the Octet device (E).

**Figure 10:** Staining profiles of CHO-S cells transfected with alternate splicing constructs for the display and secretion of an IgG1 antibody using the B7-1 transmembrane region with different transmembrane domains of 22-23 hydrophobic amino acids. The analysis was performed the day after transfection. As positive control, the alternate splicing construct coding for secreted and membrane displayed IgG1 with B7-1 transmembrane region including B7-1 transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different transmembrane domains is presented as filled histogram, the staining of cells transfected with the construct using the B7-1 transmembrane region including B7-1 transmembrane domain is included as overlaying black line (B-G). The histograms were used in order to determine the percentage of stained cells (H) and the mean fluorescence of staining (I) of the alternate splicing constructs and the control. Titers of secreted antibody were determined 4 days after transfection using the Octet device (G).

**Figure 11:** Staining profiles of CHO-S cells transfected with alternate splicing constructs for the display and secretion of an IgG1 antibody using the B7-1 transmembrane region with different transmembrane domains of 21-24 hydrophobic amino acids containing hydrophobic, polar and charged residues. The analysis was performed the day after transfection. As positive control, the alternate splicing construct coding for secreted and membrane displayed IgG1 with B7-1 transmembrane region including the B7-1 transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different transmembrane domains is presented as filled histogram, the staining of cells transfected with the construct using the B7-1 transmembrane region including the B7-1 transmembrane domain is included as overlaying black line (B-H). The staining performed for the cells transfected with constructs coding for secreted and membrane displayed IgG1 using PTCRA transmembrane domains is presented as filled histogram, the staining of cells transfected with non-splicing constructs coding for secreted IgG1 antibody is included as overlaying black line (I). The histograms were used in order to determine the percentage of stained cells (J) and the mean fluorescence of staining (L) of the alternate splicing constructs and the control. Titers of secreted antibody were determined 4 days after transfection using the Octet device (K).

**Figure 12:** Staining profiles of CHO-S cells transfected with alternate splicing constructs for the display and secretion of an IgG1 antibody using the B7-1 transmembrane region including PTCRA transmembrane domain with variation of the hydrophobic residues numbers in PTCRA transmembrane domain. The analysis was performed the day after transfection. As positive control, the alternate splicing construct coding for secreted and membrane displayed IgG1 with B7-1 transmembrane region and the wild-type PTCRA transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different modifications of PTCRA transmembrane domains is presented as filled histogram, the staining of cells transfected with the construct using the B7-1 transmembrane region with the wild-type PTCRA transmembrane domain is included as overlaying black line (B-H). The histograms were used in order to determine the percentage of stained cells (I) and the mean fluorescence of staining (K) of the alternate splicing constructs and the control. Titers of secreted antibody were determined 4 days after transfection using the Octet device (J).

**Figure 13:** Staining profiles of CHO-S cells transfected with alternate splicing constructs for the display and secretion of an IgG1 antibody using the B7-1 transmembrane region with different transmembrane domains of 17-19 hydrophobic amino acids containing hydrophobic, polar and charged residues. The analysis was performed the day after transfection. As positive control, the alternate splicing construct coding for secreted and membrane displayed IgG1 with B7-1 transmembrane region including B7-1 transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different transmembrane domains is presented as filled histogram, the staining of cells transfected with the construct using the B7-1 transmembrane region including B7-1 transmembrane domain is included as overlaying black line (B-D). The histograms were used in order to determine the percentage of stained cells (E) and the mean fluorescence of staining (G) of the alternate splicing constructs and the control. Titers of secreted antibody were determined 4 days after transfection using the Octet device (F).

**Figure 14:** Staining profiles of CHO-S cells transfected with alternate splicing constructs for the display and secretion of an IgG1 antibody using the B7-1 transmembrane region with different transmembrane domains of 26-27 hydrophobic amino acids containing hydrophobic, polar and charged residues. The analysis was performed the day after transfection. As positive control, the alternate splicing construct coding for secreted and membrane displayed IgG1 with B7-1 transmembrane region including the B7-1 transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different transmembrane domains is presented as filled histogram, the staining of cells transfected with the construct using the B7-1 transmembrane region including the B7-1 transmembrane domain is included as overlaying black line (B-E). The histograms were used in order to determine the percentage of stained cells (F) and the mean fluorescence of staining (H) of the alternate splicing constructs and the control. Titers of secreted antibody were determined 4 days after transfection using the Octet device (G).

**Figure 15:** Staining profiles of CHO-S cells transfected with alternate splicing constructs for the display and secretion of an IgG1 antibody using the B7-1 transmembrane region with shortened or elongated B7-1 transmembrane domains. The analysis was performed the day after transfection. As positive control, the alternate splicing construct coding for secreted and membrane displayed IgG1 with B7-1 transmembrane region including the wild-type B7-1 transmembrane domain was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different transmembrane domains is presented as filled histogram, the staining of cells transfected with the construct using the B7-1 transmembrane region including the B7-1 wild-type transmembrane domain is included as overlaying black line (B-E). The histograms were used in order to determine the percentage of stained cells (F) and the mean fluorescence of staining (H) of the alternate splicing constructs and the control. Titers of secreted antibody were determined 4 days after transient expression using the Octet device (G).

**Figure 16:** Staining profiles of CHO-S cells transfected with alternate splicing constructs for the display and secretion of an IgG1 antibody using the B7-1 transmembrane region with different cytosolic tails. The analysis was performed the day after transfection. As positive control, the alternate splicing construct coding for secreted and membrane displayed IgG1 with the B7-1 transmembrane region including the B7-1 cytosolic tail was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using different cytosolic tails is presented as filled histogram, the staining of cells transfected with the construct using the B7-1 transmembrane region including the B7-1 cytosolic tail is included as overlaying black line (B-N). The histograms were used in order to determine the percentage of stained cells (O) and the mean fluorescence of staining (Q) of the alternate splicing constructs and the control. Titers of secreted antibody were determined 4 days after transfection using the Octet device (P).

**Figure 17:**
Staining profiles of CHO-S cells transfected with alternate splicing constructs for the display and secretion of an IgG1 antibody using the M1M2 transmembrane region with different cytosolic tails. The analysis was performed the day after transfection. As positive control, the alternate splicing construct coding for secreted and membrane displayed IgG1 with M1M2 transmembrane region including the M1M2 cytosolic tail was transfected (filled histogram in (A)) and as a negative control, CHO-S cells were transfected with non-splicing constructs coding for secreted IgG1 antibody (overlaying black line in (A)). The staining performed for the cells transfected with constructs coding for secreted and membrane displayed IgG1 using the M1M2 transmembrane region with the B7-1 cytosolic tail is presented as filled histogram, the staining of cells transfected with the construct using the M1M2 transmembrane region including the M1M2 cytosolic tail is included as overlaying black line (B). The histograms were used in order to determine the percentage of stained cells (C) and the mean fluorescence of staining (E) of the alternate splicing constructs and the control. Titers of secreted antibody were determined 4 days after transfection using the Octet device (D).

**Figure 18:** Assembly variants of the three subunits of the BEAT bispecific antibody.

**Figure 19:** Example of a dot plot obtained after dual kappa light chain (LC) and Fc fragment staining of a selected stable cell pool. The potential molecules detected by the staining on the cell membrane are displayed on the right. Fc fragment were detected using a PE conjugated goat anti-human Fc gamma specific antibody. Kappa light chains were detected using a mouse anti-human kappa LC APC labelled antibody.

**Figure 20:** Correlation between surface staining and secretion profiles of stable pools transfected with alternate splicing vectors. The percentage of Q6* and Q7* plotted refer to 100% being the entire producing cell population (excluding Q8).

**Figure 21:** Dot plot obtained after surface staining of a stable cell pool prior cell sorting. The staining uses soluble Target 1 and 2 for the detection of the binders displayed on the cell surface. The potential molecules detected by the staining on the cell membrane are indicated in the corresponding quadrants.

**Figure 22:** Correlation between the fraction of BEAT® molecule detected in the supernatants of a 14 days fed-batch and the fraction of cells displaying a BEAT® phenotype on their cell surface.

**Figure 23:** Example of the measured surface Fc profile obtained for a selected stable cell pool transfected with the alternate splicing constructs. IgG present on the cell membrane were detected using a PE conjugated goat anti-human Fc gamma specific antibody. Living cells were gated in a FSC vs SSC dot plot (g1 in A) to display the fluorescence distribution of the tested alternate splicing pools (filled histogram, B). The staining of an IgG secreting clone lacking the alternate splicing construct was used a negative control and is presented as an overlay (dashed line).

**Figure 24:** Correlation between surface staining intensity and expression level of secreting cells assayed during a batch process: correlation between surface intensity and IgG titer on day 1 of a supplemented batch (A) and correlation between the surface intensity on day 1 and the qP measured during the exponential phase (day 1-3) (B)

**Figure 25:** Surface staining intensity on day 1 is predictive of the accumulated secreted IgG on day 7.

**Figure 26:** Selection of high producers according to the IgG surface display. Cells displaying "low", "medium" and "high" density of IgG on their membrane were selected by flow cytometric cell sorting (A). The distribution of the surface fluorescence was determined two weeks following sorting (B) and the specific productivity of the sorted clones was assessed in a supplemented batch (C).

**Detailed Description of the Invention**

The present invention provides expression constructs and methods for the cell membrane expression of polypeptides, especially heteromultimeric polypeptides. In particular the protein of interest may be recombinant antibodies or fragments thereof or bispecific antibodies, in host cells using alternate splicing. The level of cell membrane display is indicative of the secretion level of the polypeptide in a recombinant host cell and for heterodimeric antibodies the cell membrane display is also indicative of the secretion profile i.e. heterodimer or homodimer expression.

The term "expression construct" or "construct" as used interchangeably herein includes a polynucleotide sequence encoding a polypeptide to be expressed and sequences controlling its expression such as a promoter and optionally an enhancer sequence, including any combination of cis-acting transcriptional control elements. The sequences controlling the expression of the gene, i.e. its transcription and the translation of the transcription product, are commonly referred to as regulatory unit. Most parts of the regulatory unit are located upstream of coding sequence of the gene and are operably linked thereto. The expression construct may also contain a downstream 3' untranslated region comprising a poly(A) site.

The regulatory unit of the invention is either operably linked to the gene to be expressed, i.e. transcription unit, or is separated therefrom by intervening DNA such as for example by the 5'-untranslated region (5'UTR) of the heterologous gene. Preferably the expression construct is flanked by one or more suitable restriction sites in order to enable the insertion of the expression construct into a vector and/or its excision from a vector. Thus, the expression construct according to the present invention can be used for the construction of an expression vector, in particular a mammalian expression vector.

The term "polynucleotide sequence encoding a polypeptide" as used herein includes DNA coding for a gene, preferably a heterologous gene expressing the polypeptide.

The terms "heterologous coding sequence", "heterologous gene sequence", "heterologous gene", "recombinant gene" or "gene" are used interchangeably. These terms refer to a DNA sequence that codes for a recombinant polypeptide, in particular a recombinant heterologous protein product that is sought to be expressed in a host cell, preferably in a mammalian cell and harvested for further use. The product of the gene can be a polypeptide, glycopeptide or lipoglycopeptides. The heterologous gene sequence may not naturally be present in the host cell and is derived from an organism of the same or a different species and may be genetically modified. Alternatively the heterologous gene sequence is naturally present in the host cell.

The terms "protein" and "polypeptide" are used interchangeably to include a series of amino acid residues connected to the other by peptide bonds between the alpha-amino and carboxy groups of adjacent residues.

The term "promoter" as used herein defines a regulatory DNA sequence generally located upstream of a gene that mediates the initiation of transcription by directing RNA polymerase to bind to DNA and initiating RNA synthesis. Promoters for use in the invention include, for example, viral, mammalian, insect and yeast promoters that provide for high levels of expression, e.g. the mammalian cytomegalovirus or CMV promoter, the SV40 promoter, or any promoter known in the art suitable for expression in eukaryotic cells. Promoters particularly suitable for use in an expression construct of the present invention can be selected from the list consisting of: SV40 promoter, human tk promoter, MPSV promoter, mouse CMV, human CMV, rat CMV, human EF1alpha, Chinese hamster EF1alpha, human GAPDH, hybrid promoters including MYC, HYK and CX promoter, synthetic promoters based on rearrangement of transcription factor binding sites. A particular preferred promoter of the present invention is the mouse CMV promoter.

The term "5' untranslated region (5'UTR)" refers to an untranslated segment in the 5' terminus of the pre-mRNA or mature mRNA. On mature mRNA, the 5'UTR typically harbours on its 5' end a 7-methylguanosine cap and is involved in many processes such as splicing, polyadenylation, mRNA export towards the cytoplasm, identification of the 5' end of the mRNA by the translational machinery and protection of the mRNAs against degradation (Cowling VH (2010) Biochem J, 425: 295-302).

The term "intron" refers to a segment of nucleic acid non-coding sequence that is transcribed and is present in the pre-mRNA but is excised by the splicing machinery based on the sequences of the splice donor site and splice acceptor site, respectively at the 5' and 3' ends of the intron, and therefore not present in the mature mRNA transcript. Typically introns have an internal site, called the branch point, located between 20 and 50 nucleotides upstream of the 3' splice site. The length of the intron used in the present invention may be between 50 and 10000 nucleotides long. A shortened intron may comprise 50 or more nucleotides. A full length intron may comprise more than 10000 nucleotides. Introns suitable for use in an expression construct of the present invention can be selected from the list consisting of: synthetic or artificial introns; or naturally occurring introns such as -globin/IgG chimeric intron, -globin intron, IgG intron, mouse CMV first intron, rat CMV first intron, human CMV first intron, Ig variable region intron and splice acceptor site (Bothwell et al., (1981) Cell, 24: 625-637; US5,024,939), introns of the chicken TNT gene and the first intron of EF1 alpha or modified versions thereof.

The intron used in the present invention may have splice acceptor and splice donors sites from different introns, e.g. the intron may comprise a splice donor site from a human IgG intron e.g. the splice donor site of the *ighg1* gene and a splice acceptor site from the chicken cTNT intron 4.

In a preferred embodiment the intron comprises a splice donor site from a human IgG intron and the splice acceptor site from the chicken cTNT intron 4. More preferred are introns comprising the splice donor site from a human IgG intron and a splice acceptor selected from the group consisting of chicken TNT intron 4 (SEQ ID NO: 69) and constructs derived from chicken TNT intron 4 (SEQ ID NOs: 70-78).

The term "exon" refers to a segment of nucleic acid sequence that is transcribed into mRNA and maintained in the mature mRNA after splicing.

The term "splice site" refers to specific nucleic acid sequences that are capable of being recognized by the splicing machinery of a eukaryotic cell as suitable for being cut and/or ligated to a corresponding splice site. Splice sites allow for the excision of introns present in a pre-mRNA transcript. Typically the 5' portion of the splice site is referred to as the splice donor site and the 3' corresponding splice site is referred to as the splice acceptor site. The term splice site includes, for example, naturally occurring splice sites, engineered splice sites, for example, synthetic splice sites, canonical or consensus splice sites, and/or non-canonical splice sites, for example, cryptic splice sites. Splice donor or acceptor sites suitable for use in an expression construct of the present invention can be selected from the list consisting of: Splice donor and acceptor from -globin/IgG chimeric intron, splice donor and acceptor from -globin intron, splice donor and acceptor from IgG intron, splice donor and acceptor from mouse CMV first intron, splice donor and acceptor from rat CMV first intron, splice donor and acceptor from human CMV first intron, splice donor and acceptor from Ig variable region intron and splice acceptor sequence (Bothwell et al., (1981) Cell, 24: 625-637; US5,024,939), splice donor and acceptor from introns of the chicken TNT gene and splice donor and acceptor from the first intron of the EF1alpha gene or fusion constructs thereof. In a preferred embodiment the splice donor site is from the human IgG intron and the splice acceptor site is selected from the group consisting of chicken TNT intron 4 (SEQ ID NO: 69) and constructs derived from chicken TNT intron 4 (SEQ ID NOs: 70- 78).

The term consensus sequence of the splice donor region as used herein refers to the sequence (C/A)AGGU(A/G)AGU (underlined sequences are part of the intron) SEQ ID NO: 345 as described in "Molecular Biology of the Cell" (Alberts et al., Garland Publishing, New York 1995).

The term consensus sequence of the splice acceptor region as used herein refers to the sequence *CTRAYY*- - - poly(Y) tract- - - NCAGG (underlined sequences are part of the intron; in italics: branch point region) SEQ ID NO: 346 as described in "Molecular Biology of the Cell" (Alberts et al., Garland Science, New York 2002).

The term "stop codon" refers to any one of the three stop codons that signal termination of protein synthesis (TAA (RNA: UAA), TAG (RNA: UAG) and TGA (RNA: UGA). In order to avoid incomplete termination efficiency or "leakiness" of a stop codon, 2,3 or multiple stop codons can be used to signal termination of protein synthesis.

The term "transmembrane region" refers to a polypeptide or protein which is encoded by a nucleic acid sequence and which comprises an optional extracellular part, a transmembrane domain and an optional cytosolic tail. A transmembrane domain is any three-dimensional protein structure which is thermodynamically stable in a membrane and usually comprises a single transmembrane alpha helix of a transmembrane protein, predominantly composed of hydrophobic amino acids. The length of the transmembrane domain is in average 21 amino acids, but might vary between 4 to 48 amino acids (Baeza-Delgado et al., 2012). A transmembrane region comprises an optional N-terminal extracellular connecting stretch of amino acids and a transmembrane domain. In some embodiments, the transmembrane region may further comprise a C-terminal cytoplasmic amino acid stretch or an intracellular domain. The transmembrane region that is found in the human *ighg1* gene e.g. is composed of a connecting stretch of amino acids followed by the two domains M1 and M2 (this transmembrane region will be referred to as "M1M2" or "IgG1 transmembrane region"). Transmembrane regions of use in the invention include, but are not limited to, the transmembrane region of the human platelet-derived growth factor receptor (PDGFR) gene (Swissprot entry P16234), human asialoglycoprotein receptor (Swissprot entry P07306), human and murine B7-1 (human: Swissprot entry P33681 and murine: Swissprot entry Q00609), human ICAM-1 (Swissprot entry P05362), human erbb1 (Swissprot entry P00533), human erbb2 (Swissprot entry P04626), human erbb3 (Swissprot entry P21860), human erbb4 (Swissprot entry Q15303), human fibroblast growth factor receptors such as FGFR 1(Swissprot entry P11362),FGFR2 (Swissprot entry P21802), FGFR3 (Swissprot entry P22607), FGFR4 (Swissprot entry P22455), human VEGFR-1 (Swissprot entry P17948), human VEGFR-2 (Swissprot entry P35968), human erythropoietin receptor (Swissprot entry P19235), human PRL-R, prolactin receptor (Swissprot entry P16471), human EphA1, Ephrin type-A receptor 1 (Swissprot entry P21709), human insulin (Swissprot entry P06213), Insulin-like growth factor 1 receptor (IGFR1, Swissprot entry P08069, SEQ ID NO: 181), human receptor-like protein tyrosine phosphatases (Swissprot entries Q12913, P23471, P23467, P18433, P23470, P23469, P23468), human neuropilin (Swissprot entry PO14786), human major histocompatibility complex class II (alpha and beta chains), human integrins (alpha and beta families), human Syndecans, human Myelin protein, human cadherins, human synaptobrevin-2 (Swissprot entry P63027), human glycophorin-A (GpA, Swissprot entry P02724, SEQ ID NO: 185), human Bnip3 (Swissprot entry Q12983), human APP (Swissprot entry P05067), amyloid precursor protein (Swissprot entry PODJI8), human T-cell receptor alpha gene (PTCRA, Swissprot entry PQ6ISU1, SEQ ID NO: 186) and T-cell receptor beta, CD3 gamma (Swissprot entry P09693), CD3 delta (Swissprot entry P04234), CD3 zeta (Swissprot entry P20963), and CD3 epsilon (CD3E, Swissprot entry P07766, SEQ ID NO: 197), human Serine/threonine-protein kinase receptor R3 (ACVL1, Swissprot entry P37023, SEQ ID NO: 174), human Anthrax toxin receptor 2 (ANTR2, Swissprot entry P58335, SEQ ID NO: 175), human T-cell surface glycoprotein CD4 (CD4, Swissprot entry P01730, SEQ ID NO: 176), human Receptor-type tyrosine-protein phosphatase mu (PTPRM, Swissprot entry P28827, SEQ ID NO: 177), human Tumor necrosis factor receptor superfamily member 5 (TNR5, Swissprot entry P25942, SEQ ID NO: 178). Human Integrin beta-1 (ITB1, Swissprot entry P05556, SEQ ID NO: 179), human HLA class I histocompatibility antigen, B-7 alpha chain (Swissprot entry P01889, SEQ ID NO: 180), human Thrombomodulin (TRBM, Swissprot entry P07204, SEQ ID NO: 182), human Interleukin-4 receptor subunit alpha (IL4RA, Swissprot entry P24394 , SEQ ID NO: 183), human Low-density lipoprotein receptor-related protein 6 (LRP6, Swissprot entry 075581, SEQ ID NO: 184), human High affinity immunoglobulin epsilon receptor subunit alpha (FCERA, Swissprot entry P12319, SEQ ID NO: 194), human Killer cell immunoglobulin-like receptor 2DL2 (KI2L2, Swissprot entry P43627, SEQ ID NO: 195), human Cytokine receptor common subunit beta (IL3RB, Swissprot entry P32927, SEQ ID NO: 196), human Integrin alpha-IIb (ITA2B, Swissprot entry P08514, SEQ ID NO: 198), human T-cell-specific surface glycoprotein CD28 (CD28, Swissprot entry P10747, SEQ ID NO: 199)

In a preferred embodiment the transmembrane region used in the present invention is selected form the group consisting of the human B7-1 transmembrane region, the murine B7-1 transmembrane region, the PDGFR transmembrane region, the human asialoglycoprotein receptor transmembrane region and the erbb-2 transmembrane region. More preferred is the murine B7-1 transmembrane region, most preferred is the murine B7-1 transmembrane region as shown in SEQ ID NO: 66.

An immunoglobulin transmembrane region includes the transmembrane region from the human immunoglobulin genes IGHA1 (NCBI access code: M60193), IGHA2 (NCBI access code: M60194), IGHD (NCBI access code: K02881), IGHE (NCBI access code: X63693), IGHG1 (NCBI access code: X52847), IGHG2 (NCBI access code: AB006775), IGHG3 (NCBI access code: D78345), IGHG4(NCBI access code: AL928742), IGHGP (NCBI access code: X52849), IGHM (NCBI access code: X14940) as well as the transmembrane regions from the murine immunoglobulin genes IGHA1 (NCBI access code:K00691), IGHD (NCBI access code: J00450), IGHE (NCBI access code: X03624, U08933), IGHG1 (NCBI access code:J00454, J00455), IGHG2A (NCBI access code: J00471), IGHG2B (NCBI access code: J00462, D78344), IGHG3 (NCBI access code: X00915, V01526), IGHM (NCBI access code: J00444).

In one embodiment of the invention the transmembrane region used is a non-immunoglobulin transmembrane region which does not comprise transmembrane regions encoded by immunoglobulin genes, in a particular a non-immunoglobulin transmembrane region which does not comprise transmembrane regions encoded by the human immunoglobulin genes IGHA1 (NCBI access code: M60193), IGHA2 (NCBI access code: M60194), IGHD (NCBI access code: K02881), IGHE (NCBI access code: X63693), IGHG1 (NCBI access code: X52847), IGHG2 (NCBI access code: AB006775), IGHG3 (NCBI access code: D78345), IGHG4(NCBI access code: AL928742), IGHGP (NCBI access code: X52849), IGHM (NCBI access code: X14940) and does not comprise transmembrane regions encoded by the murine immunoglobulin genes IGHA1 (NCBI access code:K00691), IGHD (NCBI access code: J00450), IGHE (NCBI access code: X03624, U08933), IGHG1 (NCBI access code:J00454, J00455), IGHG2A (NCBI access code: J00471), IGHG2B (NCBI access code: J00462, D78344), IGHG3 (NCBI access code: X00915, V01526), IGHM (NCBI access code: J00444).

The term "poly(Y) tract" refers to the stretch of nucleic acids found between the branch point of the intron and the intron-exon border. This stretch of nucleic acids has an abundance of pyrimidines (Ys), meaning an abundance of the pyrimidine bases C or T.

The term "3’untranslated region (3UTR)" refers to an untranslated segment in the 3’terminus of the pre-mRNAs or mature mRNAs. On mature mRNAs this region harbours the poly(A) tail and is known to have many roles in mRNA stability, translation initiation and mRNA export (Jia J et al., (2013) Curr Opin Genet Dev, 23(1): 29-34).

The term "enhancer" as used herein defines a nucleotide sequence that acts to potentiate the transcription of genes independent of the identity of the gene, the position of the sequence in relation to the gene, or the orientation of the sequence. The vectors of the present invention optionally include enhancers.

The term "polyadenylation signal" or "poly(A) signal" or "poly(A)" or "poly(A) site" refers to a nucleic acid sequence present in the mRNA transcripts, that allows for the transcripts, when in the presence of the poly(A) polymerase, to be polyadenylated on the polyadenylation site located 10 to 30 bases downstream the poly(A) signal. Many poly(A) signals are known in the art and may be useful in the present invention. Examples include the human variant growth hormone poly(A) signal, the SV40 late poly(A) signal and the bovine growth hormone poly(A) signal.

The terms "functionally linked" and "operably linked" are used interchangeably and refer to a functional relationship between two or more DNA segments, in particular gene sequences to be expressed and those sequences controlling their expression. For example, a promoter and/or enhancer sequence, including any combination of cis-acting transcriptional control elements is operably linked to a coding sequence if it stimulates or modulates the transcription of the coding sequence in an appropriate host cell or other expression system. Promoter regulatory sequences that are operably linked to the transcribed gene sequence are physically contiguous to the transcribed sequence.

"Orientation" refers to the order of nucleotides in a given DNA sequence. For example, an orientation of a DNA sequence in opposite direction in relation to another DNA sequence is one in which the 5’ to 3’ order of the sequence in relation to another sequence is reversed when compared to a point of reference in the DNA from which the sequence was obtained. Such reference points can include the direction of transcription of other specified DNA sequences in the source DNA and/or the origin of replication of replicable vectors containing the sequence.

The term "nucleic acid sequence homology" or "nucleotide sequence homology" as used herein include the percentage of nucleotides in the candidate sequence that are identical with the nucleotide sequence of the comparison sequence after aligning the sequences and introducing gaps, if necessary, to achieve the maximum per cent sequence identity. Thus sequence identity can be determined by standard methods that are commonly used to compare the similarity in position of the nucleotides of two nucleotide sequences.

The term "expression vector" as used herein includes an isolated and purified DNA molecule which upon transfection into an appropriate host cell provides for an appropriate expression level of a recombinant gene product within the host cell. In addition to the DNA sequence coding for the recombinant or gene product the expression vector comprises regulatory DNA sequences that are required for an efficient transcription of the DNA coding sequence into mRNA and for an efficient translation of the mRNAs into proteins in the host cell line.

The terms "host cell" or "host cell line" as used herein include any cells, in particular mammalian cells, which are capable of growing in culture and expressing a desired recombinant product protein.

Recombinant polypeptides and proteins can be produced in various expression systems such as prokaryotic (e.g. *E.coli*), eukaryotic (e.g. yeast, insect, vertebrate, mammalian), and *in vitro* expression systems. Most commonly used methods for the large-scale production of protein-based biologics rely on the introduction of genetic material into host cells by transfection of DNA vectors. Transient expression of polypeptides can be achieved with transient transfection of host cells. Integration of vector DNA into the host cell genome results in a cell line that is stably transfected and propagation of such a stable cell line can be used for the large-scale production of polypeptides and proteins.

Methods for producing multiple polypeptides in a eukaryotic cell by means of alternate splicing are described in WO2005/089285. Two different expression cassettes are under control of a single promoter wherein the expression cassettes have splice sites which allow for their alternative splicing and expression as two or more independent gene products at a desired ratio.

The present inventors have found however, that such an approach is limited for the expression of a soluble polypeptide variant and a plasma-membrane-bound variant as two entirely independent proteins will be produced and hence, the plasma-membrane-bound variant does not represent the desired product quality of the soluble polypeptide variant.

Methods for selecting eukaryotic cells expressing a heterologous protein and expression of a soluble polypeptide variant and a plasma-membrane-bound variant from an alternatively spliceable nucleic acid are described in WO2007/131774. The plasma-membrane-bound variant is connected to the cell expressing it and can be used as a marker to isolate cells that have been successfully transfected.

The present inventors have found however, that such an approach is limited as there is no means to control the ratio of soluble polypeptide expression to membrane displayed polypeptide. Alternate splicing, as mentioned above, is a process by which different variants of a polypeptide can be expressed but since the mechanisms of alternate splicing are highly variable, without any ability to control the splicing ratio, the amounts of polypeptide variants expressed will vary.

A cell expression system whereby a portion of the expressed polypeptide is re-directed to be expressed on the cell membrane will therefore have reduced titres of soluble polypeptide. Whilst cell membrane expression of polypeptides is a useful marker to isolate high expressing clones, without any form of control of the amount of polypeptide expressed on the cell membrane, the titres of soluble polypeptide can be reduced significantly. Furthermore, no two polypeptides express at the same level under the same culture conditions, therefore a method developed for the soluble and cell membrane expression of one polypeptide is unlikely to be suitable for all polypeptides.

In contrast to the alternate splicing approaches described previously, the present applicants have designed an alternate splicing approach for the expression of both soluble and cell membrane displayed polypeptide, in which the components of the expression construct can be modified to optimize the amount of soluble polypeptide expressed whilst still permitting cell membrane expression of the polypeptide at an amount sufficient to enable cell clone selection.

In an exemplary expression construct of the present invention for the expression of an antibody heavy chain, the IgG1 heavy chain constant region contains a weak splice donor upstream of the stop codon terminating the open reading frame of the secreted heavy chain. A splice acceptor site and the DNA sequence coding for a transmembrane region followed by a stop codon and a poly(A) site located 3' of the stop codon so that the transmembrane region is in frame with the open reading frame of the IgG1 heavy chain after splicing. The resulting alternative open reading frames are terminated by a stop codon and a poly(A) site, respectively. The applicants have found that by modifying this expression construct they can alter the splice ratio and therefore the amount of soluble polypeptide versus membrane displayed polypeptide.

These modifications can include, for example, use of a heterologous transmembrane region. The naturally occurring transmembrane region of IgG1 is composed by a connecting stretch of amino acids and the domains M1 and M2. By replacement of the IgG1 transmembrane region with a transmembrane region which is a non-immunoglobulin transmembrane region e.g. by replacement with the murine B7-1 transmembrane region, the amount of membrane displayed polypeptide can be increased. As is shown in Figure 2 of Example 2, almost 40% of the transiently transfected cells were positive for membrane displayed IgG1, which is a significant increase over the percentage of cells transfected with a construct comprising the IgG1 transmembrane region (up to 20%). Such a result is surprising as it would be expected that use of the natural IgG1 transmembrane region would be most efficient for the cell membrane display of IgG1, not a heterologous transmembrane region. Preferably, the transmembrane region used in an expression construct of the present invention comprises a transmembrane region selected from the group consisting of the transmembrane region of human platelet-derived growth factor receptor (PDGFR) gene, human asialoglycoprotein receptor, human and murine B7-1, human ICAM-1, human erbb1, human erbb2, human erbb3, human erbb4, human fibroblast growth factor receptors such as FGFR 1,FGFR2, FGFR3, FGFR4, human VEGFR-1, human VEGFR-2, human erythropoietin receptor, human PRL-R, prolactin receptor, human EphA1, Ephrin type-A receptor 1, human insulin, IGF-1 receptors, human receptor-like protein tyrosine phosphatases, human neuropilin, human major histocompatibility complex class II (alpha and beta chains), human integrins (alpha and beta families), human Syndecans, human Myelin protein, human cadherins, human synaptobrevin-2, human glycophorin-A, human Bnip3, human APP, amyloid precursor protein, human T-cell receptor alpha (PTCRA) and T-cell receptor beta, CD3 gamma, CD3 delta, CD3 zeta, and CD3 epsilon. In a preferred embodiment the transmembrane region used in this invention is selected form the group consisting of the human B7-1 transmembrane region, the murine B7-1 transmembrane region, the PDGFR transmembrane region, the human asialoglycoprotein receptor transmembrane region and the erbb-2 transmembrane region. More preferred is the murine B7-1 transmembrane region, most preferred is the murine B7-1 transmembrane region as shown in SEQ ID NO: 66.

Further modifications to alter the splice ratio of the amount of secreted polypeptide versus membrane displayed polypeptide include altering the number of pyrimidine bases in a polypyrimidine (poly(Y)) tract upstream of the exon coding for the transmembrane region and/or modifying the splice donor and acceptor consensus sequences. Decreasing the number of pyrimidine bases (Ys) has been shown in Example 3 to cause a shift in the splice ratio towards secreted protein. If no pyrimidine bases are included in the construct, there is no surface expression of the protein of interest. Such a mechanism to shift the splice ratio away from cell membrane expression could be useful for a protein which is expressed strongly on the cell membrane but shows weaker soluble expression. The number of pyrimidine bases in the poly(Y) tract may comprise between 0 and 30 bases. Preferably, the poly(Y) tract comprises 20 pyrimidine bases or less, more preferably 15 bases or less, even more preferably 10 bases or less.

Since an increase in the amount of membrane displayed protein can have a negative impact on the titres of soluble polypeptide, the inventors have developed modifications to the expression construct that can increase soluble polypeptide expression in the host cell without impacting on the amount of membrane displayed polypeptide. The addition of a poly(A) site in the intron was found to increase soluble polypeptide titre by a maximum of 50 % (for the M1M2 transmembrane region) while maintaining a significant level of cell membrane display. In a further embodiment of the present invention, a poly(A) site is located in the intron of the expression construct.

In an aspect of the present invention, the expression construct is suitable for polypeptide multimers and proteins for example antibodies or fragments thereof or bispecific antibodies or fragments thereof.

The term "antibody" as referred to herein includes whole antibodies and any antigen binding fragments or single chains thereof. An "antibody" refers to a glycoprotein comprising at least two heavy (H) chains and two light (L) chains inter-connected by disulfide bonds, or an antigen binding fragment thereof. Each heavy chain is comprised of a heavy chain variable region (abbreviated herein as VH) and a heavy chain constant region. The heavy chain constant region is comprised of three domains, CH1, CH2 and CH3. Each light chain is comprised of a light chain variable region (abbreviated herein as VL) and a light chain constant region. The light chain constant region is comprised of one domain, CL. The VH and VL regions can be further subdivided into regions of hypervariability, termed complementarity determining regions (CDR) which are hypervariable in sequence and/or involved in antigen recognition and/or usually form structurally defined loops, interspersed with regions that are more conserved, termed framework regions (FR or FW). Each VH and VL is composed of three CDRs and four FWs, arranged from amino- terminus to carboxy-terminus in the following order: FW1, CDR1, FW2, CDR2, FW3, CDR3, FW4. The amino acid sequences of FW1, FW2, FW3, and FW4 all together constitute the "non-CDR region" or "non-extended CDR region" of VH or VL as referred to herein.

The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system.

Antibodies are grouped into classes, also referred to as isotypes, as determined genetically by the constant region. Human constant light chains are classified as kappa (CK) and lambda (C) light chains. Heavy chains are classified as mu ($\mu$), delta ($\delta$), gamma ($\gamma$), alpha ($\alpha$), or epsilon ($\varepsilon$), and define the antibody's isotype as IgM, IgD, IgG, IgA, and IgE, respectively. The IgG class is the most commonly used for therapeutic purposes. In humans this class comprises subclasses IgG1, IgG2, IgG3 and IgG4.

The term "Fab" or "Fab region" as used herein includes the polypeptides that comprise the VH, CH1, VL, and CL immunoglobulin domains. Fab may refer to this region in isolation, or this region in the context of a full length antibody or antibody fragment.

The term "Fc" or "Fc region", as used herein includes the polypeptide comprising the constant region of an antibody excluding the first constant region immunoglobulin domain. Thus Fc refers to the last two constant region immunoglobulin domains of IgA, IgD, and IgG, and the last three constant region immunoglobulin domains of IgE and IgM, and the flexible hinge N-terminal to these domains. For IgA and IgM, Fc may include the J chain. For IgG, Fc comprises immunoglobulin domains C gamma 2 and C gamma 3 (C2 and C3) and the hinge between C gamma 1 (C1) and C gamma 2 (C2). Although the boundaries of the Fc region may vary, the human IgG heavy chain Fc region is usually defined to comprise residues C226 or P230 to its carboxyl-terminus, wherein the numbering is according to the EU numbering system. For human IgG1 the Fc region is herein defined to comprise residue P232 to its carboxyl-terminus, wherein the numbering is according to the EU numbering system (Edelman GM et al., (1969) Proc Natl Acad Sci USA, 63(1): 78-85). Fc may refer to this region in isolation or this region in the context of an Fc polypeptide, for example an antibody.

The term "full length antibody" as used herein includes the structure that constitutes the natural biological form of an antibody, including variable and constant regions. For example, in most mammals, including humans and mice, the full length antibody of the IgG class is a tetramer and consists of two identical pairs of two immunoglobulin chains, each pair having one light and one heavy chain, each light chain comprising immunoglobulin domains VL and CL, and each heavy chain comprising immunoglobulin domains VH, CH1 (C1), CH2 (C2), and CH3 (C3). In some mammals, for ex ample in camels and llamas, IgG antibodies may consist of only two heavy chains, each heavy chain comprising a variable domain attached to the Fc region.

Antibody fragments include, but are not limited to, (i) the Fab fragment consisting of VL, VH, CL and CH1 domains, including Fab and Fab -SH, (ii) the Fd fragment consisting of the VH and CH1 domains, (iii) the Fv fragment consisting of the VL and VH domains of a single antibody; (iv) the dAb fragment (Ward ES et al., (1989) Nature, 341: 544-546) which consists of a single variable, (v) $F(ab')_2$ fragments, a bivalent fragment comprising two linked Fab fragments (vi) single chain Fv molecules (scFv), wherein a VH domain and a VL domain are linked by a peptide linker which allows the two domains to associate to form an antigen binding site (Bird RE et al., (1988) Science 242: 423-426; Huston JS et al., (1988) Proc. Natl. Acad. Sci. USA, 85: 5879-83), (vii) bispecific single chain Fv dimers (PCT/US92/09965), (viii) "diabodies" or "triabodies", multivalent or multispecific fragments constructed by gene fusion (Tomlinson I & Hollinger P (2000) Methods Enzymol. 326: 461-79; WO94/13804; Holliger P et al., (1993) Proc Natl Acad Sci USA, 90: 6444-48) and (ix) scFv genetically fused to the same or a different antibody (Coloma MJ & Morrison SL (1997) Nature Biotech, 15(2): 159-163).

Antibodies and fragment thereof that can be expressed by an expression construct as described herein may bind to an antigen selected from the group consisting of: AXL, Bcl2, HER2, HER3, EGF, EGFR, VEGF, VEGFR, IGFR, PD-1, PD-1L, BTLA, CTLA-4, GITR, mTOR, CS1, CD3, CD16, CD16a, CD19, CD20, CD22, CD25, CD27, CD28, CD30, CD32b, CD33, CD38, CD40, CD52, CD64, CD79, CD89, CD137, CD138, CA125, cMet, CCR6, MUCI, PEM antigen, Ep-CAM, EphA2, 17-1a, CEA, AFP, HLA class II, HLA-DR, HSG, IgE, IL-12, IL-17a, IL-18, IL-23, IL-1alpha, IL-1beta, GD2-ganglioside, MCSP, NG2, SK-I antigen, Lag3, PAR2, PDGFR, PSMA, Tim3, TF, CTLA4, TL1A, TIGIT, SIRPa, ICOS, Treml2, NCR3, HVEM, OX40, VLA-2 and 4-1BB.

Bispecific or heterodimeric antibodies have been available in the art for many years. However the generation of such antibodies is often associated with the presence of mispaired byproducts, which reduces significantly the production yield of the desired bispecific antibody and requires sophisticated purification procedures to achieve product homogeneity. The mispairing of immunoglobulin heavy chains can be reduced by using several rational design strategies, most of which engineer the antibody heavy chains for heterodimerisation via the design of man-made complementary heterodimeric interfaces between the two subunits of the CH3 domain homodimer. The first report of an engineered CH3 heterodimeric domain pair was made by Carter *et al.* describing a "protuberance-into-cavity" approach for generating a hetero-dimeric Fc moiety (US5,807,706; 'knobs-into-holes'; Merchant AM et al., (1998) Nat Biotechnol, 16(7):677-81). Alternative designs have been recently developed and involved either the design of a new CH3 module pair by modifying the core composition of the modules as described in WO2007110205 or the design of complementary salt bridges between modules as described in WO2007147901 or WO2009089004. The disadvantage of the CH3 engineering strategies is that these techniques still result in the production of a significant amount of undesirable homo-dimers. A more preferred technique for generating bispecific antibodies in which predominantly heterodimers are produced is described in PCT Publication No: WO2012/131555. Bispecific antibodies can be generated to a number of targets, for example, a target located on tumour cells and/or a target located on effector cells. Preferably, a bispecific antibody can bind to two targets selected from the group consisting of: AXL, Bcl2, HER2, HER3, EGF, EGFR, VEGF, VEGFR, IGFR, PD-1, PD-1L, BTLA, CTLA-4, GITR, mTOR, CS1, CD3, CD16, CD16a, CD19, CD20, CD22, CD25, CD27, CD28, CD30, CD32b, CD33, CD38, CD40, CD52, CD64, CD79, CD89, CD137, CD138, CA125, cMet, CCR6, MUCI, PEM antigen, Ep-CAM, EphA2, 17-1a, CEA, AFP, HLA class II, HLA-DR, HSG, IgE, IL-12, IL-17a, IL-18, IL-23, IL-1alpha, IL-1beta, GD2-ganglioside, MCSP, NG2, SK-I antigen, Lag3, PAR2, PDGFR, PSMA, Tim3, TF, CTLA4, TL1A, TIGIT, SIRPa, ICOS, Treml2, NCR3, HVEM, OX40, VLA-2 and 4-1BB.

In a further aspect, the present invention provides a host cell comprising an expression construct or an expression vector as described *supra.* The host cell can be a human or non-human cell. Usually the host cell is selected from the group consisting of a mammalian cell, an insect cell and a yeast cell. Preferred host cells are mammalian cells. Preferred examples of mammalian host cells include, without being restricted to, Human embryonic kidney cells (Graham FL et al., (1977) J. Gen. Virol. 36: 59-74), MRC5 human fibroblasts, 983M human melanoma cells, MDCK canine kidney cells, RF cultured rat lung fibroblasts isolated from Sprague-Dawley rats, B16BL6 murine melanoma cells, P815 murine mastocytoma cells, MT1 A2 murine mammary adenocarcinoma cells, PER:C6 cells (Leiden, Netherlands) and Chinese hamster ovary (CHO) cells or cell lines (Puck TT et al., (1958), J. Exp. Med. 108: 945-955).

In a particular preferred embodiment the host cell is a Chinese hamster ovary (CHO) cell or cell line. Suitable CHO cell lines include e.g. CHO-S (Invitrogen, Carlsbad, CA, USA), CHO K1 (ATCC CCL-61), CHO pro3-, CHO DG44, CHO P12 or the dhfr- CHO cell line DUK-BII (Urlaub G & Chasin LA (1980) PNAS 77(7): 4216-4220), DUXBI 1 (Simonsen CC & Levinson AD (1983) PNAS 80(9): 2495-2499), or CHO-K1SV (Lonza, Basel, Switzerland).

In a further aspect, the present disclosure provides a method for the selection of a host cell expressing a polypeptide of interest comprising transfecting a host cell with the expression construct or an expression vector as described *supra,* culturing the host cell, detecting cell membrane expression of the polypeptide of interest and selecting the host cell clone with the desired cell membrane expression. In a further step the polypeptide of interest can be recovered from the host cell. The polypeptide is preferably a heterologous, more preferably a human polypeptide. As is shown in Examples, the level of cell membrane expression of the polypeptide of interest is directly proportional to the level of soluble polypeptide expressed. Therefore this enables the quantitative selection of a host cell clone expressing high levels of the polypeptide of interest.

In a preferred embodiment, the present invention provides an *in vitro* method for the selection of a host cell expressing a multimeric protein, preferable a heterodimeric antibody by transfecting a host cell with the expression constructs or expression vectors as described *supra,* culturing the host cell, detecting cell membrane expression of the multimeric protein of interest and selecting the host cell clone with the desired cell membrane expression. In a further step the multimeric protein of interest, for example a heterodimeric antibody can be recovered from the host cell. As is shown in the Examples, a host cell which displays more heteromodimeric antibody over unwanted homodimeric antibody species can be easily selected for i.e. allowing for qualitative selection of a host cell clone.

For transfecting the expression construct or the expression vector into a host cell according to the present invention any transfection technique such as those well-known in the art, e.g. electroporation, calcium phosphate co-precipitation, cationic polymer mediated transfection, lipofection, can be employed if appropriate for a given host cell type. It is to be noted that the host cell transfected with the expression construct or the expression vector of the present invention is to be construed as being a transiently or stably transfected cell line. Thus, according to the present invention the present expression construct or the expression vector can be maintained episomally i.e. transiently transfected or can be stably integrated in the genome of the host cell i.e. stably transfected.

A transient transfection is characterised by non-appliance of any selection pressure for a vector borne selection marker. In transient expression experiments which commonly last two to up to ten days post transfection, the transfected expression construct or expression vector are maintained as episomal elements and are not yet integrated into the genome. That is the transfected DNA does not usually integrate into the host cell genome. The host cells tend to lose the transfected DNA and the cells having lost the transfected DNA tend to overgrow transfected cells in the population upon culture of the transiently transfected cell pool. Therefore expression is strongest in the period immediately following transfection and decreases with time. Preferably, a transient transfectant according to the present invention is understood as a cell that is maintained in cell culture in the absence of selection pressure up to a time of two to ten days post transfection.

In a preferred embodiment of the invention the host cell e.g. the CHO host cell is stably transfected with the expression construct or the expression vector of the present invention. Stable transfection means that newly introduced foreign DNA such as vector DNA is becoming incorporated into genomic DNA, usually by random, non-homologous recombination events. The copy number of the vector DNA and concomitantly the amount of the gene product can be increased by selecting cell lines in which the vector sequences have been amplified after integration into the DNA of the host cell. Therefore, it is possible that such stable integration gives rise, upon exposure to further increases in selection pressure for gene amplification, to double minute chromosomes in CHO cells. Furthermore, a stable transfection may result in loss of vector sequence parts not directly related to expression of the recombinant gene product, such as e.g. bacterial copy number control regions rendered superfluous upon genomic integration. Therefore, a transfected host cell has integrated at least part or different parts of the expression construct or the expression vector into the genome.

In a further aspect, the present disclosure provides the use of the expression construct or an expression vector as described *supra* for the expression of a heterologous polypeptide from a mammalian host cell, in particular the use of the expression construct or an expression vector as described *supra* for the *in vitro* expression of a heterologous polypeptide from a mammalian host cell. In a preferred embodiment the expression constructs or expression vectors as described *supra* are used for the *in vitro* expression of a heterodimeric antibody from a mammalian host cell.

An expression construct as described in the present invention can be used in a method for selecting a recombinant host cell expressing a polypeptide or protein of interest. Preferably, the protein of interest is an antibody. The method comprises:

(i) co-transfecting a host cell with:

(a) an expression construct comprising in a 5' to 3' direction:

a promoter;
an exon encoding an antibody heavy chain;
a splice donor site, an intron and a splice acceptor site, wherein a first stop codon is located between the splice donor site and the splice acceptor site within said intron; a second exon encoding a transmembrane region which is selected from a modified immunoglobulin transmembrane region or a modified or unmodified non-immunoglobulin transmembrane region;
a second stop codon; and
a poly(A) site,

(b) an expression construct encoding an antibody light chain,

(ii) culturing the transfected host cell under conditions suitable for expression of the antibody;
(iii) detecting cell membrane expression of the antibody; and
(iv) selecting the host cell displaying the antibody on the surface of the cell membrane.

Furthermore, the above detailed method can be used for the selection of a recombinant host cell expressing a bispecific antibody whereby the host cell is co-transfected with a third expression construct encoding a scFv-Fc or a third expression construct encoding a scFv-Fc with splicing of a non-immunoglobulin transmembrane region, such as a murine B7-1 transmembrane region.
Selection of the desired host cell can be made according to methods known in the art. Such methods include, fluorescence, ELISA, Western blotting, SDS polyacrylamide gel electrophoresis, radioimmunoassay or by using antibodies or specific molecules such as aptamers that recognise and bind to the protein of interest. Preferably, the protein of interest displayed on the membrane of the host cell is detected by a fluorescent probe, or linked to a fluorescent label, therefore permitting selection using fluorescence activated cell sorting or FACS.
Expression and recovering of the protein can be carried out according to methods known to the person skilled in the art.
In a further aspect, the present disclosure provides the use of the expression construct or the expression vector as described *supra* for the preparation of a medicament for the treatment of a disorder.
In a further aspect, the present disclosure provides the expression construct or the expression vector as described *supra* for use as a medicament for the treatment of a disorder.
In a further aspect, the present disclosure provides the expression construct or the expression vector as described *supra* for use in gene therapy.

## Examples

### Example 1: Cloning of the vectors

Introduction

The alternate splicing constructs that were prepared comprised the splice donor sequence of the human *ighg1* gene. The introns (including the splice acceptor site) were derived from the chicken cTNT intron 4, whereas the exon was derived from the transmembrane region of the *ighg1* gene (in the following referred to as M1M2), the transmembrane region of the T-cell receptor alpha (PTCRA), or the murine B7-1 gene. In several constructs, the murine B7-1 transmembrane domain was replaced by other transmembrane domains, including naturally occurring transmembrane domains and modifications of these. Furthermore, the B7-1 cytosolic tail in the B7-1 transmembrane region was replaced by several other cytosolic tails with or without an ER exportation signal.
For the expression of a full length antibody of IgG1 or IgG4 format, the simultaneous expression of heavy and light chain was necessary. All subunits were expressed using separate plasmids using a co-transfection strategy. The alternate splicing construct was used for the expression of the IgG1 or IgG4 heavy chain. The light chain of the IgG1 or IgG4 was cloned in the same expression vector backbone, but without alternate splicing.
For expression of the in-house generated bispecific antibody formats (BEAT® technology; described in WO2012/131555), three different subunits had to be transfected in cells: heavy chain, light chain and the scFv-Fc. In order to evaluate the best strategy for membrane display of the bispecific antibody format, spliced and alternate splicing constructs were cloned for expression of the heavy chain and the scFv-Fc. The light chain was cloned in the same expression vector backbone, but without alternate splicing. Table 1 summarises all the constructs generated in this example.

**Table 1: Summary of all constructs generated** (*)

| Plasmid name | Plasmid batch number | DNA Sequence SEQ ID NO | Protein Sequence SEQ ID NO |
|---|---|---|---|
| pGLEX41_IgG4-HC | GSB59 | 79 | 126 |

(continued)

| Plasmid name | Plasmid batch number | DNA Sequence SEQ ID NO | Protein Sequence SEQ ID NO |
|---|---|---|---|
| pGLEX41_HC | GSC314 | 48 | 286 |
| pGLEX41_LC | GSC315 | 294 | 295 |
| pGLEX41_IG4-LC | GSC3704 | 319 | 320 |
| pGLEX41_HC-AS | GSC3836 | 287 | 288 |
| pGLEX41_HC-cTNTintron4 | GSC3848 | 289 | 288 |
| pGLEX41_HC-I4-M1M2-M1M2-M1M2 | GSC3899 | 36 | 290 |
| pGLEX41_HC-I4(0Y)-M1M2-M1M2-M1M2 | GSC4398 | 37 | 290 |
| pGLEX41_HC-I4(polyA)-M1M2-M1M2-M1M2 | GSC4401 | 38 | 290 |
| pGLEX41_HC-I4-M1M2-M1M2-M1M2-SV40ter | GSC4402 | 39 | 290 |
| pGLEX41_BEAT-HC-I4-M1M2-M1M2-M1M2 | GSC5537 | 50 | 299 |
| pGLEX41_scFv-Fc-I4-M1M2-M1M2-M1M2 | GSC5538 | 52 | 301 |
| pGLEX41_BEAT-LC | GSC5540 | 302 | 303 |
| pGLEX4LBEAT-HC-His | GSC5607 | 296 | 297 |
| pGLEX41_scFv-Fc | GSC5608 | 51 | 300 |
| pGLEX41_BEAT-HC | GSC5644 | 49 | 298 |
| pGLEX41_HC-I4-PTCRA | GSC5854 | 40 | 291 |
| pGLEX41_HC-I4(0Y)-PTCRA | GSC5855 | 41 | 291 |
| pGLEX41_HC-I4-PTCRA-SV40ter | GSC5856 | 43 | 291 |
| pGLEX41_HC-I4(polyA)-PTCRA | GSC5857 | 42 | 291 |
| pGLEX41_HC-I4-M1M2-PTCRA-M1M2 | GSC6030 | 44 | 292 |
| pGLEX41_HC-I4-M1M2-PTCRA-M1M2-SV40ter | GSC6046 | 47 | 292 |
| pGLEX41_HC-I4(polyA)-M1M2-PTCRA-M1M2 | GSC6047 | 46 | 292 |
| pGLEX41_HC-I4(0Y)-M1M2-PTCRA-M1M2 | GSC6048 | 45 | 292 |
| pGLEX41_HC-I4-B7-B7-B7 | GSC7056 | 55 | 293 |
| pGLEX41_HC-I4(0Y)-B7-B7-B7 | GSC7057 | 53 | 293 |
| pGLEX41_HC-I4(polyA)-B7-B7-B7 | GSC7058 | 54 | 293 |
| pGLEX41_HC-I4-B7-B7-B7-SV40ter | GSC7059 | 56 | 293 |
| pGLEX41_HC-I4(5Y)-I4-B7-B7-B7 | GSC9763 | 61 | 293 |
| pGLEX41_HC-SD_CCC-I4-B7-B7-B7 | GSC9764 | 58 | 293 |
| pGLEX41_HC-SD_GGC-I4-B7-B7-B7 | GSC9765 | 57 | 293 |
| pGLEX41_HC-I4(5Y-5)-I4-B7-B7-B7 | GSC9768 | 60 | 293 |

(continued)

| Plasmid name | Plasmid batch number | DNA Sequence SEQ ID NO | Protein Sequence SEQ ID NO |
|---|---|---|---|
| pGLEX41_HC-I4(3Y)-I4-B7-B7-B7 | GSC9770 | 62 | 293 |
| pGLEX41_HC-I4(9Y)-I4-B7-B7-B7 | GSC9949 | 59 | 293 |
| pGLEX41_HC_I4(15Y-3')-I4-B7-B7-B7 | GSC9950 | 63 | 293 |
| pGLEX41_scFv-Fc-I4-B7-B7-B7 | GSC 10487 | 323 | 324 |
| pGLEX4LBEAT-HC-I4-B7-B7-B7 | GSC10488 | 321 | 322 |
| pGLEX41_HC-I4-B7-PTCRA_K13V-B7 | GSC11203 | 95 | 142 |
| pGLEX41_HC-I4-B7-PTCRA-B7 | GSC11204 | 93 | 140 |
| pGLEX41_HC-I4-B7-PTPMR-B7 | GSC11205 | 84 | 131 |
| pGLEX41_HC-I4-M1M2-PTCRA-M1M2_version 1 | GSC11206 | 284 | 285 |
| pGLEX41_HC-I4-B7-B7-6His | GSC11207 | 112 | 159 |
| pGLEX41_HC-I4-B7-PTCRA_K13V_D18V-B7 | GSC11208 | 99 | 146 |
| pGLEX41_HC-I4-B7-B7-KCFCK | GSC11209 | 113 | 160 |
| pGLEX41_HC-I4-B7-CD4-B7 | GSC11210 | 83 | 130 |
| pGLEX41_HC-I4-B7-PTCRA_D18V-B7 | GSC11211 | 96 | 143 |
| pGLEX41_HC-I4-B7-PTCRA_R8V_K13V-B7 | GSC11212 | 97 | 144 |
| pGLEX41-HC-14-B7-ACVL1-B7 | GSC11213 | 81 | 128 |
| pGLEX41_HC-I4-B7-ANTR2-B7 | GSC11214 | 82 | 129 |
| pGLEX41_HC-I4-B7-PTCRA_R8V-B7 | GSC11216 | 94 | 141 |
| pGLEX41_HC-I4-B7-TNR5-B7 | GSC11217 | 85 | 132 |
| pGLEX41_IgG4-HC-B7-B7-B7 | GSC11218 | 80 | 127 |
| pGLEX4LHC-I4-B7-PTCRA_R8V_K13V_D18V-B7 | GSC11291 | 100 | 147 |
| pGLEX41_HC-I4-B7-B7(26)-B7 | GSC11292 | 111 | 158 |
| pGLEX41_HC-I4-B7-IGF1R-B7 | GSC11295 | 88 | 135 |
| pGLEX41_HC-I4-B7-1B07-B7 | GSC11296 | 87 | 134 |
| pGLEX41_HC-I4-B7-FCERA-B7 | GSC11297 | 101 | 148 |
| pGLEX41_HC-I4-B7-KI2L2-B7 | GSC11298 | 102 | 149 |
| pGLEX41_HC-I4-B7-M1M2-B7 | GSC11389 | 107 | 154 |
| pGLEX41_HC-I4-B7-TRBM-B7 | GSC11390 | 89 | 136 |
| pGLEX41_HC-I4-B7-IL4RA-B7 | GSC11391 | 90 | 137 |
| pGLEX41_HC-I4-B7-LRP6-B7 | GSC11392 | 91 | 138 |
| pGLEX41_HC-I4-B7-IL3RB-B7 | GSC11393 | 103 | 150 |
| pGLEX41_HC-I4-B7-CD3E-B7 | GSC11394 | 104 | 151 |
| pGLEX41_HC-I4-B7-ITA2B-B7 | GSC11395 | 105 | 152 |
| pGLEX41_HC-I4-B7-CD28-B7 | GSC11396 | 106 | 153 |

(continued)

| Plasmid name | Plasmid batch number | DNA Sequence SEQ ID NO | Protein Sequence SEQ ID NO |
|---|---|---|---|
| pGLEX41_HC-I4-B7-GpA-B7 | GSC11397 | 92 | 139 |
| pGLEX41_HC-I4-B7-PTCRA_R8V_ D18V-B7 | GSC11398 | 98 | 145 |
| pGLEX41_HC-14-B7-B7-GAT1noER | GSC11483 | 118 | 165 |
| pGLEX41_HC-I4-B7-B7(24)-B7 | GSC11484 | 110 | 157 |
| pGLEX41_HC-I4-B7-B7(18)-B7 | GSCI1677 | 108 | 155 |
| pGLEX41_HC-I4-B7-B7-ERGIC53_noER | GSC11678 | 116 | 163 |
| pGLEX41_HC-I4-B7-B7(20)-B7 | GSC11679 | 109 | 156 |
| pGLEX4LHC-I4-B7-ITB1-B7 | GSC11756 | 86 | 133 |
| pGLEX4LHC-I4-B7-B7-M1M2 | GSC11758 | 114 | 161 |
| pGLEX4LHC-I4-B7-B7-GAT1 | GSC11759 | 117 | 164 |
| pGLEX41_HC-I4-B7-B7-AGTR2 | GSCI1760 | 119 | 166 |
| pGLEX41_HC-I4-B7-B7-AGTR2_noER | GSC11761 | 120 | 167 |
| pGLEX41_HC-I4-B7-B7-V1BR | GSC11762 | 121 | 168 |
| pGLEX41_HC-I4-B7-B7-VGLG_noER | GSC11763 | 124 | 171 |
| pGLEX41_HC-I4-B7-B7-ERGIC53 | GSC11764 | 115 | 162 |
| pGLEX41_HC-I4-B7-B7-V1BR_noER | GSC11765 | 122 | 169 |
| pGLEX4LHC-I4-B7-B7-VGLG | GSC11766 | 123 | 170 |
| pGLEX41_HC-I4-M1M2-M1M2-B7 | GSC11294 | 125 | 172 |
| *Sequence listed stretches from the last 5 amino acids of the non-spliced ORF to the end of the expression construct for DNA sequences and from the same amino acids to the end of the fused transmembrane region for protein sequences, except for GSC314 (SEQ ID NO: 48), GSC315 (SEQ ID NO: 294), GSC5607 (SEQ ID NO: 296), GSC5644 (SEQ ID NO: 49), GSC5608 (SEQ ID NO: 51), GSC5540 (SEQ ID NO: 302), GSB59 (SEQ ID NO: 79) (complete ORF sequence). | | | |

Materials and Methods

*LB culture plates*

500 ml of water were mixed and boiled with 16 g of LB Agar (Invitrogen, Carlsbad, CA, USA) (1 liter of LB contains 10 g tryptone, 5 g yeast extract and 10 g NaCl). After cooling, the respective antibiotic was added to the solution which was then plated (ampicillin plates at 100 g/ml and kanamycin plates at 50 g/ml ).

*Polymerase Chain Reaction (PCR)*

All PCRs were performed using 1 1 of dNTPs (10 mM for each dNTP; Invitrogen, Carlsbad, CA, USA), 2 units of Phusion® DNA Polymerase (Finnzymes Oy, Espoo, Finland), 25 nmol of Primer A (Microsynth, Balgach, Switzerland or Operon, Ebersberg, Germany), 25 nmol of Primer B (Microsynth, Balgach, Switzerland or Operon, Ebersberg, Germany), 10 l of 5X HF buffer (7.5 mM $MgCl_2$, Finnzymes, Espoo, Finland), 1.5 l of Dimethyl sulfoxide (DMSO, Finnzymes, Espoo, Finland) and 1-3 l of the template (10 -20 ng) in a 50 l final volume. All primers used are listed in Table 2.

The PCRs were started by an initial denaturation at 98°C for 3 min, followed by 35 cycles of 30 sec denaturation at 98°C, 30 sec annealing at a primer-specific temperature (according to GC content) and 2 min elongation at 72°C. A final elongation at 72°C for 10 min was performed before cooling and keeping at 4°C.

**Table 2: Summary of primers used in PCRs. Primers were obtained either from Operon (Ebersberg, Germany) or from Microsynth (Balgach, Switzerland).**

| Primer name | Primer sequence | SEQ ID NO |
|---|---|---|
| GlnPr269 | CAGGGGGGCGGAGCCTATGG | 1 |
| GlnPr497 | GCCCCCCTCCCGCGAGGAGATGACCAAGAACCAGGTGTCCCTG | 2 |
| GlnPr498 | TTTCTAGATTCATCACTTGCCGGGGGACAGGCTC | 3 |
| GlnPr501 | TATAAAGCTTCCACCATGGAGAGCCCTGCCC | 4 |
| GlnPr502 | TATATCTAGATTCATCAGCACTCGCCCCGGTTG | 5 |
| GlnPr1285 | CGGAAGAA TTCAGCCACAGCTTT AAGGCACCTGGCT AAC | 259 |
| GlnPr1437 | CCAAATCGATTTATCAGCACTCGCCCCGGTTGAAG | 6 |
| GlnPr1452 | ACGTATCGATTCATCACTTGCCGGGGGACAG | 327 |
| GlnPr1487 | CTGCTGCTGTGGCTGCCCGACGGGGTGCACGCTGAGATCGTGCTGACA CAGAG | 325 |
| GlnPr1488 | CTGCTGCTGTGGCTGCCCGACGGGGTGCACGCTCAGGTCACACTGAAA GAGTC | 328 |
| GlnPr1491 | CCAAATCGATTTATCAGCACTCGCCCCGGTTGAAG | 326 |
| GlnPr1494 | ACGAACTAGTCCACCATGGAAAGCCCAGCCCAGCTGCTGTTCCTGCTG CTGCTGTGGCTGCCCGAC | 260 |
| GlnPr1500 | CCGGGATCGATTTATCAGTGATGGTGATGGTGATG | 7 |
| GlnPr1502 | GGCCGATCGATTTATCACTTGCCAGGAGACAG | 8 |
| GlnPr1516 | GATGAAAGCTTGGTAGGTGATCCTCCTG | 9 |
| GlnPr1517 | ATGTATTCGAATATCTCCACCGGTTGCAGCTCTGAGCCAGGGAGGAGG GAAG | 10 |
| GlnPr1518 | TAAGCTAGCCCACCATGGAGAGCCCTGC | 11 |
| GlnPr1519 | GAGGCTCTTCTGCGTGTAGTGGTTGTGCAAGGCCTCGTGCATCACGCT G | 12 |
| GlnPr1520 | CTACCAAGCTTTCATCATTTACCCGGAGACAGGGAGAGGCTCTTCTGC GTG | 13 |
| GlnPr1521 | GGGAGCTGGACGGGCTGTGGACGACCATCACCATCTTCATCACACTCT TCCTGTTAAGCGTGTGCTACAGTGCCACCGTCACCTTCTTCAAG | 14 |
| GlnPr1522 | GTAGTCGGGGATGATGGTCTGCTTCAGGTCCACCACCGAGGAGAAGA TCCACTTCACCTTGAAGAAGGTGACGGTGGCACTGTAGCACACGC | 15 |
| GlnPr1523 | ATATACCGGTGGAGAGCTGTGCGGAGGCGCAGGACGGGGAGCTGGAC GGGCTGTG | 16 |
| GlnPr1524 | ATATTTCGAATCACTAGGCCCCCTGTCCGATCATGTTCCTGTAGTCGGG GATGATGGTC | 17 |
| GlnPr1649 | TCTCCACCGGTTGCAGCTCTGTTCCCTCCTCCCTCGGTTAG | 18 |
| GlnPr1650 | GCGCCATCGATACAGACATGATAAGATACATTG | 19 |
| GlnPr1651 | GCGCGATCGATACTTGTTTATTGCAGCTTATAATGG | 20 |

(continued)

| Primer name | Primer sequence | SEQ ID NO |
|---|---|---|
| GlnPr1689 | ATATGCTAGCCCACCATGCGGAGCCCTGCCCAGCTGCTGTTCCTGCTG CTGCTG | 21 |
| GlnPr1690 | TGCTGTTCCTGCTGCTGCTGTGGATCCCTGGCACCAACGCCGAGGTGC AGCTGGTCGAGTC | 22 |
| GlnPr1725 | ATATACTAGTCCACCATGCGGAGCCCTGCCCAGCTGCTGTTCCTGCTG CTGCTG | 23 |
| GlnPr1726 | CTGTTCCTGCTGCTGCTGTGGATCCCTGGCACCAACGCCCAGGTGCAG CTGCAGCAGTC | 24 |
| GlnPr1727 | CTGTTCCTGCTGCTGCTGTGGATCCCTGGCACCAACGCCATGGAGACA GACACACTCC | 25 |
| GlnPr1735 | GACTTCGAATCATCAATGATGGTGGTGGTGATGGGCGCTGGCGAGGA GCAGGTCGAACAGGAGCAGCTTG | 26 |
| GlnPr1760 | CCGCATCGATTTATCATTTACCCGGGCTCAGGCTCAG | 27 |
| GlnPr1799 | ATATACCGGTGGAGGCCCTGTGGCTGGGCGTGCTGAGACTGCTGCTGT TCAAGCTGCTCCTGTTCGACCTG | 28 |
| GlnPr1800 | GTAAGCTTTCATCATTTACCCGGAGACAGGGAGAGGCTCTTCTGCGTG AATCGGTTGTGC | 29 |
| GlnPr1801 | GCGCGAAGCTTTCATCATTTACCCGGAGACAGGGAGAGGCTCTTCTGC GTATAATGATTG | 30 |
| GlnPr1840 | ATATACCGGTGGAGGCCCTGTGGCTGGGCGTGCTG | 31 |
| GlnPr2099 | TCCTCGGGGGGAGATCCACCACCACCTGAGCCAGGGAGGAGGGAAG | 32 |
| GlnPr2100 | GCGCTTCGAATCATCACAGAAACACGGTCTG | 33 |
| GlnPr2101 | TGGTGGTGGATCTCCCCCCGAGGACCCCCCTGACTCCAAG | 34 |
| GlnPr2102 | TCCTCGGGGGGAGATCCACCACCACCTGTTCCCTCCTCCCTCGGTTAG | 35 |
| GlnPr2160 | CCAAGCTTTCATCATTTGCCCGGAGACAGGGAGAGG | 312 |
| GlnPr2161 | CCAAGCTTTCATCATTTACCGGGAGACAGGGAGAGGCTCTTC | 313 |
| GlnPr2162 | GTCCTCGGGGGGAGATCCACCACCACCTGTCCCAGAAGGAGACGGTT AG | 314 |
| GlnPr2163 | GTCCTCGGGGGGAGATCCACCACCACCTGTGCAATCCTCCCAGGGTTA G | 315 |
| GlnPr2164 | GTCCTCGGGGGGAGATCCACCACCACCTGTGCAATAAGGACAGGGTT AG | 316 |
| GlnPr2165 | GTCCTCGGGGGGAGATCCACCACCACCTGTCCCCTCAGGTCTCGGTTA G | 317 |
| GlnPr2166 | GTCCTCGGGGGGAGATCCACCACCACCTGAGCCAGGTAGCAGGGAAG GG | 318 |
| GlnPr2294 | CTACCAAGCTTTCATCATTTACCCGGAGACAGGGAGAGGCTCTTCTGC GTGTAGTGGTTGTGCAAGGCCTCG | 261 |

(continued)

| Primer name | Primer sequence | SEQ ID NO |
|---|---|---|
| GlnPr2376 | GGCCGTTCGAATCATCACTTGCAGAAGCACTTGATAATC | 219 |
| GlnPr2377 | GTCATTTCGAATCATCAATGATGGTGGTGATGGGCGCTGGCGATAATCACGACGATCACGAC | 220 |
| GlnPr2378 | TTGAACAGCAGCAGTCTCAGCACGCCCAGCCACAGGGCCCCGTCCAGCTCCCCGTCCTG | 221 |
| GlnPr2379 | CTGAGACTGCTGCTGTTCAAGCTGCTCCTGTTCGACCTGCTCCTCAAGTGGATCTTCTCCTCGGTG | 222 |
| GlnPr2380 | GCTTGAACAGCAGCAGTCTCAGCACGCCCAGCCACAGGGCATTCTTGGAGTCAGGGGGGTCCTC | 223 |
| GlnPr2381 | CAGGACCGGTGCTTGCAGAAGCACTTGAGGAGCAGGTCGAACAGGAGCAGCTTGAACAGCAGCAGTCTC | 224 |
| GlnPr2382 | GCTTGAACAGCAGCAGTACCAGCACGCCCAGCCACAGGGCATTCTTGGAGTCAGGGGGGTCCTC | 225 |
| GlnPr2383 | CAGGACCGGTGCTTGCAGAAGCACTTGAGGAGCAGGTCGAACAGGAGCAGCTTGAACAGCAGCAGTACC | 226 |
| GlnPr2384 | GACCGGTGCTTGCAGAAGCACTTGAGGAGCAGGTCGAACAGGAGCAGCACGAACAGCAGCAGTCTC | 227 |
| GlnPr2385 | GACCGGTGCTTGCAGAAGCACTTGAGGAGCAGGACGAACAGGAGCAGCTTGAACAGCAGCAGTCTC | 228 |
| GlnPr2386 | CAGGACCGGTGCTTGCAGAAGCACTTGAGGAGCAGGTCGAACAGGAGCAGCACGAACAGCAGCAGTACC | 229 |
| GlnPr2387 | CAGGACCGGTGCTTGCAGAAGCACTTGAGGAGCAGGACGAACAGGAGCAGCTTGAACAGCAGCAGTACC | 230 |
| GlnPr2388 | GACCGGTGCTTGCAGAAGCACTTGAGGAGCAGGACGAACAGGAGCAGCACGAACAGCAGCAGTCTC | 231 |
| GlnPr2389 | CAGGACCGGTGCTTGCAGAAGCACTTGAGGAGCAGGACGAACAGGAGCAGCACGAACAGCAGCAGTACC | 232 |
| GlnPr2390 | GGTCGTGATCGTCGTGATTATCAAGTGGATCTTCTCCTCGGTGGTGG | 233 |
| GlnPr2391 | CCACCACCGAGGAGAAGATCCACTTGATAATCACGACGATCACGACC | 234 |
| GlnPr2392 | CTTCGGCGCCACCGTGGTCGTGATCGTCGTGATTATCAAGTG | 235 |
| GlnPr2393 | CAGGACCGGTGCTTGCAGAAGCACTTGATAATCACGACGATC | 236 |
| GlnPr2394 | CTTCGGCGCCACCGTGATCGTCGTGATTATCAAGTGCTTC | 237 |
| GlnPr2395 | CAGGACCGGTGCTTGCAGAAGCACTTGATAATCACGAC | 238 |
| GlnPr2396 | CTTCGGCGCCGTGGTCATTGTAACCGTGGTCGTGATCGTCGTG | 239 |
| GlnPr2397 | CAGGACCGGTGCTTGCAGAAGCACTTGATAATCACGACGATCACGACCAC | 240 |
| GlnPr2398 | CTTCGGCGCCGTGGTCATTGTAGTCGTAACCGTGGTCGTGATCGTCGTG | 241 |

(continued)

| Primer name | Primer sequence | SEQ ID NO |
|---|---|---|
| GlnPr2399 | CAGGACCGGTGCTTGCAGAAGCACTTGATAATCACGACGATCACGAC CACGG | 242 |
| GlnPr2400 | GAGCCACCAGTGCCAGCAGAGCCAGCACAGGTCCCAGGATCAGAGCC AGATTCTTGGAGTCAGGGGGGTC | 243 |
| GlnPr2401 | CAGGACCGGTGCTTGCAGAAGCACTTCCACAGTCCCAGCACTCCCAGA GCCACCAGTGCCAGCAG | 244 |
| GlnPr2402 | CGATGCCCAGCAGCAGGAGCAACACCAGGATCACGATGATGGCGGCG ATATTCTTGGAGTCAGGGGGGTC | 245 |
| GlnPr2403 | CAGGACCGGTGCTTGCAGAAGCACTTCCAGAACCACCACATCAGGCC GATGCCCAGCAGCAGGAG | 246 |
| GlnPr2404 | GATAAACAGCAGCAGGCCAGCCACTCCGCCCAGCACAATCAGGGCCA TATTCTTGGAGTCAGGGGGGTC | 247 |
| GlnPr2405 | CAGGACCGGTGCTTGCAGAAGCACTTGAAGAAGATGCCCAGGCCGAT AAACAGCAGCAGGCC | 248 |
| GlnPr2406 | GTCCAATCAGCACGATGCCAGCCACCACGCCGGCCACGATAGGGATG ATATTCTTGGAGTCAGGGGGGTC | 249 |
| GlnPr2407 | CAGGACCGGTGCTTGCAGAAGCACTTCCAGATCAGCAGCAGGGCCAG TCCAATCAGCACGATGCC | 250 |
| GlnPr2408 | CCAGGAAGATGATCACGAACAGCAGGATGCCAGCGATCACGCCAGCG ATATTCTTGGAGTCAGGGGGGTC | 251 |
| GlnPr2409 | CAGGACCGGTGCTTGCAGAAGCACTTCATCACGAGCACCACGCCCAG GAAGATGATCACGAA | 252 |
| GlnPr2410 | GCAGGATGGCGAACAGGATGCCGAAGATGATGGGGATCACGACCAGA GCATTCTTGGAGTCAGGGGGGTC | 253 |
| GlnPr2411 | CAGGACCGGTGCTTGCAGAAGCACTTGATGAACACCAGCACCAGCAG GATGGCGAACAGGATG | 254 |
| GlnPr2412 | CTTCGGCGCCGTGATTACCGTGGTCGTGATCGTCGTGATTATCAGGTC CCAGCAGGAGGCCGCCG | 255 |
| GlnPr2413 | GTCATTTCGAATCATCAGAAGAACTTCTTGGCGGCGGCCTCCTGCTGG GAC | 256 |
| GlnPr2414 | GTCATTTCGAATCATCACCACCACTTCTTGGCGGCGGCCTCCTGCTGG GAC | 257 |
| GlnPr2415 | GGTCATTTCGAATCATCAGGAGCCCTTCAGGGTCAGGAACATGATAAT CACGACGATCACGAC | 258 |

*Restriction digest*

For all restriction digests around 1 g of plasmid DNA (quantified with NanoDrop, ND -1000 Spectrophotometer (Thermo Scientific, Wilmington, DE, USA)) was mixed to 10-20 units of each enzyme, 4 l of corresponding 10X NEBuffer (NEB, Ipswich, MA, USA), and the volume was made up to 40 l with sterile $H_2O$. The reactions were incubated for 1h at the temperature necessary for the enzymes.

After each preparative digestion of backbone, 1 unit of Calf Intestinal Alkaline Phosphatase (CIP; NEB, Ipswich, MA, USA) was added and the mix was incubated 30 min at 37°C.

*PCR clean up*

To allow digestion, all PCR fragments were cleaned prior to restriction digests using the Macherey Nagel Extract II kit (Macherey Nagel, Oensingen, Switzerland) or Gel and PCR clean-up kit (new brand name of the same kit) following the manual of the manufacturer using 40 µl of elution buffer. This protocol was also used for changing buffers of DNA samples.

*DNA extraction*

Gel electrophoresis was performed using 1-2% agarose gels. These were prepared using the necessary amount of UltraPure™ Agarose (Invitrogen, Carlsbad, CA, USA) and 1X Tris Acetic Acid EDTA buffer (TAE, pH 8.3; Bio RAD, Munich, Germany). For staining of DNA 1 l of Gel Red Dye (Biotum, Hayward, CA, USA) was added to 100 ml of agarose gel. As size marker 2 l of the 1 kb DNA ladder (NEB, Ipswich, MA, USA) was used. The electrophoresis was run for around 1 hour at 125 Volts.

The bands of interests were cut out from the agarose gel and purified using the kit Extract II (Macherey-Nagel, Oensingen, Switzerland) or Gel and PCR clean-up kit (new brand name of the same kit) or Qiaquick Gel extraction kit (Qiagen, Hilden, Germany), following the manual of the manufacturer using 40 µl of elution buffer.

*Ligation*

For each ligation, 4 l of insert was mixed with 11 of vector, 400 units of ligase (T4 DNA ligase, NEB, Ipswich, MA, USA) and 1 1 of 10X ligase buffer (T4 DNA ligase buffer; NEB, Ipswich, MA, USA), filled up to a 10 l final volume using UHP water. The mix was incubated for 1-2 h at RT.

*Transformation of ligation products into competent bacteria*

For transformation of ligation products, the competent bacteria "TOP 10" (One Shot® TOP 10 Competent *E. coli;* Life Technologies, Carlsbad, CA, USA) were used. 25-50 l of bacteria were thawed on ice for 5 minutes. Then, 3-5 l of ligation product were added to competent bacteria and incubated for 20-30 min on ice before a short heat shock of 1 min at 42°C. Then, 500 1 of S.O.C medium (Invitrogen, Carlsbad, CA, USA) were added per tube and incubate d for 1 h at 37°C under agitation. Finally, the bacteria were put on a LB plate with ampicillin or kanamycin (Sigma-Aldrich, St. Louis, MO, USA) and incubated overnight at 37°C.

*Minipreparation of plasmids*

For minipreparation, colonies of transformed bacteria were grown for 6-16 hours in 2.5 ml of LB and ampicillin or kanamycin at 37°C, 200 rpm. The DNA was extracted with a plasmid purification kit for *E.coli* (NucleoSpin QuickPure or NucleoSpin Plasmid (Macherey Nagel, Oensingen, Switzerland) or Qiagen QuickLyse Miniprep (Qiagen)), following the manufacturer's manual.

Plasmid DNA from minipreparations was quantified once with the NanoDrop ND-1000 Spectrophotometer (Thermo Scientific, Wilmington, DE, USA) by measuring the absorbance at 260 nm and assessing the ratio of the OD260 nm/OD280 nm that had to be between 1.8 and 2. A control digestion was performed before sending the sample to Fasteris SA (Plan-les-Ouates, Switzerland) for sequence confirmation.

*Midipreparation of plasmids*

For midipreparation, transformed bacteria were grown at 37°C overnight in 200 ml of LB and ampicillin (or kanamycin). Then, the culture was centrifuged 20 min at 725 g and the plasmid was purified using a commercial kit (NucleoBond Xtra Midi; Macherey Nagel, Oensingen, Switzerland) following the protocol provided in the manufacturer's manual.

Plasmid DNA from midipreparation was quantified three times with the NanoDrop ND-1000 Spectrophotometer, by measuring the absorbance at 260 nm and assessing the ratio of the OD260 nm/OD280 nm that had to be between 1.8 and 2 as confirmed by restriction digest. The samples were then sent for sequencing (Fasteris SA, Plan-les-Ouates, Switzerland).

*Maxipreparation of plasmids*

For maxipreparation, transformed bacteria were grown at 37°C overnight in 500 ml of LB and ampicillin (or kanamycin). The culture was centrifuged for 20 min at 725 g and the plasmid was purified using a commercial kit (NucleoBond PC500 or NucleoBond Xtra Maxi; Macherey Nagel, Oensingen, Switzerland) following the protocol provided in the manufacturer's manual.

Plasmid DNA from maxipreparation was quantified three times with the NanoDrop ND-1000 Spectrophotometer, by measuring the absorbance at 260 nm and assessing the ratio of the OD260 nm/OD280 nm that had to be between 1.8 and 2 as confirmed by restriction digest. The samples were then sent for sequencing (Fasteris SA, Plan-les-Ouates, Switzerland).

*Gigapreparation of plasmids*

For gigapreparation, transformed bacteria were grown at 37°C overnight in 1500 ml of LB and ampicillin (or kanamycin). The culture was centrifuged for 20 min at 725 g and the plasmid was purified using a commercial kit (NucleoBond PC10000; Macherey Nagel, Oensingen, Switzerland) following the protocol provided in the manufacturer's manual.

Plasmid DNA from gigapreparation was quantified three times with the NanoDrop ND-1000 Spectrophotometer, by measuring the absorbance at 260 nm and assessing the ratio of the OD260 nm/OD280 nm that had to be between 1.8 and 2 as confirmed by restriction digest. The samples were then sent for sequencing (Fasteris SA, Plan-les-Ouates, Switzerland).

Results

*Cloning of the heavy chain of an IgG1 antibody*

The gene coding for the IgG1 heavy chain used in this patent was optimized for expression in Chinese hamster cells by GeneArt (Regensburg, Germany). The variable part and the constant part of the heavy chain were provided in two different plasmids by GeneArt. After reception, GeneArt constructs were solubilised in water and the heavy chain constant region and the heavy chain variable region were cloned together using the enzymes KpnI and ApaI. This work was performed by an external CRO and the fused product was delivered to Glenmark. After sequencing of this fused construct, a sequence variation in the heavy chain constant region compared to the theoretical sequence was identified in the CH2 domain of the Fc region (EEMTK to DELTK). Primers were designed to change this region by megaprimer PCR and simultaneously introduced convenient restriction sites 5' and 3' of the open reading frame.

A first PCR using the primers GlnPr497 (SEQ ID NO:2) and GlnPr498 (SEQ ID NO:3) was performed on the original construct. The obtained amplicon (304 bps) was used in different concentrations as a megaprimer in a second PCR, using the second primer GlnPr501 and the original construct as template. The final PCR product was gel-purified and cloned into the shuttle vector pCR-blunt (Zero Blunt® PCR Cloning Kit, Invitrogen, LifeTechnologies). After miniprep screening it appeared that although the insert sequence was correct, the restriction sites 5' and 3' to the open reading frame were missing. Therefore the insert was re-amplified using the primers GlnPr501 (SEQ ID NO:4) and GlnPr498 (SEQ ID NO: 3) and a higher annealing temperature (64°C). The amplicon was gel-purified and cloned into pCR-blunt (Zero Blunt® PCR Cloning Kit, Invitrogen, LifeTechnologies). One miniprep with the correct restriction pattern was chosen for subcloning in pSEL1, a shuttle vector, using restriction enzymes HindIII and XbaI. After miniprep screening, a maxiprep was produced. The insert of this maxiprep was again cut out using the restriction enzymes XbaI and HindIII and cloned into the backbone of the plasmid pGLEX41 (GSC281, SEQ ID NO: 304), opened with the same enzymes and treated with CIP. pGLEX41 is the standard in-house vector for expression and contains an expression cassette consisting of the mouse CMV promoter followed by a constitutively spliced intron, a multiple cloning site (MCS) and a SV40 poly(A) sequence. After miniprep screening, a maxiprep of pGLEX41_HC was produced and received the plasmid batch number GSC314. Further midipreps or gigapreps of the same DNA received the plasmid batch numbers GSC314a, GSC314b, GSC314c, GSC314d and GSC314e (all these preps were confirmed to be identical by DNA sequencing and will be referred to as GSC314 plasmid (SEQ ID NO: 48)).

*Cloning of the alternate splicing expression vector for membrane displayed heavy chain of an IgG1 antibody*

The coding region of the heavy chain of an antibody of the IgG1 format was amplified by PCR using the primers GlnPr1518 (SEQ ID NO: 11), GlnPr1519 (SEQ ID NO: 12) and GlnPr1520 (SEQ ID NO: 13) and the plasmid pGLEX41_HC (GSC314, SEQ ID NO: 48) as template. The resulting PCR product contained a NheI restriction site at the 5'end, and the 3' end of the amplicon was modified in order to contain precisely the last 40 bps of NCBI database entry for the *ighg1* gene (SEQ ID NO: 65) as well as a HindIII restriction site. The 3'end modification was done in order to reconstitute the natural splice donor site in the C-terminal region of the constant part of the IgG1 heavy chain. The PCR product was cut using the restriction enzymes NheI and HindIII and cloned in pGLEX41 (GSC281, SEQ ID NO: 304), cut using the same enzymes and CIPed. The resulting vector was called pGLEX41_HC-AS (plasmid batch number GSC3836, SEQ ID NO: 287) and was confirmed by restriction digest and sequence analysis.

The chicken troponin (cTNT) intron number 4 (SEQ ID NO: 69) was amplified from an in-house plasmid containing its sequence (GSC2819, SEQ ID NO: 67) with the primers GlnPr1516 (SEQ ID NO: 9) and GlnPr1517 (SEQ ID NO: 10). Troponin is expressed exclusively in cardiac muscle and embryonic skeletal muscle. Over 90 % of the mRNAs include the exon in early embryonic heart and skeletal muscle, whereas >95 % of mRNAs in the adult exclude the exon (Cooper & Ordahl (1985) J Biol Chem, 260(20): 11140-8). The primers added a HindIII site 5' and an AgeI site followed by BstBI to the 3' terminus. The amplicon was gel-purified, digested using HindIII and BstBI and cloned into the vector pGLEX41_HC-IgAS (GSC3836, SEQ ID NO: 287) opened using the same enzymes and CIPed. The resulting vector was called pGLEX41_HC-cTNTintron4 (plasmid batch number GSC3848, SEQ ID NO: 289) and was confirmed by restriction digest and sequence analysis.

The sequence coding for the transmembrane region of the human *ighg1* gene (M1M2) was assembled using plasmids GlnPr1521 (SEQ ID NO: 14) and GlnPr1522 (SEQ ID NO: 15). These primers overlap partially and were completed to double stranded DNA using PCR. The blunt-ended PCR product was cloned into pCR-blunt (Zero Blunt® PCR Cloning Kit, Invitrogen, LifeTechnologies), leading to the plasmid pCRblunt-M1M2 which was confirmed by sequencing. The insert was re-amplified with the primers GlnPr1523 (SEQ ID NO: 16) and GlnPr1524 (SEQ ID NO: 17) to complete the M1M2 transmembrane domain sequence. The amplicon was gel-purified, digested using AgeI and BstBI, and cloned in vector pGLEX41_HC-cTNTintron4 (GSC3848, SEQ ID NO: 289). The resulting plasmid was named pGLEX41_HC-I4-M1M2-M1M2-M1M2 and was produced in maxiprep scale, received the plasmid batch number GSC3899 and was confirmed by sequence analysis (SEQ ID NO: 36).

In order to increase the splice ratio between expressed and membrane displayed antibody, the number of pyrimidines in the poly(Y) tract upstream of the exon coding for the transmembrane region was reduced. For this the I4(0Y) fragment (SEQ ID NO: 70) was amplified from an in-house vector (GSC3469, SEQ ID No. 68) using the primers GlnPr1516 (SEQ ID NO: 9) and GlnPr1649 (SEQ ID NO: 18). The amplicon was digested using the restriction enzymes HindIII and AgeI and cloned into the backbone of the pGLEX41_HC-I4-M1M2-M1M2-M1M2 (GSC3899, SEQ ID NO: 36) backbone opened using the same enzymes and treated with CIP. After miniprep screening, the midiprep pGLEX41_HC-I4(0Y)-M1M2-M1M2-M1M2 was prepared, received the batch number GSC4398 and was confirmed by sequencing (SEQ ID NO: 37).

A poly(A) was introduced in the intron separating the exon coding for the IgG1 and the alternate exon coding for the transmembrane region. The SV40 poly(A) signal was amplified from an in-house vector (GSC3469, SEQ ID NO: 305) with the primers GlnPr1650 (SEQ ID NO: 19) and GlnPr1651 (SEQ ID NO: 20). The amplicon was digested using the restriction enzyme ClaI and cloned into the vector pGLEX41_HC-M1M2-M1M2-M1M2 (GSC3899, SEQ ID NO: 36) digested using ClaI and CIPed, resulting in the I4(polyA) intron (SEQ ID NO: 71) After miniprep screening and confirmation of the right orientation of the insert, the pGLEX41_HC-I4(polyA)-M1M2-M1M2-M1M2 midiprep was prepared, received the batch number GSC4401 and was confirmed by sequencing (SEQ ID NO: 38).

The gastrin terminator sequence had been previously added to the SV40 poly(A) signal of an in-house vector (GSC23, SEQ ID NO: 306) sharing the same general backbone than pGLEX41 (GSC281, SEQ ID NO: 304) but bearing a different promoter. The fusion of SV40 poly(A) and gastrin terminator was designated as "SV40ter" (SEQ ID NO: 306) in the plasmid nomenclature. The expression cassette of GSC23 (SEQ ID NO: 306) (promoter + coding sequence) was replaced by the expression cassette of pGLEX41-HC-14-M1M2-M1M2-M1M1 (GSC3899, SEQ ID NO: 36) except for the SV40 poly(A) signal (i.e. promoter and coding sequence). The insert was cut out with the restriction enzymes BstBI and NruI and cloned into the backbone of GSC23 (SEQ ID NO: 306) digested with the same enzymes and treated with CIP. After miniprep screening, a midiprep of plasmid pGLEX41_HC-I4-M1M2-M1M2-M1M2-SV40ter was prepared, received the plasmid batch number GSC4402 and was confirmed by sequencing (SEQ ID NO: 39).

Another preferred construct comprises a poly(A) signal with the gastrin terminator in the intron. The sequence of I4(SV40ter) is given in SEQ ID NO: 77.

*Cloning of the alternate splicing expression vector for membrane displayed heavy chain of an* IgG1 *antibody using the human T-cell receptor alpha transmembrane domain (PTCRA)*

In order to replace the M1M2 transmembrane region in the alternate splicing constructs with the transmembrane domain of the human T-cell receptor alpha (PTCRA) gene, the complementary primers GlnPr1799 (SEQ ID NO: 28) and GlnPr1735 (SEQ ID NO: 26) were used for generation of the insert fragment. These primers overlap partially and were completed to double stranded DNA using PCR. The insert fragment was cut using AgeI and BstBI and cloned into the backbone of pGLEX41_HC-I4-M1M2-M1M2-M1M2 (GSC3899, SEQ ID NO: 36) digested with the same enzymes and treated with CIP. As a part of the insert was missing from the resulting miniprep a new amplification was performed on this miniprep using the primers GlnPr1799 (SEQ ID NO: 28) and GlnPr269 (SEQ ID NO: 1). The amplified insert fragment was digested with Agel and BstBI and cloned into the backbone of pGLEX4LHC-I4(0Y)-M1M2-M1M2-M1M2 (GSC4398, SEQ ID NO: 37) digested with the same enzymes and treated with CIP. One miniprep was confirmed by sequencing, and was used for another PCR with the primers GlnPr1840 (SEQ ID NO: 31) and GlnPr269 (SEQ ID NO: 1). The amplicon was digested with Agel and BstBI and cloned in the backbones pGLEX41_HC-I4-M1M2-M1M2-M1M2 (GSC3899, SEQ ID NO: 36), pGLEX41_HC-I4(0Y)-M1M2-M1M2-M1M2 (GSC4398, SEQ ID NO: 37), pGLEX41_HC-I4(polyA)-M1M2-M1M2-M1M2 (GSC4401, SEQ ID NO: 38) and pGLEX41_HC-I4-M1M2-M1M2-M1M2-SV40ter (GSC4402, SEQ ID NO: 39) digested using the same enzymes and treated with CIP. The ligations resulted in the midipreps pGLEX41_HC-I4-PTCRA (GSC5854, SEQ ID NO: 40), pGLEX41_HC-I4(0Y)-PTCRA (GSC5855, SEQ ID NO: 41), pGLEX41_HC-I4(polyA)-PTCRA (GSC5857, SEQ ID NO: 42) and pGLEX41_HC-I4-PTCRA-SV40ter (GSC5856, SEQ ID NO: 43), respectively. All the midipreps were verified by sequencing.

*Cloning of the alternate splicing expression vector for membrane displayed heavy chain of an IgG1 antibody using an M1M2-PTCRA fusion construct*

The insert coding for the M1M2-PTCRA fusion construct was ordered from GeneArt with the name GeneArt_Seq32 (SEQ ID NO: 64). This fragment contains the regular M1M2 transmembrane region of an IgG1 heavy chain, except for the transmembrane domain that was replaced by the transmembrane domain of the T-cell receptor alpha subunit (PTCRA). The plasmid provided by GeneArt was cut using the restriction enzymes Agel and BstBI and the purified insert was ligated in the backbone of pGLEX41_HC-I4-M1M2-M1M2-M1M2 (GSC3899, SEQ ID NO: 36), pGLEX41_HC-I4(0Y)-M1M2-M1M2-M1M2 (GSC4398, SEQ ID NO: 37), pGLEX41_HC-I4(polyA)-M1M2-M1M2-M1M2 (GSC4401, SEQ ID NO: 38) and pGLEX4LHC-I4-M1M2-M1M2-M1M2-SV40ter (GSC4402, SEQ ID NO: 39), respectively, digested with the same enzymes and treated with CIP.

The ligations resulted in the midipreps pGLEX41-HC-14-M1M2-PTCRA-M1M2 (GSC6030, SEQ ID NO: 44), pGLEX41_HC-I4(0Y)-M1M2-PTCRA-M1M2 (GSC6048, SEQ ID NO: 45), pGLEX41-HC-I4(polyA)-M1M2-PTCRA-M1M2(GSC6047, SEQ ID NO: 46) and pGLEX41_HC-I4-M1M2-PTCRA-M1M2-SV40ter (GSC6046, SEQ ID NO: 47). All midipreps were confirmed by sequencing.

A first M1M2-PTCRA fusion was generated, these constructs allowed membrane display of the IgG1 and showed the same behaviour regarding modifications of introns as the native M1M2 constructs (see Figure 6 in Example 3).

A further construct pGLEX41_HC-I4-M1M2-PTCRA-M1M2_corrected was designed to allow a correct analysis of the impact of the modification of the transmembrane domain in the transmembrane region.

For this construct, a fusion PCR was performed. A portion of the cTNT 4 intron and the M1M2 extracellular part of the M1M2 transmembrane region were amplified from the plasmid pGLEX41_HC-I4-M1M2-M1M2-M1M2- (GSC3899, SEQ ID NO: 36) with the primers GlnPr1285 (SEQ ID NO: 259) and GlnPr2378 (SEQ ID NO: 221). The cytosolic tail of the M1M2 transmembrane region as well as the SV40 poly(A) signal were amplified with the primers GlnPr2379 (SEQ ID NO: 222) and GlnPr1650 (SEQ ID NO: 19) from the same template. The primers GlnPr2378 (SEQ ID NO: 221) and GlnPr2379 (SEQ ID NO: 222) allowed the creation of overlapping ends of the two PCR products creating the corrected PTCRA transmembrane domain. An overlapping PCR was then performed from these two first PCR products with the primers GlnP1285 (SEQ ID NO: 259) and GlnPr1650 (SEQ ID NO:19). The resulting fusion PCR product was digested with Agel and BstBI and ligated in the backbone pGLEX41_HC-I4-M1M2-M1M2-M1M2 (GSC3899, SEQ ID NO: 36) digested with the same enzymes and treated with CIP. After miniprep screening, the midiprep pGLEX41_HC-I4-M1M2-PTCRA-M1M2_corrected was produced, received the plasmid batch number GSC11206 and was confirmed by sequencing (SEQ ID NO: 284).

*Cloning of the alternate splicing expression vector for membrane displayed heavy chain of an IgG1 antibody using the murine B7-1 transmembrane region*

In order to change the transmembrane region to the transmembrane region of the murine B7-1 gene, fusion PCRs were performed.

First, the sequence coding for the murine B7-1 transmembrane region was amplified by PCR from an in-house construct (ordered from GeneArt as GeneArt_Seq43, SEQ ID NO: 66) using the primers GlnPr2100 (SEQ ID NO: 33) and GlnPr2101 (SEQ ID NO:34).

For cloning of vector pGLEX41_HC-I4(0Y)-B7-B7-B7, the fragment coding for cTNT-I4(0Y) was amplified by PCR from template pGLEX41_HC-I4(0Y)-M1M2-M1M2-M1M2 (GSC4398, SEQ ID NO: 37) using primers GlnPr1516 (SEQ ID NO: 9) and GlnPr2102 (SEQ ID NO:35). The sequence coding for the murine B7-1 transmembrane region was amplified by PCR from an in-house construct (ordered from GeneArt as GeneArt_Seq43, SEQ ID NO: 66) using the primers GlnPr2100 (SEQ ID NO: 33) and GlnPr2101 (SEQ ID NO:34). Both PCR products were fused using the primers GlnPr1516 (SEQ ID NO: 9) and GlnPr2100 (SEQ ID NO:33). The resulting PCR product was digested with BstBI and HindIII, and was ligated into pGLEX41_HC-I4-M1M2-M1M2-M1M2 (GSC3899, SEQ ID NO: 36) digested with the same enzymes and treated with CIP. After miniprep screening, the midiprep of pGLEX41_HC-I4(0Y)-B7-B7-B7 was produced, received the batch number GSC7057 and was confirmed by restriction digest and sequencing (SEQ ID NO: 53).

For cloning of vector pGLEX41_HC-I4(polyA)-B7-B7-B7, the fragment coding for cTNT-I4(polyA) was amplified by PCR from template pGLEX41-HC-I4(polyA)-M1M2-M1M2-M1M2 (GSC4401, SEQ ID NO: 38) using primers GlnPr1516 (SEQ ID NO: 9) and GlnPr2099 (SEQ ID NO:32). The sequence coding for the murine B7-1 transmembrane region was amplified by PCR from an in-house construct (ordered from GeneArt as GeneArt_Seq43, SEQ ID NO: 66) using the primers GlnPr2100 (SEQ ID NO: 33) and GlnPr2101 (SEQ ID NO:34). Both PCR products were fused using the primers GlnPr1516 (SEQ ID NO: 9) and GlnPr2100 (SEQ ID NO:33). The resulting PCR product was digested with BstBI and HindIII, and was ligated into pGLEX41_HC-I4-M1M2-M1M2-M1M2 (GSC3899, SEQ ID NO: 36) digested with the same enzymes and treated with CIP. After miniprep screening, the resulting pGLEX41_HC-I4(polyA)-B7-B7-B7 midiprep was given the batch number GSC7058 and was confirmed by restriction digest and sequencing (SEQ ID NO: 54).

For cloning of the vector pGLEX41_HC-I4-B7-B7-B7, the cTNT-I4 was amplified by PCR from template pGLEX41_HC-I4-M1M2-M1M2-M1M2 (GSC3899, SEQ ID NO: 36) using primers GlnPr1516 (SEQ ID NO: 9) and GlnPr2099 (SEQ ID NO:32). The sequence coding for the murine B7-1 transmembrane region was amplified by PCR from an in-house construct (ordered from GeneArt as GeneArt_Seq43, SEQ ID NO: 66) using the primers GlnPr2100 (SEQ ID NO: 33) and GlnPr2101 (SEQ ID NO:34). Both PCR products were fused using the primers GlnPr1516 (SEQ ID NO: 9) and GlnPr2100 (SEQ ID NO:33). The resulting PCR product was digested with BstBI and HindIII, and was ligated into pGLEX41_HC-I4-M1M2-M1M2-M1M2 (GSC3899, SEQ ID NO: 36) digested with the same enzymes and treated with CIP. After miniprep screening, the resulting pGLEX41_HC-I4-B7-B7-B7 midiprep was given the batch number GSC7056 and was confirmed by restriction digest and sequencing (SEQ ID NO: 55).

For cloning of vector pGLEX41_HC-I4-B7-B7-B7-SV40ter, the same PCR product as for pGLEX41_HC- I4-B7-B7-B7 construct was used, digested with HindIII and BstBI and ligated into pGLEX41_HC-I4-M1M2-M1M2-M1M2-SV40ter (GSC4402, SEQ ID NO: 39) digested with the same enzymes and treated with CIP. After miniprep screening, the resulting pGLEX41_HC-I4-B7-B7-B7-SV40ter midiprep was given the batch number GSC7059 and was confirmed by restriction digest and by sequencing (SEQ ID NO: 56).

*Further reduction of the splice ratio of secreted to membrane displayed heavy chain of an IgG1 antibody using the murine B7-1 transmembrane region*

It has been shown that the membrane display of chimeric proteins is highly efficient using the murine B7-1 transmembrane region (Chou W-C et al., (1999) Biotechnol Bioeng, 65: 160-9; Liao K-W et al., (2001) Biotechnol Bioeng, 73: 313-23). It has also been shown herein that decreasing the amount of Ys in the poly(Y) tract of the splice-acceptor to 0 lowered the amount of displayed antibody on the cell membrane significantly (see Figure 6F in Example 3).

In order to assess the effect of the reduction of the amount of pyrimidines in the poly(Y) tract of the intron, several constructs were produced by PCR fusion.

**Table 3: Modification of the intron: Primer pairs and templates used for PCR.**

| Construct ID | Intron Sequence SEQ ID NO | Number of Y in the intron | Template PCR 1 | Primer pairs PCR 1 |
|---|---|---|---|---|
| pGLEX41_HC-14(3Y)-I4-B7-B7-B7 | 72 | 3Y | GSC4340 (SEQ ID NO: 307) | GlnPr1516 (SEQ ID NO: 9) / GlnPr2165 (SEQ ID NO:317) |
| pGLEX41_HC-I4(5Y)-I4-B7-B7-B7 | 73 | 10Y | GSC4222 (SEQ ID NO: 308) | GlnPr1516 (SEQ ID NO: 9) / GlnPr2164 (SEQ ID NO:316) |
| pGLEX41_HC-I4(5Y-5)-I4-B7-B7-B7 | 74 | 5Y | GSC2617 (SEQ ID NO: 309) | GlnPrl516 (SEQ ID NO: 9)/ GlnPr2163 (SEQ ID NO:315) |
| pGLEX41_HC-I4(9Y)-I4-B7-B7-B7 | 75 | 9Y | GSC4335 (SEQ ID NO: 310) | GlnPrl516 (SEQ ID NO: 9) / GlnPr2162 (SEQ ID NO:314) |

(continued)

| Construct ID | Intron Sequence SEQ ID NO | Number of Y in the intron | Template PCR 1 | Primer pairs PCR 1 |
|---|---|---|---|---|
| pGLEX41_HC-I4(15Y-3')-I4-B7-B7-B7 | 76 | 25Y | GSC2332 (SEQ ID NO: 311) | GlnPrl516 (SEQ ID NO: 9)/ GlnPr2166 (SEQ ID NO:318) |

A 1st PCR allowed amplifying the desired intron from in-house backbones containing the modified introns. The primers and templates used for each construct are listed in Table 3. A 2nd PCR (common for all constructs) allowed the amplification of the B7-1 transmembrane domains from the construct pGLEX41_HC-I4-B7-B7-B7 (GSC7056, SEQ ID NO: 55) with the primers GlnPr2101 (SEQ ID NO: 34) and GlnPr2100 (SEQ ID NO:33). After purification of these PCR products, the introns and transmembrane domain were fused by overlapping extension PCR with the primers GlnPr1516 (SEQ ID NO: 9) and GlnPr2100 (SEQ ID NO:33). The purified PCR products were then digested with HindIII and BstBI and ligated in the backbone of pGLEX41_HC-I4-B7-B7-B7 (GSC7056, SEQ ID NO: 55) digested with the same enzymes and treated with CIP. After miniprep screening, midipreps were produced for each of the constructs and verified by sequencing. The plasmid batch numbers attributed to each constructs are the following:

| | |
|---|---|
| pGLEX41_HC-I4(3Y)-I4-B7-B7-B7 | GSC9770 (SEQ ID NO: 62) |
| pGLEX41_HC-I4(5Y)-I4-B7-B7-B7 | GSC9763 (SEQ ID NO: 61) |
| pGLEX41_HC-I4(5Y-5)-I4-B7-B7-B7 | GSC9768 (SEQ ID NO: 60) |
| pGLEX41_HC-I4(9Y)-I4-B7-B7-B7 | GSC9949 (SEQ ID NO: 59) |
| pGLEX41_HC-I4(15Y-3')-I4-B7-B7-B7 | GSC9950 (SEQ ID NO: 63) |

Another way to reduce the splicing was to weaken the splice donor site. The splice donor is characterized by a consensus sequence defining the 5'end of the intron. Therefore, two constructs were designed, where the DNA sequence of the splice donor consensus was modified without impact on the protein sequence expressed by the gene.

PCR amplifying the IgG1 heavy chain with modified 3' end sequence was performed on the template pGLEX41_HC-I4-B7-B7-B7 (GSC7056, SEQ ID NO: 55) with the primer GlnPr1518 (SEQ ID NO: 11) and GlnPr2161 (SEQ ID NO: 313) for the SD_CCC construct and GlnPr1518 (SEQ ID NO: 11) and GlnPr2160 (SEQ ID NO: 314) for the SD_GGC construct. The PCR were digested with NheI and HindIII and ligated in the backbone pGLEX41_HC-I4-B7-B7-B7 (GSC7056, SEQ ID NO: 55) digested with the same enzymes and treated with CIP. After miniprep screening, the midipreps pGLEX41_HC-SD_CCC-I4-B7-B7-B7 (plasmid batch number GSC9764, SEQ ID NO: 58) and pGLEX41_HC-SD_GGC-I4-B7-B7-B7 (plasmid batch number GSC9765, SEQ ID NO: 57) were produced and confirmed by sequencing. The intron sequence of the construct pGLEX41_HC-SD_GGC-I4-B7-B7-B7 is given in SEQ ID NO: 78.

*Replacement of B7-1 transmembrane domain by transmembrane domains of similar length with only hydrophobic residues*

To assess the effect of the transmembrane domain on the membrane display, several constructs were designed. Transmembrane helices containing mainly hydrophobic residues are thought to be favourable for the stabilization of the surface display, and thus, as 1st approach, the B7-1 transmembrane domain of the B7-1 transmembrane region was replaced by transmembrane domains of other proteins containing only hydrophobic residues. Moreover, as the length of the transmembrane helice might have an impact on surface display, these first constructs were only composed of transmembrane domains with a length similar to the B7-1 transmembane domain.

The sequences and characteristics of these transmembrane domains are summarized in Table 4.

**Table 4: Transmembrane domains with 22-23 amino acids (all hydrophobic): characteristics and sequences.**

| Transmembrane domain | Length | Hydrophobic | Polar | Charged | Sequence | SEQ ID NO |
|---|---|---|---|---|---|---|
| B7 | 22 | 20 | 2 | 0 | TLVLFGAGFGAVITVVVIVVII | 173 |
| ACLV1 | 23 | 23 | 0 | 0 | LALILGPVLALLALVALGVLGLW | 174 |
| ANTR2 | 23 | 23 | 0 | 0 | IAAIIVILVLLLLLGIGLMWWFW | 175 |
| CD4 | 22 | 22 | 0 | 0 | MALIVLGGVAGLLLFIGLGIFF | 176 |
| ITB1 | 23 | 23 | 0 | 0 | IAGVIAGILLFVIIFLGVVLVM | 179 |
| PTPRM | 22 | 22 | 0 | 0 | IAGVIAGILLFVIIFLGVVLVM | 177 |
| TNR5 | 22 | 22 | 0 | 0 | ALVVIPIIFGILFAILLVLVFI | 178 |

**Table 5: Primer pairs used for the cloning of hydrophobic transmembrane domains.**

| Transmembrane domain | Primer 1st PCR | Primers 2nd PCR |
|---|---|---|
| ACVL1 | GlnPr1516 (SEQ ID NO: 9) / GlnPr2400 (SEQ ID NO:243) | GlnPrl516 (SEQ ID NO: 9) / GlnPr2401 (SEQ ID NO:244) |
| ANTR2 | GlnPr1516 (SEQ ID NO: 9) / GlnPr2402 (SEQ ID NO:245) | GlnPrl516 (SEQ ID NO: 9) / GlnPr2403 (SEQ ID NO:246) |
| CD4 | GlnPr1516 (SEQ ID NO: 9) / GlnPr2404 (SEQ ID NO:247) | GlnPrl516 (SEQ ID NO: 9) / GlnPr2405 (SEQ ID NO:248) |
| PTPRM | GlnPrl516 (SEQ ID NO: 9) / GlnPr2408 (SEQ ID NO:251) | GlnPrl516 (SEQ ID NO: 9) / GlnPr2409 (SEQ ID NO:252) |
| TNR5 | GlnPr1516 (SEQ ID NO: 9) / GlnPr2410 (SEQ ID NO:253) | GlnPrl516 (SEQ ID NO: 9) / GlnPr2411 (SEQ ID NO:254) |
| ITB1 | GlnPr1516 (SEQ ID NO: 9) / GlnPr2406 (SEQ ID NO:249 | GlnPrl516 (SEQ ID NO: 9) / GlnPr2407 (SEQ ID NO:250) |

The different transmembrane domains were created by 2 step PCR amplification of a portion of the cTNT intron 4 with primers modifying the 3' end of the transmembrane domain. The template was always the pGLEX41_HC-I4-B7-B7-B7 (GSC7056, SEQ ID NO: 55) construct. For each construct, a first round PCR was performed with the respective primers listed in Table 5. After clean-up of this 1st round PCR products, a 2nd round of PCR was performed with the primers listed in the Table 5. After clean-up, these PCR products were digested with AgeI and ClaI and ligated in the backbone pGLEX41_HC-I4-B7-B7-B7 (GSC7056, SEQ ID NO: 55) digested with the same enzymes and treated with CIP. After confirmation of the sequences at the miniprep level, midiprep of the different contracts were produced and the following GSC numbers were assigned to these plasmid batches:

| | |
|---|---|
| pGLEX41-GBR200HC-I4-B7-ACVL1-B7 | GSC11213 (SEQ ID NO : 81) |
| pGLEX41-GBR200HC-I4-B7-ANTR2-B7 | GSC11214 (SEQ ID NO : 82) |
| pGLEX41-GBR200HC-I4-B7-CD4-B7 | GSC11210 (SEQ ID NO : 83) |
| pGLEX41-GBR200HC-I4-B7-PTPRM-B7 | GSC11205 (SEQ ID NO : 84) |
| pGLEX41-GBR200HC-I4-B7-TNR5-B7 | GSC11217 (SEQ ID NO : 85) |
| pGLEX41-GBR200HC-I4-B7-ITB1-B7 | GSC11756 (SEQ ID NO : 86) |

All midipreps were confirmed by sequencing.

*Replacement of B7-1 transmembrane domain by transmembrane domains of similar length containing charged and/or polar residues*

As transmembrane domains containing only hydrophobic residues have no impact on surface display (see Figure 10 in Example 4), other transmembrane domains were chosen having a similar length than B7-1 transmembrane domain but containing one or more charged and/or polar residues.

The sequences and characteristics of these transmembrane domains are summarized in the Table 6.

**Table 6: Transmembrane domains with 21-24 amino acids containing charged and/or polar residues: characteristics and sequences.**

| Transmembrane domain | Length | Hydrophobic | Polar | Charged | Sequence | SEQ ID NO |
|---|---|---|---|---|---|---|
| **B7** | 22 | 20 | 2 | 0 | TLVLFGAGFGAVITVVVIVVII | 173 |
| **IGF1R** | 24 | 22 | 1 | 1 | LIIALPVAVLLIVGGLVIMLYVFH | 181 |
| **1B07** | 24 | 22 | 1 | 1 | GIVAGLAVLAVVVIGAVVAAVMCR | 180 |
| **TRBM** | 24 | 19 | 4 | 1 | GLLIGISIASLCLVVALLALLCHL | 182 |
| **IL4RA** | 24 | 16 | 8 | 0 | LLLGVSVSCIVILAVCLLCYVSIT | 183 |
| **LRP6** | 23 | 15 | 8 | 0 | TNTVGSVIGVIVTIFVSGTVYFI | 184 |
| **GpA** | 23 | 19 | 4 | 0 | ITLIIFGVMAGVIGTILLISYGI | 185 |
| **PTCRA** | 21 | 18 | 0 | 3 | ALWLGVLRLLLFKLLLFDLLL | 186 |

**Table 7: GeneArt constructs used for the cloning of transmembrane domains with 21-24 amino acids containing charged and/or polar residues.**

| Transmembrane domain | GeneArt Seq name | GeneArt Seq SEQ ID NO |
|---|---|---|
| 1B07 | GeneArt_Seq84 | 266 |
| IGFIR | GeneArt_Seq 83 | 265 |

(continued)

| Transmembrane domain | GeneArt Seq name | GeneArt Seq SEQ ID NO |
|---|---|---|
| TRBM | GeneArt_Seq 85 | 267 |
| IL4RA | GeneArt_Seq 86 | 268 |
| LRP6 | GeneArt_Seq 87 | 269 |
| GpA | GeneArt_Seq 94 | 276 |

The different transmembrane domains (except PTCRA) were ordered from GeneArt with the names indicated in the Table 7. Once received, GeneArt constructs were resuspended in water and then digested with AgeI and ClaI. The resulting inserts were ligated in the backbone pGLEX41_HC-I4-B7-B7-B7 (GSC7056, SEQ ID NO: 55) digested with the same enzymes and treated with CIP. After miniprep screening, midipreps were produced and received the following batch numbers:

| | |
|---|---|
| pGLEX41-GBR200HC-I4-B7-1B07-B7 | GSC11296 (SEQ ID NO : 87) |
| pGLEX41-GBR200HC-I4-B7-IGF1R-B7 | GSC11295 (SEQ ID NO : 88) |
| pGLEX41-GBR200HC-I4-B7-TRBM-B7 | GSC11390 (SEQ ID NO : 89) |
| pGLEX41-GBR200HC-I4-B7-IL4RA-B7 | GSC11391 (SEQ ID NO : 90) |
| pGLEX41-GBR200HC-I4-B7-LRP6-B7 | GSC11392 (SEQ ID NO : 91) |
| pGLEX41-GBR200HC-I4-B7-GpA-B7 | GSC11397 (SEQ ID NO : 92) |

For the construct pGLEX41_HC-I4-B7-PTCRA-B7, a 2 step PCR was performed. The cTNT intron with the B7 extracellular part of the B7-1 transmembrane region was amplified using the primers GlnPr1516 (SEQ ID NO: 9) and GlnPr2380 (SEQ ID NO: 223) and pGLEX41_HC-I4-B7-B7-B7 (GSC7056, SEQ ID NO: 55) as template. The resulting PCR product was re-amplified with the primers GlnPr1516 (SEQ ID NO: 9) and GlnPr2381 (SEQ ID NO: 224). The primers GlnPr2380 (SEQ ID NO: 223) and GlnPr2381 (SEQ ID NO: 224) allowed fusing the PTCRA transmembrane domain to the B7-1 extracellular part of the B7-1 transmembane region. The resulting PCR product was digested with ClaI and AgeI and recloned in the backbone pGLEX41_HC-I4-B7-B7-B7 (GSC7056, SEQ ID NO: 55) digested with the same enzymes and treated with CIP. After miniprep screening, the midiprep pGLEX41_HC-I4-B7-PTCRA-B7 was prepared. It received the batch number GSC11204 and was confirmed by sequencing (SEQ ID NO: 93).

*Modifications of charged residues in PTCRA transmembrane domain*

The PTCRA transmembrane domain is 21 amino acids long, 3 of which being charged residues. The presence of these charged residues might be an explanation for the relative poor performance of this particular transmembrane domain in the surface display system (see Figure 11 in Example 4). A mutation of these residues to the hydrophobic amino acid valine might thus be beneficial in term of surface display.

**Table 8: Mutations of charged residues in PTCRA transmembrane domain: sequences, primers and DNA batch numbers.**

| Construct name | Transmembrane domain sequence | SEQ ID NO | Primer 1st round PCR | Primer pair 2nd round PCR |
|---|---|---|---|---|
| pGLEX41_HC-I4-B7-PTCRA_R8V-B7 | ALWLGVLVLLLFKLLLFDLLL | 187 | GlnPr238 2 (SEQ ID NO: 225) | GlnPr 2383 (SEQ ID NO: 226) |
| pGLEX41_HC-I4-B7-PTCRA_K13V-B7 | ALWLGVLRLLLFVLLLFDLLL | 188 | GlnPr 2380 (SEQ ID NO: 223) | GlnPr 2384 (SEQ ID NO: 227) |
| pGLEX41_HC-I4-B7-PTCRA_D18V-B7 | ALWLGVLRLLLFKLLLFVLLL | 189 | GlnPr 2380 (SEQ ID NO: 223) | GlnPr 2385 (SEQ ID NO: 228) |

(continued)

| Construct name | Transmembrane domain sequence | SEQ ID NO | Primer 1st round PCR | Primer pair 2nd round PCR |
|---|---|---|---|---|
| pGLEX41_HC-I4-B7-PTCRA_R8V_K13V-B7 | ALWLGVLVLLLFVLLLFDLLL | 190 | GlnPr 2382 (SEQ ID NO: 225) | GlnPr 2386 (SEQ ID NO: 229) |
| pGLEX41_HC-I4-B7- | ALWLGVLVLLLFKLLLFVLLL | 191 | GlnPr | GlnPr |
| PTCRA_R8V_D18V-B7 | | | 2382 (SEQ ID NO:225) | 2387 (SEQ ID NO:230) |
| pGLEX41_HC-I4-B7-PTCRA_K13V_D18V-B7 | ALWLGVLRLLLFVLLLFVLLL | 192 | GlnPr 2380 (SEQ ID NO: 223) | GlnPr 2388 (SEQ ID NO: 231) |
| pGLEX41_HC-I4-B7-PTCRA_R8V_K13V_D18V-B7 | ALWLGVLVLLLFVLLLFVLLL | 193 | GlnPr 2382 (SEQ ID NO: 225) | GlnPr 2389 (SEQ ID NO: 232) |

All possible combinations of non-mutated and mutated charged residues in PTCRA transmembrane domain were generated by two step PCRs using pGLEX41_HC-I4-B7-B7-B7 (GSC7056, SEQ ID NO: 55) as template, with GlnPr1516 (SEQ ID NO: 9) as forward primer and the reverse primer described in Table 8 for the different constructs. After the 1st round of PCR, PCR products were cleaned up and a second round of PCR was performed with GlnPr1516 (SEQ ID NO: 9) as forward primer and the reverse primer described in Table 8. The resulting PCR products were cleaned up, digested with ClaI and Age I and ligated in the backbone pGLEX41_HC-I4-B7-B7-B7 (GSC7056, SEQ ID NO: 55) digested with the same enzymes and treated with CIP. After miniprep screening, the positive constructs were prepared at midiprep scale and were confirmed by sequencing. The batch numbers of the respective constructs are the following:

| | |
|---|---|
| pGLEX41_HC-I4-B7-PTCRA_R8V-B7 | GSC11216 (SEQ ID NO: 94) |
| pGLEX41_HC-I4-B7-PTCRA_K13V-B7 | GSC11203 (SEQ ID NO: 95) |
| pGLEX41_HC-I4-B7-PTCRA_D18V-B7 | GSC11211 (SEQ ID NO: 96) |
| pGLEX41_HC-I4-B7-PTCRA_R8V_K13V-B7 | GSC11212 (SEQ ID NO: 97) |
| pGLEX41_HC-I4-B7-PTCRA_R8V_D18V-B7 | GSC11398 (SEQ ID NO: 98) |
| pGLEX41_HC-I4-B7-PTCRA_K13V_D18V-B7 | GSC11208 (SEQ ID NO: 99) |
| pGLEX41_HC-I4-B7-PTCRA_R8V_K13V_D18V-B7 | GSC11291 (SEQ ID NO: 100) |

*Replacement of B7-1 transmembrane domain by transmembrane domains of different lengths containing charged and/or polar residues*

As the presence of charged and residues in the transmembrane domains does not abrogate the surface display (see Figure 12 in Example 4), the length of the transmembrane domain was assessed using transmembrane domains shorter or longer than B7-1 transmembrane domain and containing this times one or more charged and/or polar residues.

The sequences and characteristics of these transmembrane domains are summarized in the Table 9.

**Table 9: Transmembrane domains of different length containing charged and/or polar residues: characteristics and sequences.**

| Transmembrane domain | Length | Hydrophobic | Polar | Charged | Sequence | SEQ ID NO |
|---|---|---|---|---|---|---|
| B7 | 22 | 20 | 2 | 0 | TLVLFGAGFGAVITVVVIVVII | 173 |
| IL3RB | 17 | 16 | 1 | 0 | VLALIVIFLTIAVLLAL | 196 |
| FCERA | 19 | 17 | 1 | 1 | FFIPLLVVILFAVDTGLFI | 194 |
| KI2L2 | 19 | 17 | 2 | 0 | ILIGTSVVIILFILLFFLL | 195 |
| CD3E | 26 | 18 | 7 | 1 | VMSVATIVIVDICITGGLLLLVYYWS | 197 |
| ITA2B | 26 | 25 | 1 | 0 | AIPIWWVLVGVLGGLLLLTILVLAMW | 198 |
| CD28 | 27 | 23 | 4 | 0 | FWVLVVVGGVLACYSLLVTVAFIIFWV | 199 |
| M1M2 | 27 | 16 | 10 | 1 | LWTTITIFITLFLLSVCYSATVTFFKV | 200 |

**Table 10: GeneArt constructs used for the cloning of transmembrane domains with different lengths containing charged and/or polar residues.**

| Transmembrane domain | GeneArt Seq name | GeneArt SEQ ID NO |
|---|---|---|
| IL3RB | GeneArt_Seq 88 | 270 |
| FCERA | GeneArt_Seq 89 | 271 |
| KI2L2 | GeneArt_Seq 90 | 272 |
| CD3E | GeneArt_Seq 91 | 273 |
| ITA2B | GeneArt_Seq 92 | 274 |
| CD28 | GeneArt_Seq 93 | 275 |
| M1M2 | GeneArt_Seq 80 | 262 |

The different transmembrane domains were ordered from GeneArt with the names indicated in the Table 10. Once received, GeneArt constructs were resuspended in water and then digested with AgeI and ClaI. The resulting inserts were ligated in the backbone pGLEX41_HC-I4-B7-B7-B7 (GSC7056, SEQ ID NO: 55) digested with the same enzymes and treated with CIP. After miniprep screening, midipreps were produced and received the following batch numbers:

| | |
|---|---|
| pGLEX41-GBR200HC-I4-B7-FCERA-B7 | GSC11297 (SEQ ID NO : 101) |
| pGLEX41-GBR200HC-I4-B7-KI2L2-B7 | GSC11298 (SEQ ID NO : 102) |
| pGLEX41-GBR200HC-I4-B7-IL3RB-B7 | GSC11393 (SEQ ID NO : 103) |
| pGLEX41-GBR200HC-I4-B7-CD3E-B7 | GSC11394 (SEQ ID NO : 104) |
| pGLEX41-GBR200HC-I4-B7-ITA2B-B7 | GSC11395 (SEQ ID NO : 105) |
| pGLEX41-GBR200HC-I4-B7-CD28-B7 | GSC11396 (SEQ ID NO : 106) |
| pGLEX41-GBR200HC-I4-B7-M1M2-B7 | GSC11389 (SEQ ID NO : 107) |

*Modification of B7-1 transmembrane domain length*

In order to assess the impact of the transmembrane length on surface display, different variants of B7-1 transmembrane domains were generated by removing or adding hydrophobic residues in the middle of B7-1 transmembrane domain sequence.

**Table 11: Modification of B7-1 transmembrane domain length: sequence and primer pairs.**

| Transmenbrane domain | Sequence | SEQ ID NO | Primers |
|---|---|---|---|
| B7 (18) | TLVLFGAGFGATVIVVII | 201 | GlnPr2395 (SEQ ID NO: 238) / GlnPr2394 (SEQ ID NO:237) |
| B7 (20) | TLVLFGAGFGATVVVIVVII | 202 | GlnPr2392 (SEQ ID NO: 235) / GlnPr2393 (SEQ ID NO:236) |
| B7 (24) | TLVLFGAGFGAVVIVTVVVIVVII | 203 | GlnPr2396 (SEQ ID NO: 239) / GlnPr2397 (SEQ ID NO:240) |
| B7 (26) | TLVLFGAGFGAVVIVVVTVVVIVVII | 204 | GlnPr2398 (SEQ ID NO: 241) / GlnPr2399 (SEQ ID NO:242) |

For this purpose, primer annealing was performed. For each variant of B7-1 transmembrane domains, primers were ordered that anneal in their 3' ends. The primer pairs used for each construct are described in Table 11. The primers were allowed to anneal and then the sequence was completed by PCR. After clean-up, the resulting PCR products were either directly digested with SfoI and AgeI and cloned into pGLEX41_HC-I4-B7-B7-B7 (GSC7056, SEQ ID NO: 55) backbone digested with the same enzymes and treated with CIP, or ligated into the pCR-blunt (Zero Blunt® PCR Cloning Kit, Invitrogen, LifeTechnologies) shuttle vector (as the annealing products are very short, the direct ligation did not work for every product). In the 2nd case, minipreps were extracted from colonies obtained after ligation of the annealed product in pCR-blunt (Zero Blunt® PCR Cloning Kit, Invitrogen, LifeTechnologies). After sequence confirmation, the positive minipreps were digested with SfoI and AgeI and the obtained fragments were ligated cloned into pGLEX41_HC-I4-B7-B7-B7 (GSC7056, SEQ ID NO: 55) backbone digested with the same enzymes and treated with CIP. After miniprep screening, midipreps were prepared for each constructs. A GSC batch number was attributed to these midipreps as described below and the preps were confirmed by sequencing.

| | |
|---|---|
| pGLEX41-GBR200HC-I4-B7-B7(18)-B7 | GSC11677 (SEQ ID NO : 108) |
| pGLEX41-GBR200HC-I4-B7-B7(20)-B7 | GSC11679 (SEQ ID NO : 109) |
| pGLEX41-GBR200HC-I4-B7-B7(24)-B7 | GSC11484 (SEQ ID NO : 110) |
| pGLEX41-GBR200HC-I4-B7-B7(26)-B7 | GSC11292 (SEQ ID NO : 111) |

*Modification of cytosolic tail in B7-1 transmembrane region*

As the cytosolic tail (with or without an ER exportation signal) may have an impact on surface display, several constructs were designed with modified cytosolic tails.

The sequences and characteristics of these cytosolic tails are summarized in the Table 12.

**Table 12: Cytosolic tails: characteristics and sequences.**

| Cytosolic tail | ER export signal | Sequence | SEQ ID NO. |
|---|---|---|---|
| B7 | Yes | KCFCKHRSCFRRNEASRETNNSLTFGPEEALAEQTVFL | 218 |
| M1M2 | ? | KWIFSSVVDLKQTIIPDYRNMIGQGA | 207 |
| 6His | No | ASAHHHHHH | 205 |
| KCFCK | No | KCFCK | 206 |
| ERGIC53 | Yes | RSQQEAAAKKFF | 208 |
| ERGIC53_noER | No | RSQQEAAAKKWW | 209 |

(continued)

| Cytosolic tail | ER export signal | Sequence | SEQ ID NO. |
|---|---|---|---|
| GAT1 | Yes | MFLTLKGSLKQRLQVMIQPSEDIVRPENGPEQPQAGSSASKEAYI | 210 |
| GAT1_noER | No | MFLTLKGS | 211 |
| AGTR2 | Yes | CFVGNRFQQKLRSVFRVPITWLQGKRETMSCRKSSSLREMDTFVS | 212 |
| AGTR2_noER | No | CFVGNRFQQKLRSVFRVPITWLQGKRETMSCRKSSSLR | 213 |
| V1BR | Yes | NSHLLPRPLRHLACCGGPQPRMRRRLSDGSLSSRHTTLLTRSSCPATLSLSLSLTLSGRPRPEESPRDLELADGEGTAETIIF | 214 |
| V1BR_noER | No | NSHEESPRDLELADGEGTAETIIF | 215 |
| VGLG | Yes | CIKLKHTKKRQIYTDIEMNRLGK | 216 |
| VGLG_noER | No | CIKLKHTKKRQIAAAAAANRLGK | 217 |

Different cloning strategies were applied according to the length of the different constructs. The first constructs were generated by PCR amplification of the cTNT intron 4 with B7-1 extracellular and transmembrane domains with primers modifying the cytosolic tail.

**Table 13: Primer pairs and enzymes used for cytosolic tail modification.**

| Cytosolic tail | Primer pair | Enzymes |
|---|---|---|
| 6His | GlnPr2377 (SEQ ID NO: 220) / GlnPr 1516 (SEQ ID NO:9) | HindIII / BstBI |
| KCFCK | GlnPr 2376 (SEQ ID NO: 219)/ GlnPr 1516 (SEQ ID NO:9) | HindIII / BstBI |
| GAT1_noER | GlnPr 2415 (SEQ ID NO: 258) / GlnPr 1516 (SEQ ID NO:9) | ClaI / BstBI |

The primers pairs used are described in the Table 13 and the template used was pGLEX41_HC-I4-B7-B7-B7 (GSC7056, SEQ ID NO: 55). The purified PCR products were digested with the enzymes described in Table 13 and cloned in the backbone pGLEX41_HC-I4-B7-B7-B7 (GSC7056, SEQ ID NO: 55) digested with the same enzymes and treated with CIP. After miniprep screening, midipreps were produced which received the following batch number:

| | |
|---|---|
| pGLEX41-GBR200HC-I4-B7-B7-6His | GSC11207 (SEQ ID NO : 112) |
| pGLEX41-GBR200HC-I4-B7-B7-KCFCK | GSC11209 (SEQ ID NO : 113) |
| pGLEX41-GBR200HC-I4-B7-B7-GAT1noER | GSC11483 (SEQ ID NO : 118) |

All midipreps were confirmed by sequencing.

In order to fuse the M1M2 cytosolic tail to the B7-1 transmembrane region, a fusion PCR was performed. The cTNT intron 4 with the B7-1 extracellular and transmembrane domains were amplified with GlnPr1516 (SEQ ID NO: 9) and GlnPr2391 (SEQ ID NO: 234) using pGLEX41_HC-I4-B7-B7-B7 (GSC7056, SEQ ID NO: 55) as template. The M1M2 cytosolic tail with the SV40 poly(A) signal was amplified with the primers GlnPr2390 (SEQ ID NO: 233) and GlnPr1650 (SEQ ID NO: 19) using pGLEX41_HC-I4-M1M2-M1M2-M1M2 (GSC3899, SEQ ID NO: 36) as template. After clean-up, both PCR products were fused in a 3rd PCR using GlnPr1516 (SEQ ID NO: 9) and GlnPr1650 (SEQ ID NO: 19) as primers. The resulting PCR product was cleaned up, digested wit SfoI and BstBI and ligated in pGLEX41_HC-I4-B7-B7-B7 (GSC7056, SEQ ID NO: 55) digested with the same enzymes and treated with CIP. After miniprep screening, the midiprep pGLEX41_HC-I4-B7-B7-M1M2 was produced, received the batch number GSC11758 and was confirmed by sequencing (SEQ ID NO: 114).

For some constructs, a strategy of primer annealing was chosen. For each cytosolic tail, primers were ordered that anneal in their 3' ends. The primer pairs used for each construct are described in Table 14. The primers were allowed to anneal and then the sequence was completed by PCR. After clean-up, the resulting PCR products were either directly digested with SfoI and BstBI and cloned into pGLEX41_HC-I4-B7-B7-B7 (GSC7056, SEQ ID NO: 55) backbone digested with the same enzymes and treated with CIP, or ligated into the pCR-blunt shuttle vector (Zero Blunt® PCR Cloning Kit, Invitrogen, LifeTechnologies) (as the annealing products are very short, the direct ligation did not work for every product). In the 2nd case, minipreps were extracted from colonies obtained after ligation of the annealed product in pCR-blunt (Zero Blunt® PCR Cloning Kit, Invitrogen, LifeTechnologies). After sequence confirmation, the positive minipreps were digested with SfoI and BstBI and the obtained fragments were ligated into pGLEX41_HC-I4-B7-B7-B7 (GSC7056, SEQ ID NO: 55) backbone digested with the same enzymes and treated with CIP. After miniprep screening, midipreps were prepared for each constructs. A GSC batch number was attributed to theses midipreps as described in Table 14 and the preps were confirmed by sequencing.

**Table 14: Primer pairs used for primer annealing for cytosolic tail modification and DNA batch numbers.**

| Cytosolic tail | Primers | Midiprep Batch number |
|---|---|---|
| ERGIC53 | GlnPr2412 (SEQ ID NO: 255) / GlnPr2413 (SEQ ID NO:256) | GSC11764 (SEQ ID NO: 115) |
| ERGIC53-noER | GlnPr2412 (SEQ ID NO: 255) / GlnPr2414 (SEQ ID NO:257) | GSC11678 (SEQ ID NO: 116) |

Finally, for the last constructs, the sequences were ordered from GeneArt with the sequence names described in the Table 15. Once received, GeneArt constructs were resuspended in water and then digested with SfoI and BstBI. The resulting inserts were ligated in the backbone pGLEX41_HC-I4-B7-B7-B7 (GSC7056, SEQ ID NO: 55) digested with the same enzymes and treated with CIP. After miniprep screening, midipreps were produced and received the batch numbers detailed in the following table.

**Table 15: GeneArt sequences ordered for cytosolic tail modification and DNA batch numbers.**

| Cytosolic tail | GeneArt Seq | Geneart SEQ ID NO | Midiprep batch number |
|---|---|---|---|
| GAT1 | GeneArt_Seq95 | 277 | GSC11759 (SEQ ID NO: 117) |
| AGTR2 | GeneArt_Seq 96 | 278 | GSC 11760 (SEQ ID NO: 119) |
| AGTR2_noER | GeneArt_Seq 97 | 279 | GSC 11761 (SEQ ID NO: 120) |
| V1BR | GeneArt_Seq 98 | 280 | GSC11762 (SEQ ID NO: 121) |
| V1BR_noER | GeneArt_Seq 99 | 281 | GSC11765 (SEQ ID NO: 122) |
| VGLG | GeneArt_Seq 100 | 282 | GSC 11766 (SEQ ID NO: 123) |
| VGLG_noER | GeneArt_Seq 101 | 283 | GSC11763 (SEQ ID NO: 124) |

In order to further evaluate the impact of the B7-1 cytosolic tail, the cytosolic tail of M1M2 transmembrane domain was replaced by the B7-1 cytosolic tail. For this purpose, the M1M2 transmembrane domain with the B7-1 cytosolic tail was ordered from GeneArt as GeneArt_Seq82 (SEQ ID NO: 264). The GeneArt construct was digested with ClaI and BstBI and the insert was ligated in the backbone pGLEX41_HC-I4-B7-B7-B7 (GSC7056, SEQ ID NO: 55) digested with the same enzymes and treated with CIP. After miniprep screening, the midiprep pGLEX41_HC-M1M2-M1M2-B7 was produced, received the plasmid batch number GSC11294 and was confirmed by sequencing (SEQ ID NO: 125).

*Cloning of the vector for the expression of the light chain of an IgG1 antibody*

The cloning vector provided from GeneArt carrying the light chain insert (0704970pGA4) was used as a template for PCR using the primers GlnPr501 (SEQ ID NO: 4) and GlnPr502 (SEQ ID NO:5). These primers amplify the open reading frame of the light chain and add a HindIII restriction site 5' and an XbaI restriction site 3' to the open reading frame. The amplicon was digested with the XbaI and HindIII and was ligated in a shuttle vector cut using XbaI and HindIII. After miniprep screening, a gigaprep was produced, which was confirmed by sequencing and digestion.

The coding region of the light chain of an antibody of the IgG1 format was cut from this gigaprep with XbaI and HindIII, and cloned into the backbone of the plasmid pGLEX41 (GSC281, SEQ ID NO: 304) opened with the same enzymes and treated with CIP. After miniprep screening, a maxiprep of pGLEX41_LC was produced, received the batch number GSC315 (SEQ ID NO: 294) and was confirmed by sequencing. Subsequently, several batches of the same DNA were produced at midiprep or gigaprep level and received the batch numbers GSC315a, GSC315b, GSC315c, GSC315d and GSC315e (as all these preps were confirmed by sequencing they wil be referred to in the following as GSC315 plasmid (SEQ ID NO: 294)).

*Cloning of the vector for the expression of the light chain of an IgG4 antibody*

The sequence coding for the IgG4 heavy chain was generated at Glenmark and was received in a shuttle vector. The IgG4 heavy chain coding sequence was amplified by PCR with the primers GlnPr1488 (SEQ ID NO: 328) and GlnPr1452 (SEQ ID NO:327). After purification, the resulting PCR product was re-amplified with the primers GlnPr1494 (SEQ ID NO: 260) and GlnPr1452 (SEQ ID NO: 327). These two rounds of PCR modified the leader peptide and added restriction sites at both ends of the coding sequence. The resulting PCR product was purified and cloned in the shuttle vector pCR-blunt (Zero Blunt® PCR Cloning Kit, Invitrogen, LifeTechnologies). After miniprep screening, a positive clone was digested with SpeI and ClaI to extract the IgG4 heavy chain coding sequence. The resulting insert was ligated in pGLEX41 (GSC281, SEQ ID NO: 304) digested with NheI and BstBI and treated with CIP. After miniprep screening, the gigaprep pGLEX41_IgG4-HC was produced, received the plasmid batch number GSB59 and was confirmed by sequencing (SEQ ID NO: 79).

*Cloning of the vector for the expression of the heavy chain of an IgG4 antibody with alternate splicing allowing the expression of the B7-1 transmembrane region*

For the addition of the alternate splicing cassette for membrane-bound IgG4 expression, the coding sequence for IgG4 heavy chain was amplified by PCR with the primers GlnPr1494 (SEQ ID NO: 260) and GlnPr2294 (SEQ ID NO: 261) using pGLEX41_IgG4-HC (GSB59, SEQ ID NO: 79) as template. The resulting PCR product was cleaned-up, digested with SpeI and HindIII and ligated into the pGLEX41_HC-I4-B7-B7-B7 (GSC7056, SEQ ID NO: 55) backbone digested with NheI and HindIII and treated with CIP. After miniprep screening, the midiprep pGLEX41_IgG4-HC-B7-B7-B7 was prepared which received the plasmid batch number GSC11218 and was confirmed by sequencing (SEQ ID NO: 80).

*Cloning of the vector for the expression of the light chain of an IgG4 antibody*

The sequence coding for the IgG4 light chain was generated at Glenmark and received in a shuttle vector. Using a single round of PCR with three different primers GlnPr1487 (SEQ ID NO:325), GlnPr1491 (SEQ ID NO: 326) and GlnPr1494 (SEQ ID NO:260), the leader peptide was exchanged and restriction sites were added at both ends of the coding sequence. The PCR product was digested with SpeI and ClaI and ligated in the pGLEX41 (GSC281, SEQ ID NO: 304) backbone opened with NheI and BstBI and treated with CIP. After miniprep screening, the midiprep pGLEX41_IgG4-LC was produced, received the plasmid batch number GSC3704 and was confirmed by sequencing (SEQ ID NO: 319).

*Cloning of the vector for the expression of secreted heavy chain of a BEAT® bispecific antibody*

The heavy chain of the bispecific antibody was generated at Glenmark as part of the bispecific BEAT® format. Using two rounds of PCR, a kozak sequence, a signal peptide and flanking restriction sites were added. In the first round, the template was amplified using primers GlnPr1726 (SEQ ID NO: 24) and GlnPr1500 (SEQ ID NO: 7). The PCR product was purified and used as template for a second round of PCR using primers GlnPr1500 (SEQ ID NO: 7) and GlnPr1725 (SEQ ID NO: 23). The resulting amplicon was purified and cut using the restriction enzymes SpeI and ClaI. This insert was cloned into the backbone pGLEX41 (GSC281, SEQ ID NO: 304), opened using NheI and BstBI and treated with CIP. After miniprep screening, the pGLEX41_BEAT-HC-His was produced in midiprep scale and received the batch number GSC5607 (SEQ ID NO: 296). The sequencing control and the restriction digest confirmed the plasmid.

This plasmid was used as template for a modifying PCR using primers GlnPr1725 (SEQ ID NO: 23) and GlnPr1760 (SEQ ID NO: 27) for removal of the His-tag from the template sequence. The amplicon was digested with SpeI and ClaI and cloned in the backbone of the vector pGLEX41 (GSC281, SEQ ID NO: 304), opened using the restriction enzymes NheI and BstBI and treated with CIP. After miniprep screening, the midiprep pGLEX41_BEAT-HC was given the plasmid batch number GSC5644 and was confirmed by sequencing (SEQ ID NO: 49).

*Cloning of the alternate splicing expression vector for membrane displayed heavy chain of a bispecific BEAT® antibody*

The template vector coding for the BEAT heavy chain (GSC5644, SEQ ID NO: 49) was amplified using the primers GlnPr1800 (SEQ ID NO: 29) and GlnPr1725 (SEQ ID NO: 23). The purified product was digested using the restriction enzymes SpeI and HindIII and cloned in the vector pGLEX41_HC-I4-M1M2-M1M2-M1M2(GSC3899, SEQ ID NO: 36) opened using the enzymes NheI and HindIII and treated with CIP. After miniprep screening, the maxiprep pGLEX41_BEAT-HC-14-M1M2-M1M2-M1M2, received the plasmid batch number GSC5537 (SEQ ID NO: 50) and was confirmed by restriction digest and sequencing.

In order to use the B7-1 transmembrane region for surface display, the insert of the construct pGLEX41_BEAT-HC-I4-M1M2-M1M2-M1M2 (GSC5537, SEQ ID NO: 50) was digested with SacI and HindIII and recloned in pGLEX41_HC-I4-B7-B7-B7 (GSC7056, SEQ ID NO: 55) digested with the same enzymes and treated with CIP. After miniprep screening, the maxiprep pGLEX41_BEAT-HC-I4-B7-B7-B7 was produced, received the plasmid batch number GSC10488 and was confirmed by sequencing (SEQ ID NO: 321).

*Cloning of the expression vector for secreted scFv-Fc of a bispecific antibody*

The scFv-Fc sequence was developed in-house as part of the bispecific BEAT® format. Amplification was performed using primers GlnPr1690 (SEQ ID NO: 22), GlnPr1689 (SEQ ID NO: 21) and GlnPr1502 (SEQ ID NO: 8). These primers add a kozak sequence, a signal peptide and restriction sites to the amplicon. The PCR product was re-amplified using primers GlnPr1689 (SEQ ID NO: 21) and GlnPr1502 (SEQ ID NO: 8) and the resulting amplicon was digested using NheI and ClaI. This insert fragment was ligated with the backbone pGLEX41 (GSC281, SEQ ID NO: 304) cut using NheI and BstBI and treated with CIP. After miniprep screening, the midiprep pGLEX41_scFv-Fc with the batch number GSC5608 (SEQ ID NO: 51) was produced and confirmed by restriction digest and sequencing.

*Cloning of the alternate splicing expression vector for membrane displayed scFv-Fc of a bispecific antibody*

The template vector coding for the scFv (GSC5608, SEQ ID NO: 51) was amplified using the primers GlnPr1801 (SEQ ID NO: 30) and GlnPr1689 (SEQ ID NO:21). The purified product was digested with NheI and HindIII and cloned in the vector pGLEX41_HC-14-M1M2-M1M2-M1M2 (GSC3899, SEQ ID NO: 36) opened using the same enzymes and treated with CIP. After miniprep screening, the midiprep pGLEX41_scFv-Fc-I4-M1M2-M1M2-M1M2 with the plasmid batch number GSC5538 (SEQ ID NO: 52) was produced and confirmed by restriction digest and sequencing.

In order to use the B7-1 transmembrane region for surface display, the insert of the construct pGLEX41_scFv-Fc-I4-M1M2-M1M2-M1M2 (GSC5537, SEQ ID NO: 50) was digested with NheI and HindIII and recloned in pGLEX41_HC-I4-B7-B7-B7 (GSC7056, SEQ ID NO: 55) digested with the same enzymes and treated with CIP. After miniprep screening, the maxiprep pGLEX41_scFv-Fc-I4-B7-B7-B7 was produced, received the plasmid batch number GSC10487 and was confirmed by sequencing (SEQ ID NO: 323).

*Cloning of the expression vector for the light chain of a bispecific antibody*

The light chain sequence was developed in-house as part of the bispecific BEAT® format. Amplification was performed using primers GlnPr1689 (SEQ ID NO: 21), GlnPr1727 (SEQ ID NO: 25) and GlnPr1437 (SEQ ID NO:6 ). These primers add a signal peptide, a Kozak sequence and flanking restriction sites to the amplicon. The amplicon was purified and digested using the restriction enzymes ClaI and NheI and cloned in the backbone of a shuttle vector, opened with NheI and ClaI and treated with CIP. After miniprep screening, a maxiprep was prepared and verified by sequencing. This shuttle vector coding for the light chain of the bispecific construct was digested using the restriction enzymes NheI and ClaI and the insert was cloned in the vector pGLEX41 (GSC281, SEQ ID NO: 304), opened with NheI and BstBI and treated with CIPed. After miniprep screening, the midiprep of pGLEX41_BEAT-LC was prepared (plasmid batch number GSC5540, SEQ ID NO: 302) and the plasmid was confirmed by restriction digest and sequencing.

## Example 2: Surface display and specific detection of translated product

Introduction

Example 1 described the cloning of alternate splicing constructs leading to expression of two different mRNAs from the same DNA template, coding either for a secreted protein or the same protein with a C-terminal transmembrane region (TM). As detailed in the definition section, a transmembrane region comprises an optional linker, a transmembrane domain and an optional cytoplasmic tail. These constructs were transfected in CHO-S cells in order to determine whether this technology could be used for displaying a fraction of the protein on the cell membrane, while maintaining an efficient secretion of the target protein. Three different proteins were used in this experiment: An antibody of the IgG1 subclass, an antibody of the IgG4 subclass and a bispecific antibody of the BEAT® format.

Material and Methods

*Transfections*

Suspension CHO-S cells were transfected with the expression vectors using polyethyleneimine (JetPEI®, Polyplus-transfection, Illkirch, France) in 50 ml bioreactor tube (Tubespins, TPP) format. For this purpose, exponential growing cells were seeded at a density of 2 E6 cells /mL in 5 mL of OptiMEM medium (#31985-047, Invitrogen) or CD-CHO Medium (#10743-011, Life Technologies). A JetPEI®:DNA complex was added to the cells in a weight ratio of 3 (μg/μg). Final DNA concentration in the cell suspension was 2.5 μg/mL. After 5 hours incubation at 37°C under shaking (200 rpm), 5 mL of fresh culture medium were added to the cell suspension. Then the cells were incubated on a shaken platform at 37°C, 5% $CO_2$ and 80% humidity.

*Surface staining*

Surface staining of the cells was performed on day 1 after transfection. A total of 1 E5 cells were harvested and transferred to a round bottom well of a 96 well plate. The cells were washed twice with washing buffer (2% FBS in PBS) and then resuspended in 100 μL washing buffer containing the detection antibody. The specific detection of the kappa light chain was performed using a mouse anti-human kappa light chain APC labelled antibody (#561323, BD Pharmingen), excited by a red laser (640 nm) and detected in the spectral range 661/19 nm. Both, heavy chain and scFv-Fc, were detected using PE conjugated goat anti-human Fc gamma specific antibody (#12-4998-82, eBioscience) excited by a blue laser (488 nm) and detected in the spectral range 583/26 nm and the scFv-Fc was detected specifically using a FITC-labelled Protein A (#P5145, Sigma) excited at 488 nm by a blue laser and detected in the spectral range 525/30. After 20 min incubation in the dark at room temperature, the cells were washed once in washing buffer and resuspended in 200 μL of washing buffer for flow cytometric analysis. The cells were analysed with a Guava flow cytometer (Merck Millipore) or with a FACSCalibur (Becton Dickinson)

The transient expression level of secreted molecules was measured on day 4-6 after transfection using the Octet QK instrument (Fortebio, Menlo Park, CA) and protein A biosensors.

Results

For IgG1 antibody expression, CHO-S cells were transfected as described in the Material and Methods section, using the expression vector pGLEX41_LC (SEQ ID NO: 294) expressing the light chain and a second vector named pGLEX41_HC-I4-B7-B7-B7 (SEQ ID NO: 55), pGLEX41_HC-I4-M1M2-M1M2-M1M2 (SEQ ID NO: 36), pGLEX41_HC-I4-PTCRA (SEQ ID NO: 40) or pGLEX41_HC-I4-M1M2-PTCRA-M1M2_corrected (SEQ ID NO: 284) , respectively. As control, the non-splicing construct coding for secreted heavy chain was used (SEQ ID NO: 48).

After transfection, the cells were stained as described in Materials and Methods and analyzed by flow cytometry. The negative control expressing the secreted version of light chain and heavy chain (no alternate splicing of a transmembrane region) only yielded a low intensity of surface staining (see unfilled histogram in Figure 2A and Figure 2E for the percentage of stained cells) with a relatively high secretion level of IgG1 (see Figure 2F). In contrast to this, cells transfected with constructs containing the M1M2 region (SEQ ID NO: 36) showed a significant positive population displaying the antibody on the cell membrane (see filled histogram in Figure 2B). The exchange of the entire M1M2 transmembrane region by the PTCRA transmembrane region (see Figure 2C; SEQ ID NO: 40), or the specific exchange of the transmembrane domain of M1M2 by the transmembrane domain of PTCRA (see Figure 2D; SEQ ID NO: 284) led to a positive, but weakly stained population of cells. The murine B7-1 transmembrane region (see Figure 2A; SEQ ID NO: 55) showed a surprisingly high level of staining with 40% of the population positive for membrane displayed IgG1.

Alternate splicing and hence, the membrane-bound expression of a fraction of the overall produced antibody seems to have a negative impact on the titers of secreted IgG. Nevertheless, the M1M2 transmembrane region (also in combination with the PTCRA transmembrane domain) and especially the B7-1 transmembrane region allowed up to 80% of the secretion level of the control construct without alternate splicing (see Figure 2F).

For IgG4 antibody expression CHO-S cells were transfected as described in the Material and Methods section, using the expression vector pGLEX41_IgG4-LC (SEQ ID NO: 319) expressing the light chain and a second vector coding for the heavy chain with alternate splicing of the B7-1 transmembrane region for membrane display (SEQ ID NO: 80). As control, the non-splicing construct coding for secreted IgG4 heavy chain was used (SEQ ID NO: 79).

After transfection, the cells were stained as described in Materials and Methods and analyzed by flow cytometry. The negative control expressing the secreted version of light chain and heavy chain (without alternate splicing of a transmembrane region) only yielded a negative population (see Figure 3A) with a substantial secretion level of IgG4 of approx. 6 μg/mL (see Figure 3C). In contrast to this, cells transfected with constructs containing the B7-1 region (SEQ ID NO: 80) showed a significant positive population displaying the antibody on the cell membrane (see Figure 3A for a histogram and Figure 3B for the percentage of staining) while the expression level remained at a similar level with and without alternate splicing (see Figure 3C).

To address whether a bispecific antibody could be displayed on the cell membrane via a transmembrane region (TM), the alternate splicing constructs described in Example 1 coding for bispecific antibodies were transiently expressed in CHO cells. The alternate splicing constructs code for a bispecific antibody format developed in-house and described in WO2012/131555 (termed "BEAT®"), which was used as a model protein in this example. The BEAT molecule is a trimer of an IgG heavy chain ("heavy chain"), a kappa light chain ("light chain") and a scFv fused to a heavy chain constant region ("scFv-Fc"). The Protein A-binding site of the heavy chain was abrogated so that only the Fc-fragment of the scFv-Fc was able to bind to Protein A. The design of the molecule and the engineering of the heavy chain Protein A binding site allowed for specific detection of every subunit of the trimer. Display on the cell membrane was achieved by either transfecting the alternate splicing construct of either the heavy chain or the scFv-Fc or transfecting both alternate splicing constructs. Membrane bound BEAT bispecific antibody displayed on the surface of single cells was specifically detected by flow cytometry.

In the following, the vectors coding for the regular expression constructs will be shortened to "pHC" for pGLEX41_BEAT-HC (SEQ ID NO: 49), "pLC" for pGLEX41_BEAT-LC (SEQ ID NO: 302) and "pScFv-Fc" for pGLEX41_scFv-Fc (SEQ ID NO: 51). The alternate splicing construct adding a transmembrane region to the HC will be termed "pHC-M1M2" (pGLEX41_BEAT-HC-I4-M1M2-M1M2-M1M2; SEQ ID NO: 50) and the construct adding a transmembrane region to the scFv-Fc will be termed "pScFv-Fc-M1M2" (pGLEX41_scFv-Fc-I4-M1M2-M1M2-M1M2; SEQ ID NO: 52). The expression vectors coding for the three subunits of the BEAT antibody were co-transfected using the alternate splicing expression constructs or regular expression constructs in different combinations. Table 16 summarizes the transfections performed and the species expected to be displayed on the cell membrane.

**Table 16: Transfection scheme and expected species displayed on the cell membrane**

| | Expected species displayed on cell membrane | | |
|---|---|---|---|
| **Transfected vectors** | **Fc** | **Kappa Lc** | **scFv-Fc** |
| pHC + pScFv-Fc + pLC | - | - | - |
| pHC-M1M2 + pScFv-Fc-M1M2 + pLC | + | + | + |
| pHC-M1M2 + pScFv-Fc + pLC | + | + | + |
| pHC + pScFv-Fc-M1M2 + pLC | + | + | + |
| pScFv-Fc-M1M1 | + | - | + |
| pHC-M1M2 + pLC | + | + | - |

The flow cytometric profiles obtained on day 1 after transfection are shown in Figure 4A and B. The first transfection cocktail (pHC + pScFv-Fc + pLC) did not include a construct with a transmembrane region for cell membrane display. Thus, the profiles obtained for this transfection (Figure 4A, D, G) were considered a negative control for the experiment and are presented as a dashed distribution in the histograms.

The transfection of the vector pScFv-Fc-M1M2 alone gave only positive cells for anti-Fc (detecting the heavy chain) and Protein A staining (Figure 4K, N, Q). In this case, no light chain was detected on the cell membrane demonstrating the specificity of the assay. Co-transfection of vectors pHC-M1M2 and pLC yielded positive cells for anti-Fc and kappa light chain staining, but no Protein A binding could be observed (Figure 4L, O, R). This was in accordance with the fact that the Protein A binding site had been abrogated in the heavy chain subunit of the BEAT molecule. Hence, although the anti-human Fc gamma antibody was not specific for the heavy chain and recognized the scFv-Fc as well, the detection of light chain allowed us to conclude on the expression of the heavy chain.

Transfections performed with plasmid cocktails coding for all three subunits, independent of the alternate splicing construct used, showed expression of the BEAT construct with at least one transmembrane region (Figure 4B, C, J, E, F, M, H, I, P). The staining was positive for the light chain and the scFv-Fc, as well as for the Fc fragments (heavy chain and scFv-Fc), indicating that all expected species were present on the cell membrane. Hence, fully folded multimers were present on the cell membrane confirming the effectiveness of the reporter system for the expression of multimeric proteins. Surprisingly, all species were detected regardless of the position of the transmembrane region. No difference was observed whether the transmembrane region (TM) was attached to both the heavy chain (HC) and the scFv-Fc subunit (Figure 4B, E, H), solely to the HC (Figure 4C, F, I) or solely to the scFv-Fc (Figure 4J, M, P).

Therefore, the approach presented here allows the distinction between a heterodimer (for instance heavy chain + scFv-Fc + light chain) and homodimers (heavy chain + light chain or scFv-Fc homodimer) displayed on the cell membrane. In the context of expression of multimeric proteins such as bispecific antibodies this quality of information is tremendously useful.

The concentrations of the secreted BEAT and scFv-Fc homodimer were measured on day 6 after transfection by Octet QK instrument using Protein A biosensors. The results are shown in Figure 5. As expected no expression could be measured with the Protein A biosensors for the transfection using the expression vectors pHC-M1M2 + pLC as the resulting product (a heavy chain-light chain homodimer) does not bind to Protein A. For the transfection using the expression vector pScFv-Fc-M1M2 a lower expression level was observed compared to the control transfection leading exclusively to secreted product. BEAT molecules or scFv-Fc homodimer could be detected in the supernatants for all transfections using alternate splicing constructs. This confirms that the BEAT heterotrimers were not only displayed on the cell membrane as previously demonstrated, but were also successfully secreted using the secretory pathway of the cells. More importantly, the expression levels obtained with the alternate splicing construct were comparable to the control (pHC + pScFv-Fc + pLC). This suggested that only a small fraction of proteins deviated from the secretory pathway for surface display, as no significant impact on the secretion level could be measured in this experimental set-up.

Summary

Based on these results it can be concluded that the alternate splicing constructs were successfully deflecting a fraction of the produced antibody to the cellular membrane by alternate splicing. While the surface staining was low for the constructs containing the PTCRA transmembrane domain, the staining was high for the cells transfected with the constructs containing the M1M2 transmembrane region and even higher for the constructs containing the B7-1 transmembrane region. Besides the signal strength, the percentage of the cellular population that could be stained was surprisingly high in the transfections with the B7-1 constructs, considering that the M1M2 transmembrane region was selected for efficient membrane display of antibodies during the evolution of B-cells and should therefore represent the most efficient construct for membrane display of immunoglobulins.

The overall antibody expression level decreased when a portion of the secreted antibody was redirected to the cell membrane, but around 80% of the expression level of the non-spliced control was reached with the different constructs.

Molecules with more than two subunits could also be successfully displayed on the cell membrane using the alternate splicing constructs of the present invention, as demonstrated by the example of the BEAT® constructs. These constructs fused a transmembrane region to a small fraction of the expressed protein. Using a bispecific antibody (BEAT) as an example, no significant differences in the secretion level could be observed compared to the control transfection leading exclusively to secreted protein. The observation that there was no, or only a minor impact of the alternate splicing on the expression level, makes the technology acceptable for industrial applications. Moreover, it could be shown that the approach as described herein allowed specific detection of the multimers displayed on the cell membrane. Surprisingly, the transmembrane region could be added to the heavy chain or to the scFv-Fc or to both subunits, without an impact on the surface display or the expression level of the secreted protein.

Since the multimeric molecules displayed on the cell membrane by alternate splicing reflect the product composition of secreted multimeric proteins of a specific cell, this technology would allow a cytometry based qualitative prediction of the secretion profile on the single cell level. This is demonstrated in the Example 5.

**Example 3: Modulation of cell surface display and expression titer by modification of intron sequence**

Introduction

As demonstrated in Example 2, alternate splicing of a transmembrane region allows redirecting a portion of an otherwise normally secreted antibody to the cell surface. Nevertheless, this modification of the secretion process might have a negative impact on the secretion level of antibody. Fine-tuning the splicing ratio between secreted and membrane displayed antibody might help recovering the expression level observed with the non-spliced antibody constructs (without an alternate splicing transmembrane region). This will be demonstrated in the following example.

Material and Methods

*Transfections*

Suspension CHO-S cells were transfected with expression vectors using polyethyleneimine (JetPEI®, Polyplus-transfection, Illkirch, France) in 50 ml bioreactor tube (Tubespins, TPP) format. For this purpose, exponential growing cells were seeded at a density of 2 E6 cells /mL in 5 mL of OptiMEM medium (#31985-047, Invitrogen). A JetPEI®:DNA complex was added to the cells in a weight ratio of 3 (μg/μg). Final DNA concentration in the cell suspension was 2.5 μg/mL. After 5 hours incubation at 37°C under shaking (200 rpm), 5 mL of fresh culture medium were added to the cell suspension. Then the cells were incubated on a shaken platform at 37°C, 5% $CO_2$ and 80% humidity.

*Surface staining*

Surface staining of the cells was performed on day 1 after transfection. A total of 1 E5 cells were harvested and transferred to a round bottom well of a 96 well plate. The cells were washed twice with washing buffer (2% FBS in PBS) and then resuspended in 100 μL washing buffer containing the detection antibody. The specific detection of the heavy chain was performed using a PE conjugated goat anti human IgG antibody (#512-4998-82, eBioscience) excited by a blue laser (488 nm) and detected in the spectral range 583/26 nm. After 20 min incubation in the dark at room temperature, the cells were washed once in washing buffer and resuspended in 200 μL of washing buffer for flow cytometric analysis. The cells were analysed with a Guava flow cytometer (Merck Millipore) or with a FACSCalibur (Becton Dickinson). Transient expression level of secreted molecules was measured on day 4-6 after transfection using the Octet QK instrument (Fortebio, Menlo Park, CA) and protein A biosensors.

Results

CHO-S cells were transfected as described in the Material and Methods section, using the expression vector pGLEX41_LC (SEQ ID NO: 294) expressing the light chain and a second vector coding for the heavy chain and containing modified versions of the intron with the M1M2 transmembrane domain (SEQ ID NO: 36-39), with the PTCRA transmembrane domain (SEQ ID NO: 40-43), with the M1M2-PTCRA fusion transmembrane domain (SEQ ID NO: 44-47) or with the B7-1 transmembrane domain (SEQ ID NO: 53-56). As control, the secreted heavy chain without alternate splicing was used (SEQ ID NO: 48).

After transfection, the cells were stained as described in Materials and Methods and analyzed by flow cytometry. As already observed in Example 2, the negative control expressing the secreted version of light chain and heavy chain (without alternate splicing of a transmembrane region) only yielded a low fraction of stained cells (see Figure 6) with a substantial level of IgG1 secretion (see Figure 7). In contrast to this, cells transfected with constructs containing the M1M2 region (SEQ ID NO: 36, Figure 6A) and the B7-1 transmembrane domain (SEQ ID NO: 55, Figure 6M) showed a significant positive population displaying the antibody on the cell membrane. With the PTCRA transmembrane domain (SEQ ID NO: 40, Figure 6E) or the M1M2-PTCRA fusion transmembrane domain (SEQ ID NO: 44, Figure 61), the positive population was weakly stained.

The reduction of the Y content in the poly(Y) tract of the intron has been shown to weaken the splice acceptor and was introduced in order to shift the splice ratio in favor of secreted antibody. For both transmembrane regions leading to successful display (M1M2 and B7-1), the constructs without Y in the poly(Y) tract of the intron (SEQ ID NO: 37 and SEQ ID NO: 53) showed no surface staining of cells (see Figure 6B and N). This suggested that the ratio in splicing was mostly shifted towards the secreted antibody.

The presence of an additional poly(A) site in the intron of the mouse immunoglobulin μ primary transcript has been shown to impact the alternate splicing event, leading to a higher fraction of secreted product (Galli et al., 1987) and less membrane displayed antibody. Based on this finding, an SV40 poly(A) site was introduced 3' of the stop codon of the secreted polypeptide and 5' of the branch point of the intron in order to shift the splice ratio in favor of antibody secretion. With the addition of the SV 40 poly(A) site in the intron the secretion level of antibody was significantly increased for the M1M2 construct compared to the original I4 one (see Figure 7). At the same time, the fraction of membrane displayed antibody for the M1M2 construct (see Figure 6C) was increased. As the staining of the B7-1 cells was already very high, no conclusions could be drawn on the impact of the poly(A) on the membrane display (see Figure 6O).

The gastrin terminator site (reported to terminate mRNA transcription) was introduced directly 3' of the poly(A) site of the expression cassette in order to avoid aberrant splice events with other splice acceptors in proximity of the expression cassette. The gastrin terminator construct increased the fraction of membrane displayed antibody especially for the M1M2 construct (Figure 6D), but did not lead to a significant increase in the secretion level. Again, no conclusion could be drawn for the B7-1 cells (Figure 6P).

Alternate splicing and hence, the membrane-bound expression of a portion of the overall produced antibody seems to have a negative impact on the overall secreted IgG titers. Nevertheless, the M1M2 transmembrane region (also in combination with the PTCRA transmembrane domain) and especially the B7-1 transmembrane region allowed up to 80% of the secretion level of the control construct without alternate splicing (see Figure 7). Furthermore, the addition of a poly(A) signal in the intron was found to be favorable for IgG secretion, whilst maintaining a significant staining on the cell membrane. The gastrin terminator on the other hand showed only a minor impact on secretion compared to the respective reference constructs (see Figure 7).

In the previous experiments, the different modifications of the intron sequence showed a similar impact on the efficiency of alternate splicing, independent of the transmembrane region of the construct. Therefore the B7-1 transmembrane region was chosen to further characterize the impact of intron sequence modifications on the alternate splice ratio and the antibody secretion level, as it was leading to the highest levels of staining.

In order to reduce the amount of cell surface displayed antibody and to restore the expression level of the control construct, the splice ratio was altered in favor of the secreted product, i.e. the splice donor and the splice acceptor site were weakened.

The poly(Y) tract present in the splice acceptor consensus sequence is known to play an important role in the splice acceptor strength (the shorter the poly(Y) tract, the weaker the splice acceptor site). In the precedent experiment, it was shown that complete reduction of the Y content in the poly(Y) tract to zero abolishes the cell surface display of the antibody. The native chicken TNT intron 4 poly(Y) tract contains 27 Y. A series of constructs with modified introns containing reduced numbers of Y in their poly(Y) tract were transfected in CHO-S cells as described previously (SEQ ID NO: 53, 55, 59-63). After surface staining and estimation of the expression level using the Octet device and protein A biosensors, an impact of the poly(Y) tract could be clearly identified on the cell surface display of the antibody: less than 9Y in the poly(Y) tract reduced dramatically the percentage of stained cells (see Figure 8 A, B, C, D, E, F, G, H) as well as the staining intensity (see Figure 8 A, B, C, D, E, F, G, J). Unfortunately, this reduction in cell surface display had no impact on the overall expression level which remained lower than the control for every construct (see Figure 8 I).

Another way to increase the splicing ratio towards the secreted product is to reduce the strength of the splice donor at the 5' end of the alternatively spliced intron by changing the DNA sequence of the splice donor. Two different DNA constructs (SD_CCC, SEQ ID NO 58 and SD_GGC, SEQ ID NO 57) coding for same amino acid sequence as the original construct could be generated by taking advantage of alternate codons coding for the same amino acid. While the first modification of the splice donor consensus sequence (SD_CCC, SEQ ID NO 58) had no effect on either percentage of stained cells, intensity of staining or expression level (see Figure 9), the second one (SD_GGC, SEQ ID NO 57) showed a dramatic decrease in surface display (both percentage and intensity, see Figure 9 C, D and F). This decrease in surface display correlated with a clear increase in expression titer, up to the level of the control without alternate transmembrane domain (see Figure 9E). Furthermore, although the cell surface display was dramatically decreased when compared to the native chicken TNT intron 4 sequence, a significant staining could be observed when compared with the control without transmembrane domain (see Figure 9C).

## Summary

The gastrin terminator increased the fraction of cells stained positive for IgG on the cell membrane especially for the M1M2 construct, but did not lead to higher expression of secreted IgG compared to the standard construct. The insertion of an additional poly(A) in the intron increased the fraction of membrane displayed antibody in the constructs M1M2 (for B7-1 no conclusion could be drawn). The M1M2 construct with the additional poly(A) also showed high levels of secreted IgG (up to 80% of the expression of the non-spliced control constructs).

Reduction of the poly(Y) tract was expected to weaken the splice acceptor and to reduce the expression of the membrane displayed splice variant. This could be confirmed for all four different basal constructs (M1M2, PTCRA, M1M2-PTCRA and B7-1). Furthermore, the level of surface display was found to be directly correlated to the length of the poly(Y) tract, as depicted by the examples with the B7-1 transmembrane domain. A minimum of 5 Y in the poly(Y) tract was found necessary for a significant surface staining in this context. At the same time, no increase in the expression levels of secreted antibody could be observed. Hence, the shift of the alternate splicing did not benefit the accumulation of secreted product. Unexpectedly, a specific modification of the splice donor consensus sequence allowed decreasing the level of surface staining while increasing the level of secreted antibody. The level of surface display was quite low, but still significant as compared to the control construct, and the secretion level was found to be the same as the control.

The inventors consider that the frequency of the alternate splice event is determined by the strength of the splice donor site. If the corresponding splice acceptor is strong enough (more than 5 Y in the poly(Y) tract), alternate splicing will lead to the formation of the alternate mRNA coding for the construct with transmembrane region and hence membrane display. If the splice acceptor is weakened by lowering the amount of Y in the poly(Y) tract to less than 5 Y, the splicing event will be less efficient and the alternate pre-mRNA might be degraded, while there is no impact on the non-spliced mRNA and thus on the secretion level. In accordance with this, less membrane display was observed after weakening of the splice acceptor, but there was no impact on the secretion level. Reducing the strength of the splice donor on the other hand might reduce the frequency of the alternate splicing event. As a consequence, more mRNA is coding for the secreted product of the non-spliced mRNA, leading to the observed increase in secreted antibody. The strong splice acceptor leads to efficient alternate splicing of mRNA, but at a lower frequency due to the weak splice donor, which might explain the significant, but weak membrane display observed.

In summary, modifications of the splice donor and / or splice acceptor sequences allowed a modulation of both the level of surface display and the secretion level of the antibody up to the secretion level of an antibody expressed without alternate splicing. The presented constructs therefore allow the fine-tuning of the display and secretion up to the desired levels by adjusting the alternate splicing efficiency.

Galli G1, Guise J, Tucker PW, Nevins JR (1988). Poly(A) site choice rather than splice site choice governs the regulated production of IgM heavy-chain RNAs. Proc Natl Acad Sci U S A. Apr;85(8):2439-43.

**Example 4: Impact of transmembrane region on cell surface display**

Introduction

The alternate splicing system for the membrane display of a fraction of antibody produced by cells might be altered by characteristics of the transmembrane region. For example, it could be speculated that the length and the structure of the transmembrane domain may influence the efficacy of the membrane display. Furthermore, the cytosolic tail of the transmembrane region might impact the cell surface display by presence or absence of an ER exportation signal. In the following example, we show that neither the amino acid composition of the transmembrane domain, its length, nor the cytosolic tail of the transmembrane region have a critical impact on surface display and secretion.

Material and Methods

*Transfections*

Suspension CHO-S cells were transfected with the expression vectors using polyethyleneimine (JetPEI®, Polyplus-transfection, Illkirch, France) in 50 ml bioreactor tube (Tubespins, TPP) format. For this purpose, exponential growing cells were seeded at a density of 2 E6 cells /mL in 5 mL of OptiMEM medium (#31985-047, Invitrogen). A JetPEI®:DNA complex was added to the cells in a weight ratio of 3 (μg/μg). Final DNA concentration in the cell suspension was 2.5 μg/mL. After 5 hours incubation at 37°C under shaking (200 rpm), 5 mL of fresh culture medium were added to the cell suspension. Then the cells were incubated on a shaken platform at 37°C, 5% $CO_2$ and 80% humidity.

*Surface staining*

Surface staining of the cells was performed on day 1 after transfection. A total of 1 E5 cells were harvested and transferred to a round bottom well of a 96 well plate. The cells were washed twice with washing buffer (2% FBS in PBS) and then resuspended in 100 μL washing buffer containing the detection antibody. The specific detection of the heavy chain was performed using a PE conjugated goat anti human IgG antibody (#512-4998-82, eBioscience) excited by a blue laser (488 nm) and detected in the spectral range 583/26 nm. After 20 min incubation in the dark at room temperature, the cells were washed once in washing buffer and resuspended in 200 μL of washing buffer for flow cytometric analysis. The cells were analysed with a Guava flow cytometer (Merck Millipore) or with a FACSCalibur (Becton Dickinson). Transient expression level of secreted molecules was measured on day 4-6 after transfection using the Octet QK instrument (Fortebio, Menlo Park, CA) and protein A biosensors.

Results

CHO-S cells were transfected as described in the Material and Methods section, using the expression vector pGLEX41_LC (SEQ ID NO: 294) expressing the light chain and a second vector coding for the heavy chain with different transmembrane regions.. As control, the secreted heavy chain without alternate splicing was used (SEQ ID NO: 48) and the construct with the B7-1 transmembrane region (SEQ ID NO: 55) was used as positive control.

After transfection, the cells were stained as described in Materials and Methods and analyzed by flow cytometry.

*Transmembrane domain length and composition*

In order to analyze the impact of the transmembrane domain on cell surface display and antibody secretion levels, the B7-1 transmembrane domain was replaced by transmembrane domains of similar length (22-23 amino acids) containing only hydrophobic residues (see SEQ ID NO: 81-86). Exchanging the transmembrane domain had no impact on cell surface display or secretion of the antibody (see Figure 10).

When the B7-1 transmembrane domain was replaced by transmembrane domains of similar length (21-24 amino acids) containing not exclusively hydrophobic residues, but also polar or charged residues (SEQ ID NO: 87-93) no impact could be seen on surface display or on secretion, except for the transmembrane domain derived from PTCRA (SEQ ID NO: 93). This transmembrane domain, without changing the fraction of cells exhibiting a product on the cell surface, reduced dramatically the surface density of the product without any impact on secretion level (see Figure 11 H, J, K and L)

Transmembrane domains are surrounded by a hydrophobic lipid phase. The energy cost of inserting an ionizable group in the hydrophobic environment of the membrane is very high, therefore there should be a strong bias against charged amino acids in the transmembrane domain (White and Wimley, 1999). A statistical analysis of transmembrane helices confirmed that transmembrane domains are composed mainly by hydrophobic amino acid residues, particularly in the hydrophobic core of the transmembrane region (Beza-Delgado, 2012). Based on this observation we wanted to identify the impact of three charged residues (R8, K13 and D18, the numbering starting at the beginning of the transmembrane helix) in the PTCRA transmembrane domain on the observed weak staining intensity. Several PTCRA constructs were designed where different charged residues were exchanged for the amino acid valine (one of the four most frequent amino acids in the transmembrane domain (Baeza-Delgado et al., 2012)) and transfected in CHO-S cells (SEQ ID NO: 94-100). The results of these transfections confirmed the impact of the charged residues on the surface staining intensity. Mutation of the single amino acid R8V (SEQ ID NO: 94), K13V (SEQ ID NO: 95) or D18V (SEQ ID NO: 96) allowed to increase significantly the density of surface displayed antibody, but had no significant impact on the secretion level of the antibody (see Figure 12 B, C, D and J). As expected from the results of the single mutations, the combinations of 2 mutated charged residues (either R8V and K13V (SEQ ID NO: 97), R8V and D18V (SEQ ID NO: 98) or K13V and D18V (SEQ ID NO: 99)) showed a level of surface display and a similar secretion level than the transmembrane domain of PTCRA (see Figure 12 E, F, G and J). Finally, the mutation of all three charged residues to valine (SEQ ID NO: 100) resulted as expected in a high surface display compared to the control. Unexpectedly, the expression level of secreted antibody, as well as the percentage of stained cells was slightly lower with this construct (see Figure 12 H and J). The reason for the reduced secretion and staining levels is currently unclear.

When the B7-1 transmembrane domain was replaced by shorter transmembrane domains (17-19 amino acids) containing not only hydrophobic residues, but also polar or charged residues (SEQ ID NO: 101-103), different effects could be identified. The results can be seen in Figure 13. Whereas the transmembrane domain of FCERA (SED ID NO: 101) and KI2L2 (SEQ ID NO: 102) had a minor detrimental effect on the percentage of stained cells (Figure 13 B, C and E), only the transmembrane domain derived from FCERA had a negative impact on the intensity of the staining (Figure 13 C and G). Furthermore, both transmembrane domains (FCERA and KI2L2) had a minor favorable effect on the secretion level (Figure 13F). For the transmembrane domain from IL3RB (SEQ ID NO: 103) on the other hand, no change compared to the B7-1 reference construct was seen for the percentage of stained cells and the secretion level (Figure 13 D, E and F), only a slight decrease in staining intensity was observed (Figure 13 G). This makes the short transmembrane domains a potential tool for fine-tuning of the product surface display.

When the B7-1 transmembrane domain was replaced by longer transmembrane domains containing not only hydrophobic residues, but also polar or charged residues (SEQ ID NO: 104-107), no effect was observed on the percentage of stained cells, nor on the staining intensity compared to the reference construct with the B7-1 transmembrane domain (see Figure 14 B, C, D, E,F and H). Nevertheless the secretion was slightly better with all these transmembrane domains than with B7-1 (see Figure 14 G).

In order to assess the impact of the transmembrane domain length in a more defined way without switching from one transmembrane domain to another, constructs with modified B7-1 transmembrane domains were designed. Several hydrophobic amino acids were removed from or added to the hydrophobic center of the B7-1 transmembrane domain (SEQ ID NO: 108-111). Shortening B7-1 to 18 amino acids (SEQ ID NO: 108) was beneficial in terms of percentage of stained cells, but slightly detrimental in term of staining intensity (see Figure 15 B, F and H). The three other constructs (20 (SEQ ID NO: 109), 24 (SEQ ID NO: 110) and 26 (SEQ ID NO: 111) amino acids, respectively) did not significantly influence the percentage of stained cells or the staining intensity (see Figure 14 C, D, E, F and H). Regarding the secretion level, shortening the length of the transmembrane domain was found beneficial, whereas constructs with elongated transmembrane domain showed a comparable secretion level as constructs with the native B7-1 (see figure 14G).

In general, a shorter transmembrane domain seems to be favorable in terms of percentage of stained cells and in terms of secretion of antibody, although the staining intensity might be slightly decreased compared to longer transmembrane domains.

*Cytosolic tail*

Literature suggests the presence of a structural, non-linear ER exportation signal in the B7-1 cytosolic tail (Lin et al., 2013). The authors found that the presence of an ER exportation signal in the cytosolic domain of a transmembrane protein targeted to the cell surface might have a positive impact on the amount of protein on the cell surface. In the case of a protein with a high turn-over, the accelerated export from the ER to the membrane may compensate for proteolytic cleavage from the plasma membrane.

In order to assess whether the ER exportation signal is relevant for the membrane display in our system, the B7-1 cytosolic tail was replaced by the cytosolic tails of several transmembrane proteins known to contain an ER exportation signal and deletion mutants without the ER exportation signal (SEQ ID NO: 112-124).

As shown in Figure 16 P, the absence of the ER exportation signal was found slightly favorable for the level of secreted antibody, while the percentage of stained cells (Figre 16 B-N and O) and the staining intensity (Figure 16 B-N and Q) were not significantly affected. Hence, our membrane displayed antibody might not be subject to a rapid turnover.

In addition to these constructs, the exchange of the M1M2 cytosolic tail by the B7-1 cytosolic tail in M1M2 transmembrane domain was performed (SEQ ID NO: 125). As shown in Figure 17 E, this exchange increased slightly the intensity of the fluorescence, but had no impact on the percentage of stained cells (Figure 17 C) nor on the secretion level (Figure 17 D).

Summary

Taken together, the data suggest that the length or the amino acid composition of transmembrane domains in our alternate splicing system had only a minor impact on the secretion level of antibodies and the product display on the cell membrane. Minor shifts in the ratio of secreted to membrane-displayed product were observed that might be used for fine-tuning of the alternate splicing system. All of the constructs allowed a significant surface display of the antibody and also a relevant secretion level.

Interestingly, a major impact of the transmembrane domain was seen on the fluorescence level of the cell surface display. Presence or absence of hydrophobic amino acids allowed to control the mean fluorescence, while it did not impact the secretion level or the percentage of cells showing cell surface display.

The ER exportation signal in the cytoplasmic tail was found to be not necessary for the cell surface display of the control antibody. A minimal cytoplasmic tail consisting of a glycine-alanine linker and 6 histidines or the first 5 amino acids of the B7-1 tail were sufficient for cell surface display. This might be different for other antibodies or proteins subject to a rapid turnover. Thus the cytosolic tail might be an interesting tool for adjusting the surface display of the protein of interest.

Taken together, the data from examples 2-4 suggests that surface display level and secretion level are influenced by the different sequences involved in the alternate splicing and the membrane integration of the membrane-bound protein. Whereas specific modification of the splice donor site allowed recovering the same secretion level than the control non-alternate splicing construct (see Example 3 Figure 9), the surface staining was significantly decreased when using B7-1 transmembrane region. On the other hand, adding a SV40 poly(A) in the intron when using M1M2 transmembrane region increased both secretion and surface display levels (see Example 3 Figures 6 and 7). Moreover, modifications of the B7-1 transmembrane domains as B7-1 reduced to 18 or 20 amino acids (see Figure 15) had a positive impact on both the secretion level and the surface display level. Finally, replacing the B7-1 cytosolic tail by a cytosolic tail containing no ER export signal as 6His or KCFCK also improved both surface display and secretion levels. Finally, the impact of Gastrin terminator at the end of the expression cassette on expression was positive, and might be even more favorable if placed in the intron. Therefore, constructs combining these different features might be even more favorable, allowing the best secretion level coupled with a high dynamic range of cell surface display wished for cell selection.

The backbone of these constructs will be the pGLEX41 (GSC281, SEQ ID NO: 304) and the sequences of the last 5 amino acids of the non-spliced ORF to the end of the expression construct are listed as DNA sequences as well as the protein sequence starting from the same amino acids with the fused transmembrane region as protein sequences in **Fehler! Ungültiger Eigenverweis auf Textmarke..**

**Table 17: Sequences of the potential constructs (*)**

| Construct name | DNA sequence SEQ ID NO: | Protein sequence SEQ ID NO: |
|---|---|---|
| pGLEX41_HC-GGC-I4(polyA)-B7-B7(18)-6His | 331 | 332 |
| pGLEX41_HC-GGC-I4(polyA)-B7-B7(20)-6His | 335 | 336 |
| pGLEX41_HC-GGC-I4(polyA)-B7-B7(18)-KCFCK | 333 | 334 |
| pGLEX41_HC-GGC-I4(polyA)-B7-B7(20)-KCFCK | 337 | 338 |
| pGLEX41_HC-GGC-I4(SV40ter)-B7-B7(18)-6His | 329 | 330 |
| pGLEX41_HC-GGC-I4(SV40ter)-B7-B7(20)-6His | 341 | 342 |
| pGLEX41_HC-GGC-I4(SV40ter)-B7-B7 (18)-KCFCK | 339 | 340 |
| pGLEX41_HC-GGC-I4(SV40ter)-B7-B7 (20)-KCFCK | 343 | 344 |
| *Sequence listed stretches from the last 5 amino acids of the non-spliced ORF to the end of the expression construct | | |

**Publications:**

Lin, Y., Chen, B., Wei-Cheng Lu, W., Chien-I Su, C., Prijovich,Z., Chung, W., Wu, P., Chen, K., Lee, I., Juan, T and Roffler, S. (2013). The B7-1 Cytoplasmic Tail Enhances Intracellular Transport and Mammalian Cell Surface Display of Chimeric Proteins in the Absence of a Linear ER Export Motif PLoS One. Sep 20;8(9)

Baeza-Delgado, C1, Marti-Renom, MA, Mingarro, I. (2012). Structure-based statistical analysis of transmembrane helices. Eur Biophys J. 2013 Mar;42(2-3):199-207

White, SH and Wimley, WC. (1999). Membrane protein folding and stability: physical principles. Annu Rev Biophys Biomol Struct. 28:319-65.

**Example 5: Qualitative prediction of product secretion using alternate splicing for surface display**

Introduction

Transfection of cells is the initial step for the establishment of a stable cell line for recombinant protein expression. For many reasons (heterogeneous initial cell population, different integration loci of plasmids in the genome, different post-translational machinery, and epigenetic regulation of expression) this step generally gives rise to heterogeneous cell populations with regard to expression and secretion level but also product quality attributes such as protein folding, glycosylation and other post-translational modification. Bispecific antibodies are composed by up to four different subunits (2 heavy chains and 2 light chains) that might assemble in all possible combinations. Depending on plasmid integration, regulation of transcription and translation as well as the folding efficacy in the ER, a particular clone may secrete preferably the correctly assembled product instead of unwanted by-products. The selection of a clone with this desired secretion pattern requires extensive screening and characterization of secreted proteins. Reducing the amount of screening needed in order to obtain a cell line producing the product of interest and minimal by-products, would be a major development.

It was demonstrated previously in Examples 2 to 4 that a fraction of secreted protein can be deviated from the secretory pathway for cell membrane display using alternate splicing technology. It was also shown that specific detection of proteins displayed on the cell membrane is possible via flow cytometry.

The aim of this example was to demonstrate that the product pattern display on the cell membrane is predictive of the secretion profile of a clone. For this purpose, the heterotrimeric bispecific BEAT® antibody presented in Example 2 was used as a model. The molecule binds to two different soluble molecules called "Target1" and "Target2" in the following. A schematic cartoon of the molecule can be seen in Figure 18, structure A. It will be abbreviated "BEAT" in the following text. After transfection not only BEAT molecules are secreted but also the monospecific heavy chain-light chain dimer binding to "Target 1". This molecule is referred to as Fab homodimer, (abbreviated "Fab-DIM"). A second by-product is the monospecific scFv-Fc homodimer (abbreviated "scFv-Fc-DIM") binding to "Target2". These by-products can be seen as structures B and C in Figure 18.

After stable transfection of cells with the expression vectorspGLEX41_BEAT-HC-I4-M1M2-M1M2-M1M2 (SEQ ID NO: 50), pGLEX41_scFv-Fc-I4-M1M2-M1M2-M1M2 (SEQ ID NO: 52) and pGLEX41_BEAT-LC (SEQ ID NO: 302) or with pGLEX41_BEAT-LC (SEQ ID NO: 302), pGLEX41_scFv-Fc-I4-B7-B7-B7 (SEQ ID NO: 323) and pGLEX41_BEAT-HC-I4-B7-B7-B7 (SEQ ID NO: 321), clones were screened by surface staining. Two approaches were followed for characterizing the surface phenotype of the cells. First a dual surface staining of the light chain and the Fc fragments was performed. While this staining did not all allow the detection of all produced species (no distinction between BEAT and Fab-DIM), it detected the monospecific contaminant scFv-Fc-DIM. This method is universally applicable for all kind of BEAT molecules. The second approach was more specific and required the soluble targets for the detection of the respective binding arms. In this case all three possible products were unequivocally detected by surface staining.

Recombinant pools and clones were generated by limiting dilution and flow cytometric cell sorting, respectively. Batch or fed batch cultivations were performed and the product accumulated in the supernatants was analysed by capillary electrophoresis. Eventually the secretion profile (BEAT, Fab-DIM or scFv-Fc-DIM fraction) measured in the supernatant was compared to the product profile displayed on the cell membrane of the recombinant cells.

Material and Methods

*Establishment of stable pools*

Stable transfected CHO cells were generated by co-transfection of the previously described vectors pGLEX41-BEAT-HC-14-M1M2-M1M2-M1M2 (abbreviated as "pHC-M1M2"; SEQ ID NO: 50), pGLEX41_scFv-Fc-I4- M1M2-M1M2-M1M2 (abbreviated as "pscFv-Fc-M1M2";SEQ ID NO: 52) and pGLEX41_BEAT-LC (abbreviated as "pLC"; SEQ ID NO:302) for the expression of the bispecific heterodimer BEAT together with two proprietary vectors for the expression of the protein providing resistance against the antibiotics puromycin and geneticin, respectively.

Suspension CHO-S cells were transfected with linearized vectors using polyethyleneimine (JetPEI®, Polyplus-transfection, Illkirch, France) in 50 ml bioreactor format. For this purpose, exponential growing cells were seeded at a density of 2 E6 cells /mL in 5 mL of OptiMEM medium (#31985-047, Invitrogen). A JetPEI®:DNA complex was added to the cells in a weight to weight ratio of 3 (μg/μg). Final DNA concentration of 2.5 μg/mL was added to the cells. After 5 hours incubation of the cells with the JetPEI®:DNA complex at 37°C under shaking (200 rpm), 5 mL of culture medium PowerCHO2 (# BE12-771Q, Lonza) were added to the cell suspension. The cells were then incubated on a shaken platform at 37°C, 5% $CO_2$ and 80% humidity. One day after transfection the cells were seeded in 96 well plates at different concentration (at 0.7, 0.5 and 0.4 E5 cells/mL) in a selective medium containing puromycin (# P8833-25mg, Sigma) and geneticin (# 11811-098, Gibco) at a concentration allowing the growth of stable pools. After 14 days of selection under static conditions 15 producing stable pools were picked from the wells and expanded into TubeSpin bioreactors.

*Establishment of recombinant clones*

Clones were generated by co-transfection of the previously described vectors pGLEX41_BEAT-HC-I4-B7-B7-B7 (abbreviated as "pHC-B7"; SEQ ID NO: 321), pGLEX41_scFv-Fc-I4-B7-B7-B7 (abbreviated as "pscFv-Fc-B7"; SEQ ID NO: 323) and pGLEX41_BEAT-LC (abbreviated as "pLC"; SEQ ID NO: 302) for the expression of the bispecific heterodimer BEAT together with two proprietary vectors for the expression of the protein providing resistance against the antibiotics puromycin and geneticin, respectively. Transfection and selection of stable transfectants were performed as previously described. After 14 days of selection under static conditions all stable transfectants were collected, pooled and passaged under dynamic conditions (orbital shaking) for a week. Single cell sorting was performed using a FACSAria II by gating on living cells excluding cell doublets by pulse processing.

*LC and Fc staining of the product displayed on the cell surface*

Dual surface staining of the light chain (LC) and the Fc fragment on the stable cells was performed on day 2 after batch seeding as described in detail in Example 2. In short, a total of 1 E5 cells were harvested and transferred to a round bottom well of a 96 well plate. The cells were washed twice with washing buffer (PBS containing 2% FBS) and then resuspended in 100 μL of detection antibody. The specific detection of the kappa light chain was performed using a mouse anti-human kappa LC APC labelled antibody (#561323, BD Pharmingen), Fc fragments were detected using PE conjugated goat anti-human Fc gamma specific antibody (#12-4998-82, eBioscience). After 20 min incubation in the dark at room temperature, the cells were washed once in washing buffer and resuspended in 200 μL for flow cytometric analysis. The cells were analysed with a Guava flow cytometer (Merck Millipore).

*Staining of the specific binders displayed on the cell surface*

For this staining the soluble targets were used for the detection of the binding arms of the molecule displayed on the cell surface. In short, a total of 1 E5 cells were harvested and transferred to a round bottom well of a 96 well plate. The cells were washed twice with washing buffer (PBS containing 2% FBS) and then resuspended in 100 μL of a mix of biotinylated Target 1 and His-tagged Target2. After 20 min incubation in the dark at room temperature, the cells were washed twice with washing buffer and resuspended in 100 μL of a mix containing Streptavidin conjugated with APC (#554067, BD Pharmingen) and an anti His-Tag antibody labelled with PE (#IC050P, RD Systems). After 20 min incubation in the dark at room temperature, the cells were washed once in washing buffer and resuspended in 200 μL for flow cytometric analysis with a Guava flow cytometer (Merck Millipore).

*Analysis of the cell secretion profile*

Recombinant pools were seeded in TubeSpins at a cell density of 0.5 E6 cells/mL in supplemented growth medium (PowerCHO2, 2 mM L-Glutamine, 8 mM Glutamax (Life Technologies, Carlsbad, CA), 15% Efficient Feed A (Life Technologies), 15% Efficient Feed B (Life Technologies). Clones were seeded in TubeSpins at a cell density of 0.5 E6 cells/mL in growth medium (PowerCHO2, 2 mM L-Glutamine) and feeds of ActiCHO Feed A & B feed, (#U050-078 , PAA Laboratories GmbH) were performed on a daily basis. On day 14 of the batch or fed-batches culture supernatants were harvested by centrifugation and filtered using a 0.2 μm syringe filter (#99722. TPP). The crude supernatants were either directly analysed or purified using Streamline Protein A beads (# 17-1281-02, GE). The proteins were analysed using the Caliper LabChip GXII protein assay. The different species were identified according to their molecular weight and the fraction of each product was determined.

Results

A total of 15 stable pools were obtained following transfection with pHC-M1M2, pScFv-Fc-M1M2 and pLC constructs. Supplemented batches were started in order to determine the secretion profile of these pools. Dual surface staining of the light chain and the Fc fragments (scFv-Fc and the heavy chain) on the pools was performed on day 2 after batch seeding. Figure 19 gives an example of the surface profile of one of the pools measured by dual staining on day 2 of a supplemented batch. The dot plot presents the intensity for kappa LC staining (y-axis) versus the intensity of human Fc staining (x-axis). The quadrant fixes arbitrary limits between negatively and positively stained cells. Quadrant Q8 represents the fraction of cells expressing no protein on the cell membrane, e.g. a sub population of non-producers. Q7 is the fraction of positive cells for the Fc fragment but negative for kappa LC thus corresponding to cells exhibiting scFv-Fc-DIM on the cell membrane. Dual positive cells, in Q6, are the cells displaying both Lc and Fc fragments on the cell membrane, e.g. BEAT or Fab-DIM molecules.

The analysis indicated a heterogeneous cell population of 33.8% of non-producers (Q8), 50.3% of scFv-Fc-DIM secretors (Q7) and 15.8% of potential BEAT or Fab-DIM secretors (Q6). In order to be able to calculate a correlation between the surface staining and the secreted product, only the producing population was taken into account. Hence the fraction of BEAT and Fab-DIM producers as well as the fraction of scFv-Fc-DIM producers within the producing cell fraction (Q6 + Q7) was re-calculated excluding the non-producer fraction Q8 (this fractions will be referred to as Q6* and Q7*). In the analysis shown in Figure 17 for example the populations were calculated as follows:

$$Q6^* = Q6/\ (Q6+Q7) = 23.9\% \text{ and } Q7^* = Q7/\ (Q6+Q7) = 76.1\%.$$

This analysis was performed for all 15 producing stable pools and used for the correlation shown in Figure 20.

The fraction of BEAT, Fab-DIM and scFv-Fc-DIM producers could be identified by surface staining according to the species display on the cell membrane by alternate splicing. The results were then confronted with the actual secretion profile of the analysed pools. For this purpose, the supernatants of the 15 producing stable pools were purified on day 14 by Protein A and each secreted molecule was identified according to the molecular weight and quantified by Caliper LabChip GXII protein assay. It should be noted that under these purification conditions only the Protein A binding species, namely BEAT and scFv-Fc-DIM molecules, are purified from the supernatant as Fab-DIM molecules lack the Protein A binding site. Figure 20 shows the relationship between the percentage of secreted molecules and the corresponding cell fraction identified by surface staining.

The data indicate that surface display and secretion profile are highly correlated ($R^2$ = 0.9) for both BEAT and scFv-Fc-DIM secretion. The nature of the relationship is however not linear. The lack of linear regression may be explained by the fact that the surface staining performed in this experiment does not distinguish between BEAT and Fab-DIM, whereas the purified secreted products excludes the Fab-DIM fraction. The percentage of secreted BEAT and scFv-Fc-DIM in the supernatant may be slightly overestimated. Nevertheless, the experiment clearly demonstrated that the higher the fraction of positive cells for the surface display of a particular molecule, the higher is the fraction of this molecule in the supernatant. In this example, the fraction of secreted BEAT molecule increased linearly when the tested stable pools harboured a percentage of positive cells higher than 20%. The selection of a stable pool harbouring more than 75% of dual positive cells (Q6) would allow the selection of a secreting cell population yielding more than 90% of the desired BEAT molecule. For a clonal population harbouring a dual positive phenotype, e.g. 100% of the cell population positive for both LC and Fc surface staining, it is reasonable to expect an even higher fraction of BEAT secretion, approaching approximately 100%.

In order to increase the sensitivity of the method and demonstrate a linear relationship between surface display and the secretion pattern, a different detection method was applied involving the soluble target as detection reagents. This way all 3 possible products are unequivocally identified by their binding patterns. Figure 21 gives an example of the surface profile of a pool measured prior cell sorting. The dot plot presents the cell surface density of the binder for Target1 (y-axis) versus the cell surface density of the binder for Target2 (x-axis). The quadrant fixes arbitrary limits between negatively and positively stained cells. Q1 is the fraction of positive cells for Target1 binding but negative for Target2 binding thus corresponding to cells exhibiting Fab-DIM on the cell membrane. Dual positive cells, in Q2, are the cells displaying both binders for Target1 and 2 on the cell membrane and thus correspond to cells displaying the BEAT on the membrane. Q3 are the cells displaying solely Target2 binders thus corresponding to scFv-Fc-DIM. Quadrant Q4 represents the fraction of cells expressing no protein on the cell membrane, e.g. a sub population of non-producers. As these cells did not contribute to the secretion of antibodies they were excluded from the analysis.

All producing clones (28) generated by flow cytometric cell sorting after transfection with pHC-B7, pScFv-Fc-B7 and pLC constructs were analysed using this approach. As previously described the fraction of BEAT, Fab-DIM and scFv-Fc-DIM producers could be identified by surface staining according to the species display on the cell membrane by alternate splicing. The actual secretion profiles of each clone were determined on day 14 of a fed batch directly in the supernatant by Caliper LabChip GXII protein assay.

Figure 22 shows the correlation between the fraction of BEAT molecule detected in the supernatants and the fraction of cells displaying a BEAT phenotype on their cell surface (% of Q2). Using this specific staining a good linear correlation ($R^2$ = 0.8) was obtained. The data clearly indicated that the aptitude of clone to secrete the BEAT molecule could be predicted by the product pattern detected on the cell membrane.

Conclusion

In this example it was demonstrated that alternate splicing technology combined with cell membrane display is an effective reporter system for the qualitative prediction of the secretion profile of single cells. A clear correlation was found between the cell membrane display pattern and the actual secretion profile of transfected cells using either a general detection method based on LC and Fc surface detection or using a product specific staining method involving the soluble targets of the molecule,. The approach does not require the time consuming screening of cell performances in batch or fed batch cultures, nor the harvest, purification and extensive characterization of secreted product.

In conclusion, the reporter system described here provides a reliable, high throughput screening tool for cell clones harbouring a particular secretion profile for heterotrimeric molecules.

In addition to the qualitative prediction of secretion profile, the quantitative prediction of the secretion level of particular cell clone is of high interest for cell line development purposes. In the following example it was investigated whether the level of membrane bound, surface displayed product using an alternate splicing approach correlated with the secretion level of a clone.

**Example 6: Quantitative prediction of secretion level using alternate splicing for surface display**

Introduction

A major challenge of the cell line development process is the selection in a time effective manner of high performing clones, for example, a high secretion rate of good quality recombinant protein. It was demonstrated previously that the display on the cell membrane of a fraction of an expressed protein via alternate splicing indicates the actual qualitative secretion profile of a clone. In this example it will be demonstrated that the level of cell membrane display correlates quantitatively with the secretion level of a clone and that high producing cells can be selected on the basis of the intensity of the surface display.

Material and Methods

*Stable cell line development*

Stable transfected CHO cells were generated by co-transfection of the vectors pGLEX41_HC-I4-M1M2-M1M2-M1M2 (SEQ ID NO: 36) and pGLEX41_LC (SEQ ID NO: 294) for the expression of a humanized IgG1 antibody, together with two propriety vectors for the expression of the protein providing resistance against the antibiotics puromycin and geneticin, respectively.

Suspension CHO-S cells were transfected with linearized vectors using polyethyleneimine (JetPEI®, Polyplus-transfection, Illkirch, France) in 50 ml bioreactor format. For this purpose, exponential growing cells were seeded at a density of 2 E6 cells /mL in 5 mL of OptiMEM medium (#31985-047, Invitrogen). A JetPEI®:DNA complex was added to the cells in a weight to weight ratio of 3 (μg/μg). A final DNA concentration of 2.5 μg/mL was added to the cells. After 5 hours incubation of the cells with the JetPEI®:DNA complex at 37°C under shaking (200 rpm), 5 mL of culture medium PowerCHO2 (# BE12-771Q, Lonza) was added to the cell suspension. The cells were then incubated on a shaken platform at 37°C, 5 % $CO_2$ and 80% humidity. One day after transfection the cells were seeded in 96 well plates at different concentration (at 0.7, 0.5 and 0.4 E5 cells/mL) in a selective medium containing 4 μg/mL puromycin (# P8833-25mg, Sigma) and 400 μg/mL geneticin (# 11811-098, Gibco). After 14 days of selection under static conditions 15 producing stable pools were picked from the wells and expanded into TubeSpin bioreactors for the assessment of the expression level.

*Assessment of the expression level*

Supplemented batches were seeded at a cell density of 0.5E6cells/mL in PowerCHO2 (# BE12-771Q, Lonza) supplemented with 2 mM L-Glutamine, 8 mM Glutamax, 15% Efficient Feed A (#A1023401, Invitrogen) and 15% Efficient FeedB (#A1024001, Invitrogen). Viable cell count (VCC) and viability were monitored on day 1, 3 and 7 using the ViaCount assay of the Guava flow cytometer. IgG titers were measured on day 1, 3 and 7 using the octet QK instrument. The specific productivity qP of the stable pools was calculated between day 1 and day 3 according to the following formula

$$qP = \frac{\Delta \mathrm{IgG}_{d3-d1}}{\Delta t_{d3-d1}} \times \frac{1}{\mathrm{VCC}_{\mathrm{mean}\ d1-d3}}$$

With:

qP = specific secretion rate or productivity [pg/cell/day]
IgG = IgG titers in [pg/mL] on day 3 and day 1
t = cultivation time [day]
$VCC_{mean\ d1-d3}$ = mean viable cell count between day 1 and day 3 [cells/mL]

Quantification of the displayed IgGs on the cell membrane was performed on day 1 during the exponential growth phase as described in the following.

*Staining of the cells*

Staining of the Fc fragments on the stable pools was performed on day 1 after batch seeding. In short, a total of 1 E5 cells were harvested and transferred to a round bottom well of a 96 well plate. The cells were washed twice with the washing buffer (PBS containing 2% FBS) and resuspended in 100 μL of detection antibody. The specific detection of the Fc fragments was performed using a PE conjugated goat anti-human Fc gamma specific antibody (#12-4998-82, eBioscience). After 20 min incubation in the dark at room temperature, the cells were washed once in washing buffer and resuspended in 200 μL for flow cytometric analysis. The cells were analysed with a Guava flow cytometer.

*Flow cytometric cell sorting*

Clones were generated by flow cytometric cell sorting form a heterogeneous pool of stable transfectants established as previously described (see section "Stable cell line development"). For this purpose a surface staining of the Fc fragment was performed according to the protocol described earlier. The sort was performed using a FACSAria II by gating on living cells excluding cell doublets by pulse processing. Three gates were defined according to the surface fluorescence intensity ("low", "medium" and "high") and single cells were distributed into 96 well plates containing 200 μL of a cloning medium. After 2 weeks of growth under static condition the cells were expanded under dynamic conditions and the performances of the selected clones were evaluated as described previously.

Results

The analysis of the surface staining profile of a stable pool is illustrated in Figure 23. The surface fluorescence of living cells gated in the region g1 (Figure 23, profile A) was displayed as a histogram (Figure 23, plot B) and the mean fluorescence of the distribution (Mean [RFU]) was computed. The mean fluorescence is used as a measure of the IgG level expressed on the cell membrane. This analysis was performed for all generated stable pools and the level of surface IgG was compared to the actual level of secreted IgGs measured in the supernatant.

Figure 24 shows the correlation between the surface fluorescence intensity and the secretion level of all stable pools measured on day 1 of a supplemented batch. A good correlation ($R^2 > 0.8$) was observed between the surface IgG expression and the titers (Figure 24, plot A) as well as between surface IgG expression and the specific productivity (Figure 24, plot B). The data demonstrate that the surface expression of alternately spliced transmembrane IgG reported the actual secretion level of stable transfected cells. A similar good correlation ($R^2 >0.8$) was observed between the surface fluorescence of the pools on day 1 and the volumetric productivity in a 7 day batch process (see Figure 25; in this batch day 7 corresponds to the end of the stationary phase). The data indicate that the performance of the pools at the end of a batch process could be predicted early on according to the detected surface IgG expression. In a batch process, the physiological state of the cells is continuously changing according to the changing medium environment. The correlation between surface display of reporter IgG via alternate splicing and the actual secretion level in a batch process is therefore independent of the physiological state of the cells (exponential growing or stationary) and the batch process phases (growth or production).

To validate the hypothesis clones were generated by flow cytometric cell sorting according to the density of antibody displayed on the cell surface. For this purpose a stable pool was stained for surface IgG using an anti-human Fc PE labelled antibody. Three regions were defined according to the surface fluorescence of the cells ("low", "medium" and "high") and the single cells were cloned accordingly in 96 well plates. The gating strategy can be seen in Figure 26A. After approximately two weeks clones were expanded and the surface intensity of the cells was again determined to verify that indeed the sorted phenotype was obtained following cloning. The distribution of the surface intensity of all clones can be seen in Figure 26B. Overall the sorting was successful as the distribution of the surface IgG of the three different sorted groups corresponded to the expected phenotype "low", "medium" and "high".

Some outliers were detected for instance in the "low" group showing a high surface fluorescence, probably due to an artefact of staining prior sorting. The performances of the generated clones were subsequently assessed in supplemented batches. Figure 26C shows the distribution of the specific productivity qP for each group. The qP of the original pool was also assessed in quadruplicate and is given in the diagram as a control. The best performers were clearly identified within the "high" surface fluorescence group with a median qP of approx. 10 pg/cell/day. Compared to the original pool (approx. 5 pg/cell/day) the productivity was significantly improved by a 2-6-fold factor, the best clone reaching more than 30 pg/cell/day. In contrast the clones of the "low" and "medium" categories were overall low performers with a median qP close to 0 pg/cell/day. Some outliers were reaching > 20 pg/cell/day but these phenotypes were also showing high surface density of IgG detected by flow cytometry.

Conclusion

In this example it was demonstrated that the level of IgG expressed via alternate splicing on the cell membrane reports the actual secretion level of recombinant cells. The cell membrane fluorescence after the specific detection of the product correlates with the accumulated IgG concentration in the supernatant and the qP of transfected stable cells. Also it was demonstrated that the correlation was valid regardless of the phase of a batch production process. Taken together, these data indicated that the quantitative secretion level of recombinant cells can be predicted by the alternate splicing surface display reporter system described herein. This was verified by selecting clones according to the density of IgG displayed on the cell surface using flow cytometric cell sorting. Indeed, high producers could be selected using this approach and clones showing industrial relevant specific productivity could be generated in a time efficient manner.

SEQUENCE LISTING

<110> Glenmark Pharmaceuticals S.A.

<120> Expression constructs and methods for selecting host cells expressing polypeptides

<130> P3125PC00

<160> 347

<170> PatentIn version 3.5

<210> 1
<211> 20
<212> DNA
<213> Artificial Sequence

<220>
<223> Primer GlnPr269

<400> 1
caggggggcg gagcctatgg 20

<210> 2
<211> 43
<212> DNA
<213> Artificial Sequence

<220>
<223> Primer GlnPr497

<400> 2
gcccccctcc cgcgaggaga tgaccaagaa ccaggtgtcc ctg 43

<210> 3
<211> 34
<212> DNA
<213> Artificial Sequence

<220>
<223> Primer GlnPr498

<400> 3
tttctagatt catcacttgc cgggggacag gctc 34

<210> 4
<211> 31
<212> DNA
<213> Artificial Sequence

<220>
<223> Primer GlnPr501

<400> 4
tataaagctt ccaccatgga gagccctgcc c 31

<210> 5
<211> 33
<212> DNA
<213> Artificial Sequence

<220>
<223> Primer GlnPr502

<400> 5
tatatctaga ttcatcagca ctcgccccgg ttg 33

<210> 6
<211> 35
<212> DNA
<213> Artificial Sequence

<220>
<223> Primer GlnPr1437

<400> 6
ccaaatcgat ttatcagcac tcgccccggt tgaag 35

<210> 7
<211> 35
<212> DNA
<213> Artificial Sequence

<220>
<223> Primer GlnPr1500

<400> 7
ccgggatcga tttatcagtg atggtgatgg tgatg 35

<210> 8
<211> 32
<212> DNA
<213> Artificial Sequence

<220>
<223> Primer GlnPr1502

<400> 8
ggccgatcga tttatcactt gccaggagac ag 32

<210> 9
<211> 28
<212> DNA
<213> Artificial Sequence

<220>
<223> Primer GlnPrl516

<400> 9
gatgaaagct tggtaggtga tcctcctg 28

<210> 10
<211> 52
<212> DNA
<213> Artificial Sequence

<220>
<223> Primer GlnPr1517

<400> 10
atgtattcga atatctccac cggttgcagc tctgagccag ggaggaggga ag 52

<210> 11
<211> 28
<212> DNA
<213> Artificial Sequence

<220>
<223> Primer GlnPr1518

<400> 11
taagctagcc caccatggag agccctgc 28

<210> 12
<211> 49
<212> DNA
<213> Artificial Sequence

<220>
<223> Primer GlnPr1519

<400> 12
gaggctcttc tgcgtgtagt ggttgtgcaa ggcctcgtgc atcacgctg 49

<210> 13
<211> 51
<212> DNA
<213> Artificial Sequence

<220>
<223> Primer GlnPr1520

<400> 13
ctaccaagct ttcatcattt acccggagac agggagaggc tcttctgcgt g 51

<210> 14
<211> 92
<212> DNA
<213> Artificial Sequence

<220>
<223> Primer GlnPr1521

<400> 14

gggagctgga cgggctgtgg acgaccatca ccatcttcat cacactcttc ctgttaagcg 60

tgtgctacag tgccaccgtc accttcttca ag 92

<210> 15
<211> 92
<212> DNA
<213> Artificial Sequence

<220>
<223> Primer GlnPr1522

<400> 15

gtagtcgggg atgatggtct gcttcaggtc caccaccgag gagaagatcc acttcacctt 60

gaagaaggtg acggtggcac tgtagcacac gc 92

<210> 16
<211> 55
<212> DNA
<213> Artificial Sequence

<220>
<223> Primer GlnPr1523

<400> 16
atataccggt ggagagctgt gcggaggcgc aggacgggga gctggacggg ctgtg 55

<210> 17
<211> 59
<212> DNA
<213> Artificial Sequence

<220>

<223> Primer GlnPr1524

<400> 17
atatttcgaa tcactaggcc ccctgtccga tcatgttcct gtagtcgggg atgatggtc 59

<210> 18
<211> 41
<212> DNA
<213> Artificial Sequence

<220>
<223> Primer GlnPr1649

<400> 18
tctccaccgg ttgcagctct gttccctcct ccctcggtta g 41

<210> 19
<211> 33
<212> DNA
<213> Artificial Sequence

<220>
<223> Primer GlnPr1650

<400> 19
gcgccatcga tacagacatg ataagataca ttg 33

<210> 20
<211> 36
<212> DNA
<213> Artificial Sequence

<220>
<223> Primer GlnPr1651

<400> 20
gcgcgatcga tacttgttta ttgcagctta taatgg 36

<210> 21
<211> 54
<212> DNA
<213> Artificial Sequence

<220>
<223> Primer GlnPr1689

<400> 21
atatgctagc ccaccatgcg gagccctgcc cagctgctgt tcctgctgct gctg 54

<210> 22
<211> 61
<212> DNA
<213> Artificial Sequence

<220>
<223> Primer GlnPr1690

<400> 22

tgctgttcct gctgctgctg tggatccctg gcaccaacgc cgaggtgcag ctggtcgagt 60

c 61

<210> 23
<211> 54
<212> DNA
<213> Artificial Sequence

<220>
<223> Primer GlnPr1725

<400> 23
atatactagt ccaccatgcg gagccctgcc cagctgctgt tcctgctgct gctg 54

<210> 24
<211> 59
<212> DNA
<213> Artificial Sequence

<220>
<223> Primer GlnPr1726

<400> 24
ctgttcctgc tgctgctgtg gatccctggc accaacgccc aggtgcagct gcagcagtc 59

<210> 25
<211> 58
<212> DNA
<213> Artificial Sequence

<220>
<223> Primer GlnPr1727

<400> 25
ctgttcctgc tgctgctgtg gatccctggc accaacgcca tggagacaga cacactcc 58

<210> 26
<211> 70
<212> DNA
<213> Artificial Sequence

<220>
<223> Primer GlnPr1735

<400> 26

gacttcgaat catcaatgat ggtggtggtg atgggcgctg gcgaggagca ggtcgaacag 60

gagcagcttg 70

<210> 27
<211> 37
<212> DNA
<213> Artificial Sequence

<220>

<223> Primer GlnPr1760

<400> 27
ccgcatcgat ttatcattta cccgggctca ggctcag 37

<210> 28
<211> 71
<212> DNA
<213> Artificial Sequence

<220>
<223> Primer GlnPr1799

<400> 28

**atataccggt ggaggccctg tggctgggcg tgctgagact gctgctgttc aagctgctcc 60**

**tgttcgacct g 71**

<210> 29
<211> 60
<212> DNA
<213> Artificial Sequence

<220>
<223> Primer GlnPr1800

<400> 29
gtaagctttc atcatttacc cggagacagg gagaggctct tctgcgtgaa tcggttgtgc 60

<210> 30
<211> 60
<212> DNA
<213> Artificial Sequence

<220>
<223> Primer GlnPr1801

<400> 30
gcgcgaagct ttcatcattt acccggagac agggagaggc tcttctgcgt ataatgattg 60

<210> 31
<211> 35
<212> DNA
<213> Artificial Sequence

<220>
<223> Primer GlnPr1840

<400> 31
atataccggt ggaggccctg tggctgggcg tgctg 35

<210> 32
<211> 46
<212> DNA
<213> Artificial Sequence

<220>
<223> Primer GlnPr2099

<400> 32
tcctcggggg gagatccacc accacctgag ccagggagga gggaag 46

<210> 33
<211> 31
<212> DNA
<213> Artificial Sequence

<220>
<223> Primer GlnPr2100

<400> 33
gcgcttcgaa tcatcacaga aacacggtct g 31

<210> 34
<211> 40
<212> DNA
<213> Artificial Sequence

<220>
<223> Primer GlnPr2101

<400> 34
tggtggtgga tctccccccg aggacccccc tgactccaag 40

<210> 35
<211> 48
<212> DNA
<213> Artificial Sequence

<220>
<223> Primer GlnPr2102

<400> 35
tcctcggggg gagatccacc accacctgtt ccctcctccc tcggttag 48

<210> 36
<211> 1082
<212> DNA
<213> Artificial Sequence

<220>
<223> Construct GSC3899

<400> 36

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct cagagctgca accggtggag agctgtgcgg 600
aggcgcagga cggggagctg gacgggctgt ggacgaccat caccatcttc atcacactct 660
tcctgttaag cgtgtgctac agtgccaccg tcaccttctt caaggtgaag tggatcttct 720
cctcggtggt ggacctgaag cagaccatca tccccgacta caggaacatg atcggacagg 780
gggcctagtg attcgaaatg accgaccaag cgacgcccaa cctgccatca cgagatttcg 840
attccaccgc cgccttctat gaaaggttgg gcttcggaat cgttttccgg gacgccggct 900
ggatgatcct ccagcgcggg gatctcatgc tggagttctt cgcccacccc aacttgttta 960
ttgcagctta taatggttac aaataaagca atagcatcac aaatttcaca aataaagcat 1020
ttttttcact gcattctagt tgtggtttgt ccaaactcat caatgtatct tatcatgtct 1080
gt 1082

<210> 37
<211> 1064
<212> DNA
<213> Artificial Sequence

<220>
<223> Construct GSC4398

<400> 37

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120

```
cattgggacc atgttctgaa tgcctccacc tcaaccaccg gccggtagga ccaaagccac      180
cccgtgtttt ctcaggatct cttttcccag ggagatccct cggcccaaag agggagatgg      240
caatgctgga tgtgtgcaca ataattcaac aggcattgga acttcagcat cgatgctgaa      300
tgcaattaac aatgctcaag cagaaccccc ggctccatca gcacagtgca ggaccaaacc      360
ccatgctgca gcagtggggc tgtctgtacg gggtgggcaa tgggaaccgg ggtctgctgg      420
ggctcctgct gcttcagtgc tgccatgcag ccacacatcc tgagagctga aagggtcggc      480
gtcctcacct ggtgcacacc gtagctctgc cccacagctt taaggcacct ggctaaccga      540
gggaggaggg aacagagctg caaccggtgg agagctgtgc ggaggcgcag gacggggagc      600
tggacgggct gtggacgacc atcaccatct tcatcacact cttcctgtta agcgtgtgct      660
acagtgccac cgtcaccttc ttcaaggtga agtggatctt ctcctcggtg gtggacctga      720
agcagaccat catccccgac tacaggaaca tgatcggaca gggggcctag tgattcgaaa      780
tgaccgacca agcgacgccc aacctgccat cacgagattt cgattccacc gccgccttct      840
atgaaaggtt gggcttcgga atcgttttcc gggacgccgg ctggatgatc ctccagcgcg      900
gggatctcat gctggagttc ttcgcccacc ccaacttgtt tattgcagct tataatggtt      960
acaaataaag caatagcatc acaaatttca caaataaagc atttttttca ctgcattcta     1020
gttgtggttt gtccaaactc atcaatgtat cttatcatgt ctgt                      1064
```

<210> 38
<211> 1219
<212> DNA
<213> Artificial Sequence

<220>
<223> Construct GSC4401

<400> 38

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatacttgt 300
ttattgcagc ttataatggt tacaaataaa gcaatagcat cacaaatttc acaaataaag 360
catttttttc actgcattct agttgtggtt tgtccaaact catcaatgta tcttatcatg 420
tctgtatcga tgctgaatgc aattaacaat gctcaagcag aacccccggc tccatcagca 480
cagtgcagga ccaaacccca tgctgcagca gtggggctgt ctgtacgggg tgggcaatgg 540
gaaccggggt ctgctggggc tcctgctgct tcagtgctgc catgcagcca cacatcctga 600
gagctgaaag ggtcggcgtc ctcacctggt gcacaccgta gctctgcccc acagctttaa 660
ggcacctggc taacctctgc gcttcttccc ttccctcctc cctggctcag agctgcaacc 720
ggtggagagc tgtgcggagg cgcaggacgg ggagctggac gggctgtgga cgaccatcac 780
catcttcatc acactcttcc tgttaagcgt gtgctacagt gccaccgtca ccttcttcaa 840
ggtgaagtgg atcttctcct cggtggtgga cctgaagcag accatcatcc ccgactacag 900
gaacatgatc ggacaggggg cctagtgatt cgaaatgacc gaccaagcga cgcccaacct 960
gccatcacga gatttcgatt ccaccgccgc cttctatgaa aggttgggct tcggaatcgt 1020
tttccgggac gccggctgga tgatcctcca gcgcggggat ctcatgctgg agttcttcgc 1080
ccaccccaac ttgtttattg cagcttataa tggttacaaa taaagcaata gcatcacaaa 1140
tttcacaaat aaagcatttt tttcactgca ttctagttgt ggtttgtcca aactcatcaa 1200
tgtatcttat catgtctgt 1219

<210> 39
<211> 1263
<212> DNA
<213> Artificial Sequence

<220>
<223> Construct GSC4402

<400> 39

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct cagagctgca accggtggag agctgtgcgg 600
aggcgcagga cggggagctg gacgggctgt ggacgaccat caccatcttc atcacactct 660
tcctgttaag cgtgtgctac agtgccaccg tcaccttctt caaggtgaag tggatcttct 720
cctcggtggt ggacctgaag cagaccatca tccccgacta caggaacatg atcggacagg 780
gggcctagtg attcgaaatg accgaccaag cgacgcccaa cctgccatca cgagatttcg 840
attccaccgc cgccttctat gaaaggttgg gcttcggaat cgttttccgg gacgccggct 900
ggatgatcct ccagcgcggg gatctcatgc tggagttctt cgcccacccc aacttgttta 960

ttgcagctta taatggttac aaataaagca atagcatcac aaatttcaca aataaagcat 1020
tttttcact gcattctagt tgtggtttgt ccaaactcat caatgtatct tatcatgtct 1080
gtataccgtc gacctctagc tagagcttgg cgtaatcatg gtcatagctg tttcctgtgt 1140
gaaattgtta tccgctcaca attccacaca acatacgagc cggggcagga taatatatgg 1200
tagggttcat agccagagta acctttttt ttaattttta ttttatttta tttttgagat 1260
cag 1263

<210> 40
<211> 977
<212> DNA
<213> Artificial Sequence

<220>
<223> Construct GSC5854

<400> 40

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct cagagctgca accggtggag gccctgtggc     600
tgggcgtgct gagactgctg ctgttcaagc tgctcctgtt cgacctgctc ctcgccagcg     660
cccatcacca ccaccatcat tgatgattcg aaatgaccga ccaagcgacg cccaacctgc     720
catcacgaga tttcgattcc accgccgcct tctatgaaag gttgggcttc ggaatcgttt     780
tccgggacgc cggctggatg atcctccagc gcggggatct catgctggag ttcttcgccc     840
accccaactt gtttattgca gcttataatg gttacaaata aagcaatagc atcacaaatt     900
tcacaaataa agcatttttt tcactgcatt ctagttgtgg tttgtccaaa ctcatcaatg     960
tatcttatca tgtctgt                                                    977
```

<210> 41
<211> 959
<212> DNA
<213> Artificial Sequence

<220>
<223> Construct GSC5855

<400> 41

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaaccga     540
gggaggaggg aacagagctg caaccggtgg aggccctgtg gctgggcgtg ctgagactgc     600
tgctgttcaa gctgctcctg ttcgacctgc tcctcgccag cgcccatcac caccaccatc     660
attgatgatt cgaaatgacc gaccaagcga cgcccaacct gccatcacga gatttcgatt     720
ccaccgccgc cttctatgaa aggttgggct tcggaatcgt tttccgggac gccggctgga     780
tgatcctcca gcgcggggat ctcatgctgg agttcttcgc ccaccccaac ttgtttattg     840
cagcttataa tggttacaaa taaagcaata gcatcacaaa tttcacaaat aaagcatttt     900
tttcactgca ttctagttgt ggtttgtcca aactcatcaa tgtatcttat catgtctgt      959
```

<210> 42
<211> 1114
<212> DNA
<213> Artificial Sequence

<220>
<223> Construct GSC5857

<400> 42

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatacttgt     300
ttattgcagc ttataatggt tacaaataaa gcaatagcat cacaaatttc acaaataaag     360
catttttttc actgcattct agttgtggtt tgtccaaact catcaatgta tcttatcatg     420
tctgtatcga tgctgaatgc aattaacaat gctcaagcag aacccccggc tccatcagca     480
cagtgcagga ccaaacccca tgctgcagca gtggggctgt ctgtacgggg tgggcaatgg     540
```

```
gaaccggggt ctgctggggc tcctgctgct tcagtgctgc catgcagcca cacatcctga      600
gagctgaaag ggtcggcgtc ctcacctggt gcacaccgta gctctgcccc acagctttaa      660
ggcacctggc taacctctgc gcttcttccc ttccctcctc cctggctcag agctgcaacc      720
ggtggaggcc ctgtggctgg gcgtgctgag actgctgctg ttcaagctgc tcctgttcga      780
cctgctcctc gccagcgccc atcaccacca ccatcattga tgattcgaaa tgaccgacca      840
agcgacgccc aacctgccat cacgagattt cgattccacc gccgccttct atgaaaggtt      900
gggcttcgga atcgttttcc gggacgccgg ctggatgatc ctccagcgcg gggatctcat      960
gctggagttc ttcgcccacc ccaacttgtt tattgcagct tataatggtt acaaataaag     1020
caatagcatc acaaatttca caaataaagc atttttttca ctgcattcta gttgtggttt     1080
gtccaaactc atcaatgtat cttatcatgt ctgt                                 1114
```

<210> 43
<211> 1158
<212> DNA
<213> Artificial Sequence

<220>
<223> Construct GSC5856

<400> 43

ctgtctccgg gtaaatgatg aaagcttggt aggtgatcct cctgctgctt tggttcaggg 60
ttttgcttga ggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct cagagctgca accggtggag gccctgtggc 600
tgggcgtgct gagactgctg ctgttcaagc tgctcctgtt cgacctgctc ctcgccagcg 660
cccatcacca ccaccatcat tgatgattcg aaatgaccga ccaagcgacg cccaacctgc 720
catcacgaga tttcgattcc accgccgcct tctatgaaag gttgggcttc ggaatcgttt 780
tccgggacgc cggctggatg atcctccagc gcggggatct catgctggag ttcttcgccc 840
accccaactt gtttattgca gcttataatg gttacaaata aagcaatagc atcacaaatt 900
tcacaaataa agcatttttt tcactgcatt ctagttgtgg tttgtccaaa ctcatcaatg 960
tatcttatca tgtctgtata ccgtcgacct ctagctagag cttggcgtaa tcatggtcat 1020

agctgtttcc tgtgtgaaat tgttatccgc tcacaattcc acacaacata cgagccgggg 1080
caggataata tatggtaggg ttcatagcca gagtaacctt ttttttaat ttttatttta 1140
ttttattttt gagatcag 1158

<210> 44
<211> 1076
<212> DNA
<213> Artificial Sequence

<220>
<223> Construct GSC6030

<400> 44

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct cagagctgca accggtggag agctgtgcgg 600
aggcgcagga cggggagctg gacgccctgt ggctgggcgt gctgagactg ctgctgttca 660
agctgctcct gttcgacctg ctcctcacct tcttcaaggt gaagtggatc ttctcctcgg 720
tggtggacct gaagcagacc atcatccccg actacaggaa catgatcgga caggggggcct 780
agtgattcga aatgaccgac caagcgacgc ccaacctgcc atcacgagat ttcgattcca 840
ccgccgcctt ctatgaaagg ttgggcttcg gaatcgtttt ccgggacgcc ggctggatga 900
tcctccagcg cggggatctc atgctggagt tcttcgccca ccccaacttg tttattgcag 960
cttataatgg ttacaaataa agcaatagca tcacaaattt cacaaataaa gcattttttt 1020
cactgcattc tagttgtggt ttgtccaaac tcatcaatgt atcttatcat gtctgt 1076

<210> 45
<211> 1058
<212> DNA
<213> Artificial Sequence

<220>
<223> Construct GSC6048

<400> 45

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaaccga 540
gggaggaggg aacagagctg caaccggtgg agagctgtgc ggaggcgcag gacggggagc 600
tggacgccct gtggctgggc gtgctgagac tgctgctgtt caagctgctc ctgttcgacc 660
tgctcctcac cttcttcaag gtgaagtgga tcttctcctc ggtggtggac ctgaagcaga 720
ccatcatccc cgactacagg aacatgatcg gacagggggc ctagtgattc gaaatgaccg 780
accaagcgac gcccaacctg ccatcacgag atttcgattc caccgccgcc ttctatgaaa 840
ggttgggctt cggaatcgtt ttccgggacg ccggctggat gatcctccag cgcggggatc 900
tcatgctgga gttcttcgcc caccccaact tgtttattgc agcttataat ggttacaaat 960
aaagcaatag catcacaaat ttcacaaata aagcattttt ttcactgcat tctagttgtg 1020
gtttgtccaa actcatcaat gtatcttatc atgtctgt 1058

<210> 46
<211> 1213
<212> DNA
<213> Artificial Sequence

<220>
<223> Construct GSC6047

<400> 46

```
ctgtctccgg gtaaatgatg aaagcttggt aggtgatcct cctgctgctt tggttcaggg        60
ttttgcttga gggggggggg tggtgatttc cttgccatgg gcagactgag cagaaaaggc       120
cattgggacc atgttctgaa tgcctccacc tcaaccaccg gccggtagga ccaaagccac       180
cccgtgtttt ctcaggatct cttttcccag ggagatccct cggcccaaag agggagatgg       240
caatgctgga tgtgtgcaca ataattcaac aggcattgga acttcagcat cgatacttgt       300
ttattgcagc ttataatggt tacaaataaa gcaatagcat cacaaatttc acaaataaag       360
catttttttc actgcattct agttgtggtt tgtccaaact catcaatgta tcttatcatg       420
tctgtatcga tgctgaatgc aattaacaat gctcaagcag aacccccggc tccatcagca       480
cagtgcagga ccaaacccca tgctgcagca gtggggctgt ctgtacgggg tgggcaatgg       540

gaaccggggt ctgctggggc tcctgctgct tcagtgctgc catgcagcca cacatcctga       600
gagctgaaag ggtcggcgtc ctcacctggt gcacaccgta gctctgcccc acagctttaa       660
ggcacctggc taacctctgc gcttcttccc ttccctcctc cctggctcag agctgcaacc       720
ggtggagagc tgtgcggagg cgcaggacgg ggagctggac gccctgtggc tgggcgtgct       780
gagactgctg ctgttcaagc tgctcctgtt cgacctgctc ctcaccttct tcaaggtgaa       840
gtggatcttc tcctcggtgg tggacctgaa gcagaccatc atccccgact acaggaacat       900
gatcggacag ggggcctagt gattcgaaat gaccgaccaa gcgacgccca acctgccatc       960
acgagatttc gattccaccg ccgccttcta tgaaaggttg ggcttcggaa tcgttttccg      1020
ggacgccggc tggatgatcc tccagcgcgg ggatctcatg ctggagttct tcgcccaccc      1080
caacttgttt attgcagctt ataatggtta caaataaagc aatagcatca caaatttcac      1140
aaataaagca tttttttcac tgcattctag ttgtggtttg tccaaactca tcaatgtatc      1200
ttatcatgtc tgt                                                         1213
```

<210> 47
<211> 1257
<212> DNA
<213> Artificial Sequence

<220>
<223> Construct GSC6046

<400> 47

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg ggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct cagagctgca accggtggag agctgtgcgg     600
aggcgcagga cggggagctg gacgccctgt ggctgggcgt gctgagactg ctgctgttca     660
agctgctcct gttcgacctg ctcctcacct tcttcaaggt gaagtggatc ttctcctcgg     720
tggtggacct gaagcagacc atcatccccg actacaggaa catgatcgga caggggggcct     780
agtgattcga aatgaccgac caagcgacgc ccaacctgcc atcacgagat ttcgattcca     840

ccgccgcctt ctatgaaagg ttgggcttcg gaatcgtttt ccgggacgcc ggctggatga     900
tcctccagcg cggggatctc atgctggagt tcttcgccca ccccaacttg tttattgcag     960
cttataatgg ttacaaataa agcaatagca tcacaaattt cacaaataaa gcattttttt    1020
cactgcattc tagttgtggt ttgtccaaac tcatcaatgt atcttatcat gtctgtatac    1080
cgtcgacctc tagctagagc ttggcgtaat catggtcata gctgtttcct gtgtgaaatt    1140
gttatccgct cacaattcca cacaacatac gagccggggc aggataatat atggtagggt    1200
tcatagccag agtaaccttt ttttttaatt tttattttat tttatttttg agatcag       1257
```

<210> 48
<211> 1738
<212> DNA
<213> Artificial Sequence

<220>
<223> Construct GSC314

<400> 48

atggagagcc ctgcccagct gctgttcctg ctgctgctgt ggctgcctga cacccaggcc 60
gaggtgcagc tggtcgagtc tggcggcgga ctggtgcagc ctggcggctc cctgcggctg 120
tcctgcgccg cctccggctt caacatcaag gacacctaca tccactgggt gcggcaggcc 180
cctggcaagg gcctggagtg ggtggcccgg atctacccta ccaacggcta caccagatac 240
gccgactccg tgaagggccg gttcaccatc tccgccgaca cctccaagaa caccgcctac 300
ctgcagatga actccctgcg ggccgaggac accgccgtgt actactgctc cagatgggga 360
ggagatggct tctacgccat ggactactgg ggccagggca ccctggtgac cgtgtcctcc 420
gcctccacca agggccccag cgtgttcccc ctggccccca gcagcaagtc taccagcggc 480
ggcacagcag ccctgggatg cctggtgaag gactacttcc ccgagcccgt gaccgtgagc 540
tggaacagcg gagccctgac ctccggcgtg cacaccttcc ccgccgtgct gcagagcagc 600
ggcctgtaca gcctgagcag cgtggtgacc gtgcccagca gcagcctggg cacccagacc 660
tacatctgca acgtgaacca caagcccagc aacaccaagg tggacaagaa ggtggagccc 720
aagagctgcg acaagaccca cacctgccct ccctgtcctg ctcctgagct gctcggcgga 780
ccctccgtgt tcctgttccc ccccaagccc aaggacaccc tgatgatcag caggaccccc 840
gaggtgacct gcgtggtggt ggacgtgagc cacgaggacc cagaggtgaa gttcaactgg 900
tacgtggacg gcgtggaggt gcacaacgcc aagaccaagc ccagagagga acagtacaac 960
agcacctaca gggtggtgtc cgtgctgacc gtgctgcacc aggactggct gaacggcaaa 1020
gagtacaagt gcaaggtctc caacaaggcc ctgccagccc ccatcgagaa aaccatcagc 1080
aaggccaagg gccagccacg ggagccccag gtgtacaccc tgccccctc ccgcgaggag 1140
atgaccaaga accaggtgtc cctgacatgt ctggtgaaag gcttctaccc cagcgacatc 1200
gccgtggagt gggagagcaa cggccagccc gagaacaact acaagaccac ccccccagtg 1260
ctggacagcg acggcagctt cttcctgtac agcaagctga ccgtggacaa gagcaggtgg 1320
cagcagggca acgtgttcag ctgcagcgtg atgcacgagg ccctgcacaa ccactacacc 1380
cagaagagcc tgagcctgtc ccccggcaag tgatgaatct agaggtaccc ccgggggcc 1440
cctcgagttc gaaatgaccg accaagcgac gcccaacctg ccatcacgag atttcgattc 1500
caccgccgcc ttctatgaaa ggttgggctt cggaatcgtt ttccgggacg ccggctggat 1560
gatcctccag cgcggggatc tcatgctgga gttcttcgcc caccccaact tgtttattgc 1620
agcttataat ggttacaaat aaagcaatag catcacaaat ttcacaaata aagcattttt 1680
ttcactgcat tctagttgtg gtttgtccaa actcatcaat gtatcttatc atgtctgt 1738

<210> 49
<211> 1410

<212> DNA
<213> Artificial Sequence

<220>
<223> Construct GSC5644

<400> 49

```
atgcggagcc ctgcccagct gctgttcctg ctgctgctgt ggatccctgg caccaacgcc      60
caggtgcagc tgcagcagtc tggcgccgaa ctggccagac caggcgccag cgtgaagatg     120
agctgcaagg ccagcggcta caccttcacc cggtacacca tgcactgggt gaaacagcgg     180
cctggacagg gcctggaatg gatcggctac atcaacccca gccggggcta caccaactac     240
aaccagaagt tcaaggacaa ggccaccctg accaccgaca agagcagcag caccgcctac     300
atgcagctga gcagcctgac cagcgaggac agcgccgtgt actactgcgc ccggtactac     360
gacgaccact actgcctgga ctactggggc cagggcacca ccctgaccgt gagcagcgcg     420
tcgaccaagg gccccagcgt gttcccgcta gcccccagca gcaagagcac cagcggcggc     480
acagccgccc tgggctgcct ggtgaaggac tacttccccg agcccgttac cgtgtcctgg     540
aactctggag ccctgacctc cggcgtgcac accttccccg ccgtgctcca gagcagcggc     600
ctgtacagcc tgagcagcgt ggtgacagtg cccagcagca gcctgggaac ccagacctac     660
atctgcaacg tgaaccacaa gcccagcaac accaaggtgg acaagaaggt ggagcccaag     720
agctgcgaca aaactcacac ctgcccaccc tgccctgccc ctgagctgct gggcggaccc     780
tccgtgttcc tgttcccccc caagcccaag gacaccctga tgatcagccg gacccccgag     840
gtgacctgcg tggtggtgga cgtgagccac gaggaccctg aggtgaagtt caattggtac     900
gtggacggcg tggaggtgca caacgccaag accaagcccc gggaggaaca gtacaacagc     960
acctaccggg tggtgtccgt gctgaccgtg ctgcaccagg actggctgaa cggcaaggaa    1020

tacaagtgca aggtctccaa caaggccctg cctgccccca tcgaaaagac catcagcaag    1080
gccaagggcc agccgcgcga accgcaggtg tacaccctgc cccccagccg ggaagagatg    1140
accaagaacc aggtgaaact ggtgtgcctg gtgacaggct tctaccccag cgatatcgcc    1200
gtggaatggg agagcagcgg ccagcctgag aacaactact acaccacccc ccccatgctg    1260
gacagcgacg gcagcttcag cctggtgtcc tggctgaacg tggacaagag ccggtggcag    1320
cagggcaaca tcttcagctg cagcgtgatg cacgaggccc tgcacaaccg gttcacccag    1380
aagtccctga gcctgagccc gggtaaatga                                    1410
```

<210> 50
<211> 1074
<212> DNA

<213> Artificial Sequence

<220>
<223> Construct GSC5537

<400> 50

```
gggtaaatga tgaaagcttg gtaggtgatc ctcctgctgc tttggttcag ggttttgctt      60
gaggggggggg ggtggtgatt tccttgccat gggcagactg agcagaaaag gccattggga    120
ccatgttctg aatgcctcca cctcaaccac cggccggtag gaccaaagcc accccgtgtt     180
ttctcaggat ctcttttccc agggagatcc ctcggcccaa agagggagat ggcaatgctg     240
gatgtgtgca caataattca acaggcattg gaacttcagc atcgatgctg aatgcaatta     300
acaatgctca agcagaaccc ccggctccat cagcacagtg caggaccaaa ccccatgctg     360
cagcagtggg gctgtctgta cggggtgggc aatgggaacc ggggtctgct ggggctcctg     420
ctgcttcagt gctgccatgc agccacacat cctgagagct gaaagggtcg gcgtcctcac     480
ctggtgcaca ccgtagctct gccccacagc tttaaggcac ctggctaacc tctgcgcttc     540
ttcccttccc tcctccctgg ctcagagctg caaccggtgg agagctgtgc ggaggcgcag     600
gacggggagc tggacgggct gtggacgacc atcaccatct tcatcacact cttcctgtta     660
agcgtgtgct acagtgccac cgtcaccttc ttcaaggtga agtggatctt ctcctcggtg     720
gtggacctga agcagaccat catccccgac tacaggaaca tgatcggaca gggggcctag     780
tgattcgaaa tgaccgacca agcgacgccc aacctgccat cacgagattt cgattccacc     840
gccgccttct atgaaaggtt gggcttcgga atcgttttcc gggacgccgg ctggatgatc     900
ctccagcgcg gggatctcat gctggagttc ttcgcccacc ccaacttgtt tattgcagct     960
tataatggtt acaaataaag caatagcatc acaaatttca caaataaagc atttttttca    1020
ctgcattcta gttgtggttt gtccaaactc atcaatgtat cttatcatgt ctgt          1074
```

<210> 51
<211> 1488
<212> DNA
<213> Artificial Sequence

<220>
<223> Construct GSC5608

<400> 51

```
atgcggagcc ctgcccagct gctgttcctg ctgctgctgt ggatccctgg caccaacgcc      60
gaggtgcagc tggtcgagtc tggcggcgga ctggtgcagc ctggcggctc cctgcggctg     120
tcctgcgccg cctccggctt caacatcaag gacacctaca tccactgggt gcggcaggcc     180
cctggcaagg gcctggagtg ggtggcccgg atctacccta ccaacggcta caccagatac     240
gccgactccg tgaagggccg gttcaccatc tccgccgaca cctccaagaa caccgcctac     300
ctgcagatga actccctgcg ggccgaggac accgccgtgt actactgctc cagatgggga     360
ggagatggct tctacgccat ggactactgg ggccagggca ccctggtgac cgtgtcctcc     420
ggtggcggtg gcagcggcgg tggtggttcc ggaggcggcg gttctgacat ccagatgacc     480
cagtccccct ccagcctgtc tgcctccgtg ggcgaccggg tgaccatcac ctgccgggcc     540
tcccaggacg tgaacaccgc cgtggcctgg tatcagcaga agcctggcaa ggcccctaag     600
ctgctgatct actccgcctc cttcctgtac tccggcgtgc cttcccggtt ctccggctcc     660
cggtccggca ccgacttcac cctgaccatc tcctccctgc agcctgagga cttcgccacc     720
tactactgcc agcagcacta caccaccccct cctaccttcg gccagggcac caaggtggag     780
atcaagaggg gaggaggagg taccgacaaa actcacacat gcccaccgtg cccagcacct     840
gaactcctgg ggggaccgtc agtcttcctc ttccccccaa aacccaagga caccctcatg     900
atctcccgga cccctgaggt cacatgcgtg gtggtggacg tgagccacga agaccctgag     960
gtcaagttca actggtacgt ggacggcgtg gaggtgcata atgccaagac aaagccgcgg    1020
gaggagcagt acaacagcac gtaccgtgtg gtcagcgtcc tcaccgtcct gcaccaggac    1080
tggctgaatg gcaaggagta caagtgcaag gtctccaaca aagccctccc agcccccatc    1140
gagaaaacca tctccaaagc caaaggacag cccagggaac ctgaggtggc cacattccca    1200
cctagccggg acgagctgac aaaaaatcag gtcaccctcg tctgtctcgt gaccggcttt    1260
taccccttccg acattgccgt ggaatgggaa tccaatgggc agcccgagaa caattacaag    1320
acagaccccc ccctgctgga atccgatggc agcttcgccc tgagcagccg gctgcgggtg    1380
gacaagtcca gatggcagca ggggaatgtc ttttcctgct ccgtcatgca tgaagccctc    1440
cacaatcatt atacacagaa aagcctgagc ctgtctcctg gcaagtga                 1488
```

<210> 52
<211> 1074
<212> DNA
<213> Artificial Sequence

<220>
<223> Construct GSC5538

<400> 52

```
gggtaaatga tgaaagcttg gtaggtgatc ctcctgctgc tttggttcag ggttttgctt      60
gaggggggggg ggtggtgatt tccttgccat gggcagactg agcagaaaag gccattggga     120
ccatgttctg aatgcctcca cctcaaccac cggccggtag gaccaaagcc accccgtgtt     180
ttctcaggat ctcttttccc agggagatcc ctcggcccaa agagggagat ggcaatgctg     240
gatgtgtgca caataattca acaggcattg gaacttcagc atcgatgctg aatgcaatta     300
acaatgctca agcagaaccc ccggctccat cagcacagtg caggaccaaa ccccatgctg     360
cagcagtggg gctgtctgta cggggtgggc aatgggaacc ggggtctgct ggggctcctg     420
ctgcttcagt gctgccatgc agccacacat cctgagagct gaaagggtcg gcgtcctcac     480
ctggtgcaca ccgtagctct gccccacagc tttaaggcac ctggctaacc tctgcgcttc     540
ttcccttccc tcctccctgg ctcagagctg caaccggtgg agagctgtgc ggaggcgcag     600
gacggggagc tggacgggct gtggacgacc atcaccatct tcatcacact cttcctgtta     660
agcgtgtgct acagtgccac cgtcaccttc ttcaaggtga agtggatctt ctcctcggtg     720
gtggacctga agcagaccat catccccgac tacaggaaca tgatcggaca gggggcctag     780
tgattcgaaa tgaccgacca agcgacgccc aacctgccat cacgagattt cgattccacc     840
gccgccttct atgaaaggtt gggcttcgga atcgttttcc gggacgccgg ctggatgatc     900
ctccagcgcg gggatctcat gctggagttc ttcgcccacc ccaacttgtt tattgcagct     960
tataatggtt acaaataaag caatagcatc acaaatttca caaataaagc atttttttca    1020
ctgcattcta gttgtggttt gtccaaactc atcaatgtat cttatcatgt ctgt          1074
```

<210> 53
<211> 1076
<212> DNA
<213> Artificial Sequence

<220>
<223> Construct GSC7057

<400> 53

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg ggtgggcaa tgggaaccgg ggtctgctgg     420
```

ggctcctgct gcttcagtgc tgccatgcag ccacacatcc tgagagctga aagggtcggc 480

gtcctcacct ggtgcacacc gtagctctgc cccacagctt taaggcacct ggctaaccga 540

gggaggaggg aacaggtggt ggtggatctc cccccgagga cccccctgac tccaagaata 600

ccctggtgct gttcggcgct ggcttcggcg ccgtgattac cgtggtcgtg atcgtcgtga 660

ttatcaagtg cttctgcaag caccggtcct gcttccggcg gaacgaggcc tccagagaga 720

caaacaactc cctgaccttc ggccccgagg aagccctggc tgagcagacc gtgtttctgt 780

gatgattcga aatgaccgac caagcgacgc ccaacctgcc atcacgagat ttcgattcca 840

ccgccgcctt ctatgaaagg ttgggcttcg gaatcgtttt ccgggacgcc ggctggatga 900

tcctccagcg cggggatctc atgctggagt tcttcgccca ccccaacttg tttattgcag 960

cttataatgg ttacaaataa agcaatagca tcacaaattt cacaaataaa gcattttttt 1020

cactgcattc tagttgtggt ttgtccaaac tcatcaatgt atcttatcat gtctgt 1076

<210> 54
<211> 1231
<212> DNA
<213> Artificial Sequence

<220>
<223> Construct GSC7058

<400> 54

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatacttgt 300
ttattgcagc ttataatggt tacaaataaa gcaatagcat cacaaatttc acaaataaag 360
cattttttc actgcattct agttgtggtt tgtccaaact catcaatgta tcttatcatg 420
tctgtatcga tgctgaatgc aattaacaat gctcaagcag aaccccccggc tccatcagca 480
cagtgcagga ccaaacccca tgctgcagca gtggggctgt ctgtacgggg tgggcaatgg 540
gaaccggggt ctgctggggc tcctgctgct tcagtgctgc catgcagcca cacatcctga 600
gagctgaaag ggtcggcgtc ctcacctggt gcacaccgta gctctgcccc acagctttaa 660
ggcacctggc taacctctgc gcttcttccc ttccctcctc cctggctcag gtggtggtgg 720
atctcccccc gaggacccc ctgactccaa gaataccctg gtgctgttcg gcgctggctt 780
cggcgccgtg attaccgtgg tcgtgatcgt cgtgattatc aagtgcttct gcaagcaccg 840
gtcctgcttc cggcggaacg aggcctccag agagacaaac aactccctga ccttcggccc 900

cgaggaagcc ctggctgagc agaccgtgtt tctgtgatga ttcgaaatga ccgaccaagc 960
gacgcccaac ctgccatcac gagatttcga ttccaccgcc gccttctatg aaaggttggg 1020
cttcggaatc gttttccggg acgccggctg gatgatcctc cagcgcgggg atctcatgct 1080
ggagttcttc gcccacccca acttgtttat tgcagcttat aatggttaca aataaagcaa 1140
tagcatcaca aatttcacaa ataaagcatt tttttcactg cattctagtt gtggtttgtc 1200
caaactcatc aatgtatctt atcatgtctg t 1231

<210> 55
<211> 1094
<212> DNA
<213> Artificial Sequence

<220>
<223> Construct GSC7056

<400> 55

```
ctgtctccgg gtaaatgatg aaagcttggt aggtgatcct cctgctgctt tggttcaggg      60
ttttgcttga ggggggggg tggtgatttc cttgccatgg gcagactgag cagaaaaggc      120
cattgggacc atgttctgaa tgcctccacc tcaaccaccg gccggtagga ccaaagccac    180
cccgtgtttt ctcaggatct cttttcccag ggagatccct cggcccaaag agggagatgg    240
caatgctgga tgtgtgcaca ataattcaac aggcattgga acttcagcat cgatgctgaa    300
tgcaattaac aatgctcaag cagaaccccc ggctccatca gcacagtgca ggaccaaacc    360
ccatgctgca gcagtggggc tgtctgtacg gggtgggcaa tgggaaccgg ggtctgctgg    420
ggctcctgct gcttcagtgc tgccatgcag ccacacatcc tgagagctga aagggtcggc    480
gtcctcacct ggtgcacacc gtagctctgc cccacagctt taaggcacct ggctaacctc    540
tgcgcttctt cccttccctc ctccctggct caggtggtgg tggatctccc cccgaggacc    600
cccctgactc caagaatacc ctggtgctgt tcggcgctgg cttcggcgcc gtgattaccg    660
tggtcgtgat cgtcgtgatt atcaagtgct tctgcaagca ccggtcctgc ttccggcgga    720
acgaggcctc cagagagaca aacaactccc tgaccttcgg ccccgaggaa gccctggctg    780
agcagaccgt gtttctgtga tgattcgaaa tgaccgacca agcgacgccc aacctgccat    840
cacgagattt cgattccacc gccgccttct atgaaaggtt gggcttcgga atcgttttcc    900
gggacgccgg ctggatgatc ctccagcgcg gggatctcat gctggagttc ttcgcccacc    960
ccaacttgtt tattgcagct tataatggtt acaaataaag caatagcatc acaaatttca    1020
caaataaagc atttttttca ctgcattcta gttgtggttt gtccaaactc atcaatgtat    1080
cttatcatgt ctgt                                                     1094
```

<210> 56
<211> 1275
<212> DNA
<213> Artificial Sequence

<220>
<223> Construct GSC7059

<400> 56

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaagtgct tctgcaagca ccggtcctgc ttccggcgga 720
acgaggcctc cagagagaca aacaactccc tgaccttcgg ccccgaggaa gccctggctg 780
agcagaccgt gtttctgtga tgattcgaaa tgaccgacca agcgacgccc aacctgccat 840
cacgagattt cgattccacc gccgccttct atgaaaggtt gggcttcgga atcgttttcc 900
gggacgccgg ctggatgatc ctccagcgcg gggatctcat gctggagttc ttcgcccacc 960
ccaacttgtt tattgcagct tataatggtt acaaataaag caatagcatc acaaatttca 1020
caaataaagc atttttttca ctgcattcta gttgtggttt gtccaaactc atcaatgtat 1080
cttatcatgt ctgtataccg tcgacctcta gctagagctt ggcgtaatca tggtcatagc 1140
tgtttcctgt gtgaaattgt tatccgctca caattccaca caacatacga gccggggcag 1200
gataatatat ggtagggttc atagccagag taaccttttt ttttaatttt tattttattt 1260
tatttttgag atcag 1275

<210> 57
<211> 1094
<212> DNA
<213> Artificial Sequence

<220>
<223> GSC9765

<400> 57

ctgtctccgg gcaaatgatg aaagcttggt aggtgatcct cctgctgctt tggttcaggg 60

```
ttttgcttga gggggggggg tggtgatttc cttgccatgg gcagactgag cagaaaaggc      120
cattgggacc atgttctgaa tgcctccacc tcaaccaccg gccggtagga ccaaagccac      180
cccgtgtttt ctcaggatct cttttcccag ggagatccct cggcccaaag agggagatgg      240
caatgctgga tgtgtgcaca ataattcaac aggcattgga acttcagcat cgatgctgaa      300
tgcaattaac aatgctcaag cagaaccccc ggctccatca gcacagtgca ggaccaaacc      360
ccatgctgca gcagtggggc tgtctgtacg gggtgggcaa tgggaaccgg ggtctgctgg      420
ggctcctgct gcttcagtgc tgccatgcag ccacacatcc tgagagctga aagggtcggc      480
gtcctcacct ggtgcacacc gtagctctgc cccacagctt taaggcacct ggctaacctc      540
tgcgcttctt cccttccctc ctccctggct caggtggtgg tggatctccc cccgaggacc      600
cccctgactc caagaatacc ctggtgctgt tcggcgctgg cttcggcgcc gtgattaccg      660
tggtcgtgat cgtcgtgatt atcaagtgct tctgcaagca ccggtcctgc ttccggcgga      720
acgaggcctc cagagagaca aacaactccc tgaccttcgg ccccgaggaa gccctggctg      780
agcagaccgt gtttctgtga tgattcgaaa tgaccgacca agcgacgccc aacctgccat      840
cacgagattt cgattccacc gccgccttct atgaaaggtt gggcttcgga atcgttttcc      900
gggacgccgg ctggatgatc ctccagcgcg gggatctcat gctggagttc ttcgcccacc      960
ccaacttgtt tattgcagct tataatggtt acaaataaag caatagcatc acaaatttca     1020
caaataaagc atttttttca ctgcattcta gttgtggttt gtccaaactc atcaatgtat     1080
cttatcatgt ctgt                                                       1094
```

<210> 58
<211> 1094
<212> DNA
<213> Artificial Sequence

<220>
<223> GSC9764

<400> 58

ctgtctcccg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540

tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaagtgct tctgcaagca ccggtcctgc ttccggcgga 720
acgaggcctc cagagagaca aacaactccc tgaccttcgg ccccgaggaa gccctggctg 780
agcagaccgt gtttctgtga tgattcgaaa tgaccgacca agcgacgccc aacctgccat 840
cacgagattt cgattccacc gccgccttct atgaaaggtt gggcttcgga atcgttttcc 900
gggacgccgg ctggatgatc ctccagcgcg gggatctcat gctggagttc ttcgcccacc 960
ccaacttgtt tattgcagct tataatggtt acaaataaag caatagcatc acaaatttca 1020
caaataaagc atttttttca ctgcattcta gttgtggttt gtccaaactc atcaatgtat 1080
cttatcatgt ctgt 1094

<210> 59
<211> 1076
<212> DNA
<213> Artificial Sequence

<220>
<223> GSC9949

<400> 59

ctgtctccgg gtaaatgatg aaagcttggt aggtgatcct cctgctgctt tggttcaggg 60

ttttgcttga gggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaaccgt 540

ctccttctgg gacaggtggt ggtggatctc cccccgagga cccccctgac tccaagaata 600

ccctggtgct gttcggcgct ggcttcggcg ccgtgattac cgtggtcgtg atcgtcgtga 660

ttatcaagtg cttctgcaag caccggtcct gcttccggcg gaacgaggcc tccagagaga 720

caaacaactc cctgaccttc ggccccgagg aagccctggc tgagcagacc gtgtttctgt 780

gatgattcga aatgaccgac caagcgacgc ccaacctgcc atcacgagat ttcgattcca 840

ccgccgcctt ctatgaaagg ttgggcttcg gaatcgtttt ccgggacgcc ggctggatga 900

tcctccagcg cggggatctc atgctggagt tcttcgccca ccccaacttg tttattgcag 960

cttataatgg ttacaaataa agcaatagca tcacaaattt cacaaataaa gcattttttt 1020

cactgcattc tagttgtggt ttgtccaaac tcatcaatgt atcttatcat gtctgt 1076

<210> 60
<211> 1076
<212> DNA
<213> Artificial Sequence

<220>
<223> GSC9768

<400> 60

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaaccct     540
gggaggattg cacaggtggt ggtggatctc cccccgagga cccccctgac tccaagaata     600
ccctggtgct gttcggcgct ggcttcggcg ccgtgattac cgtggtcgtg atcgtcgtga     660
ttatcaagtg cttctgcaag caccggtcct gcttccggcg gaacgaggcc tccagagaga     720
caaacaactc cctgaccttc ggccccgagg aagccctggc tgagcagacc gtgtttctgt     780
gatgattcga aatgaccgac caagcgacgc ccaacctgcc atcacgagat ttcgattcca     840
ccgccgcctt ctatgaaagg ttgggcttcg gaatcgtttt ccgggacgcc ggctggatga     900
tcctccagcg cggggatctc atgctggagt tcttcgccca ccccaacttg tttattgcag     960
cttataatgg ttacaaataa agcaatagca tcacaaattt cacaaataaa gcattttttt    1020
cactgcattc tagttgtggt ttgtccaaac tcatcaatgt atcttatcat gtctgt        1076
```

<210> 61
<211> 1076
<212> DNA
<213> Artificial Sequence

<220>
<223> GSC9763

<400> 61

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
```

cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg ggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaaccct 540
gtccttattg cacaggtggt ggtggatctc cccccgagga cccccctgac tccaagaata 600
ccctggtgct gttcggcgct ggcttcggcg ccgtgattac cgtggtcgtg atcgtcgtga 660
ttatcaagtg cttctgcaag caccggtcct gcttccggcg gaacgaggcc tccagagaga 720
caaacaactc cctgaccttc ggccccgagg aagccctggc tgagcagacc gtgtttctgt 780
gatgattcga aatgaccgac caagcgacgc ccaacctgcc atcacgagat ttcgattcca 840
ccgccgcctt ctatgaaagg ttgggcttcg gaatcgtttt ccgggacgcc ggctggatga 900
tcctccagcg cggggatctc atgctggagt tcttcgccca ccccaacttg tttattgcag 960
cttataatgg ttacaaataa agcaatagca tcacaaattt cacaaataaa gcattttttt 1020
cactgcattc tagttgtggt ttgtccaaac tcatcaatgt atcttatcat gtctgt 1076

<210> 62
<211> 1076
<212> DNA
<213> Artificial Sequence

<220>
<223> GSC9770

<400> 62

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaaccga     540
gacctgaggg gacaggtggt ggtggatctc cccccgagga cccccctgac tccaagaata     600
ccctggtgct gttcggcgct ggcttcggcg ccgtgattac cgtggtcgtg atcgtcgtga     660
ttatcaagtg cttctgcaag caccggtcct gcttccggcg gaacgaggcc tccagagaga     720
caaacaactc cctgaccttc ggccccgagg aagccctggc tgagcagacc gtgtttctgt     780
gatgattcga aatgaccgac caagcgacgc ccaacctgcc atcacgagat ttcgattcca     840
ccgccgcctt ctatgaaagg ttgggcttcg gaatcgtttt ccgggacgcc ggctggatga     900
tcctccagcg cggggatctc atgctggagt tcttcgccca ccccaacttg tttattgcag     960
cttataatgg ttacaaataa agcaatagca tcacaaattt cacaaataaa gcattttttt    1020
cactgcattc tagttgtggt ttgtccaaac tcatcaatgt atcttatcat gtctgt        1076
```

<210> 63
<211> 1094
<212> DNA
<213> Artificial Sequence

<220>
<223> GSC9950

<400> 63

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctg ctacctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaagtgct tctgcaagca ccggtcctgc ttccggcgga 720
acgaggcctc cagagagaca aacaactccc tgaccttcgg ccccgaggaa gccctggctg 780
agcagaccgt gtttctgtga tgattcgaaa tgaccgacca agcgacgccc aacctgccat 840
cacgagattt cgattccacc gccgccttct atgaaaggtt gggcttcgga atcgttttcc 900
gggacgccgg ctggatgatc ctccagcgcg gggatctcat gctggagttc ttcgcccacc 960
ccaacttgtt tattgcagct tataatggtt acaaataaag caatagcatc acaaatttca 1020
caaataaagc atttttttca ctgcattcta gttgtggttt gtccaaactc atcaatgtat 1080
cttatcatgt ctgt 1094

<210> 64
<211> 211
<212> DNA
<213> Artificial Sequence

<220>
<223> GeneArt_Seq32

<400> 64

accggtggag agctgtgcgg aggcgcagga cggggagctg gacgccctgt ggctgggcgt 60
gctgagactg ctgctgttca agctgctcct gttcgacctg ctcctcacct tcttcaaggt 120
gaagtggatc ttctcctcgg tggtggacct gaagcagacc atcatccccg actacaggaa 180
catgatcgga caggggcct agtgattcga a 211

<210> 65
<211> 2009
<212> DNA
<213> Homo sapiens

<400> 65

```
agctttctgg ggcaggccag gcctgacctt ggctttgggg cagggagggg gctaaggtga      60
ggcaggtggc gccagcaggt gcacacccaa tgcccatgag cccagacact ggacgctgaa     120
cctcgcggac agttaagaac ccaggggcct ctgcgcctgg gcccagctct gtcccacacc     180
gcggtcacat ggcaccacct ctcttgcagc ctccaccaag ggcccatcgg tcttccccct     240
ggcaccctcc tccaagagca cctctggggg cacagcggcc ctgggctgcc tggtcaagga     300
ctacttcccc gaaccggtga cggtgtcgtg gaactcaggc gccctgacca gcggcgtgca     360
caccttcccg gctgtcctac agtcctcagg actctactcc ctcagcagcg tggtgaccgt     420
gccctccagc agcttgggca cccagaccta catctgcaac gtgaatcaca agcccagcaa     480
caccaaggtg gacaagaaag ttggtgagag gccagcacag ggagggaggg tgtctgctgg     540
aagcaggctc agcgctcctg cctggacgca tcccggctat gcagccccag tccagggcag     600
caaggcaggc cccgtctgcc tcttcacccg gagcctctgc ccgccccact catgctcagg     660
gagagggtct tctggctttt tcccaggctc tgggcaggca caggctaggt gcccctaacc     720
caggccctgc acacaaaggg gcaggtgctg ggctcagacc tgccaagagc catatccggg     780
aggaccctgc ccctgaccta agcccacccc aaaggccaaa ctctccactc cctcagctcg     840
gacaccttct ctcctcccag attccagtaa ctcccaatct tctctctgca gagcccaaat     900
cttgtgacaa aactcacaca tgcccaccgt gcccaggtaa gccagcccag gcctcgccct     960
ccagctcaag gcgggacagg tgccctagag tagcctgcat ccagggacag gccccagccg    1020
ggtgctgaca cgtccacctc catctcttcc tcagcacctg aactcctggg gggaccgtca    1080
gtcttcctct tccccccaaa acccaaggac accctcatga tctcccggac ccctgaggtc    1140
```

acatgcgtgg tggtggacgt gagccacgaa gaccctgagg tcaagttcaa ctggtacgtg 1200

gacggcgtgg aggtgcataa tgccaagaca aagccgcggg aggagcagta caacagcacg 1260

taccgggtgg tcagcgtcct caccgtcctg caccaggact ggctgaatgg caaggagtac 1320

aagtgcaagg tctccaacaa agccctccca gcccccatcg agaaaaccat ctccaaagcc 1380

aaaggtggga cccgtggggt gcgagggcca catggacaga ggccggctcg gcccaccctc 1440

tgccctgaga gtgaccgctg taccaacctc tgtcctacag ggcagccccg agaaccacag 1500

gtgtacaccc tgcccccatc ccgggatgag ctgaccaaga accaggtcag cctgacctgc 1560

ctggtcaaag gcttctatcc cagcgacatc gccgtggagt gggagagcaa tgggcagccg 1620

gagaacaact acaagaccac gcctcccgtg ctggactccg acggctcctt cttcctctac 1680

agcaagctca ccgtggacaa gagcaggtgg cagcagggga acgtcttctc atgctccgtg 1740

atgcatgagg ctctgcacaa ccactacacg cagaagagcc tctccctgtc tccgggtaaa 1800

tgagtgcgac ggccggcaag ccccgctccc cgggctctcg cggtcgcacg aggatgcttg 1860

gcacgtaccc cctgtacata cttcccgggc gcccagcatg gaaataaagc acccagcgct 1920

gccctgggcc cctgcgagac tgtgatggtt ctttccacgg gtcaggccga gtctgaggcc 1980

tgagtggcat gagggaggca gagcgggtc 2009

<210> 66
<211> 216
<212> DNA
<213> Artificial Sequence

<220>
<223> GeneArt_Seq43

<400> 66

ccccccgagg acccccctga ctccaagaat accctggtgc tgttcggcgc tggcttcggc 60

gccgtgatta ccgtggtcgt gatcgtcgtg attatcaagt gcttctgcaa gcaccggtcc 120

tgcttccggc ggaacgaggc ctccagagag acaaacaact ccctgacctt cggccccgag 180

gaagccctgg ctgagcagac cgtgtttctg tgatga 216

<210> 67
<211> 546
<212> DNA
<213> Artificial Sequence

<220>
<223> Construct GSC2819

<400> 67

```
ggtaggtgat cctcctgctg ctttggttca gggttttgct tgaggggggg gggtggtgat      60
ttccttgcca tgggcagact gagcagaaaa ggccattggg accatgttct gaatgcctcc     120
acctcaacca ccggccggta ggaccaaagc caccccgtgt tttctcagga tctcttttcc     180
cagggagatc cctcggccca aagagggaga tggcaatgct ggatgtgtgc acaataattc     240
aacaggcatt ggaacttcag catcgatgct gaatgcaatt aacaatgctc aagcagaacc     300
cccggctcca tcagcacagt gcaggaccaa accccatgct gcagcagtgg ggctgtctgt     360
acggggtggg caatgggaac cggggtctgc tggggctcct gctgcttcag tgctgccatg     420
cagccacaca tcctgagagc tgaaagggtc ggcgtcctca cctggtgcac accgtagctc     480
tgccccacag ctttaaggca cctggctaac ctctgcgctt cttcccttcc ctcctccctg     540
gctcag                                                                546
```

<210> 68
<211> 528
<212> DNA
<213> Artificial Sequence

<220>
<223> Construct GSC3469

<400> 68

```
ggtaggtgat cctcctgctg ctttggttca gggttttgct tgaggggggg gggtggtgat      60
ttccttgcca tgggcagact gagcagaaaa ggccattggg accatgttct gaatgcctcc     120
acctcaacca ccggccggta ggaccaaagc caccccgtgt tttctcagga tctcttttcc     180
cagggagatc cctcggccca aagagggaga tggcaatgct ggatgtgtgc acaataattc     240
aacaggcatt ggaacttcag catcgatgct gaatgcaatt aacaatgctc aagcagaacc     300
cccggctcca tcagcacagt gcaggaccaa accccatgct gcagcagtgg ggctgtctgt     360
acggggtggg caatgggaac cggggtctgc tggggctcct gctgcttcag tgctgccatg     420
cagccacaca tcctgagagc tgaaagggtc ggcgtcctca cctggtgcac accgtagctc     480
tgccccacag ctttaaggca cctggctaac cgagggagga gggaacag                  528
```

<210> 69
<211> 563
<212> DNA
<213> Artificial Sequence

<220>
<223> Intron cTNT-I4

<400> 69

gtaaatgatg aaagcttggt aggtgatcct cctgctgctt tggttcaggg ttttgcttga 60

gggggggggg tggtgatttc cttgccatgg gcagactgag cagaaaaggc cattgggacc 120

atgttctgaa tgcctccacc tcaaccaccg gccggtagga ccaaagccac cccgtgtttt 180

ctcaggatct cttttcccag ggagatccct cggcccaaag agggagatgg caatgctgga 240

tgtgtgcaca ataattcaac aggcattgga acttcagcat cgatgctgaa tgcaattaac 300

aatgctcaag cagaaccccc ggctccatca gcacagtgca ggaccaaacc ccatgctgca 360

gcagtggggc tgtctgtacg gggtgggcaa tgggaaccgg ggtctgctgg ggctcctgct 420

gcttcagtgc tgccatgcag ccacacatcc tgagagctga aagggtcggc gtcctcacct 480

ggtgcacacc gtagctctgc cccacagctt taaggcacct ggctaacctc tgcgcttctt 540

cccttccctc ctccctggct cag 563

<210> 70
<211> 545
<212> DNA
<213> Artificial Sequence

<220>
<223> Intron I4(0Y)

<400> 70

gtaaatgatg aaagcttggt aggtgatcct cctgctgctt tggttcaggg ttttgcttga 60

gggggggggg tggtgatttc cttgccatgg gcagactgag cagaaaaggc cattgggacc 120

atgttctgaa tgcctccacc tcaaccaccg gccggtagga ccaaagccac cccgtgtttt 180

ctcaggatct cttttcccag ggagatccct cggcccaaag agggagatgg caatgctgga 240

tgtgtgcaca ataattcaac aggcattgga acttcagcat cgatgctgaa tgcaattaac 300

aatgctcaag cagaaccccc ggctccatca gcacagtgca ggaccaaacc ccatgctgca 360

gcagtggggc tgtctgtacg gggtgggcaa tgggaaccgg ggtctgctgg ggctcctgct 420

gcttcagtgc tgccatgcag ccacacatcc tgagagctga aagggtcggc gtcctcacct 480

ggtgcacacc gtagctctgc cccacagctt taaggcacct ggctaaccga gggaggaggg 540

aacag 545

<210> 71
<211> 700
<212> DNA
<213> Artificial Sequence

<220>
<223> Intron 14(polyA)

<400> 71

gtaaatgatg aaagcttggt aggtgatcct cctgctgctt tggttcaggg ttttgcttga 60
gggggggggg tggtgatttc cttgccatgg gcagactgag cagaaaaggc cattgggacc 120
atgttctgaa tgcctccacc tcaaccaccg gccggtagga ccaaagccac cccgtgtttt 180
ctcaggatct cttttcccag ggagatccct cggcccaaag agggagatgg caatgctgga 240
tgtgtgcaca ataattcaac aggcattgga acttcagcat cgatacttgt ttattgcagc 300
ttataatggt tacaaataaa gcaatagcat cacaaatttc acaaataaag catttttttc 360
actgcattct agttgtggtt tgtccaaact catcaatgta tcttatcatg tctgtatcga 420
tgctgaatgc aattaacaat gctcaagcag aacccccggc tccatcagca cagtgcagga 480
ccaaacccca tgctgcagca gtggggctgt ctgtacgggg tgggcaatgg gaaccggggt 540
ctgctggggc tcctgctgct tcagtgctgc catgcagcca cacatcctga gagctgaaag 600
ggtcggcgtc ctcacctggt gcacaccgta gctctgcccc acagctttaa ggcacctggc 660
taacctctgc gcttcttccc ttccctcctc cctggctcag 700

<210> 72
<211> 545
<212> DNA
<213> Artificial Sequence

<220>
<223> Intron I4(3Y)

<400> 72

gtaaatgatg aaagcttggt aggtgatcct cctgctgctt tggttcaggg ttttgcttga 60
gggggggggg tggtgatttc cttgccatgg gcagactgag cagaaaaggc cattgggacc 120
atgttctgaa tgcctccacc tcaaccaccg gccggtagga ccaaagccac cccgtgtttt 180
ctcaggatct cttttcccag ggagatccct cggcccaaag agggagatgg caatgctgga 240
tgtgtgcaca ataattcaac aggcattgga acttcagcat cgatgctgaa tgcaattaac 300
aatgctcaag cagaaccccc ggctccatca gcacagtgca ggaccaaacc ccatgctgca 360
gcagtggggc tgtctgtacg ggtgggcaa tgggaaccgg ggtctgctgg ggctcctgct 420
gcttcagtgc tgccatgcag ccacacatcc tgagagctga aagggtcggc gtcctcacct 480
ggtgcacacc gtagctctgc cccacagctt taaggcacct ggctaaccga gacctgaggg 540
gacag 545

<210> 73
<211> 545
<212> DNA
<213> Artificial Sequence

<220>
<223> Intron I4(5Y)

<400> 73

gtaaatgatg aaagcttggt aggtgatcct cctgctgctt tggttcaggg ttttgcttga 60
gggggggggg tggtgatttc cttgccatgg gcagactgag cagaaaaggc cattgggacc 120
atgttctgaa tgcctccacc tcaaccaccg gccggtagga ccaaagccac cccgtgtttt 180
ctcaggatct cttttcccag ggagatccct cggcccaaag agggagatgg caatgctgga 240
tgtgtgcaca ataattcaac aggcattgga acttcagcat cgatgctgaa tgcaattaac 300
aatgctcaag cagaaccccc ggctccatca gcacagtgca ggaccaaacc ccatgctgca 360
gcagtggggc tgtctgtacg ggtgggcaa tgggaaccgg ggtctgctgg ggctcctgct 420
gcttcagtgc tgccatgcag ccacacatcc tgagagctga aagggtcggc gtcctcacct 480
ggtgcacacc gtagctctgc cccacagctt taaggcacct ggctaaccct gtccttattg 540
cacag 545

<210> 74
<211> 545
<212> DNA
<213> Artificial Sequence

<220>
<223> Intron I4(5Y-5)

<400> 74

gtaaatgatg aaagcttggt aggtgatcct cctgctgctt tggttcaggg ttttgcttga 60
gggggggggg tggtgatttc cttgccatgg gcagactgag cagaaaaggc cattgggacc 120
atgttctgaa tgcctccacc tcaaccaccg gccggtagga ccaaagccac cccgtgtttt 180
ctcaggatct cttttcccag ggagatccct cggcccaaag agggagatgg caatgctgga 240
tgtgtgcaca ataattcaac aggcattgga acttcagcat cgatgctgaa tgcaattaac 300
aatgctcaag cagaaccccc ggctccatca gcacagtgca ggaccaaacc ccatgctgca 360
gcagtggggc tgtctgtacg ggtgggcaa tgggaaccgg ggtctgctgg ggctcctgct 420
gcttcagtgc tgccatgcag ccacacatcc tgagagctga aagggtcggc gtcctcacct 480
ggtgcacacc gtagctctgc cccacagctt taaggcacct ggctaaccct gggaggattg 540
cacag 545

<210> 75
<211> 545
<212> DNA
<213> Artificial Sequence

<220>
<223> Intron I4(9Y)

<400> 75

```
gtaaatgatg aaagcttggt aggtgatcct cctgctgctt tggttcaggg ttttgcttga      60
ggggggggg tggtgatttc cttgccatgg gcagactgag cagaaaaggc cattgggacc      120
atgttctgaa tgcctccacc tcaaccaccg gccggtagga ccaaagccac cccgtgtttt      180
ctcaggatct cttttcccag ggagatccct cggcccaaag agggagatgg caatgctgga      240
tgtgtgcaca ataattcaac aggcattgga acttcagcat cgatgctgaa tgcaattaac      300
aatgctcaag cagaaccccc ggctccatca gcacagtgca ggaccaaacc ccatgctgca      360
gcagtggggc tgtctgtacg gggtgggcaa tgggaaccgg ggtctgctgg ggctcctgct      420
gcttcagtgc tgccatgcag ccacacatcc tgagagctga aagggtcggc gtcctcacct      480
ggtgcacacc gtagctctgc cccacagctt taaggcacct ggctaaccgt ctccttctgg      540
gacag                                                                 545
```

<210> 76
<211> 563
<212> DNA
<213> Artificial Sequence

<220>
<223> Intron I4(15Y-3\222)

<400> 76

```
gtaaatgatg aaagcttggt aggtgatcct cctgctgctt tggttcaggg ttttgcttga      60
ggggggggg tggtgatttc cttgccatgg gcagactgag cagaaaaggc cattgggacc      120
atgttctgaa tgcctccacc tcaaccaccg gccggtagga ccaaagccac cccgtgtttt      180
ctcaggatct cttttcccag ggagatccct cggcccaaag agggagatgg caatgctgga      240
tgtgtgcaca ataattcaac aggcattgga acttcagcat cgatgctgaa tgcaattaac      300
aatgctcaag cagaaccccc ggctccatca gcacagtgca ggaccaaacc ccatgctgca      360
gcagtggggc tgtctgtacg gggtgggcaa tgggaaccgg ggtctgctgg ggctcctgct      420
gcttcagtgc tgccatgcag ccacacatcc tgagagctga aagggtcggc gtcctcacct      480
ggtgcacacc gtagctctgc cccacagctt taaggcacct ggctaacctc tgcgcttctt      540
cccttccctg ctacctggct cag                                             563
```

<210> 77
<211> 1035
<212> DNA
<213> Artificial Sequence

<220>
<223> Intron I4(SV40ter)

<400> 77

```
gtaaatgatg aaagcttggt aggtgatcct cctgctgctt tggttcaggg ttttgcttga      60
gggggggggg tggtgatttc cttgccatgg gcagactgag cagaaaaggc cattgggacc     120
atgttctgaa tgcctccacc tcaaccaccg gccggtagga ccaaagccac cccgtgtttt     180
ctcaggatct cttttcccag ggagatccct cggcccaaag agggagatgg caatgctgga     240
tgtgtgcaca ataattcaac aggcattgga acttcagcat cgaaatgacc gaccaagcga     300
cgcccaacct gccatcacga gatttcgatt ccaccgccgc cttctatgaa aggttgggct     360
tcggaatcgt tttccgggac gccggctgga tgatcctcca gcgcggggat ctcatgctgg     420
agttcttcgc ccaccccaac ttgtttattg cagcttataa tggttacaaa taaagcaata     480
gcatcacaaa tttcacaaat aaagcatttt tttcactgca ttctagttgt ggtttgtcca     540

aactcatcaa tgtatcttat catgtctgta taccgtcgac ctctagctag agcttggcgt     600
aatcatggtc atagctgttt cctgtgtgaa attgttatcc gctcacaatt ccacacaaca     660
tacgagccgg ggcaggataa tatatggtag ggttcatagc cagagtaacc tttttttta      720
atttttattt tattttattt ttgagatcag ttcgatgctg aatgcaatta acaatgctca     780
agcagaaccc ccggctccat cagcacagtg caggaccaaa ccccatgctg cagcagtggg     840
gctgtctgta cggggtgggc aatgggaacc ggggtctgct ggggctcctg ctgcttcagt     900
gctgccatgc agccacacat cctgagagct gaaagggtcg gcgtcctcac ctggtgcaca     960
ccgtagctct gccccacagc tttaaggcac ctggctaacc tctgcgcttc ttcccttccc    1020
tcctccctgg ctcag                                                     1035
```

<210> 78
<211> 563
<212> DNA
<213> Artificial Sequence

<220>
<223> Intron I4(SD_GGC)

<400> 78

```
gcaaatgatg aaagcttggt aggtgatcct cctgctgctt tggttcaggg ttttgcttga        60
gggggggggg tggtgatttc cttgccatgg gcagactgag cagaaaaggc cattgggacc       120
atgttctgaa tgcctccacc tcaaccaccg gccggtagga ccaaagccac cccgtgtttt       180
ctcaggatct cttttcccag ggagatccct cggcccaaag agggagatgg caatgctgga       240
tgtgtgcaca ataattcaac aggcattgga acttcagcat cgatgctgaa tgcaattaac       300
aatgctcaag cagaaccccc ggctccatca gcacagtgca ggaccaaacc ccatgctgca       360
gcagtggggc tgtctgtacg gggtgggcaa tgggaaccgg ggtctgctgg ggctcctgct       420
gcttcagtgc tgccatgcag ccacacatcc tgagagctga aagggtcggc gtcctcacct       480
ggtgcacacc gtagctctgc cccacagctt taaggcacct ggctaacctc tgcgcttctt       540
cccttccctc ctccctggct cag                                               563
```

<210> 79
<211> 375
<212> DNA
<213> Artificial Sequence

<220>
<223> GSB59abc

<400> 79

```
ctgtccctgg gcaagtgata gaagcttgcg gccgcgctta gcgatatcga attctctaga        60
ggtacccccg gggggcccct cgagttcgaa atgaccgacc aagcgacgcc caacctgcca       120
tcacgagatt tcgattccac cgccgccttc tatgaaaggt tgggcttcgg aatcgttttc       180
cgggacgccg gctggatgat cctccagcgc ggggatctca tgctggagtt cttcgcccac       240
cccaacttgt ttattgcagc ttataatggt tacaaataaa gcaatagcat cacaaatttc       300
acaaataaag catttttttc actgcattct agttgtggtt tgtccaaact catcaatgta       360
tcttatcatg tctgt                                                        375
```

<210> 80
<211> 1094
<212> DNA
<213> Artificial Sequence

<220>
<223> GSC11218

<400> 80

```
ctgtctccgg gtaaatgatg aaagcttggt aggtgatcct cctgctgctt tggttcaggg       60
ttttgcttga gggggggggg tggtgatttc cttgccatgg gcagactgag cagaaaaggc      120
cattgggacc atgttctgaa tgcctccacc tcaaccaccg gccggtagga ccaaagccac      180
cccgtgtttt ctcaggatct cttttcccag ggagatccct cggcccaaag agggagatgg      240
caatgctgga tgtgtgcaca ataattcaac aggcattgga acttcagcat cgatgctgaa      300
tgcaattaac aatgctcaag cagaaccccc ggctccatca gcacagtgca ggaccaaacc      360
ccatgctgca gcagtggggc tgtctgtacg gggtgggcaa tgggaaccgg ggtctgctgg      420
ggctcctgct gcttcagtgc tgccatgcag ccacacatcc tgagagctga aagggtcggc      480
gtcctcacct ggtgcacacc gtagctctgc cccacagctt taaggcacct ggctaacctc      540
tgcgcttctt cccttccctc ctccctggct caggtggtgg tggatctccc cccgaggacc      600
cccctgactc caagaatacc ctggtgctgt tcggcgctgg cttcggcgcc gtgattaccg      660
tggtcgtgat cgtcgtgatt atcaagtgct tctgcaagca ccggtcctgc ttccggcgga      720
acgaggcctc cagagagaca aacaactccc tgaccttcgg ccccgaggaa gccctggctg      780
agcagaccgt gtttctgtga tgattcgaaa tgaccgacca agcgacgccc aacctgccat      840
cacgagattt cgattccacc gccgccttct atgaaaggtt gggcttcgga atcgttttcc      900
gggacgccgg ctggatgatc ctccagcgcg gggatctcat gctggagttc ttcgcccacc      960
ccaacttgtt tattgcagct tataatggtt acaaataaag caatagcatc acaaatttca     1020
caaataaagc atttttttca ctgcattcta gttgtggttt gtccaaactc atcaatgtat     1080
cttatcatgt ctgt                                                       1094
```

<210> 81
<211> 1097
<212> DNA
<213> Artificial Sequence

<220>
<223> GSC11213

<400> 81

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatctg gctctgatcc tgggacctgt gctggctctg ctggcactgg 660
tggctctggg agtgctggga ctgtggaagt gcttctgcaa gcaccggtcc tgcttccggc 720
ggaacgaggc ctccagagag acaaacaact ccctgacctt cggccccgag gaagccctgg 780
ctgagcagac cgtgtttctg tgatgattcg aaatgaccga ccaagcgacg cccaacctgc 840
catcacgaga tttcgattcc accgccgcct tctatgaaag gttgggcttc ggaatcgttt 900
tccgggacgc cggctggatg atcctccagc gcggggatct catgctggag ttcttcgccc 960
accccaactt gtttattgca gcttataatg gttacaaata aagcaatagc atcacaaatt 1020
tcacaaataa agcatttttt tcactgcatt ctagttgtgg tttgtccaaa ctcatcaatg 1080
tatcttatca tgtctgt 1097

<210> 82
<211> 1097
<212> DNA
<213> Artificial Sequence

<220>
<223> GSC11214

<400> 82

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatatc gccgccatca tcgtgatcct ggtgttgctc ctgctgctgg 660

gcatcggcct gatgtggtgg ttctggaagt gcttctgcaa gcaccggtcc tgcttccggc 720

ggaacgaggc ctccagagag acaaacaact ccctgacctt cggccccgag gaagccctgg 780

ctgagcagac cgtgtttctg tgatgattcg aaatgaccga ccaagcgacg cccaacctgc 840

catcacgaga tttcgattcc accgccgcct tctatgaaag gttgggcttc ggaatcgttt 900

tccgggacgc cggctggatg atcctccagc gcggggatct catgctggag ttcttcgccc 960

accccaactt gtttattgca gcttataatg gttacaaata aagcaatagc atcacaaatt 1020

tcacaaataa agcatttttt tcactgcatt ctagttgtgg tttgtccaaa ctcatcaatg 1080

tatcttatca tgtctgt 1097

<210> 83
<211> 1094
<212> DNA
<213> Artificial Sequence

<220>
<223> GSC11210

<400> 83

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg ggtgggcaa tgggaaccgg ggtctgctgg      420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatatg gccctgattg tgctgggcgg agtggctggc ctgctgctgt     660
ttatcggcct gggcatcttc ttcaagtgct tctgcaagca ccggtcctgc ttccggcgga     720
acgaggcctc cagagagaca aacaactccc tgaccttcgg ccccgaggaa gccctggctg     780
agcagaccgt gtttctgtga tgattcgaaa tgaccgacca agcgacgccc aacctgccat     840
cacgagattt cgattccacc gccgccttct atgaaaggtt gggcttcgga atcgttttcc     900
gggacgccgg ctggatgatc ctccagcgcg gggatctcat gctggagttc ttcgcccacc     960
ccaacttgtt tattgcagct tataatggtt acaaataaag caatagcatc acaaatttca    1020
caaataaagc atttttttca ctgcattcta gttgtggttt gtccaaactc atcaatgtat    1080
cttatcatgt ctgt                                                      1094
```

<210> 84
<211> 1094
<212> DNA
<213> Artificial Sequence

<220>
<223> GSC11205

<400> 84

ctgtctccgg gtaaatgatg aaagcttggt aggtgatcct cctgctgctt tggttcaggg 60

ttttgcttga gggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatatc gctggcgtga tcgctggcat cctgctgttc gtgatcatct 660

tcctgggcgt ggtgctcgtg atgaagtgct tctgcaagca ccggtcctgc ttccggcgga 720

acgaggcctc cagagagaca aacaactccc tgaccttcgg ccccgaggaa gccctggctg 780

agcagaccgt gtttctgtga tgattcgaaa tgaccgacca agcgacgccc aacctgccat 840

cacgagattt cgattccacc gccgccttct atgaaaggtt gggcttcgga atcgttttcc 900

gggacgccgg ctggatgatc ctccagcgcg gggatctcat gctggagttc ttcgcccacc 960

ccaacttgtt tattgcagct tataatggtt acaaataaag caatagcatc acaaatttca 1020

caaataaagc atttttttca ctgcattcta gttgtggttt gtccaaactc atcaatgtat 1080

cttatcatgt ctgt 1094

<210> 85
<211> 1094
<212> DNA
<213> Artificial Sequence

<220>
<223> GSC11217

<400> 85

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatgct ctggtcgtga tccccatcat cttcggcatc ctgttcgcca     660
tcctgctggt gctggtgttc atcaagtgct tctgcaagca ccggtcctgc ttccggcgga     720
acgaggcctc cagagagaca aacaactccc tgaccttcgg ccccgaggaa gccctggctg     780
agcagaccgt gtttctgtga tgattcgaaa tgaccgacca agcgacgccc aacctgccat     840
cacgagattt cgattccacc gccgccttct atgaaaggtt gggcttcgga atcgttttcc     900
gggacgccgg ctggatgatc ctccagcgcg gggatctcat gctggagttc ttcgcccacc     960
ccaacttgtt tattgcagct tataatggtt acaaataaag caatagcatc acaaatttca    1020
caaataaagc atttttttca ctgcattcta gttgtggttt gtccaaactc atcaatgtat    1080
cttatcatgt ctgt                                                       1094
```

<210> 86
<211> 1097
<212> DNA
<213> Artificial Sequence

<220>
<223> GSC11756

<400> 86

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
```

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatatc atccctatcg tggccggcgt ggtggctggc atcgtgctga 660

ttggactggc cctgctgctg atctggaagt gcttctgcaa gcaccggtcc tgcttccggc 720

ggaacgaggc ctccagagag acaaacaact ccctgacctt cggccccgag gaagccctgg 780

ctgagcagac cgtgtttctg tgatgattcg aaatgaccga ccaagcgacg cccaacctgc 840

catcacgaga tttcgattcc accgccgcct tctatgaaag gttgggcttc ggaatcgttt 900

tccgggacgc cggctggatg atcctccagc gcggggatct catgctggag ttcttcgccc 960

accccaactt gtttattgca gcttataatg gttacaaata aagcaatagc atcacaaatt 1020

tcacaaataa agcatttttt tcactgcatt ctagttgtgg tttgtccaaa ctcatcaatg 1080

tatcttatca tgtctgt 1097

<210> 87
<211> 1100
<212> DNA
<213> Artificial Sequence

<220>
<223> GSC11296

<400> 87

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatgga atcgtggctg gactggctgt gctggccgtg gtcgtgattg     660
gagctgtggt ggccgccgtg atgtgcagaa agtgcttctg caagcaccgg tcctgcttcc     720
ggcggaacga ggcctccaga gagacaaaca actccctgac cttcggcccc gaggaagccc     780
tggctgagca gaccgtgttt ctgtgatgat tcgaaatgac cgaccaagcg acgcccaacc     840
tgccatcacg agatttcgat tccaccgccg ccttctatga aaggttgggc ttcggaatcg     900
ttttccggga cgccggctgg atgatcctcc agcgcgggga tctcatgctg gagttcttcg     960
cccaccccaa cttgtttatt gcagcttata atggttacaa ataaagcaat agcatcacaa    1020
atttcacaaa taaagcattt ttttcactgc attctagttg tggtttgtcc aaactcatca    1080
atgtatctta tcatgtctgt                                                1100
```

<210> 88
<211> 1100
<212> DNA
<213> Artificial Sequence

<220>
<223> GSC11295

<400> 88

```
ctgtctccgg gtaaatgatg aaagcttggt aggtgatcct cctgctgctt tggttcaggg       60
ttttgcttga gggggggggg tggtgatttc cttgccatgg gcagactgag cagaaaaggc      120
cattgggacc atgttctgaa tgcctccacc tcaaccaccg gccggtagga ccaaagccac      180
cccgtgtttt ctcaggatct cttttcccag ggagatccct cggcccaaag agggagatgg      240
caatgctgga tgtgtgcaca ataattcaac aggcattgga acttcagcat cgatgctgaa      300
tgcaattaac aatgctcaag cagaaccccc ggctccatca gcacagtgca ggaccaaacc      360
ccatgctgca gcagtggggc tgtctgtacg gggtgggcaa tgggaaccgg ggtctgctgg      420
ggctcctgct gcttcagtgc tgccatgcag ccacacatcc tgagagctga aagggtcggc      480
gtcctcacct ggtgcacacc gtagctctgc cccacagctt taaggcacct ggctaacctc      540
tgcgcttctt cccttccctc ctccctggct caggtggtgg tggatctccc cccgaggacc      600
cccctgactc caagaatctg atcattgccc tgcctgtggc cgtgctgctg atcgtgggag      660
gcctcgtgat catgctgtac gtgttccaca agtgcttctg caagcaccgg tcctgcttcc      720
ggcggaacga ggcctccaga gagacaaaca actccctgac cttcggcccc gaggaagccc      780
tggctgagca gaccgtgttt ctgtgatgat tcgaaatgac cgaccaagcg acgcccaacc      840
tgccatcacg agatttcgat tccaccgccg ccttctatga aaggttgggc ttcggaatcg      900
ttttccggga cgccggctgg atgatcctcc agcgcgggga tctcatgctg gagttcttcg      960
cccaccccaa cttgtttatt gcagcttata atggttacaa ataaagcaat agcatcacaa     1020
atttcacaaa taaagcattt ttttcactgc attctagttg tggtttgtcc aaactcatca     1080
atgtatctta tcatgtctgt                                                 1100
```

<210> 89
<211> 1100
<212> DNA
<213> Artificial Sequence

<220>
<223> GSC11390

<400> 89

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatggc ctgctgatcg gcatctccat cgcctccctg tgcctggtgg 660
tggccctgct ggccctgctg tgccacctga agtgcttctg caagcaccgg tcctgcttcc 720
ggcggaacga ggcctccaga gagacaaaca actccctgac cttcggcccc gaggaagccc 780
tggctgagca gaccgtgttt ctgtgatgat tcgaaatgac cgaccaagcg acgcccaacc 840
tgccatcacg agatttcgat tccaccgccg ccttctatga aaggttgggc ttcggaatcg 900
ttttccggga cgccggctgg atgatcctcc agcgcgggga tctcatgctg gagttcttcg 960
cccaccccaa cttgtttatt gcagcttata atggttacaa ataaagcaat agcatcacaa 1020
atttcacaaa taaagcattt ttttcactgc attctagttg tggtttgtcc aaactcatca 1080
atgtatctta tcatgtctgt 1100

<210> 90
<211> 1100
<212> DNA
<213> Artificial Sequence

<220>
<223> GSC11391

<400> 90

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120

cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg ggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatctg ctgctgggcg tgtccgtgtc ctgcatcgtg atcctggccg 660
tgtgcctgct gtgctacgtg tccatcacca agtgcttctg caagcaccgg tcctgcttcc 720
ggcggaacga ggcctccaga gagacaaaca actccctgac cttcggcccc gaggaagccc 780
tggctgagca gaccgtgttt ctgtgatgat tcgaaatgac cgaccaagcg acgcccaacc 840
tgccatcacg agatttcgat tccaccgccg ccttctatga aaggttgggc ttcggaatcg 900
ttttccggga cgccggctgg atgatcctcc agcgcgggga tctcatgctg gagttcttcg 960
cccaccccaa cttgtttatt gcagcttata atggttacaa ataaagcaat agcatcacaa 1020
atttcacaaa taaagcattt ttttcactgc attctagttg tggtttgtcc aaactcatca 1080
atgtatctta tcatgtctgt 1100

<210> 91
<211> 1097
<212> DNA
<213> Artificial Sequence

<220>
<223> GSC11392

<400> 91

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc aacaccgtgg gctccgtgat cggcgtgatc gtgaccatct 660
tcgtgtccgg caccgtgtac ttcatcaagt gcttctgcaa gcaccggtcc tgcttccggc 720
ggaacgaggc ctccagagag acaaacaact ccctgacctt cggccccgag gaagccctgg 780
ctgagcagac cgtgtttctg tgatgattcg aaatgaccga ccaagcgacg cccaacctgc 840
catcacgaga tttcgattcc accgccgcct tctatgaaag gttgggcttc ggaatcgttt 900
tccgggacgc cggctggatg atcctccagc gcggggatct catgctggag ttcttcgccc 960
accccaactt gtttattgca gcttataatg gttacaaata aagcaatagc atcacaaatt 1020
tcacaaataa agcatttttt tcactgcatt ctagttgtgg tttgtccaaa ctcatcaatg 1080
tatcttatca tgtctgt 1097

<210> 92
<211> 1097
<212> DNA
<213> Artificial Sequence

<220>
<223> GSC11397

<400> 92

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatatc accctgatca tcttcggcgt gatggccggc gtgatcggca     660
ccatcctgct gatctcctac ggcatcaagt gcttctgcaa gcaccggtcc tgcttccggc     720
ggaacgaggc ctccagagag acaaacaact ccctgacctt cggccccgag gaagccctgg     780
ctgagcagac cgtgtttctg tgatgattcg aaatgaccga ccaagcgacg cccaacctgc     840
catcacgaga tttcgattcc accgccgcct tctatgaaag gttgggcttc ggaatcgttt     900
tccgggacgc cggctggatg atcctccagc gcggggatct catgctggag ttcttcgccc     960
accccaactt gtttattgca gcttataatg gttacaaata aagcaatagc atcacaaatt    1020

tcacaaataa agcatttttt tcactgcatt ctagttgtgg tttgtccaaa ctcatcaatg    1080
tatcttatca tgtctgt                                                   1097
```

<210> 93
<211> 1091
<212> DNA
<213> Artificial Sequence

<220>
<223> GSC11204

<400> 93

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatgcc ctgtggctgg gcgtgctgag actgctgctg ttcaagctgc     660
tcctgttcga cctgctcctc aagtgcttct gcaagcaccg gtcctgcttc cggcggaacg     720
aggcctccag agagacaaac aactccctga ccttcggccc cgaggaagcc ctggctgagc     780
agaccgtgtt tctgtgatga ttcgaaatga ccgaccaagc gacgcccaac ctgccatcac     840
gagatttcga ttccaccgcc gccttctatg aaaggttggg cttcggaatc gttttccggg     900
acgccggctg gatgatcctc cagcgcgggg atctcatgct ggagttcttc gcccacccca     960
acttgtttat tgcagcttat aatggttaca aataaagcaa tagcatcaca aatttcacaa    1020
ataaagcatt tttttcactg cattctagtt gtggtttgtc caaactcatc aatgtatctt    1080
atcatgtctg t                                                         1091
```

<210> 94
<211> 1091
<212> DNA
<213> Artificial Sequence

<220>
<223> GSC11216

<400> 94

```
ctgtctccgg gtaaatgatg aaagcttggt aggtgatcct cctgctgctt tggttcaggg      60
```

```
ttttgcttga gggggggggg tggtgatttc cttgccatgg gcagactgag cagaaaaggc      120
cattgggacc atgttctgaa tgcctccacc tcaaccaccg gccggtagga ccaaagccac      180
cccgtgtttt ctcaggatct cttttcccag ggagatccct cggcccaaag agggagatgg      240
caatgctgga tgtgtgcaca ataattcaac aggcattgga acttcagcat cgatgctgaa      300
tgcaattaac aatgctcaag cagaaccccc ggctccatca gcacagtgca ggaccaaacc      360
ccatgctgca gcagtggggc tgtctgtacg gggtgggcaa tgggaaccgg ggtctgctgg      420
ggctcctgct gcttcagtgc tgccatgcag ccacacatcc tgagagctga aagggtcggc      480
gtcctcacct ggtgcacacc gtagctctgc cccacagctt taaggcacct ggctaacctc      540
tgcgcttctt cccttccctc ctccctggct caggtggtgg tggatctccc cccgaggacc      600
cccctgactc caagaatgcc ctgtggctgg gcgtgctggt actgctgctg ttcaagctgc      660
tcctgttcga cctgctcctc aagtgcttct gcaagcaccg gtcctgcttc cggcggaacg      720
aggcctccag agagacaaac aactccctga ccttcggccc cgaggaagcc ctggctgagc      780
agaccgtgtt tctgtgatga ttcgaaatga ccgaccaagc gacgcccaac ctgccatcac      840
gagatttcga ttccaccgcc gccttctatg aaaggttggg cttcggaatc gttttccggg      900
acgccggctg gatgatcctc cagcgcgggg atctcatgct ggagttcttc gcccacccca      960
acttgtttat tgcagcttat aatggttaca aataaagcaa tagcatcaca aatttcacaa     1020
ataaagcatt tttttcactg cattctagtt gtggtttgtc caaactcatc aatgtatctt     1080
atcatgtctg t                                                          1091
```

<210> 95
<211> 1091
<212> DNA
<213> Artificial Sequence

<220>
<223> GSC11203

<400> 95

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc ctgtggctgg gcgtgctgag actgctgctg ttcgtgctgc 660
tcctgttcga cctgctcctc aagtgcttct gcaagcaccg gtcctgcttc cggcggaacg 720
aggcctccag agagacaaac aactccctga ccttcggccc cgaggaagcc ctggctgagc 780
agaccgtgtt tctgtgatga ttcgaaatga ccgaccaagc gacgcccaac ctgccatcac 840
gagatttcga ttccaccgcc gccttctatg aaaggttggg cttcggaatc gttttccggg 900
acgccggctg gatgatcctc cagcgcgggg atctcatgct ggagttcttc gcccacccca 960
acttgtttat tgcagcttat aatggttaca aataaagcaa tagcatcaca aatttcacaa 1020
ataaagcatt tttttcactg cattctagtt gtggtttgtc caaactcatc aatgtatctt 1080
atcatgtctg t 1091

<210> 96
<211> 1091
<212> DNA
<213> Artificial Sequence

<220>
<223> GSC11211

<400> 96

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc ctgtggctgg gcgtgctgag actgctgctg ttcaagctgc 660
tcctgttcgt cctgctcctc aagtgcttct gcaagcaccg gtcctgcttc cggcggaacg 720
aggcctccag agagacaaac aactccctga ccttcggccc cgaggaagcc ctggctgagc 780
agaccgtgtt tctgtgatga ttcgaaatga ccgaccaagc gacgcccaac ctgccatcac 840
gagatttcga ttccaccgcc gccttctatg aaaggttggg cttcggaatc gttttccggg 900
acgccggctg gatgatcctc cagcgcgggg atctcatgct ggagttcttc gcccacccca 960
acttgtttat tgcagcttat aatggttaca aataaagcaa tagcatcaca aatttcacaa 1020
ataaagcatt tttttcactg cattctagtt gtggtttgtc caaactcatc aatgtatctt 1080
atcatgtctg t 1091

<210> 97
<211> 1091
<212> DNA
<213> Artificial Sequence

<220>
<223> GSC11212

<400> 97

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc ctgtggctgg gcgtgctggt actgctgctg ttcgtgctgc 660
tcctgttcga cctgctcctc aagtgcttct gcaagcaccg gtcctgcttc cggcggaacg 720
aggcctccag agagacaaac aactccctga ccttcggccc cgaggaagcc ctggctgagc 780
agaccgtgtt tctgtgatga ttcgaaatga ccgaccaagc gacgcccaac ctgccatcac 840
gagatttcga ttccaccgcc gccttctatg aaaggttggg cttcggaatc gttttccggg 900
acgccggctg gatgatcctc cagcgcgggg atctcatgct ggagttcttc gcccacccca 960
acttgtttat tgcagcttat aatggttaca aataaagcaa tagcatcaca aatttcacaa 1020
ataaagcatt tttttcactg cattctagtt gtggtttgtc caaactcatc aatgtatctt 1080
atcatgtctg t 1091

<210> 98
<211> 1091
<212> DNA
<213> Artificial Sequence

<220>
<223> GSC11398

<400> 98

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatgcc ctgtggctgg gcgtgctggt actgctgctg ttcaagctgc     660
tcctgttcgt cctgctcctc aagtgcttct gcaagcaccg gtcctgcttc cggcggaacg     720
aggcctccag agagacaaac aactccctga ccttcggccc cgaggaagcc ctggctgagc     780
agaccgtgtt tctgtgatga ttcgaaatga ccgaccaagc gacgcccaac ctgccatcac     840
gagatttcga ttccaccgcc gccttctatg aaaggttggg cttcggaatc gttttccggg     900
acgccggctg gatgatcctc cagcgcgggg atctcatgct ggagttcttc gcccacccca     960
acttgtttat tgcagcttat aatggttaca aataaagcaa tagcatcaca aatttcacaa    1020
ataaagcatt tttttcactg cattctagtt gtggtttgtc caaactcatc aatgtatctt    1080
atcatgtctg t                                                          1091
```

<210> 99
<211> 1091
<212> DNA
<213> Artificial Sequence

<220>
<223> GSC11208

<400> 99

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc ctgtggctgg gcgtgctgag actgctgctg ttcgtgctgc 660
tcctgttcgt cctgctcctc aagtgcttct gcaagcaccg gtcctgcttc cggcggaacg 720
aggcctccag agagacaaac aactccctga ccttcggccc cgaggaagcc ctggctgagc 780
agaccgtgtt tctgtgatga ttcgaaatga ccgaccaagc gacgcccaac ctgccatcac 840
gagatttcga ttccaccgcc gccttctatg aaaggttggg cttcggaatc gttttccggg 900
acgccggctg gatgatcctc cagcgcgggg atctcatgct ggagttcttc gcccacccca 960
acttgtttat tgcagcttat aatggttaca aataaagcaa tagcatcaca aatttcacaa 1020
ataaagcatt tttttcactg cattctagtt gtggtttgtc caaactcatc aatgtatctt 1080
atcatgtctg t 1091

<210> 100
<211> 1091
<212> DNA
<213> Artificial Sequence

<220>
<223> GSC11291

<400> 100

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg ggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc ctgtggctgg gcgtgctggt actgctgctg ttcgtgctgc 660
tcctgttcgt cctgctcctc aagtgcttct gcaagcaccg gtcctgcttc cggcggaacg 720
aggcctccag agagacaaac aactccctga ccttcggccc cgaggaagcc ctggctgagc 780
agaccgtgtt tctgtgatga ttcgaaatga ccgaccaagc gacgcccaac ctgccatcac 840

gagatttcga ttccaccgcc gccttctatg aaaggttggg cttcggaatc gttttccggg 900
acgccggctg gatgatcctc cagcgcgggg atctcatgct ggagttcttc gcccacccca 960
acttgtttat tgcagcttat aatggttaca aataaagcaa tagcatcaca aatttcacaa 1020
ataaagcatt tttttcactg cattctagtt gtggtttgtc caaactcatc aatgtatctt 1080
atcatgtctg t 1091

<210> 101
<211> 1085
<212> DNA
<213> Artificial Sequence

<220>
<223> GSC11297

<400> 101

```
ctgtctccgg gtaaatgatg aaagcttggt aggtgatcct cctgctgctt tggttcaggg    60
ttttgcttga gggggggggg tggtgatttc cttgccatgg gcagactgag cagaaaaggc   120
cattgggacc atgttctgaa tgcctccacc tcaaccaccg gccggtagga ccaaagccac   180
cccgtgtttt ctcaggatct cttttcccag ggagatccct cggcccaaag agggagatgg   240
caatgctgga tgtgtgcaca ataattcaac aggcattgga acttcagcat cgatgctgaa   300
tgcaattaac aatgctcaag cagaaccccc ggctccatca gcacagtgca ggaccaaacc   360
ccatgctgca gcagtggggc tgtctgtacg gggtgggcaa tgggaaccgg ggtctgctgg   420
ggctcctgct gcttcagtgc tgccatgcag ccacacatcc tgagagctga aagggtcggc   480
gtcctcacct ggtgcacacc gtagctctgc cccacagctt taaggcacct ggctaacctc   540
tgcgcttctt cccttccctc ctccctggct caggtggtgg tggatctccc cccgaggacc   600
cccctgactc caagaatttc ttcatccccc tgctggtggt gatcctgttc gccgtggaca   660
ccggcctgtt catcaagtgc ttctgcaagc accggtcctg cttccggcgg aacgaggcct   720
ccagagagac aaacaactcc ctgaccttcg gccccgagga agccctggct gagcagaccg   780
tgtttctgtg atgattcgaa atgaccgacc aagcgacgcc caacctgcca tcacgagatt   840
tcgattccac cgccgccttc tatgaaaggt tgggcttcgg aatcgttttc cgggacgccg   900
gctggatgat cctccagcgc ggggatctca tgctggagtt cttcgcccac cccaacttgt   960
ttattgcagc ttataatggt tacaaataaa gcaatagcat cacaaatttc acaaataaag  1020
catttttttc actgcattct agttgtggtt tgtccaaact catcaatgta tcttatcatg  1080
tctgt                                                              1085
```

<210> 102
<211> 1085
<212> DNA
<213> Artificial Sequence

<220>
<223> GSC11298

<400> 102

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatatc ctgatcggca cctccgtggt gatcatcctg ttcatcctgc     660
tgttcttcct gctgaagtgc ttctgcaagc accggtcctg cttccggcgg aacgaggcct     720
ccagagagac aaacaactcc ctgaccttcg gccccgagga agccctggct gagcagaccg     780
tgtttctgtg atgattcgaa atgaccgacc aagcgacgcc caacctgcca tcacgagatt     840
tcgattccac cgccgccttc tatgaaaggt tgggcttcgg aatcgttttc cgggacgccg     900
gctggatgat cctccagcgc ggggatctca tgctggagtt cttcgcccac cccaacttgt     960
ttattgcagc ttataatggt tacaaataaa gcaatagcat cacaaatttc acaaataaag    1020
catttttttc actgcattct agttgtggtt tgtccaaact catcaatgta tcttatcatg    1080
tctgt                                                                1085
```

<210> 103
<211> 1079
<212> DNA
<213> Artificial Sequence

<220>
<223> GSC11393

<400> 103

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
```

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatgtg ctggccctga tcgtgatctt cctgaccatc gccgtgctgc 660

tggccctgaa gtgcttctgc aagcaccggt cctgcttccg gcggaacgag gcctccagag 720

agacaaacaa ctccctgacc ttcggccccg aggaagccct ggctgagcag accgtgtttc 780

tgtgatgatt cgaaatgacc gaccaagcga cgcccaacct gccatcacga gatttcgatt 840

ccaccgccgc cttctatgaa aggttgggct tcggaatcgt tttccgggac gccggctgga 900

tgatcctcca gcgcggggat ctcatgctgg agttcttcgc ccaccccaac ttgtttattg 960

cagcttataa tggttacaaa taaagcaata gcatcacaaa tttcacaaat aaagcatttt 1020

tttcactgca ttctagttgt ggtttgtcca aactcatcaa tgtatcttat catgtctgt 1079

<210> 104
<211> 1106
<212> DNA
<213> Artificial Sequence

<220>
<223> GSC11394

<400> 104

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgtg atgtccgtgg ccaccatcgt gatcgtggac atctgcatca 660
ccggcggcct gctgctgctg gtgtactact ggtccaagtg cttctgcaag caccggtcct 720
gcttccggcg gaacgaggcc tccagagaga caaacaactc cctgaccttc ggccccgagg 780
aagccctggc tgagcagacc gtgtttctgt gatgattcga aatgaccgac caagcgacgc 840

ccaacctgcc atcacgagat ttcgattcca ccgccgcctt ctatgaaagg ttgggcttcg 900
gaatcgtttt ccgggacgcc ggctggatga tcctccagcg cggggatctc atgctggagt 960
tcttcgccca ccccaacttg tttattgcag cttataatgg ttacaaataa agcaatagca 1020
tcacaaattt cacaaataaa gcattttttt cactgcattc tagttgtggt ttgtccaaac 1080
tcatcaatgt atcttatcat gtctgt 1106

<210> 105
<211> 1106
<212> DNA
<213> Artificial Sequence

<220>
<223> GSC11395

<400> 105

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatgcc atccccatct ggtgggtgct ggtgggcgtg ctgggcggcc 660
tgctgctgct gaccatcctg gtgctggcca tgtggaagtg cttctgcaag caccggtcct 720
gcttccggcg gaacgaggcc tccagagaga caaacaactc cctgaccttc ggccccgagg 780
aagccctggc tgagcagacc gtgtttctgt gatgattcga aatgaccgac caagcgacgc 840
ccaacctgcc atcacgagat ttcgattcca ccgccgcctt ctatgaaagg ttgggcttcg 900
gaatcgtttt ccgggacgcc ggctggatga tcctccagcg cggggatctc atgctggagt 960
tcttcgccca ccccaacttg tttattgcag cttataatgg ttacaaataa agcaatagca 1020
tcacaaattt cacaaataaa gcattttttt cactgcattc tagttgtggt ttgtccaaac 1080
tcatcaatgt atcttatcat gtctgt 1106

<210> 106
<211> 1109
<212> **DNA**
<213> Artificial Sequence

<220>
<223> GSC11396

<400> 106

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatttc tgggtgctgg tggtggtggg cggcgtgctg gcctgctact     660
ccctgctggt gaccgtggcc ttcatcatct tctgggtgaa gtgcttctgc aagcaccggt     720
cctgcttccg gcggaacgag gcctccagag agacaaacaa ctccctgacc ttcggccccg     780
aggaagccct ggctgagcag accgtgtttc tgtgatgatt cgaaatgacc gaccaagcga     840
cgcccaacct gccatcacga gatttcgatt ccaccgccgc cttctatgaa aggttgggct     900
tcggaatcgt tttccgggac gccggctgga tgatcctcca gcgcggggat ctcatgctgg     960
agttcttcgc ccaccccaac ttgtttattg cagcttataa tggttacaaa taaagcaata    1020
gcatcacaaa tttcacaaat aaagcatttt tttcactgca ttctagttgt ggtttgtcca    1080
aactcatcaa tgtatcttat catgtctgt                                      1109
```

<210> 107
<211> 1109
<212> DNA
<213> Artificial Sequence

<220>
<223> GSC11389

<400> 107

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
```

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatctg tggacgacca tcaccatctt catcacactc ttcctgttaa 660

gcgtgtgcta cagtgccacc gtcaccttct tcaaggtgaa gtgcttctgc aagcaccggt 720

cctgcttccg gcggaacgag gcctccagag agacaaacaa ctccctgacc ttcggccccg 780

aggaagccct ggctgagcag accgtgtttc tgtgatgatt cgaaatgacc gaccaagcga 840

cgcccaacct gccatcacga gatttcgatt ccaccgccgc cttctatgaa aggttgggct 900

tcggaatcgt tttccgggac gccggctgga tgatcctcca gcgcggggat ctcatgctgg 960

agttcttcgc ccaccccaac ttgtttattg cagcttataa tggttacaaa taaagcaata 1020

gcatcacaaa tttcacaaat aaagcatttt tttcactgca ttctagttgt ggtttgtcca 1080

aactcatcaa tgtatcttat catgtctgt 1109

<210> 108
<211> 1082
<212> **DNA**
<213> Artificial Sequence

<220>
<223> GSC11677

<400> 108

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc accgtgatcg 660
tcgtgattat caagtgcttc tgcaagcacc ggtcctgctt ccggcggaac gaggcctcca 720
gagagacaaa caactccctg accttcggcc ccgaggaagc cctggctgag cagaccgtgt 780
ttctgtgatg attcgaaatg accgaccaag cgacgcccaa cctgccatca cgagatttcg 840
attccaccgc cgccttctat gaaaggttgg gcttcggaat cgttttccgg gacgccggct 900
ggatgatcct ccagcgcggg gatctcatgc tggagttctt cgcccacccc aacttgttta 960
ttgcagctta taatggttac aaataaagca atagcatcac aaatttcaca aataaagcat 1020
tttttcact gcattctagt tgtggtttgt ccaaactcat caatgtatct tatcatgtct 1080
gt 1082

<210> 109
<211> 1088
<212> DNA
<213> Artificial Sequence

<220>
<223> GSC11679

<400> 109

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc accgtggtcg 660
tgatcgtcgt gattatcaag tgcttctgca agcaccggtc ctgcttccgg cggaacgagg 720
cctccagaga gacaaacaac tccctgacct tcggccccga ggaagccctg gctgagcaga 780
ccgtgtttct gtgatgattc gaaatgaccg accaagcgac gcccaacctg ccatcacgag 840
atttcgattc caccgccgcc ttctatgaaa ggttgggctt cggaatcgtt ttccgggacg 900
ccggctggat gatcctccag cgcggggatc tcatgctgga gttcttcgcc caccccaact 960
tgtttattgc agcttataat ggttacaaat aaagcaatag catcacaaat ttcacaaata 1020
aagcattttt ttcactgcat tctagttgtg gtttgtccaa actcatcaat gtatcttatc 1080
atgtctgt 1088

<210> 110
<211> 1100
<212> **DNA**
<213> Artificial Sequence

<220>
<223> GSC11484

<400> 110

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtggtcattg 660
taaccgtggt cgtgatcgtc gtgattatca agtgcttctg caagcaccgg tcctgcttcc 720
ggcggaacga ggcctccaga gagacaaaca actccctgac cttcggcccc gaggaagccc 780
tggctgagca gaccgtgttt ctgtgatgat tcgaaatgac cgaccaagcg acgcccaacc 840
tgccatcacg agatttcgat tccaccgccg ccttctatga aaggttgggc ttcggaatcg 900
ttttccggga cgccggctgg atgatcctcc agcgcgggga tctcatgctg gagttcttcg 960
cccaccccaa cttgtttatt gcagcttata atggttacaa ataaagcaat agcatcacaa 1020
atttcacaaa taaagcattt ttttcactgc attctagttg tggtttgtcc aaactcatca 1080
atgtatctta tcatgtctgt 1100

<210> 111
<211> 1106
<212> DNA
<213> Artificial Sequence

<220>
<223> GSC11292

<400> 111

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg ggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatacc ctggtgctgt tcggcgctgg cttcggcgcc gtggtcattg 660

tagtcgtaac cgtggtcgtg atcgtcgtga ttatcaagtg cttctgcaag caccggtcct 720

gcttccggcg gaacgaggcc tccagagaga caaacaactc cctgaccttc ggccccgagg 780

aagccctggc tgagcagacc gtgtttctgt gatgattcga aatgaccgac caagcgacgc 840

ccaacctgcc atcacgagat ttcgattcca ccgccgcctt ctatgaaagg ttgggcttcg 900

gaatcgtttt ccgggacgcc ggctggatga tcctccagcg cggggatctc atgctggagt 960

tcttcgccca ccccaacttg tttattgcag cttataatgg ttacaaataa agcaatagca 1020

tcacaaattt cacaaataaa gcattttttt cactgcattc tagttgtggt ttgtccaaac 1080

tcatcaatgt atcttatcat gtctgt 1106

<210> 112
<211> 1007
<212> DNA
<213> Artificial Sequence

<220>
<223> GSC11207

<400> 112

ctgtctccgg gtaaatgatg aaagcttggt aggtgatcct cctgctgctt tggttcaggg 60

ttttgcttga ggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatacc ctggtgctgt tcggcgctgg cttcggcgcc gtgattaccg 660

tggtcgtgat cgtcgtgatt atcgccagcg cccatcacca ccaccatcat tgatgattcg 720

aaatgaccga ccaagcgacg cccaacctgc catcacgaga tttcgattcc accgccgcct 780

tctatgaaag gttgggcttc ggaatcgttt tccgggacgc cggctggatg atcctccagc 840

gcggggatct catgctggag ttcttcgccc accccaactt gtttattgca gcttataatg 900

gttacaaata aagcaatagc atcacaaatt tcacaaataa agcatttttt tcactgcatt 960

ctagttgtgg tttgtccaaa ctcatcaatg tatcttatca tgtctgt 1007

<210> 113
<211> 995
<212> DNA
<213> Artificial Sequence

<220>
<223> GSC11209

<400> 113

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atcaagtgct tctgcaagtg atgattcgaa atgaccgacc     720
aagcgacgcc caacctgcca tcacgagatt tcgattccac cgccgccttc tatgaaaggt     780
tgggcttcgg aatcgttttc cgggacgccg gctggatgat cctccagcgc ggggatctca     840
tgctggagtt cttcgcccac cccaacttgt ttattgcagc ttataatggt tacaaataaa     900
gcaatagcat cacaaatttc acaaataaag catttttttc actgcattct agttgtggtt     960
tgtccaaact catcaatgta tcttatcatg tctgt                                995
```

<210> 114
<211> 1058
<212> **DNA**
<213> Artificial Sequence

<220>
<223> GSC11758

<400> 114

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atcaagtgga tcttctcctc ggtggtggac ctgaagcaga     720
ccatcatccc cgactacagg aacatgatcg gacagggggc ctagtgattc gaaatgaccg     780
accaagcgac gcccaacctg ccatcacgag atttcgattc caccgccgcc ttctatgaaa     840
ggttgggctt cggaatcgtt ttccgggacg ccggctggat gatcctccag cgcggggatc     900
tcatgctgga gttcttcgcc caccccaact tgtttattgc agcttataat ggttacaaat     960
aaagcaatag catcacaaat ttcacaaata aagcattttt ttcactgcat tctagttgtg    1020
gtttgtccaa actcatcaat gtatcttatc atgtctgt                            1058
```

<210> 115
<211> 1016
<212> PRT
<213> Artificial Sequence

<220>
<223> GSC11764

<400> 115

```
Cys Thr Gly Thr Cys Thr Cys Cys Gly Gly Gly Thr Ala Ala Ala Thr
1               5                   10                  15

Gly Ala Thr Gly Ala Ala Ala Gly Cys Thr Thr Gly Gly Thr Ala Gly
            20                  25                  30

Gly Thr Gly Ala Thr Cys Cys Thr Cys Cys Thr Gly Cys Thr Gly Cys
        35                  40                  45
```

```
Thr Thr Thr Gly Gly Thr Thr Cys Ala Gly Gly Gly Thr Thr Thr Thr
    50                  55                  60

Gly Cys Thr Thr Gly Ala Gly Gly Gly Gly Gly Gly Gly Gly Gly Gly
65                  70                  75                  80

Thr Gly Gly Thr Gly Ala Thr Thr Thr Cys Cys Thr Thr Gly Cys Cys
                85                  90                  95

Ala Thr Gly Gly Gly Cys Ala Gly Ala Cys Thr Gly Ala Gly Cys Ala
            100                 105                 110

Gly Ala Ala Ala Ala Gly Gly Cys Cys Ala Thr Thr Gly Gly Gly Ala
        115                 120                 125

Cys Cys Ala Thr Gly Thr Thr Cys Thr Gly Ala Ala Thr Gly Cys Cys
    130                 135                 140

Thr Cys Cys Ala Cys Cys Thr Cys Ala Ala Cys Cys Ala Cys Cys Gly
145                 150                 155                 160

Gly Cys Cys Gly Gly Thr Ala Gly Gly Ala Cys Cys Ala Ala Ala Gly
                165                 170                 175

Cys Cys Ala Cys Cys Cys Cys Gly Thr Gly Thr Thr Thr Thr Cys Thr
            180                 185                 190

Cys Ala Gly Gly Ala Thr Cys Thr Cys Thr Thr Thr Thr Cys Cys Cys
        195                 200                 205

Ala Gly Gly Gly Ala Gly Ala Thr Cys Cys Cys Thr Cys Gly Gly Cys
    210                 215                 220

Cys Cys Ala Ala Ala Gly Ala Gly Gly Gly Ala Gly Ala Thr Gly Gly
225                 230                 235                 240

Cys Ala Ala Thr Gly Cys Thr Gly Gly Ala Thr Gly Thr Gly Thr Gly
                245                 250                 255

Cys Ala Cys Ala Ala Thr Ala Ala Thr Thr Cys Ala Ala Cys Ala Gly
            260                 265                 270

Gly Cys Ala Thr Thr Gly Gly Ala Ala Cys Thr Thr Cys Ala Gly Cys
        275                 280                 285

Ala Thr Cys Gly Ala Thr Gly Cys Thr Gly Ala Ala Thr Gly Cys Ala
    290                 295                 300
```

```
Ala Thr Thr Ala Ala Cys Ala Ala Thr Gly Cys Thr Cys Ala Ala Gly
305                 310                 315                 320

Cys Ala Gly Ala Ala Cys Cys Cys Cys Cys Gly Gly Cys Thr Cys Cys
                325                 330                 335

Ala Thr Cys Ala Gly Cys Ala Cys Ala Gly Thr Gly Cys Ala Gly Gly
            340                 345                 350

Ala Cys Cys Ala Ala Ala Cys Cys Cys Cys Ala Thr Gly Cys Thr Gly
        355                 360                 365

Cys Ala Gly Cys Ala Gly Thr Gly Gly Gly Gly Cys Thr Gly Thr Cys
    370                 375                 380

Thr Gly Thr Ala Cys Gly Gly Gly Gly Thr Gly Gly Gly Cys Ala Ala
385                 390                 395                 400

Thr Gly Gly Gly Ala Ala Cys Cys Gly Gly Gly Gly Thr Cys Thr Gly
                405                 410                 415

Cys Thr Gly Gly Gly Gly Cys Thr Cys Cys Thr Gly Cys Thr Gly Cys
            420                 425                 430

Thr Thr Cys Ala Gly Thr Gly Cys Thr Gly Cys Cys Ala Thr Gly Cys
        435                 440                 445

Ala Gly Cys Cys Ala Cys Ala Cys Ala Thr Cys Cys Thr Gly Ala Gly
    450                 455                 460

Ala Gly Cys Thr Gly Ala Ala Ala Gly Gly Gly Thr Cys Gly Gly Cys
465                 470                 475                 480

Gly Thr Cys Cys Thr Cys Ala Cys Cys Thr Gly Gly Thr Gly Cys Ala
                485                 490                 495

Cys Ala Cys Cys Gly Thr Ala Gly Cys Thr Cys Thr Gly Cys Cys Cys
            500                 505                 510

Cys Ala Cys Ala Gly Cys Thr Thr Thr Ala Ala Gly Gly Cys Ala Cys
        515                 520                 525

Cys Thr Gly Gly Cys Thr Ala Ala Cys Cys Thr Cys Thr Gly Cys Gly
    530                 535                 540

Cys Thr Thr Cys Thr Thr Cys Cys Cys Thr Thr Cys Cys Cys Thr Cys
545                 550                 555                 560
```

Cys Thr Cys Cys Cys Thr Gly Gly Cys Thr Cys Ala Gly Gly Thr Gly
565 570 575

Gly Thr Gly Gly Thr Gly Gly Ala Thr Cys Thr Cys Cys Cys Cys Cys
580 585 590

Cys Gly Ala Gly Gly Ala Cys Cys Cys Cys Cys Cys Thr Gly Ala Cys
595 600 605

Thr Cys Cys Ala Ala Gly Ala Ala Thr Ala Cys Cys Cys Thr Gly Gly
610 615 620

Thr Gly Cys Thr Gly Thr Thr Cys Gly Gly Cys Gly Cys Thr Gly Gly
625 630 635 640

Cys Thr Thr Cys Gly Gly Cys Gly Cys Cys Gly Thr Gly Ala Thr Thr
645 650 655

Ala Cys Cys Gly Thr Gly Gly Thr Cys Gly Thr Gly Ala Thr Cys Gly
660 665 670

Thr Cys Gly Thr Gly Ala Thr Thr Ala Thr Cys Ala Gly Gly Thr Cys
675 680 685

Cys Cys Ala Gly Cys Ala Gly Gly Ala Gly Gly Cys Cys Gly Cys Cys
690 695 700

Gly Cys Cys Ala Ala Gly Ala Ala Gly Thr Thr Cys Thr Thr Cys Thr
705 710 715 720

Gly Ala Thr Gly Ala Thr Thr Cys Gly Ala Ala Ala Thr Gly Ala Cys
725 730 735

Cys Gly Ala Cys Cys Ala Ala Gly Cys Gly Ala Cys Gly Cys Cys Cys
740 745 750

Ala Ala Cys Cys Thr Gly Cys Cys Ala Thr Cys Ala Cys Gly Ala Gly
755 760 765

Ala Thr Thr Thr Cys Gly Ala Thr Thr Cys Cys Ala Cys Cys Gly Cys
770 775 780

Cys Gly Cys Cys Thr Thr Cys Thr Ala Thr Gly Ala Ala Ala Gly Gly
785 790 795 800

Thr Thr Gly Gly Gly Cys Thr Thr Cys Gly Gly Ala Ala Thr Cys Gly

```
                805                 810                 815

Thr Thr Thr Thr Cys Cys Gly Gly Gly Ala Cys Gly Cys Cys Gly Gly
            820                 825                 830

Cys Thr Gly Gly Ala Thr Gly Ala Thr Cys Cys Thr Cys Cys Ala Gly
        835                 840                 845

Cys Gly Cys Gly Gly Gly Gly Ala Thr Cys Thr Cys Ala Thr Gly Cys
    850                 855                 860

Thr Gly Gly Ala Gly Thr Thr Cys Thr Thr Cys Gly Cys Cys Cys Ala
865                 870                 875                 880

Cys Cys Cys Cys Ala Ala Cys Thr Thr Gly Thr Thr Thr Ala Thr Thr
                885                 890                 895

Gly Cys Ala Gly Cys Thr Thr Ala Thr Ala Ala Thr Gly Gly Thr Thr
            900                 905                 910

Ala Cys Ala Ala Ala Thr Ala Ala Ala Gly Cys Ala Ala Thr Ala Gly
        915                 920                 925

Cys Ala Thr Cys Ala Cys Ala Ala Ala Thr Thr Thr Cys Ala Cys Ala
    930                 935                 940

Ala Ala Thr Ala Ala Ala Gly Cys Ala Thr Thr Thr Thr Thr Thr Thr
945                 950                 955                 960

Cys Ala Cys Thr Gly Cys Ala Thr Thr Cys Thr Ala Gly Thr Thr Gly
                965                 970                 975

Thr Gly Gly Thr Thr Thr Gly Thr Cys Cys Ala Ala Ala Cys Thr Cys
            980                 985                 990

Ala Thr Cys Ala Ala Thr Gly Thr  Ala Thr Cys Thr Thr  Ala Thr Cys
        995                 1000                 1005

Ala Thr  Gly Thr Cys Thr Gly  Thr
    1010                 1015
```

<210> 116
<211> 1016
<212> DNA
<213> Artificial Sequence

<220>
<223> GSC11678

<400> 116

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atcaggtccc agcaggaggc cgccgccaag aagtggtggt     720
gatgattcga aatgaccgac caagcgacgc ccaacctgcc atcacgagat ttcgattcca     780
ccgccgcctt ctatgaaagg ttgggcttcg gaatcgtttt ccgggacgcc ggctggatga     840
tcctccagcg cggggatctc atgctggagt tcttcgccca ccccaacttg tttattgcag     900
cttataatgg ttacaaataa agcaatagca tcacaaattt cacaaataaa gcattttttt     960
cactgcattc tagttgtggt ttgtccaaac tcatcaatgt atcttatcat gtctgt        1016
```

<210> 117
<211> 1115
<212> DNA
<213> Artificial Sequence

<220>
<223> GSC11759

<400> 117

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atcatgttcc tgaccctgaa gggctccctg aagcagaggc     720
tgcaggtgat gatccagccc tccgaggaca tcgtgaggcc cgagaacggc cccgagcagc     780
cccaggccgg ctcctccgcc tccaaggagg cctacatctg atgattcgaa atgaccgacc     840
aagcgacgcc caacctgcca tcacgagatt tcgattccac cgccgccttc tatgaaaggt     900
tgggcttcgg aatcgttttc cgggacgccg gctggatgat cctccagcgc ggggatctca     960
tgctggagtt cttcgcccac cccaacttgt ttattgcagc ttataatggt tacaaataaa    1020
gcaatagcat cacaaatttc acaaataaag catttttttc actgcattct agttgtggtt    1080
tgtccaaact catcaatgta tcttatcatg tctgt                               1115
```

<210> 118
<211> 1004
<212> DNA
<213> Artificial Sequence

<220>
<223> GSC11483

<400> 118

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcatgttcc tgaccctgaa gggctcctga tgattcgaaa 720
tgaccgacca agcgacgccc aacctgccat cacgagattt cgattccacc gccgccttct 780
atgaaaggtt gggcttcgga atcgttttcc gggacgccgg ctggatgatc ctccagcgcg 840
gggatctcat gctggagttc ttcgcccacc ccaacttgtt tattgcagct tataatggtt 900
acaaataaag caatagcatc acaaatttca caaataaagc atttttttca ctgcattcta 960
gttgtggttt gtccaaactc atcaatgtat cttatcatgt ctgt 1004

<210> 119
<211> 1115
<212> DNA
<213> Artificial Sequence

<220>
<223> GSC11760

<400> 119

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atctgcttcg tgggcaacag gttccagcag aagctgaggt 720
ccgtgttcag ggtgcccatc acctggctgc agggcaagag ggagaccatg tcctgcagga 780
agtcctcctc cctgagggag atggacacct tcgtgtcctg atgattcgaa atgaccgacc 840
aagcgacgcc caacctgcca tcacgagatt tcgattccac cgccgccttc tatgaaaggt 900
tgggcttcgg aatcgttttc cgggacgccg gctggatgat cctccagcgc ggggatctca 960
tgctggagtt cttcgcccac cccaacttgt ttattgcagc ttataatggt tacaaataaa 1020
gcaatagcat cacaaatttc acaaataaag catttttttc actgcattct agttgtggtt 1080
tgtccaaact catcaatgta tcttatcatg tctgt 1115

<210> 120
<211> 1094
<212> DNA
<213> Artificial Sequence

<220>
<223> GSC11761

<400> 120

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg ggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatacc ctggtgctgt tcggcgctgg cttcggcgcc gtgattaccg 660

tggtcgtgat cgtcgtgatt atctgcttcg tgggcaacag gttccagcag aagctgaggt 720

ccgtgttcag ggtgcccatc acctggctgc agggcaagag ggagaccatg tcctgcagga 780

agtcctcctc cctgaggtga tgattcgaaa tgaccgacca agcgacgccc aacctgccat 840

cacgagattt cgattccacc gccgccttct atgaaaggtt gggcttcgga atcgttttcc 900

gggacgccgg ctggatgatc ctccagcgcg gggatctcat gctggagttc ttcgcccacc 960

ccaacttgtt tattgcagct tataatggtt acaaataaag caatagcatc acaaatttca 1020

caaataaagc atttttttca ctgcattcta gttgtggttt gtccaaactc atcaatgtat 1080

cttatcatgt ctgt 1094

<210> 121
<211> 1229
<212> DNA
<213> Artificial Sequence

<220>
<223> GSC11762

<400> 121

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaactccc acctgctgcc caggcccctg aggcacctgg 720
cctgctgcgg cggcccccag cccaggatga ggaggaggct gtccgacggc tccctgtcct 780
ccaggcacac caccctgctg accaggtcct cctgccccgc caccctgtcc ctgtccctgt 840
ccctgaccct gtccggcagg cccaggcccg aggagtcccc cagggacctg gagctggccg 900
acggcgaggg caccgccgag accatcatct tctgatgatt cgaaatgacc gaccaagcga 960
cgcccaacct gccatcacga gatttcgatt ccaccgccgc cttctatgaa aggttgggct 1020
tcggaatcgt tttccgggac gccggctgga tgatcctcca gcgcggggat ctcatgctgg 1080
agttcttcgc ccaccccaac ttgtttattg cagcttataa tggttacaaa taaagcaata 1140
gcatcacaaa tttcacaaat aaagcatttt tttcactgca ttctagttgt ggtttgtcca 1200
aactcatcaa tgtatcttat catgtctgt 1229

<210> 122
<211> 1052
<212> DNA
<213> Artificial Sequence

<220>
<223> GSC11765

<400> 122

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaactccc acgaggagtc ccccagggac ctggagctgg 720
ccgacggcga gggcaccgcc gagaccatca tcttctgatg attcgaaatg accgaccaag 780
cgacgcccaa cctgccatca cgagatttcg attccaccgc cgccttctat gaaaggttgg 840
gcttcggaat cgttttccgg gacgccggct ggatgatcct ccagcgcggg gatctcatgc 900
tggagttctt cgcccacccc aacttgttta ttgcagctta taatggttac aaataaagca 960
atagcatcac aaatttcaca aataaagcat ttttttcact gcattctagt tgtggtttgt 1020
ccaaactcat caatgtatct tatcatgtct gt 1052

<210> 123
<211> 1049
<212> DNA
<213> Artificial Sequence

<220>
<223> GSC11766

<400> 123

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg gggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atctgcatca agctgaagca caccaagaag aggcagatct     720
acaccgacat cgagatgaac aggctgggca agtgatgatt cgaaatgacc gaccaagcga     780
cgcccaacct gccatcacga gatttcgatt ccaccgccgc cttctatgaa aggttgggct     840
tcggaatcgt tttccgggac gccggctgga tgatcctcca gcgcggggat ctcatgctgg     900
agttcttcgc ccaccccaac ttgtttattg cagcttataa tggttacaaa taaagcaata     960
gcatcacaaa tttcacaaat aaagcatttt tttcactgca ttctagttgt ggtttgtcca    1020
aactcatcaa tgtatcttat catgtctgt                                      1049
```

<210> 124
<211> 1049
<212> DNA
<213> Artificial Sequence

<220>
<223> GSC11763

<400> 124

```
ctgtctccgg gtaaatgatg aaagcttggt aggtgatcct cctgctgctt tggttcaggg      60
```

ttttgcttga ggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct caggtggtgg tggatctccc cccgaggacc 600

cccctgactc caagaatacc ctggtgctgt tcggcgctgg cttcggcgcc gtgattaccg 660

tggtcgtgat cgtcgtgatt atctgcatca agctgaagca caccaagaag aggcagatcg 720

ccgccgccgc cgccgccaac aggctgggca agtgatgatt cgaaatgacc gaccaagcga 780

cgcccaacct gccatcacga gatttcgatt ccaccgccgc cttctatgaa aggttgggct 840

tcggaatcgt tttccgggac gccggctgga tgatcctcca gcgcggggat ctcatgctgg 900

agttcttcgc ccaccccaac ttgtttattg cagcttataa tggttacaaa taaagcaata 960

gcatcacaaa tttcacaaat aaagcatttt tttcactgca ttctagttgt ggtttgtcca 1020

aactcatcaa tgtatcttat catgtctgt 1049

<210> 125
<211> 1118
<212> DNA
<213> Artificial Sequence

<220>
<223> GSC11294

<400> 125

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540

tgcgcttctt cccttccctc ctccctggct cagagctgca accggtggag agctgtgcgg 600
aggcgcagga cggggagctg gacgggctgt ggacgaccat caccatcttc atcacactct 660
tcctgttaag cgtgtgctac agtgccaccg tcaccttctt caaggtgaag tgcttctgca 720
agcaccggtc ctgcttccgg cggaacgagg cctccagaga gacaaacaac tccctgacct 780
tcggccccga ggaagccctg gctgagcaga ccgtgtttct gtagtgattc gaaatgaccg 840
accaagcgac gcccaacctg ccatcacgag atttcgattc caccgccgcc ttctatgaaa 900
ggttgggctt cggaatcgtt ttccgggacg ccggctggat gatcctccag cgcggggatc 960
tcatgctgga gttcttcgcc caccccaact tgtttattgc agcttataat ggttacaaat 1020
aaagcaatag catcacaaat ttcacaaata aagcattttt ttcactgcat tctagttgtg 1080
gtttgtccaa actcatcaat gtatcttatc atgtctgt 1118

<210> 126
<211> 466
<212> PRT
<213> Artificial Sequence

<220>
<223> GSB59abc

<400> 126

```
Met Glu Ser Pro Ala Gln Leu Leu Phe Leu Leu Leu Leu Trp Leu Pro
1               5                   10                  15

Asp Gly Val His Ala Gln Val Thr Leu Lys Glu Ser Gly Pro Ala Leu
            20                  25                  30

Val Lys Pro Thr Gln Thr Leu Thr Leu Thr Cys Ser Phe Ser Gly Phe
        35                  40                  45

Ser Leu Ser Thr Ser Gly Met Gly Val Gly Trp Ile Arg Gln Pro Pro
    50                  55                  60

Gly Lys Ala Leu Glu Trp Ile Ala His Ile Trp Trp Asp Asp Asp Lys
65                  70                  75                  80

Tyr Tyr Asn Thr Ala Leu Lys Thr Arg Leu Thr Ile Ser Lys Asp Thr
                85                  90                  95

Ser Lys Asn Gln Val Val Leu Thr Met Thr Asn Met Asp Pro Val Asp
            100                 105                 110

Thr Ala Thr Tyr Tyr Cys Ala Arg Ile Asp Trp Asp Gly Phe Ala Tyr
        115                 120                 125
```

Trp Gly Gln Gly Thr Leu Val Thr Val Ser Ser Ala Ser Thr Lys Gly
    130                 135                 140

Pro Ser Val Phe Pro Leu Ala Pro Cys Ser Arg Ser Thr Ser Glu Ser
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Lys Thr Tyr Thr Cys Asn Val
    210                 215                 220

Asp His Lys Pro Ser Asn Thr Lys Val Asp Lys Arg Val Glu Ser Lys
225                 230                 235                 240

Tyr Gly Pro Pro Cys Pro Pro Cys Pro Ala Pro Glu Phe Leu Gly Gly
                245                 250                 255

Pro Ser Val Phe Leu Phe Pro Pro Lys Pro Lys Asp Thr Leu Met Ile
            260                 265                 270

Ser Arg Thr Pro Glu Val Thr Cys Val Val Val Asp Val Ser Gln Glu
        275                 280                 285

Asp Pro Glu Val Gln Phe Asn Trp Tyr Val Asp Gly Val Glu Val His
    290                 295                 300

Asn Ala Lys Thr Lys Pro Arg Glu Glu Gln Phe Asn Ser Thr Tyr Arg
305                 310                 315                 320

Val Val Ser Val Leu Thr Val Leu His Gln Asp Trp Leu Asn Gly Lys
                325                 330                 335

Glu Tyr Lys Cys Lys Val Ser Asn Lys Gly Leu Pro Ser Ser Ile Glu
            340                 345                 350

Lys Thr Ile Ser Lys Ala Lys Gly Gln Pro Arg Glu Pro Gln Val Tyr
        355                 360                 365

Thr Leu Pro Pro Ser Gln Glu Glu Met Thr Lys Asn Gln Val Ser Leu
    370                 375                 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Arg Leu Thr Val Asp
420 425 430

Lys Ser Arg Trp Gln Glu Gly Asn Val Phe Ser Cys Ser Val Met His
435 440 445

Glu Ala Leu His Asn His Tyr Thr Gln Lys Ser Leu Ser Leu Ser Leu
450 455 460

Gly Lys
465

<210> 127
<211> 80
<212> PRT
<213> Artificial Sequence

<220>
<223> GSC11218

<400> 127

Leu Ser Leu Ser Pro Gly Gly Gly Gly Ser Pro Pro Glu Asp Pro Pro
1 5 10 15

Asp Ser Lys Asn Thr Leu Val Leu Phe Gly Ala Gly Phe Gly Ala Val
20 25 30

Ile Thr Val Val Val Ile Val Val Ile Ile Lys Cys Phe Cys Lys His
35 40 45

Arg Ser Cys Phe Arg Arg Asn Glu Ala Ser Arg Glu Thr Asn Asn Ser
50 55 60

Leu Thr Phe Gly Pro Glu Glu Ala Leu Ala Glu Gln Thr Val Phe Leu
65 70 75 80

<210> 128
<211> 81
<212> PRT
<213> Artificial Sequence

<220>
<223> GSC11213

<400> 128

Leu Ser Leu Ser Pro Gly Gly Gly Gly Ser Pro Pro Glu Asp Pro Pro
1               5                   10                  15

Asp Ser Lys Asn Leu Ala Leu Ile Leu Gly Pro Val Leu Ala Leu Leu
            20                  25                  30

Ala Leu Val Ala Leu Gly Val Leu Gly Leu Trp Lys Cys Phe Cys Lys
        35                  40                  45

His Arg Ser Cys Phe Arg Arg Asn Glu Ala Ser Arg Glu Thr Asn Asn
    50                  55                  60

Ser Leu Thr Phe Gly Pro Glu Glu Ala Leu Ala Glu Gln Thr Val Phe
65                  70                  75                  80

Leu

<210> 129
<211> 81
<212> PRT
<213> Artificial Sequence

<220>
<223> GSC11214

<400> 129

Leu Ser Leu Ser Pro Gly Gly Gly Gly Ser Pro Pro Glu Asp Pro Pro
1               5                   10                  15

Asp Ser Lys Asn Ile Ala Ala Ile Ile Val Ile Leu Val Leu Leu Leu
            20                  25                  30

Leu Leu Gly Ile Gly Leu Met Trp Trp Phe Trp Lys Cys Phe Cys Lys
        35                  40                  45

His Arg Ser Cys Phe Arg Arg Asn Glu Ala Ser Arg Glu Thr Asn Asn
    50                  55                  60

Ser Leu Thr Phe Gly Pro Glu Glu Ala Leu Ala Glu Gln Thr Val Phe
65                  70                  75                  80

Leu

<210> 130
<211> 80
<212> PRT
<213> Artificial Sequence

<220>
<223> GSC11210

<400> 130

Leu Ser Leu Ser Pro Gly Gly Gly Gly Ser Pro Pro Glu Asp Pro Pro
1 5 10 15

Asp Ser Lys Asn Met Ala Leu Ile Val Leu Gly Gly Val Ala Gly Leu
20 25 30

Leu Leu Phe Ile Gly Leu Gly Ile Phe Phe Lys Cys Phe Cys Lys His
35 40 45

Arg Ser Cys Phe Arg Arg Asn Glu Ala Ser Arg Glu Thr Asn Asn Ser
50 55 60

Leu Thr Phe Gly Pro Glu Glu Ala Leu Ala Glu Gln Thr Val Phe Leu
65 70 75 80

<210> 131
<211> 80
<212> PRT
<213> Artificial Sequence

<220>
<223> GSC11205

<400> 131

Leu Ser Leu Ser Pro Gly Gly Gly Gly Ser Pro Pro Glu Asp Pro Pro
1 5 10 15

Asp Ser Lys Asn Ile Ala Gly Val Ile Ala Gly Ile Leu Leu Phe Val
20 25 30

Ile Ile Phe Leu Gly Val Val Leu Val Met Lys Cys Phe Cys Lys His
35 40 45

Arg Ser Cys Phe Arg Arg Asn Glu Ala Ser Arg Glu Thr Asn Asn Ser
50 55 60

Leu Thr Phe Gly Pro Glu Glu Ala Leu Ala Glu Gln Thr Val Phe Leu
65 70 75 80

<210> 132
<211> 80
<212> PRT
<213> Artificial Sequence

<220>
<223> GSC11217

<400> 132

Leu Ser Leu Ser Pro Gly Gly Gly Gly Ser Pro Pro Glu Asp Pro Pro
1               5                   10                  15

Asp Ser Lys Asn Ala Leu Val Val Ile Pro Ile Ile Phe Gly Ile Leu
            20                  25                  30

Phe Ala Ile Leu Leu Val Leu Val Phe Ile Lys Cys Phe Cys Lys His
        35                  40                  45

Arg Ser Cys Phe Arg Arg Asn Glu Ala Ser Arg Glu Thr Asn Asn Ser
    50                  55                  60

Leu Thr Phe Gly Pro Glu Glu Ala Leu Ala Glu Gln Thr Val Phe Leu
65                  70                  75                  80

<210> 133
<211> 81
<212> PRT
<213> Artificial Sequence

<220>
<223> GSC11756

<400> 133

Leu Ser Leu Ser Pro Gly Gly Gly Gly Ser Pro Pro Glu Asp Pro Pro
1               5                   10                  15

Asp Ser Lys Asn Ile Ile Pro Ile Val Ala Gly Val Val Ala Gly Ile
            20                  25                  30

Val Leu Ile Gly Leu Ala Leu Leu Leu Ile Trp Lys Cys Phe Cys Lys
        35                  40                  45

His Arg Ser Cys Phe Arg Arg Asn Glu Ala Ser Arg Glu Thr Asn Asn
    50                  55                  60

Ser Leu Thr Phe Gly Pro Glu Glu Ala Leu Ala Glu Gln Thr Val Phe
65                  70                  75                  80

Leu

<210> 134
<211> 82
<212> PRT
<213> Artificial Sequence

<220>
<223> GSC11296

<400> 134

Leu Ser Leu Ser Pro Gly Gly Gly Gly Ser Pro Pro Glu Asp Pro Pro
1               5                   10                  15

Asp Ser Lys Asn Gly Ile Val Ala Gly Leu Ala Val Leu Ala Val Val
            20                  25                  30

Val Ile Gly Ala Val Val Ala Ala Val Met Cys Arg Lys Cys Phe Cys
        35                  40                  45

Lys His Arg Ser Cys Phe Arg Arg Asn Glu Ala Ser Arg Glu Thr Asn
    50                  55                  60

Asn Ser Leu Thr Phe Gly Pro Glu Glu Ala Leu Ala Glu Gln Thr Val
65                  70                  75                  80

Phe Leu

<210> 135
<211> 82
<212> PRT
<213> Artificial Sequence

<220>
<223> GSC11295

<400> 135

Leu Ser Leu Ser Pro Gly Gly Gly Gly Ser Pro Pro Glu Asp Pro Pro
1               5                   10                  15

Asp Ser Lys Asn Leu Ile Ile Ala Leu Pro Val Ala Val Leu Leu Ile
            20                  25                  30

Val Gly Gly Leu Val Ile Met Leu Tyr Val Phe His Lys Cys Phe Cys
        35                  40                  45

Lys His Arg Ser Cys Phe Arg Arg Asn Glu Ala Ser Arg Glu Thr Asn
    50                  55                  60

Asn Ser Leu Thr Phe Gly Pro Glu Glu Ala Leu Ala Glu Gln Thr Val
65                  70                  75                  80

Phe Leu

<210> 136
<211> 82
<212> PRT
<213> Artificial Sequence

<220>
<223> GSC11390

<400> 136

Leu Ser Leu Ser Pro Gly Gly Gly Gly Ser Pro Pro Glu Asp Pro Pro
1               5                   10                  15

Asp Ser Lys Asn Gly Leu Leu Ile Gly Ile Ser Ile Ala Ser Leu Cys
            20                  25                  30

Leu Val Val Ala Leu Leu Ala Leu Leu Cys His Leu Lys Cys Phe Cys
        35                  40                  45

Lys His Arg Ser Cys Phe Arg Arg Asn Glu Ala Ser Arg Glu Thr Asn
    50                  55                  60

Asn Ser Leu Thr Phe Gly Pro Glu Glu Ala Leu Ala Glu Gln Thr Val
65                  70                  75                  80

Phe Leu

<210> 137
<211> 82
<212> PRT
<213> Artificial Sequence

<220>
<223> GSC11391

<400> 137

Leu Ser Leu Ser Pro Gly Gly Gly Gly Ser Pro Pro Glu Asp Pro Pro
1               5                   10                  15

Asp Ser Lys Asn Leu Leu Leu Gly Val Ser Val Ser Cys Ile Val Ile
            20                  25                  30

Leu Ala Val Cys Leu Leu Cys Tyr Val Ser Ile Thr Lys Cys Phe Cys
        35                  40                  45

Lys His Arg Ser Cys Phe Arg Arg Asn Glu Ala Ser Arg Glu Thr Asn
    50                  55                  60

Asn Ser Leu Thr Phe Gly Pro Glu Glu Ala Leu Ala Glu Gln Thr Val
65                  70                  75                  80

Phe Leu

<210> 138

<211> 81
<212> PRT
<213> Artificial Sequence

<220>
<223> GSC11392

<400> 138

```
Leu Ser Leu Ser Pro Gly Gly Gly Gly Ser Pro Pro Glu Asp Pro Pro
1               5                   10                  15

Asp Ser Lys Asn Thr Asn Thr Val Gly Ser Val Ile Gly Val Ile Val
            20                  25                  30

Thr Ile Phe Val Ser Gly Thr Val Tyr Phe Ile Lys Cys Phe Cys Lys
        35                  40                  45

His Arg Ser Cys Phe Arg Arg Asn Glu Ala Ser Arg Glu Thr Asn Asn
    50                  55                  60

Ser Leu Thr Phe Gly Pro Glu Glu Ala Leu Ala Glu Gln Thr Val Phe
65                  70                  75                  80

Leu
```

<210> 139
<211> 81
<212> PRT
<213> Artificial Sequence

<220>
<223> GSC11397

<400> 139

```
Leu Ser Leu Ser Pro Gly Gly Gly Gly Ser Pro Pro Glu Asp Pro Pro
1               5                   10                  15

Asp Ser Lys Asn Ile Thr Leu Ile Ile Phe Gly Val Met Ala Gly Val
            20                  25                  30

Ile Gly Thr Ile Leu Leu Ile Ser Tyr Gly Ile Lys Cys Phe Cys Lys
        35                  40                  45

His Arg Ser Cys Phe Arg Arg Asn Glu Ala Ser Arg Glu Thr Asn Asn
    50                  55                  60
```

Ser Leu Thr Phe Gly Pro Glu Glu Ala Leu Ala Glu Gln Thr Val Phe
65 70 75 80

Leu

<210> 140
<211> 79
<212> PRT
<213> Artificial Sequence

<220>
<223> GSC11204

<400> 140

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Arg Leu Leu Leu Phe
20 25 30

Lys Leu Leu Leu Phe Asp Leu Leu Leu Lys Cys Phe Cys Lys His Arg
35 40 45

Ser Cys Phe Arg Arg Asn Glu Ala Ser Arg Glu Thr Asn Asn Ser Leu
50 55 60

Thr Phe Gly Pro Glu Glu Ala Leu Ala Glu Gln Thr Val Phe Leu
65 70 75

<210> 141
<211> 79
<212> PRT
<213> Artificial Sequence

<220>
<223> GSC11216

<400> 141

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Val Leu Leu Leu Phe
20 25 30

Lys Leu Leu Leu Phe Asp Leu Leu Leu Lys Cys Phe Cys Lys His Arg
35 40 45

Ser Cys Phe Arg Arg Asn Glu Ala Ser Arg Glu Thr Asn Asn Ser Leu

50 55 60

Thr Phe Gly Pro Glu Glu Ala Leu Ala Glu Gln Thr Val Phe Leu
65 70 75

<210> 142
<211> 79
<212> PRT
<213> Artificial Sequence

<220>
<223> GSC11203

<400> 142

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Arg Leu Leu Leu Phe
20 25 30

Val Leu Leu Leu Phe Asp Leu Leu Leu Lys Cys Phe Cys Lys His Arg
35 40 45

Ser Cys Phe Arg Arg Asn Glu Ala Ser Arg Glu Thr Asn Asn Ser Leu
50 55 60

Thr Phe Gly Pro Glu Glu Ala Leu Ala Glu Gln Thr Val Phe Leu
65 70 75

<210> 143
<211> 79
<212> PRT
<213> Artificial Sequence

<220>
<223> GSC11211

<400> 143

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Arg Leu Leu Leu Phe
20 25 30

Lys Leu Leu Leu Phe Val Leu Leu Leu Lys Cys Phe Cys Lys His Arg
35 40 45

Ser Cys Phe Arg Arg Asn Glu Ala Ser Arg Glu Thr Asn Asn Ser Leu
50 55 60

Thr Phe Gly Pro Glu Glu Ala Leu Ala Glu Gln Thr Val Phe Leu
65 70 75

<210> 144
<211> 79
<212> PRT
<213> Artificial Sequence

<220>
<223> GSC11212

<400> 144

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Val Leu Leu Leu Phe
20 25 30

Val Leu Leu Leu Phe Asp Leu Leu Leu Lys Cys Phe Cys Lys His Arg
35 40 45

Ser Cys Phe Arg Arg Asn Glu Ala Ser Arg Glu Thr Asn Asn Ser Leu
50 55 60

Thr Phe Gly Pro Glu Glu Ala Leu Ala Glu Gln Thr Val Phe Leu
65 70 75

<210> 145
<211> 79
<212> PRT
<213> Artificial Sequence

<220>
<223> GSC11398

<400> 145

Leu Ser Leu Ser Pro Gly Gly Gly Gly Ser Pro Pro Glu Asp Pro Pro
1 5 10 15

Asp Ser Lys Asn Ala Leu Trp Leu Gly Val Leu Val Leu Leu Leu Phe
20 25 30

Lys Leu Leu Leu Phe Val Leu Leu Leu Lys Cys Phe Cys Lys His Arg
35 40 45

Ser Cys Phe Arg Arg Asn Glu Ala Ser Arg Glu Thr Asn Asn Ser Leu
50 55 60

Thr Phe Gly Pro Glu Glu Ala Leu Ala Glu Gln Thr Val Phe Leu
65 70 75

<210> 146
<211> 79
<212> PRT
<213> Artificial Sequence

<220>
<223> GSC11208

<400> 146

```
Leu Ser Leu Ser Pro Gly Gly Gly Gly Ser Pro Pro Glu Asp Pro Pro
1               5                   10                  15

Asp Ser Lys Asn Ala Leu Trp Leu Gly Val Leu Arg Leu Leu Leu Phe
            20                  25                  30

Val Leu Leu Leu Phe Val Leu Leu Leu Lys Cys Phe Cys Lys His Arg
        35                  40                  45

Ser Cys Phe Arg Arg Asn Glu Ala Ser Arg Glu Thr Asn Asn Ser Leu
    50                  55                  60

Thr Phe Gly Pro Glu Glu Ala Leu Ala Glu Gln Thr Val Phe Leu
65                  70                  75
```

<210> 147
<211> 79
<212> PRT
<213> Artificial Sequence

<220>
<223> GSC11291

<400> 147

```
Leu Ser Leu Ser Pro Gly Gly Gly Gly Ser Pro Pro Glu Asp Pro Pro
1               5                   10                  15

Asp Ser Lys Asn Ala Leu Trp Leu Gly Val Leu Val Leu Leu Leu Phe
            20                  25                  30

Val Leu Leu Leu Phe Val Leu Leu Leu Lys Cys Phe Cys Lys His Arg
        35                  40                  45

Ser Cys Phe Arg Arg Asn Glu Ala Ser Arg Glu Thr Asn Asn Ser Leu
    50                  55                  60

Thr Phe Gly Pro Glu Glu Ala Leu Ala Glu Gln Thr Val Phe Leu
65                  70                  75
```

<210> 148
<211> 77

<212> PRT
<213> Artificial Sequence

<220>
<223> GSC11297

<400> 148

```
Leu Ser Leu Ser Pro Gly Gly Gly Gly Ser Pro Pro Glu Asp Pro Pro
1               5                   10                  15

Asp Ser Lys Asn Phe Phe Ile Pro Leu Leu Val Val Ile Leu Phe Ala
            20                  25                  30

Val Asp Thr Gly Leu Phe Ile Lys Cys Phe Cys Lys His Arg Ser Cys
        35                  40                  45

Phe Arg Arg Asn Glu Ala Ser Arg Glu Thr Asn Asn Ser Leu Thr Phe
    50                  55                  60

Gly Pro Glu Glu Ala Leu Ala Glu Gln Thr Val Phe Leu
65                  70                  75
```

<210> 149
<211> 77
<212> PRT
<213> Artificial Sequence

<220>
<223> GSC11298

<400> 149

```
Leu Ser Leu Ser Pro Gly Gly Gly Gly Ser Pro Pro Glu Asp Pro Pro
1               5                   10                  15

Asp Ser Lys Asn Ile Leu Ile Gly Thr Ser Val Val Ile Ile Leu Phe
            20                  25                  30

Ile Leu Leu Phe Phe Leu Leu Lys Cys Phe Cys Lys His Arg Ser Cys
        35                  40                  45

Phe Arg Arg Asn Glu Ala Ser Arg Glu Thr Asn Asn Ser Leu Thr Phe
    50                  55                  60

Gly Pro Glu Glu Ala Leu Ala Glu Gln Thr Val Phe Leu
65                  70                  75
```

<210> 150
<211> 75
<212> PRT
<213> Artificial Sequence

<220>
<223> GSC11393

<400> 150

```
Leu Ser Leu Ser Pro Gly Gly Gly Gly Ser Pro Pro Glu Asp Pro Pro
1               5                   10                  15

Asp Ser Lys Asn Val Leu Ala Leu Ile Val Ile Phe Leu Thr Ile Ala
            20                  25                  30

Val Leu Leu Ala Leu Lys Cys Phe Cys Lys His Arg Ser Cys Phe Arg
        35                  40                  45

Arg Asn Glu Ala Ser Arg Glu Thr Asn Asn Ser Leu Thr Phe Gly Pro
    50                  55                  60

Glu Glu Ala Leu Ala Glu Gln Thr Val Phe Leu
65                  70                  75
```

<210> 151
<211> 84
<212> PRT
<213> Artificial Sequence

<220>
<223> GSC11394

<400> 151

```
Leu Ser Leu Ser Pro Gly Gly Gly Gly Ser Pro Pro Glu Asp Pro Pro
1               5                   10                  15

Asp Ser Lys Asn Val Met Ser Val Ala Thr Ile Val Ile Val Asp Ile
            20                  25                  30

Cys Ile Thr Gly Gly Leu Leu Leu Leu Val Tyr Tyr Trp Ser Lys Cys
        35                  40                  45

Phe Cys Lys His Arg Ser Cys Phe Arg Arg Asn Glu Ala Ser Arg Glu
    50                  55                  60

Thr Asn Asn Ser Leu Thr Phe Gly Pro Glu Glu Ala Leu Ala Glu Gln
65                  70                  75                  80

Thr Val Phe Leu
```

<210> 152
<211> 84
<212> PRT
<213> Artificial Sequence

<220>
<223> GSC11395

<400> 152

Leu Ser Leu Ser Pro Gly Gly Gly Gly Ser Pro Pro Glu Asp Pro Pro
1               5                   10                  15

Asp Ser Lys Asn Ala Ile Pro Ile Trp Trp Val Leu Val Gly Val Leu
            20                  25                  30

Gly Gly Leu Leu Leu Leu Thr Ile Leu Val Leu Ala Met Trp Lys Cys
        35                  40                  45

Phe Cys Lys His Arg Ser Cys Phe Arg Arg Asn Glu Ala Ser Arg Glu
    50                  55                  60

Thr Asn Asn Ser Leu Thr Phe Gly Pro Glu Glu Ala Leu Ala Glu Gln
65                  70                  75                  80

Thr Val Phe Leu

<210> 153
<211> 85
<212> PRT
<213> Artificial Sequence

<220>
<223> GSC11396

<400> 153

Leu Ser Leu Ser Pro Gly Gly Gly Gly Ser Pro Pro Glu Asp Pro Pro
1               5                   10                  15

Asp Ser Lys Asn Phe Trp Val Leu Val Val Val Gly Gly Val Leu Ala
            20                  25                  30

Cys Tyr Ser Leu Leu Val Thr Val Ala Phe Ile Ile Phe Trp Val Lys
        35                  40                  45

Cys Phe Cys Lys His Arg Ser Cys Phe Arg Arg Asn Glu Ala Ser Arg
    50                  55                  60

Glu Thr Asn Asn Ser Leu Thr Phe Gly Pro Glu Glu Ala Leu Ala Glu
65                  70                  75                  80

Gln Thr Val Phe Leu
                85

<210> 154
<211> 85
<212> PRT
<213> Artificial Sequence

<220>
<223> GSC11389

<400> 154

```
Leu Ser Leu Ser Pro Gly Gly Gly Gly Ser Pro Pro Glu Asp Pro Pro
1               5                   10                  15

Asp Ser Lys Asn Leu Trp Thr Thr Ile Thr Ile Phe Ile Thr Leu Phe
            20                  25                  30

Leu Leu Ser Val Cys Tyr Ser Ala Thr Val Thr Phe Phe Lys Val Lys
        35                  40                  45

Cys Phe Cys Lys His Arg Ser Cys Phe Arg Arg Asn Glu Ala Ser Arg
    50                  55                  60

Glu Thr Asn Asn Ser Leu Thr Phe Gly Pro Glu Glu Ala Leu Ala Glu
65                  70                  75                  80

Gln Thr Val Phe Leu
                85
```

<210> 155
<211> 76
<212> PRT
<213> Artificial Sequence

<220>
<223> GSC11677

<400> 155

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Thr
            20                  25                  30

Val Ile Val Val Ile Ile Lys Cys Phe Cys Lys His Arg Ser Cys Phe
        35                  40                  45

Arg Arg Asn Glu Ala Ser Arg Glu Thr Asn Asn Ser Leu Thr Phe Gly
    50                  55                  60

Pro Glu Glu Ala Leu Ala Glu Gln Thr Val Phe Leu
65                  70                  75

<210> 156
<211> 78
<212> PRT
<213> Artificial Sequence

<220>
<223> GSC11679

<400> 156

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Thr
            20                  25                  30

Val Val Val Ile Val Val Ile Ile Lys Cys Phe Cys Lys His Arg Ser
        35                  40                  45

Cys Phe Arg Arg Asn Glu Ala Ser Arg Glu Thr Asn Asn Ser Leu Thr
    50                  55                  60

Phe Gly Pro Glu Glu Ala Leu Ala Glu Gln Thr Val Phe Leu
65                  70                  75

<210> 157
<211> 82
<212> PRT
<213> Artificial Sequence

<220>
<223> GSC11484

<400> 157

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Val Ile Val Thr Val Val Val Ile Val Val Ile Ile Lys Cys Phe Cys
        35                  40                  45

Lys His Arg Ser Cys Phe Arg Arg Asn Glu Ala Ser Arg Glu Thr Asn
    50                  55                  60

Asn Ser Leu Thr Phe Gly Pro Glu Glu Ala Leu Ala Glu Gln Thr Val
65                  70                  75                  80

Phe Leu

<210> 158
<211> 84
<212> PRT
<213> Artificial Sequence

<220>
<223> GSC11292

<400> 158

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Val Ile Val Val Val Thr Val Val Val Ile Val Val Ile Ile Lys Cys
        35                  40                  45

Phe Cys Lys His Arg Ser Cys Phe Arg Arg Asn Glu Ala Ser Arg Glu
    50                  55                  60

Thr Asn Asn Ser Leu Thr Phe Gly Pro Glu Glu Ala Leu Ala Glu Gln
65                  70                  75                  80

Thr Val Phe Leu

<210> 159
<211> 51
<212> PRT
<213> Artificial Sequence

<220>

<223> GSC11207

<400> 159

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Ala Ser Ala His His His
        35                  40                  45

His His His
    50
```

<210> 160
<211> 47
<212> PRT
<213> Artificial Sequence

<220>
<223> GSC11209

<400> 160

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Lys Cys Phe Cys Lys
        35                  40                  45
```

<210> 161
<211> 68
<212> PRT
<213> Artificial Sequence

<220>
<223> GSC11758

<400> 161

Leu Ser Leu Ser Pro Gly Gly Gly Gly Ser Pro Pro Glu Asp Pro Pro
1 5 10 15

Asp Ser Lys Asn Thr Leu Val Leu Phe Gly Ala Gly Phe Gly Ala Val
20 25 30

Ile Thr Val Val Val Ile Val Val Ile Ile Lys Trp Ile Phe Ser Ser
35 40 45

Val Val Asp Leu Lys Gln Thr Ile Ile Pro Asp Tyr Arg Asn Met Ile
50 55 60

Gly Gln Gly Ala
65

<210> 162
<211> 54
<212> PRT
<213> Artificial Sequence

<220>
<223> GSC11764

<400> 162

Leu Ser Leu Ser Pro Gly Gly Gly Gly Ser Pro Pro Glu Asp Pro Pro

1 5 10 15

Asp Ser Lys Asn Thr Leu Val Leu Phe Gly Ala Gly Phe Gly Ala Val
20 25 30

Ile Thr Val Val Val Ile Val Val Ile Ile Arg Ser Gln Gln Glu Ala
35 40 45

Ala Ala Lys Lys Phe Phe
50

<210> 163
<211> 54
<212> PRT
<213> Artificial Sequence

<220>
<223> GSC11678

<400> 163

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Arg Ser Gln Gln Glu Ala
        35                  40                  45

Ala Ala Lys Lys Trp Trp
    50

<210> 164
<211> 87
<212> PRT
<213> Artificial Sequence

<220>
<223> GSC11759

<400> 164

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Met Phe Leu Thr Leu Lys
        35                  40                  45

Gly Ser Leu Lys Gln Arg Leu Gln Val Met Ile Gln Pro Ser Glu Asp
    50                  55                  60

Ile Val Arg Pro Glu Asn Gly Pro Glu Gln Pro Gln Ala Gly Ser Ser
65                  70                  75                  80

Ala Ser Lys Glu Ala Tyr Ile
                85

<210> 165
<211> 50
<212> PRT
<213> Artificial Sequence

<220>
<223> GSC11483

<400> 165

Leu Ser Leu Ser Pro Gly Gly Gly Gly Ser Pro Pro Glu Asp Pro Pro
1 5 10 15

Asp Ser Lys Asn Thr Leu Val Leu Phe Gly Ala Gly Phe Gly Ala Val
20 25 30

Ile Thr Val Val Val Ile Val Val Ile Ile Met Phe Leu Thr Leu Lys
35 40 45

Gly Ser
50

<210> 166
<211> 87
<212> PRT
<213> Artificial Sequence

<220>
<223> GSC11760

<400> 166

Leu Ser Leu Ser Pro Gly Gly Gly Gly Ser Pro Pro Glu Asp Pro Pro
1 5 10 15

Asp Ser Lys Asn Thr Leu Val Leu Phe Gly Ala Gly Phe Gly Ala Val
20 25 30

Ile Thr Val Val Val Ile Val Val Ile Ile Cys Phe Val Gly Asn Arg
35 40 45

Phe Gln Gln Lys Leu Arg Ser Val Phe Arg Val Pro Ile Thr Trp Leu
50 55 60

Gln Gly Lys Arg Glu Thr Met Ser Cys Arg Lys Ser Ser Ser Leu Arg
65 70 75 80

Glu Met Asp Thr Phe Val Ser
85

<210> 167
<211> 80
<212> PRT
<213> Artificial Sequence

<220>
<223> GSC11761

<400> 167

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Cys Phe Val Gly Asn Arg
        35                  40                  45

Phe Gln Gln Lys Leu Arg Ser Val Phe Arg Val Pro Ile Thr Trp Leu
    50                  55                  60

Gln Gly Lys Arg Glu Thr Met Ser Cys Arg Lys Ser Ser Ser Leu Arg
65                  70                  75                  80
```

<210> 168
<211> 125
<212> PRT
<213> Artificial Sequence

<220>
<223> GSC11762

<400> 168

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Asn Ser His Leu Leu Pro
        35                  40                  45

Arg Pro Leu Arg His Leu Ala Cys Cys Gly Gly Pro Gln Pro Arg Met
    50                  55                  60

Arg Arg Arg Leu Ser Asp Gly Ser Leu Ser Ser Arg His Thr Thr Leu
65                  70                  75                  80

Leu Thr Arg Ser Ser Cys Pro Ala Thr Leu Ser Leu Ser Leu Ser Leu
                85                  90                  95

Thr Leu Ser Gly Arg Pro Arg Pro Glu Glu Ser Pro Arg Asp Leu Glu
            100                 105                 110

Leu Ala Asp Gly Glu Gly Thr Ala Glu Thr Ile Ile Phe
        115                 120                 125
```

<210> 169
<211> 66
<212> PRT
<213> Artificial Sequence

<220>
<223> GSC11765

<400> 169

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Asn Ser His Glu Glu Ser
        35                  40                  45

Pro Arg Asp Leu Glu Leu Ala Asp Gly Glu Gly Thr Ala Glu Thr Ile
    50                  55                  60

Ile Phe
65
```

<210> 170
<211> 65
<212> PRT
<213> Artificial Sequence

<220>
<223> GSC11766

<400> 170

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val


            20                  25                  30

Ile Thr Val Val Val Ile Val Val Ile Ile Cys Ile Lys Leu Lys His
        35                  40                  45

Thr Lys Lys Arg Gln Ile Tyr Thr Asp Ile Glu Met Asn Arg Leu Gly
    50                  55                  60

Lys
65
```

<210> 171
<211> 65
<212> PRT
<213> Artificial Sequence

<220>
<223> GSC11763

<400> 171

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Cys Ile Lys Leu Lys His
        35                  40                  45

Thr Lys Lys Arg Gln Ile Ala Ala Ala Ala Ala Ala Asn Arg Leu Gly
    50                  55                  60

Lys
65
```

<210> 172
<211> 88
<212> PRT
<213> Artificial Sequence

<220>
<223> GSC11294

<400> 172

```
Leu Ser Leu Ser Pro Glu Leu Gln Pro Val Glu Ser Cys Ala Glu Ala
1               5                   10                  15

Gln Asp Gly Glu Leu Asp Gly Leu Trp Thr Thr Ile Thr Ile Phe Ile
            20                  25                  30
```

Thr Leu Phe Leu Leu Ser Val Cys Tyr Ser Ala Thr Val Thr Phe Phe
35 40 45

Lys Val Lys Cys Phe Cys Lys His Arg Ser Cys Phe Arg Arg Asn Glu
50 55 60

Ala Ser Arg Glu Thr Asn Asn Ser Leu Thr Phe Gly Pro Glu Glu Ala
65 70 75 80

Leu Ala Glu Gln Thr Val Phe Leu
85

<210> 173
<211> 22
<212> PRT
<213> Artificial Sequence

<220>
<223> B7 TM domain

<400> 173

Thr Leu Val Leu Phe Gly Ala Gly Phe Gly Ala Val Ile Thr Val Val
1 5 10 15

Val Ile Val Val Ile Ile
20

<210> 174
<211> 23
<212> PRT
<213> Homo sapiens

<400> 174

Leu Ala Leu Ile Leu Gly Pro Val Leu Ala Leu Leu Ala Leu Val Ala
1 5 10 15

Leu Gly Val Leu Gly Leu Trp
20

<210> 175
<211> 23
<212> PRT
<213> Homo sapiens

<400> 175

```
Ile Ala Ala Ile Ile Val Ile Leu Val Leu Leu Leu Leu Leu Gly Ile
1               5                   10                  15

Gly Leu Met Trp Trp Phe Trp
            20
```

<210> 176
<211> 22
<212> PRT
<213> Homo sapiens

<400> 176

```
Met Ala Leu Ile Val Leu Gly Gly Val Ala Gly Leu Leu Leu Phe Ile
1               5                   10                  15

Gly Leu Gly Ile Phe Phe
            20
```

<210> 177
<211> 22
<212> PRT
<213> Homo sapiens

<400> 177

```
Ile Ala Gly Val Ile Ala Gly Ile Leu Leu Phe Val Ile Ile Phe Leu
1               5                   10                  15

Gly Val Val Leu Val Met
            20
```

<210> 178
<211> 22
<212> PRT
<213> Homo sapiens

<400> 178

```
Ala Leu Val Val Ile Pro Ile Ile Phe Gly Ile Leu Phe Ala Ile Leu
1               5                   10                  15

Leu Val Leu Val Phe Ile
            20
```

<210> 179
<211> 23
<212> PRT
<213> Homo sapiens

<400> 179

Ile Ile Pro Ile Val Ala Gly Val Val Ala Gly Ile Val Leu Ile Gly
1 5 10 15

Leu Ala Leu Leu Leu Ile Trp
20

<210> 180
<211> 24
<212> PRT
<213> Homo sapiens

<400> 180

Gly Ile Val Ala Gly Leu Ala Val Leu Ala Val Val Val Ile Gly Ala
1 5 10 15

Val Val Ala Ala Val Met Cys Arg
20

<210> 181
<211> 24
<212> **PRT**
<213> Homo sapiens

<400> 181

Leu Ile Ile Ala Leu Pro Val Ala Val Leu Leu Ile Val Gly Gly Leu
1 5 10 15

Val Ile Met Leu Tyr Val Phe His
20

<210> 182
<211> 24
<212> **PRT**
<213> Homo sapiens

<400> 182

Gly Leu Leu Ile Gly Ile Ser Ile Ala Ser Leu Cys Leu Val Val Ala
1 5 10 15

Leu Leu Ala Leu Leu Cys His Leu
20

<210> 183
<211> 24
<212> PRT
<213> Homo sapiens

<400> 183

Leu Leu Leu Gly Val Ser Val Ser Cys Ile Val Ile Leu Ala Val Cys
1               5                   10                  15

Leu Leu Cys Tyr Val Ser Ile Thr
            20

<210> 184
<211> 23
<212> PRT
<213> Homo sapiens

<400> 184

Thr Asn Thr Val Gly Ser Val Ile Gly Val Ile Val Thr Ile Phe Val
1               5                   10                  15

Ser Gly Thr Val Tyr Phe Ile
            20

<210> 185
<211> 23
<212> PRT
<213> Homo sapiens

<400> 185

Ile Thr Leu Ile Ile Phe Gly Val Met Ala Gly Val Ile Gly Thr Ile
1               5                   10                  15

Leu Leu Ile Ser Tyr Gly Ile
            20

<210> 186
<211> 21
<212> PRT
<213> Artificial Sequence

<220>
<223> PTCRA TM domain

<400> 186

Ala Leu Trp Leu Gly Val Leu Arg Leu Leu Leu Phe Lys Leu Leu Leu
1               5                   10                  15

Phe Asp Leu Leu Leu
            20

<210> 187
<211> 21
<212> PRT
<213> Artificial Sequence

<220>

<223> PTCRA_R8V TM domain

<400> 187

Ala Leu Trp Leu Gly Val Leu Val Leu Leu Leu Phe Lys Leu Leu Leu
1 5 10 15

Phe Asp Leu Leu Leu
20

<210> 188
<211> 21
<212> PRT
<213> Artificial Sequence

<220>
<223> PTCRA_K13V TM domain

<400> 188

Ala Leu Trp Leu Gly Val Leu Arg Leu Leu Leu Phe Val Leu Leu Leu
1 5 10 15

Phe Asp Leu Leu Leu
20

<210> 189
<211> 21
<212> PRT
<213> Artificial Sequence

<220>
<223> PTCRA_D18V TM domain

<400> 189

Ala Leu Trp Leu Gly Val Leu Arg Leu Leu Leu Phe Lys Leu Leu Leu
1 5 10 15

Phe Val Leu Leu Leu
20

<210> 190
<211> 21
<212> PRT
<213> Artificial Sequence

<220>
<223> PTCRA_R8V_K13V TM domain

<400> 190

Ala Leu Trp Leu Gly Val Leu Val Leu Leu Leu Phe Val Leu Leu Leu
1 5 10 15

Phe Asp Leu Leu Leu
20

<210> 191
<211> 21
<212> PRT
<213> Artificial Sequence

<220>
<223> PTCRA_R8V_D18V TM domain

<400> 191

Ala Leu Trp Leu Gly Val Leu Val Leu Leu Leu Phe Lys Leu Leu Leu

1 5 10 15

Phe Val Leu Leu Leu
20

<210> 192
<211> 21
<212> PRT
<213> Artificial Sequence

<220>
<223> PTCRA_K13V_D18V TM domain

<400> 192

Ala Leu Trp Leu Gly Val Leu Arg Leu Leu Leu Phe Val Leu Leu Leu
1 5 10 15

Phe Val Leu Leu Leu
20

<210> 193
<211> 21
<212> PRT
<213> Artificial Sequence

<220>
<223> PTCRA_R8V_K13V_D18V TM domain

<400> 193

Ala Leu Trp Leu Gly Val Leu Val Leu Leu Leu Phe Val Leu Leu Leu
1               5                   10                  15

Phe Val Leu Leu Leu
            20

<210> 194
<211> 19
<212> PRT
<213> Homo sapiens

<400> 194

Phe Phe Ile Pro Leu Leu Val Val Ile Leu Phe Ala Val Asp Thr Gly
1               5                   10                  15

Leu Phe Ile

<210> 195
<211> 19
<212> PRT
<213> Homo sapiens

<400> 195

Ile Leu Ile Gly Thr Ser Val Val Ile Ile Leu Phe Ile Leu Leu Phe
1               5                   10                  15

Phe Leu Leu

<210> 196
<211> 17
<212> PRT
<213> Homo sapiens

<400> 196

Val Leu Ala Leu Ile Val Ile Phe Leu Thr Ile Ala Val Leu Leu Ala
1               5                   10                  15

Leu

<210> 197
<211> 26
<212> PRT
<213> Homo sapiens

<400> 197

Val Met Ser Val Ala Thr Ile Val Ile Val Asp Ile Cys Ile Thr Gly
1               5                   10                  15

Gly Leu Leu Leu Leu Val Tyr Tyr Trp Ser
            20                  25

<210> 198
<211> 26
<212> PRT
<213> Homo sapiens

<400> 198

Ala Ile Pro Ile Trp Trp Val Leu Val Gly Val Leu Gly Gly Leu Leu
1               5                   10                  15

Leu Leu Thr Ile Leu Val Leu Ala Met Trp
            20                  25

<210> 199
<211> 27
<212> PRT
<213> Homo sapiens

<400> 199

Phe Trp Val Leu Val Val Val Gly Gly Val Leu Ala Cys Tyr Ser Leu

1               5                   10                  15

Leu Val Thr Val Ala Phe Ile Ile Phe Trp Val
            20                  25

<210> 200
<211> 27
<212> PRT
<213> Artificial Sequence

<220>
<223> M1M2 TM domain

<400> 200

Leu Trp Thr Thr Ile Thr Ile Phe Ile Thr Leu Phe Leu Leu Ser Val
1               5                   10                  15

Cys Tyr Ser Ala Thr Val Thr Phe Phe Lys Val
            20                  25

<210> 201
<211> 18
<212> PRT
<213> Artificial Sequence

<220>
<223> B7(18) TM domain

<400> 201

Thr Leu Val Leu Phe Gly Ala Gly Phe Gly Ala Thr Val Ile Val Val
1               5                   10                  15

Ile Ile

<210> 202
<211> 20
<212> PRT
<213> Artificial Sequence

<220>
<223> B7(20) TM domain

<400> 202

Thr Leu Val Leu Phe Gly Ala Gly Phe Gly Ala Thr Val Val Val Ile
1               5                   10                  15

Val Val Ile Ile
            20

<210> 203
<211> 24
<212> PRT
<213> Artificial Sequence

<220>
<223> B7(24) TM domain

<400> 203

Thr Leu Val Leu Phe Gly Ala Gly Phe Gly Ala Val Val Ile Val Thr
1               5                   10                  15

Val Val Val Ile Val Val Ile Ile
            20

<210> 204
<211> 26
<212> PRT
<213> Artificial Sequence

<220>
<223> B7(26) TM domain

<400> 204

Thr Leu Val Leu Phe Gly Ala Gly Phe Gly Ala Val Val Ile Val Val
1               5                   10                  15

Val Thr Val Val Val Ile Val Val Ile Ile
            20                  25

<210> 205
<211> 9
<212> PRT
<213> Artificial Sequence

<220>
<223> 6His cyto tail

<400> 205

Ala Ser Ala His His His His His His
1               5

<210> 206
<211> 5
<212> PRT
<213> Artificial Sequence

<220>
<223> KCFCK cyto tail

<400> 206

Lys Cys Phe Cys Lys
1               5

<210> 207
<211> 26
<212> PRT
<213> Artificial Sequence

<220>
<223> M1M2 cyto tail

<400> 207

Lys Trp Ile Phe Ser Ser Val Val Asp Leu Lys Gln Thr Ile Ile Pro
1               5                   10                  15

Asp Tyr Arg Asn Met Ile Gly Gln Gly Ala
            20                  25

<210> 208
<211> 12
<212> PRT
<213> Artificial Sequence

<220>
<223> ERGIC53 cyto tail

<400> 208

Arg Ser Gln Gln Glu Ala Ala Ala Lys Lys Phe Phe
1               5                   10

<210> 209
<211> 12
<212> PRT
<213> Artificial Sequence

<220>
<223> ERGICnoER cyto tail

<400> 209

Arg Ser Gln Gln Glu Ala Ala Ala Lys Lys Trp Trp
1               5                   10

<210> 210
<211> 45
<212> PRT
<213> Artificial Sequence

<220>
<223> GAT1 cyto tail

<400> 210

Met Phe Leu Thr Leu Lys Gly Ser Leu Lys Gln Arg Leu Gln Val Met
1               5                   10                  15

Ile Gln Pro Ser Glu Asp Ile Val Arg Pro Glu Asn Gly Pro Glu Gln
            20                  25                  30

Pro Gln Ala Gly Ser Ser Ala Ser Lys Glu Ala Tyr Ile
        35                  40                  45

<210> 211
<211> 8
<212> PRT
<213> Artificial Sequence

<220>
<223> GAT1noER cyto tail

<400> 211

Met Phe Leu Thr Leu Lys Gly Ser
1               5

<210> 212
<211> 45
<212> PRT
<213> Artificial Sequence

<220>
<223> AGTR2 cyto tail

<400> 212

```
Cys Phe Val Gly Asn Arg Phe Gln Gln Lys Leu Arg Ser Val Phe Arg
1               5                   10                  15

Val Pro Ile Thr Trp Leu Gln Gly Lys Arg Glu Thr Met Ser Cys Arg
            20                  25                  30

Lys Ser Ser Ser Leu Arg Glu Met Asp Thr Phe Val Ser
        35                  40                  45
```

<210> 213
<211> 38
<212> PRT
<213> Artificial Sequence

<220>
<223> AGTR2noER cyto tail

<400> 213

```
Cys Phe Val Gly Asn Arg Phe Gln Gln Lys Leu Arg Ser Val Phe Arg
1               5                   10                  15

Val Pro Ile Thr Trp Leu Gln Gly Lys Arg Glu Thr Met Ser Cys Arg
            20                  25                  30

Lys Ser Ser Ser Leu Arg
        35
```

<210> 214
<211> 83
<212> PRT
<213> Artificial Sequence

<220>
<223> V1BR cyto tail

<400> 214

Asn Ser His Leu Leu Pro Arg Pro Leu Arg His Leu Ala Cys Cys Gly
1               5                   10                  15

Gly Pro Gln Pro Arg Met Arg Arg Arg Leu Ser Asp Gly Ser Leu Ser
            20                  25                  30

Ser Arg His Thr Thr Leu Leu Thr Arg Ser Ser Cys Pro Ala Thr Leu
        35                  40                  45

Ser Leu Ser Leu Ser Leu Thr Leu Ser Gly Arg Pro Arg Pro Glu Glu
    50                  55                  60

Ser Pro Arg Asp Leu Glu Leu Ala Asp Gly Glu Gly Thr Ala Glu Thr
65                  70                  75                  80

Ile Ile Phe

<210> 215
<211> 24
<212> PRT
<213> Artificial Sequence

<220>
<223> V1BRnoER cyto tail

<400> 215

Asn Ser His Glu Glu Ser Pro Arg Asp Leu Glu Leu Ala Asp Gly Glu
1               5                   10                  15

Gly Thr Ala Glu Thr Ile Ile Phe
            20

<210> 216
<211> 23
<212> PRT
<213> Artificial Sequence

<220>
<223> VGLG cyto tail

<400> 216

Cys Ile Lys Leu Lys His Thr Lys Lys Arg Gln Ile Tyr Thr Asp Ile
1               5                   10                  15

Glu Met Asn Arg Leu Gly Lys
            20

<210> 217
<211> 23
<212> PRT
<213> Artificial Sequence

<220>
<223> VGLGnoER cyto tail

<400> 217

Cys Ile Lys Leu Lys His Thr Lys Lys Arg Gln Ile Ala Ala Ala Ala
1               5                   10                  15

Ala Ala Asn Arg Leu Gly Lys
            20

<210> 218
<211> 38
<212> PRT
<213> Artificial Sequence

<220>
<223> B7 cyto tail

<400> 218

Lys Cys Phe Cys Lys His Arg Ser Cys Phe Arg Arg Asn Glu Ala Ser
1               5                   10                  15

Arg Glu Thr Asn Asn Ser Leu Thr Phe Gly Pro Glu Glu Ala Leu Ala
            20                  25                  30

Glu Gln Thr Val Phe Leu
        35

<210> 219
<211> 39
<212> DNA
<213> Artificial Sequence

<220>
<223> Primer GlnPr2376

<400> 219
ggccgttcga atcatcactt gcagaagcac ttgataatc 39

<210> 220
<211> 65
<212> DNA
<213> Artificial Sequence

<220>
<223> Primer GlnPr2377

<400> 220

gtcatttcga atcatcaatg atggtggtgg tgatgggcgc tggcgataat cacgacgatc 60

acgac 65

<210> 221
<211> 59
<212> PRT
<213> Artificial Sequence

<220>
<223> Primer GlnPr2378

<400> 221

```
Thr Thr Gly Ala Ala Cys Ala Gly Cys Ala Gly Cys Ala Gly Thr Cys
1               5                   10                  15

Thr Cys Ala Gly Cys Ala Cys Gly Cys Cys Cys Ala Gly Cys Cys Ala
            20                  25                  30

Cys Ala Gly Gly Gly Cys Cys Cys Cys Gly Thr Cys Cys Ala Gly Cys
        35                  40                  45

Thr Cys Cys Cys Cys Gly Thr Cys Cys Thr Gly
    50                  55
```

<210> 222
<211> 66
<212> DNA
<213> Artificial Sequence

<220>
<223> Primer GlnPr2379

<400> 222

```
ctgagactgc tgctgttcaa gctgctcctg ttcgacctgc tcctcaagtg gatcttctcc        60

tcggtg                                                                    66
```

<210> 223
<211> 64
<212> DNA
<213> Artificial Sequence

<220>
<223> Primer GlnPr2380

<400> 223

```
gcttgaacag cagcagtctc agcacgccca gccacagggc attcttggag tcaggggggt        60

cctc                                                                      64
```

<210> 224
<211> 69
<212> PRT
<213> Artificial Sequence

<220>
<223> Primer GlnPr2381

<400> 224

Cys Ala Gly Gly Ala Cys Cys Gly Gly Thr Gly Cys Thr Thr Gly Cys
1 5 10 15

Ala Gly Ala Ala Gly Cys Ala Cys Thr Thr Gly Ala Gly Gly Ala Gly
20 25 30

Cys Ala Gly Gly Thr Cys Gly Ala Ala Cys Ala Gly Gly Ala Gly Cys
35 40 45

Ala Gly Cys Thr Thr Gly Ala Ala Cys Ala Gly Cys Ala Gly Cys Ala
50 55 60

Gly Thr Cys Thr Cys
65

<210> 225
<211> 64
<212> DNA
<213> Artificial Sequence

<220>
<223> Primer GlnPr2382

<400> 225

gcttgaacag cagcagtacc agcacgccca gccacagggc attcttggag tcaggggggt 60

cctc 64

<210> 226
<211> 69
<212> DNA
<213> Artificial Sequence

<220>
<223> Primer GlnPr2383

<400> 226

caggaccggt gcttgcagaa gcacttgagg agcaggtcga acaggagcag cttgaacagc 60

agcagtacc 69

<210> 227
<211> 66
<212> DNA
<213> Artificial Sequence

<220>
<223> Primer GlnPr2384

<400> 227

gaccggtgct tgcagaagca cttgaggagc aggtcgaaca ggagcagcac gaacagcagc 60

agtctc 66

<210> 228
<211> 66
<212> DNA
<213> Artificial Sequence

<220>
<223> Primer GlnPr2385

<400> 228

gaccggtgct tgcagaagca cttgaggagc aggacgaaca ggagcagctt gaacagcagc 60

agtctc 66

<210> 229
<211> 69
<212> DNA
<213> Artificial Sequence

<220>
<223> Primer GlnPr2386

<400> 229

caggaccggt gcttgcagaa gcacttgagg agcaggtcga acaggagcag cacgaacagc 60

agcagtacc 69

<210> 230
<211> 69
<212> DNA
<213> Artificial Sequence

<220>
<223> Primer GlnPr2387

<400> 230

caggaccggt gcttgcagaa gcacttgagg agcaggacga acaggagcag cttgaacagc 60

agcagtacc 69

<210> 231
<211> 66
<212> DNA
<213> Artificial Sequence

<220>
<223> Primer GlnPr2388

<400> 231

gaccggtgct tgcagaagca cttgaggagc aggacgaaca ggagcagcac gaacagcagc 60

agtctc 66

<210> 232
<211> 69
<212> DNA
<213> Artificial Sequence

<220>
<223> Primer GlnPr2389

<400> 232

caggaccggt gcttgcagaa gcacttgagg agcaggacga acaggagcag cacgaacagc 60

agcagtacc 69

<210> 233
<211> 47
<212> DNA
<213> Artificial Sequence

<220>
<223> Primer GlnPr2390

<400> 233
ggtcgtgatc gtcgtgatta tcaagtggat cttctcctcg gtggtgg 47

<210> 234
<211> 47
<212> DNA
<213> Artificial Sequence

<220>
<223> Primer GlnPr2391

<400> 234
ccaccaccga ggagaagatc cacttgataa tcacgacgat cacgacc 47

<210> 235
<211> 42
<212> DNA
<213> Artificial Sequence

<220>
<223> Primer GlnPr2392

<400> 235
cttcggcgcc accgtggtcg tgatcgtcgt gattatcaag tg 42

<210> 236
<211> 42
<212> DNA

<213> Artificial Sequence

<220>
<223> Primer GlnPr2393

<400> 236
caggaccggt gcttgcagaa gcacttgata atcacgacga tc 42

<210> 237
<211> 40
<212> DNA
<213> Artificial Sequence

<220>
<223> Primer GlnPr2394

<400> 237
cttcggcgcc accgtgatcg tcgtgattat caagtgcttc 40

<210> 238
<211> 38
<212> DNA
<213> Artificial Sequence

<220>
<223> Primer GlnPr2395

<400> 238
caggaccggt gcttgcagaa gcacttgata atcacgac 38

<210> 239
<211> 43
<212> DNA
<213> Artificial Sequence

<220>
<223> Primer GlnPr2396

<400> 239
cttcggcgcc gtggtcattg taaccgtggt cgtgatcgtc gtg 43

<210> 240
<211> 50
<212> DNA
<213> Artificial Sequence

<220>
<223> Primer GlnPr2397

<400> 240
caggaccggt gcttgcagaa gcacttgata atcacgacga tcacgaccac 50

<210> 241
<211> 49
<212> DNA
<213> Artificial Sequence

<220>

<223> Primer GlnPr2398

<400> 241
cttcggcgcc gtggtcattg tagtcgtaac cgtggtcgtg atcgtcgtg 49

<210> 242
<211> 52
<212> DNA
<213> Artificial Sequence

<220>
<223> Primer GlnPr2399

<400> 242
caggaccggt gcttgcagaa gcacttgata atcacgacga tcacgaccac gg 52

<210> 243
<211> 70
<212> DNA
<213> Artificial Sequence

<220>
<223> Primer GlnPr2400

<400> 243

gagccaccag tgccagcaga gccagcacag gtcccaggat cagagccaga ttcttggagt 60

cagggggtc 70

<210> 244
<211> 65
<212> DNA
<213> Artificial Sequence

<220>
<223> > Seq Id No. 244 Primer GlnPr2401

<400> 244

caggaccggt gcttgcagaa gcacttccac agtcccagca ctcccagagc caccagtgcc 60

agcag 65

<210> 245
<211> 70
<212> DNA
<213> Artificial Sequence

<220>
<223> Primer GlnPr2402

<400> 245

cgatgcccag cagcaggagc aacaccagga tcacgatgat ggcggcgata ttcttggagt 60

caggggggtc 70

<210> 246
<211> 65
<212> DNA
<213> Artificial Sequence

<220>
<223> Primer GlnPr2403

<400> 246

caggaccggt gcttgcagaa gcacttccag aaccaccaca tcaggccgat gcccagcagc 60

aggag 65

<210> 247
<211> 69
<212> DNA
<213> Artificial Sequence

<220>
<223> Primer GlnPr2404

<400> 247

gataaacagc agcaggccag ccactccgcc cagcacaatc agggccatat tcttggagtc 60

agggggtc 69

<210> 248
<211> 62
<212> DNA
<213> Artificial Sequence

<220>
<223> Primer GlnPr2405

<400> 248

caggaccggt gcttgcagaa gcacttgaag aagatgccca ggccgataaa cagcagcagg 60

cc 62

<210> 249
<211> 70
<212> DNA
<213> Artificial Sequence

<220>
<223> Primer GlnPr2406

<400> 249

gtccaatcag cacgatgcca gccaccacgc cggccacgat agggatgata ttcttggagt 60

cagggggtc 70

<210> 250
<211> 65
<212> DNA
<213> Artificial Sequence

<220>
<223> Primer GlnPr2407

<400> 250

caggaccggt gcttgcagaa gcacttccag atcagcagca gggccagtcc aatcagcacg 60

atgcc 65

<210> 251
<211> 70
<212> DNA
<213> Artificial Sequence

<220>
<223> Primer GlnPr2408

<400> 251

ccaggaagat gatcacgaac agcaggatgc cagcgatcac gccagcgata ttcttggagt 60

cagggggtc 70

<210> 252
<211> 62
<212> DNA
<213> Artificial Sequence

<220>
<223> Primer GlnPr2409

<400> 252

caggaccggt gcttgcagaa gcacttcatc acgagcacca cgcccaggaa gatgatcacg 60

aa 62

<210> 253
<211> 70
<212> DNA
<213> Artificial Sequence

<220>
<223> Primer GlnPr2410

<400> 253

gcaggatggc gaacaggatg ccgaagatga tggggatcac gaccagagca ttcttggagt 60

cagggggtc 70

<210> 254
<211> 63
<212> DNA
<213> Artificial Sequence

<220>
<223> Primer GlnPr2411

<400> 254

caggaccggt gcttgcagaa gcacttgatg aacaccagca ccagcaggat ggcgaacagg 60

atg 63

<210> 255
<211> 65
<212> DNA
<213> Artificial Sequence

<220>
<223> Primer GlnPr2412

<400> 255

cttcggcgcc gtgattaccg tggtcgtgat cgtcgtgatt atcaggtccc agcaggaggc 60

cgccg 65

<210> 256
<211> 51
<212> DNA
<213> Artificial Sequence

<220>
<223> Primer GlnPr2413

<400> 256
gtcatttcga atcatcagaa gaacttcttg gcggcggcct cctgctggga c 51

<210> 257
<211> 51
<212> DNA
<213> Artificial Sequence

<220>
<223> Primer GlnPr2414

<400> 257

gtcatttcga atcatcacca ccacttcttg gcggcggcct cctgctggga c 51

<210> 258
<211> 63
<212> DNA
<213> Artificial Sequence

<220>
<223> Primer GlnPr2415

<400> 258

ggtcatttcg aatcatcagg agcccttcag ggtcaggaac atgataatca cgacgatcac 60

gac 63

<210> 259
<211> 39
<212> DNA
<213> Artificial Sequence

<220>
<223> Primer GlnPr1285

<400> 259
cggaagaatt cagccacagc tttaaggcac ctggctaac 39

<210> 260
<211> 66
<212> DNA
<213> Artificial Sequence

<220>
<223> Primer GlnPr1494

<400> 260

acgaactagt ccaccatgga aagcccagcc cagctgctgt tcctgctgct gctgtggctg 60

cccgac 66

<210> 261
<211> 72
<212> DNA
<213> Artificial Sequence

<220>
<223> Primer GlnPr2294

<400> 261

ctaccaagct ttcatcattt acccggagac agggagaggc tcttctgcgt gtagtggttg 60

tgcaaggcct cg 72

<210> 262
<211> 432
<212> DNA
<213> Artificial Sequence

<220>
<223> GeneArt_Seq80

<400> 262

```
atcgatgctg aatgcaatta acaatgctca agcagaaccc ccggctccat cagcacagtg       60
caggaccaaa ccccatgctg cagcagtggg gctgtctgta cggggtgggc aatgggaacc      120
ggggtctgct ggggctcctg ctgcttcagt gctgccatgc agccacacat cctgagagct      180
gaaagggtcg gcgtcctcac ctggtgcaca ccgtagctct gccccacagc tttaaggcac      240
ctggctaacc tctgcgcttc ttcccttccc tcctccctgg ctcaggtggt ggtggatctc      300
cccccgagga ccccccctgac tccaagaatc tgtggacgac catcaccatc ttcatcacac      360
tcttcctgtt aagcgtgtgc tacagtgcca ccgtcacctt cttcaaggtg aagtgcttct      420
gcaagcaccg gt                                                          432
```

<210> 263
<211> 500
<212> DNA
<213> Artificial Sequence

<220>
<223> GeneArt_Seq81

<400> 263

```
atcgatgctg aatgcaatta acaatgctca agcagaaccc ccggctccat cagcacagtg       60
caggaccaaa ccccatgctg cagcagtggg gctgtctgta cggggtgggc aatgggaacc      120
ggggtctgct ggggctcctg ctgcttcagt gctgccatgc agccacacat cctgagagct      180
gaaagggtcg gcgtcctcac ctggtgcaca ccgtagctct gccccacagc tttaaggcac      240
ctggctaacc tctgcgcttc ttcccttccc tcctccctgg ctcaggtggt ggtggatctc      300
cccccgagga ccccccctgac tccaagaatc tgtggacgac catcaccatc ttcatcacac      360
tcttcctgtt aagcgtgtgc tacagtgcca ccgtcacctt cttcaaggtg aagtggatct      420
tctcctcggt ggtggacctg aagcagacca tcatccccga ctacaggaac atgatcggac      480
agggggccta gtgattcgaa                                                  500
```

<210> 264
<211> 545
<212> DNA
<213> Artificial Sequence

<220>
<223> GeneArt_Seq82

<400> 264

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcagagctg caaccggtgg 300
agagctgtgc ggaggcgcag gacggggagc tggacgggct gtggacgacc atcaccatct 360
tcatcacact cttcctgtta agcgtgtgct acagtgccac cgtcaccttc ttcaaggtga 420
agtgcttctg caagcaccgg tcctgcttcc ggcggaacga ggcctccaga gagacaaaca 480
actccctgac cttcggcccc gaggaagccc tggctgagca gaccgtgttt ctgtagtgat 540
tcgaa 545

<210> 265
<211> 423
<212> DNA
<213> Artificial Sequence

<220>
<223> GeneArt_Seq83

<400> 265

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaatc tgatcattgc cctgcctgtg gccgtgctgc 360
tgatcgtggg aggcctcgtg atcatgctgt acgtgttcca caagtgcttc tgcaagcacc 420
ggt 423

<210> 266
<211> 423
<212> DNA
<213> Artificial Sequence

<220>
<223> GeneArt_Seq84

<400> 266

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaatg gaatcgtggc tggactggct gtgctggccg 360
tggtcgtgat tggagctgtg gtggccgccg tgatgtgcag aaagtgcttc tgcaagcacc 420
ggt 423

<210> 267
<211> 423
<212> DNA
<213> Artificial Sequence

<220>
<223> GeneArt_Seq85

<400> 267

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaatg gcctgctgat cggcatctcc atcgcctccc 360
tgtgcctggt ggtggccctg ctggccctgc tgtgccacct gaagtgcttc tgcaagcacc 420
ggt 423

<210> 268
<211> 423
<212> DNA
<213> Artificial Sequence

<220>
<223> GeneArt_Seq86

<400> 268

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga ccccccctgac tccaagaatc tgctgctggg cgtgtccgtg tcctgcatcg 360
tgatcctggc cgtgtgcctg ctgtgctacg tgtccatcac caagtgcttc tgcaagcacc 420
ggt 423

<210> 269
<211> 420
<212> DNA
<213> Artificial Sequence

<220>
<223> GeneArt_Seq87

<400> 269

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga ccccccctgac tccaagaata ccaacaccgt gggctccgtg atcggcgtga 360
tcgtgaccat cttcgtgtcc ggcaccgtgt acttcatcaa gtgcttctgc aagcaccggt 420

<210> 270
<211> 402
<212> DNA
<213> Artificial Sequence

<220>
<223> GeneArt_Seq88

<400> 270

```
atcgatgctg aatgcaatta acaatgctca agcagaaccc ccggctccat cagcacagtg      60
caggaccaaa ccccatgctg cagcagtggg gctgtctgta cggggtgggc aatgggaacc     120
ggggtctgct ggggctcctg ctgcttcagt gctgccatgc agccacacat cctgagagct     180
gaaagggtcg gcgtcctcac ctggtgcaca ccgtagctct gccccacagc tttaaggcac     240
ctggctaacc tctgcgcttc ttcccttccc tcctccctgg ctcaggtggt ggtggatctc     300
cccccgagga cccccctgac tccaagaatg tgctggccct gatcgtgatc ttcctgacca     360
tcgccgtgct gctggccctg aagtgcttct gcaagcaccg gt                        402
```

<210> 271
<211> 408
<212> DNA
<213> Artificial Sequence

<220>
<223> GeneArt_Seq89

<400> 271

```
atcgatgctg aatgcaatta acaatgctca agcagaaccc ccggctccat cagcacagtg      60
caggaccaaa ccccatgctg cagcagtggg gctgtctgta cggggtgggc aatgggaacc     120
ggggtctgct ggggctcctg ctgcttcagt gctgccatgc agccacacat cctgagagct     180
gaaagggtcg gcgtcctcac ctggtgcaca ccgtagctct gccccacagc tttaaggcac     240
ctggctaacc tctgcgcttc ttcccttccc tcctccctgg ctcaggtggt ggtggatctc     300
cccccgagga cccccctgac tccaagaatt tcttcatccc cctgctggtg gtgatcctgt     360
tcgccgtgga caccggcctg ttcatcaagt gcttctgcaa gcaccggt                  408
```

<210> 272
<211> 408
<212> DNA
<213> Artificial Sequence

<220>
<223> GeneArt_Seq90

<400> 272

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaata tcctgatcgg cacctccgtg gtgatcatcc 360
tgttcatcct gctgttcttc ctgctgaagt gcttctgcaa gcaccggt 408

<210> 273
<211> 429
<212> DNA
<213> Artificial Sequence

<220>
<223> GeneArt_Seq91

<400> 273

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaatg tgatgtccgt ggccaccatc gtgatcgtgg 360
acatctgcat caccggcggc ctgctgctgc tggtgtacta ctggtccaag tgcttctgca 420
agcaccggt 429

<210> 274
<211> 429
<212> DNA
<213> Artificial Sequence

<220>
<223> GeneArt_Seq92

<400> 274

atcgatgctg aatgcaatta acaatgctca agcagaaccc ccggctccat cagcacagtg 60

caggaccaaa ccccatgctg cagcagtggg gctgtctgta cggggtgggc aatgggaacc 120

ggggtctgct ggggctcctg ctgcttcagt gctgccatgc agccacacat cctgagagct 180

gaaagggtcg gcgtcctcac ctggtgcaca ccgtagctct gccccacagc tttaaggcac 240

ctggctaacc tctgcgcttc ttcccttccc tcctccctgg ctcaggtggt ggtggatctc 300

cccccgagga ccccccctgac tccaagaatg ccatccccat ctggtgggtg ctggtgggcg 360

tgctgggcgg cctgctgctg ctgaccatcc tggtgctggc catgtggaag tgcttctgca 420

agcaccggt 429

<210> 275
<211> 432
<212> DNA
<213> Artificial Sequence

<220>
<223> GeneArt_Seq93

<400> 275

atcgatgctg aatgcaatta acaatgctca agcagaaccc ccggctccat cagcacagtg 60

caggaccaaa ccccatgctg cagcagtggg gctgtctgta cggggtgggc aatgggaacc 120

ggggtctgct ggggctcctg ctgcttcagt gctgccatgc agccacacat cctgagagct 180

gaaagggtcg gcgtcctcac ctggtgcaca ccgtagctct gccccacagc tttaaggcac 240

ctggctaacc tctgcgcttc ttcccttccc tcctccctgg ctcaggtggt ggtggatctc 300

cccccgagga ccccccctgac tccaagaatt tctgggtgct ggtggtggtg ggcggcgtgc 360

tggcctgcta ctccctgctg gtgaccgtgg ccttcatcat cttctgggtg aagtgcttct 420

gcaagcaccg gt 432

<210> 276
<211> 420
<212> DNA
<213> Artificial Sequence

<220>
<223> GeneArt_Seq94

<400> 276

atcgatgctg aatgcaatta acaatgctca agcagaaccc ccggctccat cagcacagtg 60
caggaccaaa ccccatgctg cagcagtggg gctgtctgta cggggtgggc aatgggaacc 120
ggggtctgct ggggctcctg ctgcttcagt gctgccatgc agccacacat cctgagagct 180
gaaagggtcg gcgtcctcac ctggtgcaca ccgtagctct gccccacagc tttaaggcac 240
ctggctaacc tctgcgcttc ttcccttccc tcctccctgg ctcaggtggt ggtggatctc 300
cccccgagga cccccctgac tccaagaata tcaccctgat catcttcggc gtgatggccg 360
gcgtgatcgg caccatcctg ctgatctcct acggcatcaa gtgcttctgc aagcaccggt 420

<210> 277
<211> 186
<212> DNA
<213> Artificial Sequence

<220>
<223> GeneArt_Seq95

<400> 277

ggcgccgtga ttaccgtggt cgtgatcgtc gtgattatca tgttcctgac cctgaagggc 60
tccctgaagc agaggctgca ggtgatgatc cagccctccg aggacatcgt gaggcccgag 120
aacggccccg agcagcccca ggccggctcc tccgcctcca aggaggccta catctgatga 180
ttcgaa 186

<210> 278
<211> 186
<212> DNA
<213> Artificial Sequence

<220>
<223> GeneArt_Seq96

<400> 278

ggcgccgtga ttaccgtggt cgtgatcgtc gtgattatct gcttcgtggg caacaggttc 60
cagcagaagc tgaggtccgt gttcagggtg cccatcacct ggctgcaggg caagagggag 120
accatgtcct gcaggaagtc ctcctccctg agggagatgg acaccttcgt gtcctgatga 180
ttcgaa 186

<210> 279
<211> 165
<212> DNA
<213> Artificial Sequence

<220>
<223> GeneArt_Seq97

<400> 279

```
ggcgccgtga ttaccgtggt cgtgatcgtc gtgattatct gcttcgtggg caacaggttc      60
cagcagaagc tgaggtccgt gttcagggtg cccatcacct ggctgcaggg caagagggag     120
accatgtcct gcaggaagtc ctcctccctg aggtgatgat tcgaa                     165
```

<210> 280
<211> 300
<212> DNA
<213> Artificial Sequence

<220>
<223> GeneArt_Seq98

<400> 280

```
ggcgccgtga ttaccgtggt cgtgatcgtc gtgattatca actcccacct gctgcccagg      60
cccctgaggc acctggcctg ctgcggcggc ccccagccca ggatgaggag gaggctgtcc     120
gacggctccc tgtcctccag gcacaccacc ctgctgacca ggtcctcctg ccccgccacc     180
ctgtccctgt ccctgtccct gaccctgtcc ggcaggccca ggcccgagga gtcccccagg     240
gacctggagc tggccgacgg cgagggcacc gccgagacca tcatcttctg atgattcgaa     300
```

<210> 281
<211> 123
<212> DNA
<213> Artificial Sequence

<220>
<223> GeneArt_Seq99

<400> 281

```
ggcgccgtga ttaccgtggt cgtgatcgtc gtgattatca actcccacga ggagtccccc      60
agggacctgg agctggccga cggcgagggc accgccgaga ccatcatctt ctgatgattc     120
gaa                                                                   123
```

<210> 282
<211> 120
<212> DNA
<213> Artificial Sequence

<220>
<223> GeneArt_Seq100

<400> 282

```
ggcgccgtga ttaccgtggt cgtgatcgtc gtgattatct gcatcaagct gaagcacacc      60
aagaagaggc agatctacac cgacatcgag atgaacaggc tgggcaagtg atgattcgaa     120
```

<210> 283
<211> 120
<212> DNA
<213> Artificial Sequence

<220>
<223> GeneArt_Seq101

<400> 283

ggcgccgtga ttaccgtggt cgtgatcgtc gtgattatct gcatcaagct gaagcacacc 60

aagaagaggc agatcgccgc cgccgccgcc gccaacaggc tgggcaagtg atgattcgaa 120

<210> 284
<211> 1064
<212> DNA
<213> Artificial Sequence

<220>
<223> GSC11206

<400> 284

ctgtctccgg gtaaatgatg aaagcttggt aggtgatcct cctgctgctt tggttcaggg 60

ttttgcttga gggggggggg tggtgatttc cttgccatgg gcagactgag cagaaaaggc 120

cattgggacc atgttctgaa tgcctccacc tcaaccaccg gccggtagga ccaaagccac 180

cccgtgtttt ctcaggatct cttttcccag ggagatccct cggcccaaag agggagatgg 240

caatgctgga tgtgtgcaca ataattcaac aggcattgga acttcagcat cgatgctgaa 300

tgcaattaac aatgctcaag cagaaccccc ggctccatca gcacagtgca ggaccaaacc 360

ccatgctgca gcagtggggc tgtctgtacg gggtgggcaa tgggaaccgg ggtctgctgg 420

ggctcctgct gcttcagtgc tgccatgcag ccacacatcc tgagagctga aagggtcggc 480

gtcctcacct ggtgcacacc gtagctctgc cccacagctt taaggcacct ggctaacctc 540

tgcgcttctt cccttccctc ctccctggct cagagctgca accggtggag agctgtgcgg 600

aggcgcagga cggggagctg gacggggccc tgtggctggg cgtgctgaga ctgctgctgt 660

tcaagctgct cctgttcgac ctgctcctca agtggatctt ctcctcggtg gtggacctga 720

agcagaccat catccccgac tacaggaaca tgatcggaca gggggcctag tgattcgaaa 780

tgaccgacca agcgacgccc aacctgccat cacgagattt cgattccacc gccgccttct 840

atgaaaggtt gggcttcgga atcgttttcc gggacgccgg ctggatgatc ctccagcgcg 900

gggatctcat gctggagttc ttcgcccacc ccaacttgtt tattgcagct tataatggtt 960

acaaataaag caatagcatc acaaatttca caaataaagc atttttttca ctgcattcta 1020

gttgtggttt gtccaaactc atcaatgtat cttatcatgt ctgt 1064

<210> 285
<211> 70
<212> PRT
<213> Artificial Sequence

<220>
<223> GSC11206

<400> 285

```
Leu Ser Leu Ser Pro Glu Leu Gln Pro Val Glu Ser Cys Ala Glu Ala
1               5                   10                  15

Gln Asp Gly Glu Leu Asp Gly Ala Leu Trp Leu Gly Val Leu Arg Leu
            20                  25                  30

Leu Leu Phe Lys Leu Leu Leu Phe Asp Leu Leu Leu Lys Trp Ile Phe
        35                  40                  45

Ser Ser Val Val Asp Leu Lys Gln Thr Ile Ile Pro Asp Tyr Arg Asn
    50                  55                  60

Met Ile Gly Gln Gly Ala
65                  70
```

<210> 286
<211> 470
<212> PRT
<213> Artificial Sequence

<220>
<223> GSC314

<400> 286

```
Met Glu Ser Pro Ala Gln Leu Leu Phe Leu Leu Leu Leu Trp Leu Pro
1               5                   10                  15

Asp Thr Gln Ala Glu Val Gln Leu Val Glu Ser Gly Gly Gly Leu Val
            20                  25                  30

Gln Pro Gly Gly Ser Leu Arg Leu Ser Cys Ala Ala Ser Gly Phe Asn
        35                  40                  45

Ile Lys Asp Thr Tyr Ile His Trp Val Arg Gln Ala Pro Gly Lys Gly
    50                  55                  60

Leu Glu Trp Val Ala Arg Ile Tyr Pro Thr Asn Gly Tyr Thr Arg Tyr
65                  70                  75                  80

Ala Asp Ser Val Lys Gly Arg Phe Thr Ile Ser Ala Asp Thr Ser Lys
                85                  90                  95
```

```
Asn Thr Ala Tyr Leu Gln Met Asn Ser Leu Arg Ala Glu Asp Thr Ala
            100                 105                 110

Val Tyr Tyr Cys Ser Arg Trp Gly Gly Asp Gly Phe Tyr Ala Met Asp
        115                 120                 125

Tyr Trp Gly Gln Gly Thr Leu Val Thr Val Ser Ser Ala Ser Thr Lys
    130                 135                 140

Gly Pro Ser Val Phe Pro Leu Ala Pro Ser Ser Lys Ser Thr Ser Gly
145                 150                 155                 160

Gly Thr Ala Ala Leu Gly Cys Leu Val Lys Asp Tyr Phe Pro Glu Pro
                165                 170                 175

Val Thr Val Ser Trp Asn Ser Gly Ala Leu Thr Ser Gly Val His Thr
            180                 185                 190

Phe Pro Ala Val Leu Gln Ser Ser Gly Leu Tyr Ser Leu Ser Ser Val
        195                 200                 205

Val Thr Val Pro Ser Ser Ser Leu Gly Thr Gln Thr Tyr Ile Cys Asn
    210                 215                 220

Val Asn His Lys Pro Ser Asn Thr Lys Val Asp Lys Lys Val Glu Pro
225                 230                 235                 240

Lys Ser Cys Asp Lys Thr His Thr Cys Pro Pro Cys Pro Ala Pro Glu
                245                 250                 255

Leu Leu Gly Gly Pro Ser Val Phe Leu Phe Pro Pro Lys Pro Lys Asp
            260                 265                 270

Thr Leu Met Ile Ser Arg Thr Pro Glu Val Thr Cys Val Val Val Asp
        275                 280                 285

Val Ser His Glu Asp Pro Glu Val Lys Phe Asn Trp Tyr Val Asp Gly
    290                 295                 300

Val Glu Val His Asn Ala Lys Thr Lys Pro Arg Glu Glu Gln Tyr Asn
305                 310                 315                 320

Ser Thr Tyr Arg Val Val Ser Val Leu Thr Val Leu His Gln Asp Trp
                325                 330                 335

Leu Asn Gly Lys Glu Tyr Lys Cys Lys Val Ser Asn Lys Ala Leu Pro
            340                 345                 350
```

```
Ala Pro Ile Glu Lys Thr Ile Ser Lys Ala Lys Gly Gln Pro Arg Glu
        355                 360                 365

Pro Gln Val Tyr Thr Leu Pro Pro Ser Arg Glu Glu Met Thr Lys Asn
    370                 375                 380

Gln Val Ser Leu Thr Cys Leu Val Lys Gly Phe Tyr Pro Ser Asp Ile
385                 390                 395                 400

Ala Val Glu Trp Glu Ser Asn Gly Gln Pro Glu Asn Asn Tyr Lys Thr
                405                 410                 415

Thr Pro Pro Val Leu Asp Ser Asp Gly Ser Phe Phe Leu Tyr Ser Lys
            420                 425                 430

Leu Thr Val Asp Lys Ser Arg Trp Gln Gln Gly Asn Val Phe Ser Cys
        435                 440                 445

Ser Val Met His Glu Ala Leu His Asn His Tyr Thr Gln Lys Ser Leu
    450                 455                 460

Ser Leu Ser Pro Gly Lys
465                 470
```

<210> 287
<211> 375
<212> DNA
<213> Artificial Sequence

<220>
<223> GSC3836

<400> 287

```
ctgtctccgg gtaaatgatg aaagcttgcg gccgcgctta gcgatatcga attctctaga      60

ggtacccccg gggggcccct cgagttcgaa atgaccgacc aagcgacgcc caacctgcca     120

tcacgagatt tcgattccac cgccgccttc tatgaaaggt tgggcttcgg aatcgttttc     180

cgggacgccg gctggatgat cctccagcgc ggggatctca tgctggagtt cttcgcccac     240

cccaacttgt ttattgcagc ttataatggt tacaaataaa gcaatagcat cacaaatttc     300

acaaataaag catttttttc actgcattct agttgtggtt tgtccaaact catcaatgta     360

tcttatcatg tctgt                                                      375
```

<210> 288
<211> 5
<212> PRT
<213> Artificial Sequence

<220>
<223> GSC3836 / GSC3848

<400> 288
Leu Ser Pro Gly Lys

1 5

<210> 289
<211> 884
<212> DNA
<213> Artificial Sequence

<220>
<223> GSC3848

<400> 289

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg ggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct cagagctgca accggtggag atattcgaaa     600
tgaccgacca agcgacgccc aacctgccat cacgagattt cgattccacc gccgccttct     660
atgaaaggtt gggcttcgga atcgttttcc gggacgccgg ctggatgatc ctccagcgcg     720
gggatctcat gctggagttc ttcgcccacc ccaacttgtt tattgcagct tataatggtt     780
acaaataaag caatagcatc acaaatttca caaataaagc atttttttca ctgcattcta     840
gttgtggttt gtccaaactc atcaatgtat cttatcatgt ctgt                      884
```

<210> 290
<211> 76
<212> PRT
<213> Artificial Sequence

<220>
<223> GSC3899 / GSC4398 / GSC4401 / GSC4402

<400> 290

Leu Ser Leu Ser Pro Glu Leu Gln Pro Val Glu Ser Cys Ala Glu Ala
1               5                   10                  15

Gln Asp Gly Glu Leu Asp Gly Leu Trp Thr Thr Ile Thr Ile Phe Ile

            20                  25                  30

Thr Leu Phe Leu Leu Ser Val Cys Tyr Ser Ala Thr Val Thr Phe Phe
        35                  40                  45

Lys Val Lys Trp Ile Phe Ser Ser Val Val Asp Leu Lys Gln Thr Ile
    50                  55                  60

Ile Pro Asp Tyr Arg Asn Met Ile Gly Gln Gly Ala
65                  70                  75

<210> 291
<211> 42
<212> PRT
<213> Artificial Sequence

<220>
<223> GSC5854 / GSC5855 / GSC5857 / GSC5856

<400> 291

Leu Ser Leu Ser Pro Ala Glu Leu Gln Pro Val Glu Ala Leu Trp Leu
1               5                   10                  15

Gly Val Leu Arg Leu Leu Leu Phe Lys Leu Leu Leu Phe Asp Leu Leu
            20                  25                  30

Leu Ala Ser Ala His His His His His His
        35                  40

<210> 292
<211> 74
<212> PRT
<213> Artificial Sequence

<220>
<223> GSC6030 / GSC6048 / GSC6047 / GSC6046

<400> 292

Leu Ser Leu Ser Pro Glu Leu Gln Pro Val Glu Ser Cys Ala Glu Ala
1 5 10 15

Gln Asp Gly Glu Leu Asp Ala Leu Trp Leu Gly Val Leu Arg Leu Leu
20 25 30

Leu Phe Lys Leu Leu Leu Phe Asp Leu Leu Leu Thr Phe Phe Lys Val
35 40 45

Lys Trp Ile Phe Ser Ser Val Val Asp Leu Lys Gln Thr Ile Ile Pro
50 55 60

Asp Tyr Arg Asn Met Ile Gly Gln Gly Ala
65 70

<210> 293
<211> 78
<212> PRT
<213> Artificial Sequence

<220>
<223> GSC7057 / GSC7058 / GSC7056 / GSC7059 / GSC9763 / GSC9764 / GSC9765 / GSC9768 / GSC9770 / GSC9949 / GSC9950

<400> 293

Leu Ser Pro Gly Gly Gly Gly Ser Pro Pro Glu Asp Pro Pro Asp Ser
1 5 10 15

Lys Asn Thr Leu Val Leu Phe Gly Ala Gly Phe Gly Ala Val Ile Thr
20 25 30

Val Val Val Ile Val Val Ile Ile Lys Cys Phe Cys Lys His Arg Ser
35 40 45

Cys Phe Arg Arg Asn Glu Ala Ser Arg Glu Thr Asn Asn Ser Leu Thr
50 55 60

Phe Gly Pro Glu Glu Ala Leu Ala Glu Gln Thr Val Phe Leu
65 70 75

<210> 294
<211> 708
<212> DNA
<213> Artificial Sequence

<220>
<223> GSC315

<400> 294

atggagagcc ctgcccagct gctgttcctg ctgctgctgt ggctgcctga cggagtccat 60

gccgacatcc agatgaccca gtccccctcc agcctgtctg cctccgtggg cgaccgggtg 120

accatcacct gccgggcctc ccaggacgtg aacaccgccg tggcctggta tcagcagaag 180

cctggcaagg cccctaagct gctgatctac tccgcctcct tcctgtactc cggcgtgcct 240

tcccggttct ccggctcccg gtccggcacc gacttcaccc tgaccatctc ctccctgcag 300

cctgaggact tcgccaccta ctactgccag cagcactaca ccacccctcc taccttcggc 360

cagggcacca aggtggagat caagcggacc gtggccgctc cttccgtgtt catcttccct 420

ccctccgacg agcagctgaa gagcggcacc gccagcgtgg tgtgcctgct gaacaacttt 480

taccctcggg aggccaaggt gcagtggaag gtggacaacg ccctgcagtc cggcaactcc 540

caggaatccg tcaccgagca ggactccaag gacagcacct actccctgtc ctccaccctg 600

accctgtcca aggccgacta cgagaagcac aaggtgtacg cctgcgaggt gacccaccag 660

ggcctgtcca gccctgtgac caagtccttc aaccggggcg agtgctga 708

<210> 295
<211> 235
<212> PRT
<213> Artificial Sequence

<220>
<223> GSC315

<400> 295

```
Met Glu Ser Pro Ala Gln Leu Leu Phe Leu Leu Leu Leu Trp Leu Pro
1               5                   10                  15

Asp Gly Val His Ala Asp Ile Gln Met Thr Gln Ser Pro Ser Ser Leu
            20                  25                  30

Ser Ala Ser Val Gly Asp Arg Val Thr Ile Thr Cys Arg Ala Ser Gln
        35                  40                  45

Asp Val Asn Thr Ala Val Ala Trp Tyr Gln Gln Lys Pro Gly Lys Ala
    50                  55                  60

Pro Lys Leu Leu Ile Tyr Ser Ala Ser Phe Leu Tyr Ser Gly Val Pro
65                  70                  75                  80

Ser Arg Phe Ser Gly Ser Arg Ser Gly Thr Asp Phe Thr Leu Thr Ile
                85                  90                  95

Ser Ser Leu Gln Pro Glu Asp Phe Ala Thr Tyr Tyr Cys Gln Gln His
            100                 105                 110

Tyr Thr Thr Pro Pro Thr Phe Gly Gln Gly Thr Lys Val Glu Ile Lys
        115                 120                 125

Arg Thr Val Ala Ala Pro Ser Val Phe Ile Phe Pro Pro Ser Asp Glu
    130                 135                 140

Gln Leu Lys Ser Gly Thr Ala Ser Val Val Cys Leu Leu Asn Asn Phe
145                 150                 155                 160

Tyr Pro Arg Glu Ala Lys Val Gln Trp Lys Val Asp Asn Ala Leu Gln
                165                 170                 175

Ser Gly Asn Ser Gln Glu Ser Val Thr Glu Gln Asp Ser Lys Asp Ser
            180                 185                 190

Thr Tyr Ser Leu Ser Ser Thr Leu Thr Leu Ser Lys Ala Asp Tyr Glu
        195                 200                 205

Lys His Lys Val Tyr Ala Cys Glu Val Thr His Gln Gly Leu Ser Ser
    210                 215                 220

Pro Val Thr Lys Ser Phe Asn Arg Gly Glu Cys
225                 230                 235
```

<210> 296
<211> 1428
<212> DNA

<213> Artificial Sequence

<220>
<223> GSC5607

<400> 296

```
atgcggagcc ctgcccagct gctgttcctg ctgctgctgt ggatccctgg caccaacgcc      60
caggtgcagc tgcagcagtc tggcgccgaa ctggccagac caggcgccag cgtgaagatg     120
agctgcaagg ccagcggcta caccttcacc cggtacacca tgcactgggt gaaacagcgg     180
cctggacagg gcctggaatg gatcggctac atcaacccca gccggggcta caccaactac     240
aaccagaagt tcaaggacaa ggccaccctg accaccgaca agagcagcag caccgcctac     300
atgcagctga gcagcctgac cagcgaggac agcgccgtgt actactgcgc ccggtactac     360
gacgaccact actgcctgga ctactggggc cagggcacca ccctgaccgt gagcagcgcg     420
tcgaccaagg gccccagcgt gttcccgcta gcccccagca gcaagagcac cagcggcggc     480
acagccgccc tgggctgcct ggtgaaggac tacttccccg agcccgttac cgtgtcctgg     540
aactctggag ccctgacctc cggcgtgcac accttccccg ccgtgctcca gagcagcggc     600
ctgtacagcc tgagcagcgt ggtgacagtg cccagcagca gcctgggaac ccagacctac     660
atctgcaacg tgaaccacaa gcccagcaac accaaggtgg acaagaaggt ggagcccaag     720
agctgcgaca aaactcacac ctgcccaccc tgccctgccc ctgagctgct gggcggaccc     780
tccgtgttcc tgttcccccc caagcccaag gacaccctga tgatcagccg gacccccgag     840
gtgacctgcg tggtggtgga cgtgagccac gaggaccctg aggtgaagtt caattggtac     900
gtggacggcg tggaggtgca caacgccaag accaagcccc gggaggaaca gtacaacagc     960
acctaccggg tggtgtccgt gctgaccgtg ctgcaccagg actggctgaa cggcaaggaa    1020
tacaagtgca aggtctccaa caaggccctg cctgccccca tcgaaaagac catcagcaag    1080
gccaagggcc agccgcgcga accgcaggtg tacaccctgc ccccccagccg ggaagagatg    1140
accaagaacc aggtgaaact ggtgtgcctg gtgacaggct tctaccccag cgatatcgcc    1200
gtggaatggg agagcagcgg ccagcctgag aacaactact acaccacccc ccccatgctg    1260
gacagcgacg gcagcttcag cctggtgtcc tggctgaacg tggacaagag ccggtggcag    1320
cagggcaaca tcttcagctg cagcgtgatg cacgaggccc tgcacaaccg gttcacccag    1380
aagtccctga gcctgagccc gggtaaacat caccatcacc atcactga                 1428
```

<210> 297
<211> 475
<212> PRT
<213> Artificial Sequence

<220>
<223> GSC5607

<400> 297

```
Met Arg Ser Pro Ala Gln Leu Leu Phe Leu Leu Leu Leu Trp Ile Pro
1               5                   10                  15

Gly Thr Asn Ala Gln Val Gln Leu Gln Gln Ser Gly Ala Glu Leu Ala
            20                  25                  30

Arg Pro Gly Ala Ser Val Lys Met Ser Cys Lys Ala Ser Gly Tyr Thr
        35                  40                  45

Phe Thr Arg Tyr Thr Met His Trp Val Lys Gln Arg Pro Gly Gln Gly
    50                  55                  60

Leu Glu Trp Ile Gly Tyr Ile Asn Pro Ser Arg Gly Tyr Thr Asn Tyr
65                  70                  75                  80

Asn Gln Lys Phe Lys Asp Lys Ala Thr Leu Thr Thr Asp Lys Ser Ser
                85                  90                  95

Ser Thr Ala Tyr Met Gln Leu Ser Ser Leu Thr Ser Glu Asp Ser Ala
            100                 105                 110

Val Tyr Tyr Cys Ala Arg Tyr Tyr Asp Asp His Tyr Cys Leu Asp Tyr
        115                 120                 125

Trp Gly Gln Gly Thr Thr Leu Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
```

180 185 190

Pro Ala Val Leu Gln Ser Ser Gly Leu Tyr Ser Leu Ser Ser Val Val
195 200 205

Thr Val Pro Ser Ser Ser Leu Gly Thr Gln Thr Tyr Ile Cys Asn Val
210 215 220

Asn His Lys Pro Ser Asn Thr Lys Val Asp Lys Lys Val Glu Pro Lys
225 230 235 240

Ser Cys Asp Lys Thr His Thr Cys Pro Pro Cys Pro Ala Pro Glu Leu
245 250 255

Leu Gly Gly Pro Ser Val Phe Leu Phe Pro Pro Lys Pro Lys Asp Thr
260 265 270

Leu Met Ile Ser Arg Thr Pro Glu Val Thr Cys Val Val Val Asp Val
275 280 285

Ser His Glu Asp Pro Glu Val Lys Phe Asn Trp Tyr Val Asp Gly Val
290 295 300

Glu Val His Asn Ala Lys Thr Lys Pro Arg Glu Glu Gln Tyr Asn Ser
305 310 315 320

Thr Tyr Arg Val Val Ser Val Leu Thr Val Leu His Gln Asp Trp Leu
325 330 335

Asn Gly Lys Glu Tyr Lys Cys Lys Val Ser Asn Lys Ala Leu Pro Ala
340 345 350

Pro Ile Glu Lys Thr Ile Ser Lys Ala Lys Gly Gln Pro Arg Glu Pro
355 360 365

Gln Val Tyr Thr Leu Pro Pro Ser Arg Glu Glu Met Thr Lys Asn Gln
370 375 380

Val Lys Leu Val Cys Leu Val Thr Gly Phe Tyr Pro Ser Asp Ile Ala
385 390 395 400

Val Glu Trp Glu Ser Ser Gly Gln Pro Glu Asn Asn Tyr Tyr Thr Thr
405 410 415

Pro Pro Met Leu Asp Ser Asp Gly Ser Phe Ser Leu Val Ser Trp Leu
420 425 430

Asn Val Asp Lys Ser Arg Trp Gln Gln Gly Asn Ile Phe Ser Cys Ser
435 440 445

Val Met His Glu Ala Leu His Asn Arg Phe Thr Gln Lys Ser Leu Ser
450 455 460

Leu Ser Pro Gly Lys His His His His His His
465 470 475

<210> 298
<211> 469
<212> PRT
<213> Artificial Sequence

<220>
<223> GSC5644

<400> 298

```
Met Arg Ser Pro Ala Gln Leu Leu Phe Leu Leu Leu Leu Trp Ile Pro
1               5                   10                  15

Gly Thr Asn Ala Gln Val Gln Leu Gln Gln Ser Gly Ala Glu Leu Ala
            20                  25                  30

Arg Pro Gly Ala Ser Val Lys Met Ser Cys Lys Ala Ser Gly Tyr Thr
        35                  40                  45

Phe Thr Arg Tyr Thr Met His Trp Val Lys Gln Arg Pro Gly Gln Gly
    50                  55                  60

Leu Glu Trp Ile Gly Tyr Ile Asn Pro Ser Arg Gly Tyr Thr Asn Tyr
65                  70                  75                  80

Asn Gln Lys Phe Lys Asp Lys Ala Thr Leu Thr Thr Asp Lys Ser Ser
                85                  90                  95

Ser Thr Ala Tyr Met Gln Leu Ser Ser Leu Thr Ser Glu Asp Ser Ala
            100                 105                 110

Val Tyr Tyr Cys Ala Arg Tyr Tyr Asp Asp His Tyr Cys Leu Asp Tyr
        115                 120                 125

Trp Gly Gln Gly Thr Thr Leu Thr Val Ser Ser Ala Ser Thr Lys Gly
    130                 135                 140

Pro Ser Val Phe Pro Leu Ala Pro Ser Ser Lys Ser Thr Ser Gly Gly
145                 150                 155                 160

Thr Ala Ala Leu Gly Cys Leu Val Lys Asp Tyr Phe Pro Glu Pro Val
```

```
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Lys Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Glu Glu Met Thr Lys Asn Gln
    370                 375                 380

Val Lys Leu Val Cys Leu Val Thr Gly Phe Tyr Pro Ser Asp Ile Ala
385                 390                 395                 400

Val Glu Trp Glu Ser Ser Gly Gln Pro Glu Asn Asn Tyr Tyr Thr Thr
                405                 410                 415
```

Pro Pro Met Leu Asp Ser Asp Gly Ser Phe Ser Leu Val Ser Trp Leu
420 425 430

Asn Val Asp Lys Ser Arg Trp Gln Gln Gly Asn Ile Phe Ser Cys Ser
435 440 445

Val Met His Glu Ala Leu His Asn Arg Phe Thr Gln Lys Ser Leu Ser
450 455 460

Leu Ser Pro Gly Lys
465

<210> 299
<211> 76
<212> PRT
<213> Artificial Sequence

<220>
<223> GSC5537

<400> 299

Leu Ser Leu Ser Pro Glu Leu Gln Pro Val Glu Ser Cys Ala Glu Ala
1 5 10 15

Gln Asp Gly Glu Leu Asp Gly Leu Trp Thr Thr Ile Thr Ile Phe Ile
20 25 30

Thr Leu Phe Leu Leu Ser Val Cys Tyr Ser Ala Thr Val Thr Phe Phe
35 40 45

Lys Val Lys Trp Ile Phe Ser Ser Val Val Asp Leu Lys Gln Thr Ile
50 55 60

Ile Pro Asp Tyr Arg Asn Met Ile Gly Gln Gly Ala
65 70 75

<210> 300
<211> 495
<212> PRT
<213> Artificial Sequence

<220>
<223> GSC5608

<400> 300

```
Met Arg Ser Pro Ala Gln Leu Leu Phe Leu Leu Leu Leu Trp Ile Pro
1               5                   10                  15

Gly Thr Asn Ala Glu Val Gln Leu Val Glu Ser Gly Gly Gly Leu Val
            20                  25                  30
```

```
Gln Pro Gly Gly Ser Leu Arg Leu Ser Cys Ala Ala Ser Gly Phe Asn
        35                  40                  45

Ile Lys Asp Thr Tyr Ile His Trp Val Arg Gln Ala Pro Gly Lys Gly
    50                  55                  60

Leu Glu Trp Val Ala Arg Ile Tyr Pro Thr Asn Gly Tyr Thr Arg Tyr
65                  70                  75                  80

Ala Asp Ser Val Lys Gly Arg Phe Thr Ile Ser Ala Asp Thr Ser Lys
                85                  90                  95

Asn Thr Ala Tyr Leu Gln Met Asn Ser Leu Arg Ala Glu Asp Thr Ala
            100                 105                 110

Val Tyr Tyr Cys Ser Arg Trp Gly Gly Asp Gly Phe Tyr Ala Met Asp
        115                 120                 125

Tyr Trp Gly Gln Gly Thr Leu Val Thr Val Ser Ser Gly Gly Gly Gly
    130                 135                 140

Ser Gly Gly Gly Gly Ser Gly Gly Gly Gly Ser Asp Ile Gln Met Thr
145                 150                 155                 160

Gln Ser Pro Ser Ser Leu Ser Ala Ser Val Gly Asp Arg Val Thr Ile
                165                 170                 175

Thr Cys Arg Ala Ser Gln Asp Val Asn Thr Ala Val Ala Trp Tyr Gln
            180                 185                 190

Gln Lys Pro Gly Lys Ala Pro Lys Leu Leu Ile Tyr Ser Ala Ser Phe
        195                 200                 205

Leu Tyr Ser Gly Val Pro Ser Arg Phe Ser Gly Ser Arg Ser Gly Thr
    210                 215                 220

Asp Phe Thr Leu Thr Ile Ser Ser Leu Gln Pro Glu Asp Phe Ala Thr
225                 230                 235                 240

Tyr Tyr Cys Gln Gln His Tyr Thr Thr Pro Pro Thr Phe Gly Gln Gly
                245                 250                 255

Thr Lys Val Glu Ile Lys Arg Gly Gly Gly Gly Thr Asp Lys Thr His
            260                 265                 270

Thr Cys Pro Pro Cys Pro Ala Pro Glu Leu Leu Gly Gly Pro Ser Val
        275                 280                 285
```

Phe Leu Phe Pro Pro Lys Pro Lys Asp Thr Leu Met Ile Ser Arg Thr
290 295 300

Pro Glu Val Thr Cys Val Val Val Asp Val Ser His Glu Asp Pro Glu
305 310 315 320

Val Lys Phe Asn Trp Tyr Val Asp Gly Val Glu Val His Asn Ala Lys
325 330 335

Thr Lys Pro Arg Glu Glu Gln Tyr Asn Ser Thr Tyr Arg Val Val Ser
340 345 350

Val Leu Thr Val Leu His Gln Asp Trp Leu Asn Gly Lys Glu Tyr Lys
355 360 365

Cys Lys Val Ser Asn Lys Ala Leu Pro Ala Pro Ile Glu Lys Thr Ile
370 375 380

Ser Lys Ala Lys Gly Gln Pro Arg Glu Pro Glu Val Ala Thr Phe Pro
385 390 395 400

Pro Ser Arg Asp Glu Leu Thr Lys Asn Gln Val Thr Leu Val Cys Leu
405 410 415

Val Thr Gly Phe Tyr Pro Ser Asp Ile Ala Val Glu Trp Glu Ser Asn
420 425 430

Gly Gln Pro Glu Asn Asn Tyr Lys Thr Asp Pro Pro Leu Leu Glu Ser
435 440 445

Asp Gly Ser Phe Ala Leu Ser Ser Arg Leu Arg Val Asp Lys Ser Arg
450 455 460

Trp Gln Gln Gly Asn Val Phe Ser Cys Ser Val Met His Glu Ala Leu
465 470 475 480

His Asn His Tyr Thr Gln Lys Ser Leu Ser Leu Ser Pro Gly Lys
485 490 495

<210> 301
<211> 76
<212> PRT
<213> Artificial Sequence

<220>
<223> GSC5538

<400> 301

Leu Ser Leu Ser Pro Glu Leu Gln Pro Val Glu Ser Cys Ala Glu Ala
1               5                   10                  15

Gln Asp Gly Glu Leu Asp Gly Leu Trp Thr Thr Ile Thr Ile Phe Ile
            20                  25                  30

Thr Leu Phe Leu Leu Ser Val Cys Tyr Ser Ala Thr Val Thr Phe Phe
        35                  40                  45

Lys Val Lys Trp Ile Phe Ser Ser Val Val Asp Leu Lys Gln Thr Ile
    50                  55                  60

Ile Pro Asp Tyr Arg Asn Met Ile Gly Gln Gly Ala
65                  70                  75

<210> 302
<211> 702
<212> DNA
<213> Artificial Sequence

<220>
<223> GSC5540

<400> 302

atgcggagcc ctgcccagct gctgttcctg ctgctgctgt ggatccctgg caccaacgcc 60

cagattgtgc tgacccagag ccccgccatc atgtctgcca gccctggcga gaaagtgacc 120

atgacctgca gcgccagcag cagcgtgtcc tacatgaact ggtatcagca gaagtccggc 180

accagcccca agcggtggat ctacgacacc agcaagctgg cctccggcgt gcccgcccac 240

tttagaggca gcggcagcgg cacctcctac tccctgacca tcagcggcat ggaagccgag 300

gacgccgcca cctactactg ccagcagtgg tccagcaacc ccttcacctt cggctccggc 360

accaaactgg aaattaaccg tacggtggcc gctcccagcg tgttcatctt cccccccagc 420

gacgagcagc tgaagagcgg caccgcctcc gtggtgtgcc tgctgaacaa cttctacccc 480

cgggaggcca aggtgcagtg gaaggtggac aacgccctcc agagcggcaa cagccaggaa 540

agcgtcaccg agcaggacag caaggactcc acctacagcc tgagcagcac cctgaccctg 600

agcaaggccg actacgagaa gcacaaggtg tacgcctgcg aggtgaccca ccagggcctg 660

tccagccccg tgaccaagag cttcaaccgg ggcgagtgct ga 702

<210> 303
<211> 233
<212> PRT
<213> Artificial Sequence

<220>
<223> GSC5540

<400> 303

```
Met Arg Ser Pro Ala Gln Leu Leu Phe Leu Leu Leu Leu Trp Ile Pro
1               5                   10                  15

Gly Thr Asn Ala Gln Ile Val Leu Thr Gln Ser Pro Ala Ile Met Ser
            20                  25                  30

Ala Ser Pro Gly Glu Lys Val Thr Met Thr Cys Ser Ala Ser Ser Ser
        35                  40                  45

Val Ser Tyr Met Asn Trp Tyr Gln Gln Lys Ser Gly Thr Ser Pro Lys
    50                  55                  60

Arg Trp Ile Tyr Asp Thr Ser Lys Leu Ala Ser Gly Val Pro Ala His
65                  70                  75                  80

Phe Arg Gly Ser Gly Ser Gly Thr Ser Tyr Ser Leu Thr Ile Ser Gly
                85                  90                  95

Met Glu Ala Glu Asp Ala Ala Thr Tyr Tyr Cys Gln Gln Trp Ser Ser
            100                 105                 110

Asn Pro Phe Thr Phe Gly Ser Gly Thr Lys Leu Glu Ile Asn Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230
```

<210> 304
<211> 1522
<212> **DNA**
<213> Artificial Sequence

<220>
<223> GSC281

<400> 304

```
gaattgtacc tgcccgtaca taaggtcaat aggggtgaat caacggaaaa gtcccattgg      60
agccaagtac actgcgtcaa tagggacttt ccattgggtt ttgcccagta cataaggtca     120
ataggggatg agtcaatggg aaaaacccat tggagccaag tacactgact caatagggac     180
tttccattgg gttttgccca gtacataagg tcaatagggg gtgagtcaac aggaaagttc     240
cattggagcc aagtacattg agtcaatagg gactttccaa tgggttttgc ccagtacata     300
aggtcaatgg gaggtaagcc aatgggtttt tcccattact ggcacgtata ctgagtcatt     360
agggactttc caatgggttt tgcccagtac ataaggtcaa taggggtgaa tcaacaggaa     420
agtcccattg gagccaagta cactgagtca atagggactt tccattgggt tttgcccagt     480
acaaaaggtc aatagggggt gagtcaatgg gtttttccca ttattggcac gtacataagg     540
tcaatagggg tgagtcattg ggtttttcca gccaatttaa ttaaaacgcc atgtactttc     600
ccaccattga cgtcaatggg ctattgaaac taatgcaacg tgacctttaa acggtacttt     660
cccatagctg attaatggga aagtaccgtt ctcgagccaa tacacgtcaa tgggaagtga     720
aagggcagcc aaaacgtaac accgccccgg ttttccctgg aaattccata ttggcacgca     780
ttctattggc tgagctgcgt tcacgtgggt ataagcagag ctcgtttagt gaaccgtcag     840
atcgcctgga gacgccatcc acgctgtttt gacctccata gaagacaccg ggaccgatcc     900
agcctccgcg gccgggaacg gtgcattgga acgcggattc cccgtgccaa gagtgacgta     960
agtaccgcct atagagtcta taggcccacc cccttggctt cttatgcgac ggatcccgta    1020
ctatgaggtg tggcaggctt gagatctggc catacacttg agtgacaatg acatccactt    1080
tgcctttctc tccacaggtg tccactccca cgtccaactg cagctcggtt cgatcgataa    1140
ttaattaagc tagcggcgcc gttaacaccg gttgatcaaa gcttgcggcc gcgcttagcg    1200
atatcgaatt ctctagaggt acccccgggg ggcccctcga gttcgaaatg accgaccaag    1260
cgacgcccaa cctgccatca cgagatttcg attccaccgc cgccttctat gaaaggttgg    1320
gcttcggaat cgttttccgg gacgccggct ggatgatcct ccagcgcggg gatctcatgc    1380
tggagttctt cgcccacccc aacttgttta ttgcagctta taatggttac aaataaagca    1440
atagcatcac aaatttcaca aataaagcat ttttttcact gcattctagt tgtggtttgt    1500
ccaaactcat caatgtatct ta                                              1522
```

<210> 305
<211> 132
<212> DNA
<213> Artificial Sequence

<220>
<223> GSC3469

<400> 305

```
aacttgttta ttgcagctta taatggttac aaataaagca atagcatcac aaatttcaca        60
aataaagcat ttttttcact gcattctagt tgtggtttgt ccaaactcat caatgtatct       120
tatcatgtct gt                                                           132
```

<210> 306
<211> 470
<212> DNA
<213> Artificial Sequence

<220>
<223> GSC23

<400> 306

```
cgaaatgacc gaccaagcga cgcccaacct gccatcacga gatttcgatt ccaccgccgc        60
cttctatgaa aggttgggct tcggaatcgt tttccgggac gccggctgga tgatcctcca       120
gcgcggggat ctcatgctgg agttcttcgc ccaccccaac ttgtttattg cagcttataa       180
tggttacaaa taaagcaata gcatcacaaa tttcacaaat aaagcatttt tttcactgca       240
ttctagttgt ggtttgtcca aactcatcaa tgtatcttat catgtctgta taccgtcgac       300
ctctagctag agcttggcgt aatcatggtc atagctgttt cctgtgtgaa attgttatcc       360
gctcacaatt ccacacaaca tacgagccgg ggcaggataa tatatggtag ggttcatagc       420
cagagtaacc tttttttta atttttattt tattttattt ttgagatcag                   470
```

<210> 307
<211> 528
<212> DNA
<213> Artificial Sequence

<220>
<223> GSC4340

<400> 307

ggtaggtgat cctcctgctg ctttggttca gggttttgct tgaggggggg gggtggtgat 60

ttccttgcca tgggcagact gagcagaaaa ggccattggg accatgttct gaatgcctcc 120

acctcaacca ccggccggta ggaccaaagc caccccgtgt tttctcagga tctcttttcc 180

cagggagatc cctcggccca aagagggaga tggcaatgct ggatgtgtgc acaataattc 240

aacaggcatt ggaacttcag catcgatgct gaatgcaatt aacaatgctc aagcagaacc 300

cccggctcca tcagcacagt gcaggaccaa accccatgct gcagcagtgg ggctgtctgt 360

acggggtggg caatgggaac cggggtctgc tggggctcct gctgcttcag tgctgccatg 420

cagccacaca tcctgagagc tgaaagggtc ggcgtcctca cctggtgcac accgtagctc 480

tgccccacag ctttaaggca cctggctaac cgagacctga ggggacag 528

<210> 308
<211> 528
<212> DNA
<213> Artificial Sequence

<220>
<223> GSC4222

<400> 308

ggtaggtgat cctcctgctg ctttggttca gggttttgct tgaggggggg gggtggtgat 60

ttccttgcca tgggcagact gagcagaaaa ggccattggg accatgttct gaatgcctcc 120

acctcaacca ccggccggta ggaccaaagc caccccgtgt tttctcagga tctcttttcc 180

cagggagatc cctcggccca aagagggaga tggcaatgct ggatgtgtgc acaataattc 240

aacaggcatt ggaacttcag catcgatgct gaatgcaatt aacaatgctc aagcagaacc 300

cccggctcca tcagcacagt gcaggaccaa accccatgct gcagcagtgg ggctgtctgt 360

acggggtggg caatgggaac cggggtctgc tggggctcct gctgcttcag tgctgccatg 420

cagccacaca tcctgagagc tgaaagggtc ggcgtcctca cctggtgcac accgtagctc 480

tgccccacag ctttaaggca cctggctaac cctgtcctta ttgcacag 528

<210> 309
<211> 528
<212> DNA
<213> Artificial Sequence

<220>
<223> GSC2617

<400> 309

ggtaggtgat cctcctgctg ctttggttca gggttttgct tgaggggggg gggtggtgat 60
ttccttgcca tgggcagact gagcagaaaa ggccattggg accatgttct gaatgcctcc 120
acctcaacca ccggccggta ggaccaaagc caccccgtgt tttctcagga tctcttttcc 180
cagggagatc cctcggccca aagagggaga tggcaatgct ggatgtgtgc acaataattc 240
aacaggcatt ggaacttcag catcgatgct gaatgcaatt aacaatgctc aagcagaacc 300
cccggctcca tcagcacagt gcaggaccaa accccatgct gcagcagtgg ggctgtctgt 360
acggggtggg caatgggaac cggggtctgc tggggctcct gctgcttcag tgctgccatg 420
cagccacaca tcctgagagc tgaaagggtc ggcgtcctca cctggtgcac accgtagctc 480
tgccccacag ctttaaggca cctggctaac cctgggagga ttgcacag 528

<210> 310
<211> 528
<212> DNA
<213> Artificial Sequence

<220>
<223> GSC4335

<400> 310

ggtaggtgat cctcctgctg ctttggttca gggttttgct tgaggggggg gggtggtgat 60
ttccttgcca tgggcagact gagcagaaaa ggccattggg accatgttct gaatgcctcc 120
acctcaacca ccggccggta ggaccaaagc caccccgtgt tttctcagga tctcttttcc 180
cagggagatc cctcggccca aagagggaga tggcaatgct ggatgtgtgc acaataattc 240
aacaggcatt ggaacttcag catcgatgct gaatgcaatt aacaatgctc aagcagaacc 300
cccggctcca tcagcacagt gcaggaccaa accccatgct gcagcagtgg ggctgtctgt 360
acggggtggg caatgggaac cggggtctgc tggggctcct gctgcttcag tgctgccatg 420
cagccacaca tcctgagagc tgaaagggtc ggcgtcctca cctggtgcac accgtagctc 480
tgccccacag ctttaaggca cctggctaac cgtctccttc tgggacag 528

<210> 311
<211> 546
<212> DNA
<213> Artificial Sequence

<220>
<223> GSC2332

<400> 311

ggtaggtgat cctcctgctg ctttggttca gggttttgct tgaggggggg gggtggtgat 60
ttccttgcca tgggcagact gagcagaaaa ggccattggg accatgttct gaatgcctcc 120
acctcaacca ccggccggta ggaccaaagc caccccgtgt tttctcagga tctcttttcc 180
cagggagatc cctcggccca aagagggaga tggcaatgct ggatgtgtgc acaataattc 240
aacaggcatt ggaacttcag catcgatgct gaatgcaatt aacaatgctc aagcagaacc 300
cccggctcca tcagcacagt gcaggaccaa accccatgct gcagcagtgg ggctgtctgt 360
acggggtggg caatgggaac cggggtctgc tggggctcct gctgcttcag tgctgccatg 420
cagccacaca tcctgagagc tgaaagggtc ggcgtcctca cctggtgcac accgtagctc 480
tgccccacag ctttaaggca cctggctaac ctctgcgctt cttcccttcc ctgctacctg 540
gctcag 546

<210> 312
<211> 36
<212> DNA
<213> Artificial Sequence

<220>
<223> Primer GlnPr2160

<400> 312
ccaagctttc atcatttgcc cggagacagg gagagg 36

<210> 313
<211> 42
<212> DNA
<213> Artificial Sequence

<220>
<223> Primer GlnPr2161

<400> 313
ccaagctttc atcatttacc gggagacagg gagaggctct tc 42

<210> 314
<211> 49
<212> DNA
<213> Artificial Sequence

<220>
<223> Primer GlnPr2162

<400> 314
gtcctcgggg ggagatccac caccacctgt cccagaagga gacggttag 49

<210> 315
<211> 49
<212> DNA
<213> Artificial Sequence

<220>

<223> Primer GlnPr2163

<400> 315
gtcctcgggg ggagatccac caccacctgt gcaatcctcc cagggttag 49

<210> 316
<211> 49
<212> DNA
<213> Artificial Sequence

<220>
<223> Primer GlnPr2164

<400> 316
gtcctcgggg ggagatccac caccacctgt gcaataagga cagggttag 49

<210> 317
<211> 49
<212> DNA
<213> Artificial Sequence

<220>
<223> Primer GlnPr2165

<400> 317
gtcctcgggg ggagatccac caccacctgt cccctcaggt ctcggttag 49

<210> 318
<211> 49
<212> DNA
<213> Artificial Sequence

<220>
<223> Primer GlnPr2166

<400> 318
gtcctcgggg ggagatccac caccacctga gccaggtagc agggaaggg 49

<210> 319
<211> 705
<212> DNA
<213> Artificial Sequence

<220>
<223> Construct GSC3704

<400> 319

atggaaagcc cagcccagct gctgttcctg ctgctgctgt ggctgcccga cggggtgcac 60

gctgagatcg tgctgacaca gagccccgcc accctgtctc tgagccctgg cgaaagagcc 120

accctgagct gtagagccag cagcagcgtg tcctacatgc actggtatca gcagaagccc 180

ggccaggcgc cgcgcccgtg gatttatgcg accagcaatc gggccacagg catccctgcc 240

agattttctg gcagcggctc cggcaccgac tacaccctga ccatctccag cctggaaccc 300

gaggacttcg ccgtgtacta ctgccagcag tggtccagca acccctggac atttggccag 360

ggcaccaaag tggaaattaa acgtacggtg gccgctccca gcgtgttcat cttccccccc 420

agcgacgagc agctgaagag cggcaccgcc tccgtggtgt gcctgctgaa caacttctac 480

ccccgggagg ccaaggtgca gtggaaggtg gacaacgccc tccagagcgg caacagccag 540

gaaagcgtca ccgagcagga cagcaaggac tccacctaca gcctgagcag caccctgacc 600

ctgagcaagg ccgactacga gaagcacaag gtgtacgcct gcgaggtgac ccaccagggc 660

ctgtccagcc ccgtgaccaa gagcttcaac cggggcgagt gctga 705

<210> 320
<211> 234
<212> PRT
<213> Artificial Sequence

<220>
<223> Construct GSC3704

<400> 320

Met Glu Ser Pro Ala Gln Leu Leu Phe Leu Leu Leu Leu Trp Leu Pro
1               5                   10                  15

Asp Gly Val His Ala Glu Ile Val Leu Thr Gln Ser Pro Ala Thr Leu

```
                        20                  25                  30

Ser Leu Ser Pro Gly Glu Arg Ala Thr Leu Ser Cys Arg Ala Ser Ser
        35                  40                  45

Ser Val Ser Tyr Met His Trp Tyr Gln Gln Lys Pro Gly Gln Ala Pro
    50                  55                  60

Arg Pro Trp Ile Tyr Ala Thr Ser Asn Arg Ala Thr Gly Ile Pro Ala
65                  70                  75                  80

Arg Phe Ser Gly Ser Gly Ser Gly Thr Asp Tyr Thr Leu Thr Ile Ser
                85                  90                  95

Ser Leu Glu Pro Glu Asp Phe Ala Val Tyr Tyr Cys Gln Gln Trp Ser
            100                 105                 110

Ser Asn Pro Trp Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

<210> 321
<211> 1094
<212> DNA
<213> Artificial Sequence

<220>
<223> Construct GSC10488

<400> 321

```
ctgtctccgg gtaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatgctgaa     300
tgcaattaac aatgctcaag cagaaccccc ggctccatca gcacagtgca ggaccaaacc     360
ccatgctgca gcagtggggc tgtctgtacg ggtgggcaa tgggaaccgg ggtctgctgg     420
ggctcctgct gcttcagtgc tgccatgcag ccacacatcc tgagagctga aagggtcggc     480
gtcctcacct ggtgcacacc gtagctctgc cccacagctt taaggcacct ggctaacctc     540
tgcgcttctt cccttccctc ctccctggct caggtggtgg tggatctccc cccgaggacc     600
cccctgactc caagaatacc ctggtgctgt tcggcgctgg cttcggcgcc gtgattaccg     660
tggtcgtgat cgtcgtgatt atcaagtgct tctgcaagca ccggtcctgc ttccggcgga     720
acgaggcctc cagagagaca aacaactccc tgaccttcgg ccccgaggaa gccctggctg     780
agcagaccgt gtttctgtga tgattcgaaa tgaccgacca agcgacgccc aacctgccat     840
cacgagattt cgattccacc gccgccttct atgaaaggtt gggcttcgga atcgttttcc     900
gggacgccgg ctggatgatc ctccagcgcg gggatctcat gctggagttc ttcgcccacc     960
ccaacttgtt tattgcagct tataatggtt acaaataaag caatagcatc acaaatttca    1020
caaataaagc atttttttca ctgcattcta gttgtggttt gtccaaactc atcaatgtat    1080
cttatcatgt ctgt                                                      1094
```

<210> 322
<211> 80
<212> PRT
<213> Artificial Sequence

<220>
<223> Construct GSC10488

<400> 322

Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Lys Cys Phe Cys Lys His
        35                  40                  45

Arg Ser Cys Phe Arg Arg Asn Glu Ala Ser Arg Glu Thr Asn Asn Ser
    50                  55                  60

Leu Thr Phe Gly Pro Glu Glu Ala Leu Ala Glu Gln Thr Val Phe Leu
65                  70                  75                  80

<210> 323
<211> 1094
<212> DNA
<213> Artificial Sequence

<220>
<223> Construct GSC10487

<400> 323

ctgtctccgg gtaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgatgctgaa 300
tgcaattaac aatgctcaag cagaaccccc ggctccatca gcacagtgca ggaccaaacc 360
ccatgctgca gcagtggggc tgtctgtacg gggtgggcaa tgggaaccgg ggtctgctgg 420
ggctcctgct gcttcagtgc tgccatgcag ccacacatcc tgagagctga aagggtcggc 480
gtcctcacct ggtgcacacc gtagctctgc cccacagctt taaggcacct ggctaacctc 540
tgcgcttctt cccttccctc ctccctggct caggtggtgg tggatctccc cccgaggacc 600
cccctgactc caagaatacc ctggtgctgt tcggcgctgg cttcggcgcc gtgattaccg 660
tggtcgtgat cgtcgtgatt atcaagtgct tctgcaagca ccggtcctgc ttccggcgga 720
acgaggcctc cagagagaca aacaactccc tgaccttcgg ccccgaggaa gccctggctg 780
agcagaccgt gtttctgtga tgattcgaaa tgaccgacca agcgacgccc aacctgccat 840
cacgagattt cgattccacc gccgccttct atgaaaggtt gggcttcgga atcgttttcc 900
gggacgccgg ctggatgatc ctccagcgcg gggatctcat gctggagttc ttcgcccacc 960
ccaacttgtt tattgcagct tataatggtt acaaataaag caatagcatc acaaatttca 1020
caaataaagc atttttttca ctgcattcta gttgtggttt gtccaaactc atcaatgtat 1080
cttatcatgt ctgt 1094

<210> 324
<211> 80
<212> PRT
<213> Artificial Sequence

<220>
<223> Construct GSC10487

<400> 324

```
Leu Ser Leu Ser Pro Gly Gly Gly Gly Ser Pro Pro Glu Asp Pro Pro
1               5                   10                  15

Asp Ser Lys Asn Thr Leu Val Leu Phe Gly Ala Gly Phe Gly Ala Val
            20                  25                  30

Ile Thr Val Val Val Ile Val Val Ile Ile Lys Cys Phe Cys Lys His
        35                  40                  45

Arg Ser Cys Phe Arg Arg Asn Glu Ala Ser Arg Glu Thr Asn Asn Ser
    50                  55                  60

Leu Thr Phe Gly Pro Glu Glu Ala Leu Ala Glu Gln Thr Val Phe Leu
65                  70                  75                  80
```

<210> 325
<211> 53
<212> DNA
<213> Artificial Sequence

<220>
<223> GlnPr1487

<400> 325
ctgctgctgt ggctgcccga cggggtgcac gctgagatcg tgctgacaca gag 53

<210> 326
<211> 35
<212> DNA
<213> Artificial Sequence

<220>
<223> GlnPr1491

<400> 326
ccaaatcgat ttatcagcac tcgccccggt tgaag 35

<210> 327
<211> 31
<212> DNA
<213> Artificial Sequence

<220>
<223> GlnPr1452

<400> 327
acgtatcgat tcatcacttg ccgggggaca g 31

<210> 328
<211> 53
<212> DNA
<213> Artificial Sequence

<220>
<223> GlnPr1488

<400> 328
ctgctgctgt ggctgcccga cggggtgcac gctcaggtca cactgaaaga gtc 53

<210> 329
<211> 1470
<212> DNA
<213> Artificial Sequence

<220>
<223> pGLEX41_HC-GGC-I4(SV40ter)-B7-B7(18)-6His

<400> 329

ctgtctccgg gcaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgaaatgacc 300
gaccaagcga cgcccaacct gccatcacga gatttcgatt ccaccgccgc cttctatgaa 360
aggttgggct tcggaatcgt tttccgggac gccggctgga tgatcctcca gcgcggggat 420
ctcatgctgg agttcttcgc ccaccccaac ttgtttattg cagcttataa tggttacaaa 480
taaagcaata gcatcacaaa tttcacaaat aaagcatttt tttcactgca ttctagttgt 540
ggtttgtcca aactcatcaa tgtatcttat catgtctgta taccgtcgac ctctagctag 600
agcttggcgt aatcatggtc atagctgttt cctgtgtgaa attgttatcc gctcacaatt 660
ccacacaaca tacgagccgg ggcaggataa tatatggtag ggttcatagc cagagtaacc 720
ttttttttta atttttattt tattttattt ttgagatcag ttcgatgctg aatgcaatta 780
acaatgctca agcagaaccc ccggctccat cagcacagtg caggaccaaa ccccatgctg 840
cagcagtggg gctgtctgta cggggtgggc aatgggaacc ggggtctgct ggggctcctg 900
ctgcttcagt gctgccatgc agccacacat cctgagagct gaaagggtcg gcgtcctcac 960
ctggtgcaca ccgtagctct gccccacagc tttaaggcac ctggctaacc tctgcgcttc 1020
ttcccttccc tcctccctgg ctcaggtggt ggtggatctc cccccgagga cccccctgac 1080
tccaagaata ccctggtgct gttcggcgct ggcttcggcg ccaccgtgat cgtcgtgatt 1140
atcgccagcg cccatcacca ccaccatcat tgatgatgat tcgaaatgac cgaccaagcg 1200
acgcccaacc tgccatcacg agatttcgat tccaccgccg ccttctatga aaggttgggc 1260
ttcggaatcg ttttccggga cgccggctgg atgatcctcc agcgcgggga tctcatgctg 1320
gagttcttcg cccaccccaa cttgtttatt gcagcttata atggttacaa ataaagcaat 1380
agcatcacaa atttcacaaa taaagcattt ttttcactgc attctagttg tggtttgtcc 1440

aaactcatca atgtatctta tcatgtctgt 1470

<210> 330
<211> 46
<212> PRT
<213> Artificial Sequence

<220>
<223> pGLEX41_HC-GGC-I4(SV40ter)-B7-B7(18)-6His

<400> 330

```
Leu Ser Leu Ser Arg Gly Gly Gly Ser Pro Pro Glu Asp Pro Pro Asp
1               5                   10                  15

Ser Lys Asn Thr Leu Val Leu Phe Gly Ala Gly Phe Gly Ala Thr Val
            20                  25                  30

Ile Val Val Ile Ile Ala Ser Ala His His His His His His
        35                  40                  45
```

<210> 331
<211> 1135
<212> DNA
<213> Artificial Sequence

<220>
<223> pGLEX41_HC-GGC-I4(polyA)-B7-B7(18)-6His

<400> 331

```
ctgtctccgg gcaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatacttgt     300
ttattgcagc ttataatggt tacaaataaa gcaatagcat cacaaatttc acaaataaag     360
catttttttc actgcattct agttgtggtt tgtccaaact catcaatgta tcttatcatg     420
tctgtatcga tgctgaatgc aattaacaat gctcaagcag aacccccggc tccatcagca     480
cagtgcagga ccaaacccca tgctgcagca gtggggctgt ctgtacgggg tgggcaatgg     540
gaaccggggt ctgctggggc tcctgctgct tcagtgctgc catgcagcca cacatcctga     600
gagctgaaag ggtcggcgtc ctcacctggt gcacaccgta gctctgcccc acagctttaa     660
ggcacctggc taacctctgc gcttcttccc ttccctcctc cctggctcag gtggtggtgg     720
atctcccccc gaggaccccc ctgactccaa gaataccctg gtgctgttcg gcgctggctt     780
cggcgccacc gtgatcgtcg tgattatcgc cagcgcccat caccaccacc atcattgatg     840

atgattcgaa atgaccgacc aagcgacgcc caacctgcca tcacgagatt tcgattccac     900
cgccgccttc tatgaaaggt tgggcttcgg aatcgttttc cgggacgccg gctggatgat     960
cctccagcgc ggggatctca tgctggagtt cttcgcccac cccaacttgt ttattgcagc    1020
ttataatggt tacaaataaa gcaatagcat cacaaatttc acaaataaag catttttttc    1080
actgcattct agttgtggtt tgtccaaact catcaatgta tcttatcatg tctgt         1135
```

<210> 332
<211> 45
<212> PRT
<213> Artificial Sequence

<220>
<223> pGLEX41_HC-GGC-I4(polyA)-B7-B7(18)-6His

<400> 332

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Ile
            20                  25                  30

Val Val Ile Ile Ala Ser Ala His His His His His His
        35                  40                  45
```

<210> 333
<211> 1123
<212> DNA
<213> Artificial Sequence

<220>
<223> pGLEX41_HC-GGC-I4(polyA)-B7-B7(18)-KCFCK

<400> 333

```
ctgtctccgg gcaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatacttgt     300
ttattgcagc ttataatggt tacaaataaa gcaatagcat cacaaatttc acaaataaag     360
catttttttc actgcattct agttgtggtt tgtccaaact catcaatgta tcttatcatg     420
tctgtatcga tgctgaatgc aattaacaat gctcaagcag aacccccggc tccatcagca     480
cagtgcagga ccaaacccca tgctgcagca gtggggctgt ctgtacgggg tgggcaatgg     540
gaaccggggt ctgctggggc tcctgctgct tcagtgctgc catgcagcca cacatcctga     600
gagctgaaag ggtcggcgtc ctcacctggt gcacaccgta gctctgcccc acagctttaa     660
ggcacctggc taacctctgc gcttcttccc ttccctcctc cctggctcag gtggtggtgg     720
atctcccccc gaggaccccc ctgactccaa gaataccctg gtgctgttcg gcgctggctt     780
cggcgccacc gtgatcgtcg tgattatcaa gtgcttctgc aagtgatgat gattcgaaat     840
gaccgaccaa gcgacgccca acctgccatc acgagatttc gattccaccg ccgccttcta     900
tgaaaggttg ggcttcggaa tcgttttccg ggacgccggc tggatgatcc tccagcgcgg     960
ggatctcatg ctggagttct tcgcccaccc caacttgttt attgcagctt ataatggtta    1020
caaataaagc aatagcatca caaatttcac aaataaagca tttttttcac tgcattctag    1080
ttgtggtttg tccaaactca tcaatgtatc ttatcatgtc tgt                      1123
```

<210> 334
<211> 41
<212> PRT
<213> Artificial Sequence

<220>
<223> pGLEX41_HC-GGC-I4(polyA)-B7-B7(18)-KCFCK

<400> 334

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Ile
            20                  25                  30

Val Val Ile Ile Lys Cys Phe Cys Lys
        35                  40
```

<210> 335
<211> 1141
<212> DNA
<213> Artificial Sequence

<220>
<223> pGLEX41_HC-GGC-I4(polyA)-B7-B7(20)-6His

<400> 335

```
ctgtctccgg gcaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgatacttgt     300
ttattgcagc ttataatggt tacaaataaa gcaatagcat cacaaatttc acaaataaag     360
cattttttc actgcattct agttgtggtt tgtccaaact catcaatgta tcttatcatg      420
```

```
tctgtatcga tgctgaatgc aattaacaat gctcaagcag aacccccggc tccatcagca      480
cagtgcagga ccaaacccca tgctgcagca gtggggctgt ctgtacgggg tgggcaatgg      540
gaaccggggt ctgctggggc tcctgctgct tcagtgctgc catgcagcca cacatcctga      600
gagctgaaag ggtcggcgtc ctcacctggt gcacaccgta gctctgcccc acagctttaa      660
ggcacctggc taacctctgc gcttcttccc ttccctcctc cctggctcag gtggtggtgg      720
atctccccc gaggaccccc ctgactccaa gaataccctg gtgctgttcg gcgctggctt      780
cggcgccacc gtggtcgtga tcgtcgtgat tatcgccagc gcccatcacc accaccatca      840
ttgatgatga ttcgaaatga ccgaccaagc gacgcccaac ctgccatcac gagatttcga      900
ttccaccgcc gccttctatg aaaggttggg cttcggaatc gttttccggg acgccggctg      960
gatgatcctc cagcgcgggg atctcatgct ggagttcttc gcccacccca acttgtttat     1020
tgcagcttat aatggttaca aataaagcaa tagcatcaca aatttcacaa ataaagcatt     1080
tttttcactg cattctagtt gtggtttgtc caaactcatc aatgtatctt atcatgtctg     1140
t                                                                     1141
```

<210> 336
<211> 47
<212> PRT
<213> Artificial Sequence

<220>
<223> pGLEX41_HC-GGC-I4(polyA)-B7-B7(20)-6His

<400> 336

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Val
            20                  25                  30

Val Ile Val Val Ile Ile Ala Ser Ala His His His His His His
        35                  40                  45
```

<210> 337
<211> 1129
<212> DNA
<213> Artificial Sequence

<220>
<223> pGLEX41_HC-GGC-I4(polyA)-B7-B7(20)-KCFCK

<400> 337

```
ctgtctccgg gcaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180

cccgtgtttt ctcaggatct cttttcccag ggagatccct cggcccaaag agggagatgg     240
caatgctgga tgtgtgcaca ataattcaac aggcattgga acttcagcat cgatacttgt     300
ttattgcagc ttataatggt tacaaataaa gcaatagcat cacaaatttc acaaataaag     360
catttttttc actgcattct agttgtggtt tgtccaaact catcaatgta tcttatcatg     420
tctgtatcga tgctgaatgc aattaacaat gctcaagcag aacccccggc tccatcagca     480
cagtgcagga ccaaacccca tgctgcagca gtggggctgt ctgtacgggg tgggcaatgg     540
gaaccggggt ctgctggggc tcctgctgct tcagtgctgc catgcagcca cacatcctga     600
gagctgaaag ggtcggcgtc ctcacctggt gcacaccgta gctctgcccc acagctttaa     660
ggcacctggc taacctctgc gcttcttccc ttccctcctc cctggctcag gtggtggtgg     720
atctccccccc gaggaccccc ctgactccaa gaataccctg gtgctgttcg gcgctggctt     780
cggcgccacc gtggtcgtga tcgtcgtgat tatcaagtgc ttctgcaagt gatgatgatt     840
cgaaatgacc gaccaagcga cgcccaacct gccatcacga gatttcgatt ccaccgccgc     900
cttctatgaa aggttgggct tcggaatcgt tttccgggac gccggctgga tgatcctcca     960
gcgcggggat ctcatgctgg agttcttcgc ccaccccaac ttgtttattg cagcttataa    1020
tggttacaaa taaagcaata gcatcacaaa tttcacaaat aaagcatttt tttcactgca    1080
ttctagttgt ggtttgtcca aactcatcaa tgtatcttat catgtctgt               1129
```

<210> 338
<211> 43
<212> PRT
<213> Artificial Sequence

<220>
<223> pGLEX41_HC-GGC-I4(polyA)-B7-B7(20)-KCFCK

<400> 338

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Val
            20                  25                  30

Val Ile Val Val Ile Ile Lys Cys Phe Cys Lys
        35                  40
```

<210> 339
<211> 1458
<212> DNA
<213> Artificial Sequence

<220>
<223> pGLEX41_HC-GGC-I4(SV40ter)-B7-B7(18)-KCFCK

<400> 339

ctgtctccgg gcaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgaaatgacc 300
gaccaagcga cgcccaacct gccatcacga gatttcgatt ccaccgccgc cttctatgaa 360
aggttgggct tcggaatcgt tttccgggac gccggctgga tgatcctcca gcgcggggat 420
ctcatgctgg agttcttcgc ccaccccaac ttgtttattg cagcttataa tggttacaaa 480
taaagcaata gcatcacaaa tttcacaaat aaagcatttt tttcactgca ttctagttgt 540
ggtttgtcca aactcatcaa tgtatcttat catgtctgta taccgtcgac ctctagctag 600
agcttggcgt aatcatggtc atagctgttt cctgtgtgaa attgttatcc gctcacaatt 660
ccacacaaca tacgagccgg ggcaggataa tatatggtag ggttcatagc cagagtaacc 720
ttttttttta atttttattt tattttattt ttgagatcag ttcgatgctg aatgcaatta 780
acaatgctca agcagaaccc ccggctccat cagcacagtg caggaccaaa ccccatgctg 840
cagcagtggg gctgtctgta cggggtgggc aatgggaacc ggggtctgct ggggctcctg 900
ctgcttcagt gctgccatgc agccacacat cctgagagct gaaagggtcg gcgtcctcac 960
ctggtgcaca ccgtagctct gccccacagc tttaaggcac ctggctaacc tctgcgcttc 1020
ttcccttccc tcctccctgg ctcaggtggt ggtggatctc cccccgagga cccccctgac 1080
tccaagaata ccctggtgct gttcggcgct ggcttcggcg ccaccgtgat cgtcgtgatt 1140
atcaagtgct tctgcaagtg atgatgattc gaaatgaccg accaagcgac gcccaacctg 1200
ccatcacgag atttcgattc caccgccgcc ttctatgaaa ggttgggctt cggaatcgtt 1260
ttccgggacg ccggctggat gatcctccag cgcggggatc tcatgctgga gttcttcgcc 1320
caccccaact tgtttattgc agcttataat ggttacaaat aaagcaatag catcacaaat 1380
ttcacaaata aagcattttt ttcactgcat tctagttgtg gtttgtccaa actcatcaat 1440
gtatcttatc atgtctgt 1458

<210> 340

<211> 41
<212> PRT
<213> Artificial Sequence

<220>
<223> pGLEX41_HC-GGC-I4(SV40ter)-B7-B7(18)-KCFCK

<400> 340

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Ile
            20                  25                  30

Val Val Ile Ile Lys Cys Phe Cys Lys
        35                  40
```

<210> 341
<211> 1476
<212> DNA
<213> Artificial Sequence

<220>
<223> pGLEX41_HC-GGC-I4(SV40ter)-B7-B7(20)-6His

<400> 341

ctgtctccgg gcaaatgatg aaagcttggt aggtgatcct cctgctgctt tggttcaggg 60
ttttgcttga gggggggggg tggtgatttc cttgccatgg gcagactgag cagaaaaggc 120
cattgggacc atgttctgaa tgcctccacc tcaaccaccg gccggtagga ccaaagccac 180
cccgtgtttt ctcaggatct cttttcccag ggagatccct cggcccaaag agggagatgg 240
caatgctgga tgtgtgcaca ataattcaac aggcattgga acttcagcat cgaaatgacc 300
gaccaagcga cgcccaacct gccatcacga gatttcgatt ccaccgccgc cttctatgaa 360
aggttgggct tcggaatcgt tttccgggac gccggctgga tgatcctcca gcgcggggat 420
ctcatgctgg agttcttcgc ccaccccaac ttgtttattg cagcttataa tggttacaaa 480
taaagcaata gcatcacaaa tttcacaaat aaagcatttt tttcactgca ttctagttgt 540
ggtttgtcca aactcatcaa tgtatcttat catgtctgta taccgtcgac ctctagctag 600
agcttggcgt aatcatggtc atagctgttt cctgtgtgaa attgttatcc gctcacaatt 660
ccacacaaca tacgagccgg ggcaggataa tatatggtag ggttcatagc cagagtaacc 720
ttttttttta atttttattt tattttattt ttgagatcag ttcgatgctg aatgcaatta 780
acaatgctca agcagaaccc ccggctccat cagcacagtg caggaccaaa ccccatgctg 840
cagcagtggg gctgtctgta cggggtgggc aatgggaacc ggggtctgct ggggctcctg 900
ctgcttcagt gctgccatgc agccacacat cctgagagct gaaagggtcg gcgtcctcac 960
ctggtgcaca ccgtagctct gccccacagc tttaaggcac ctggctaacc tctgcgcttc 1020
ttcccttccc tcctccctgg ctcaggtggt ggtggatctc cccccgagga cccccctgac 1080
tccaagaata ccctggtgct gttcggcgct ggcttcggcg ccaccgtggt cgtgatcgtc 1140
gtgattatcg ccagcgccca tcaccaccac catcattgat gatgattcga aatgaccgac 1200
caagcgacgc ccaacctgcc atcacgagat ttcgattcca ccgccgcctt ctatgaaagg 1260
ttgggcttcg gaatcgtttt ccgggacgcc ggctggatga tcctccagcg cggggatctc 1320
atgctggagt tcttcgccca ccccaacttg tttattgcag cttataatgg ttacaaataa 1380
agcaatagca tcacaaattt cacaaataaa gcattttttt cactgcattc tagttgtggt 1440
ttgtccaaac tcatcaatgt atcttatcat gtctgt 1476

<210> 342
<211> 47
<212> **PRT**
<213> **Artificial** Sequence

<220>
<223> **pGLEX41_HC-GGC-I4(SV40ter)-B7-B7(20)-6His**

<400> 342

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Val
            20                  25                  30

Val Ile Val Val Ile Ile Ala Ser Ala His His His His His His
        35                  40                  45
```

<210> 343
<211> 1464
<212> DNA
<213> Artificial Sequence

<220>
<223> pGLEX41_HC-GGC-I4(SV40ter)-B7-B7(20)-KCFCK

<400> 343

```
ctgtctccgg gcaaatgatg aaagcttggt aggtgatcct cctgctgctt tggttcaggg      60
ttttgcttga gggggggggg tggtgatttc cttgccatgg gcagactgag cagaaaaggc     120
cattgggacc atgttctgaa tgcctccacc tcaaccaccg gccggtagga ccaaagccac     180
cccgtgtttt ctcaggatct cttttcccag ggagatccct cggcccaaag agggagatgg     240
caatgctgga tgtgtgcaca ataattcaac aggcattgga acttcagcat cgaaatgacc     300
gaccaagcga cgcccaacct gccatcacga gatttcgatt ccaccgccgc cttctatgaa     360
aggttgggct tcggaatcgt tttccgggac gccggctgga tgatcctcca gcgcggggat     420
ctcatgctgg agttcttcgc ccaccccaac ttgtttattg cagcttataa tggttacaaa     480
taaagcaata gcatcacaaa tttcacaaat aaagcatttt tttcactgca ttctagttgt     540
ggtttgtcca aactcatcaa tgtatcttat catgtctgta taccgtcgac ctctagctag     600
agcttggcgt aatcatggtc atagctgttt cctgtgtgaa attgttatcc gctcacaatt     660
ccacacaaca tacgagccgg ggcaggataa tatatggtag ggttcatagc cagagtaacc     720
```

```
ttttttttta atttttattt tattttattt ttgagatcag ttcgatgctg aatgcaatta      780
acaatgctca agcagaaccc ccggctccat cagcacagtg caggaccaaa ccccatgctg      840
cagcagtggg gctgtctgta cggggtgggc aatgggaacc ggggtctgct ggggctcctg      900
ctgcttcagt gctgccatgc agccacacat cctgagagct gaaagggtcg gcgtcctcac      960
ctggtgcaca ccgtagctct gccccacagc tttaaggcac ctggctaacc tctgcgcttc     1020
ttcccttccc tcctccctgg ctcaggtggt ggtggatctc cccccgagga cccccctgac     1080
tccaagaata ccctggtgct gttcggcgct ggcttcggcg ccaccgtggt cgtgatcgtc     1140
gtgattatca agtgcttctg caagtgatga tgattcgaaa tgaccgacca agcgacgccc     1200
aacctgccat cacgagattt cgattccacc gccgccttct atgaaaggtt gggcttcgga     1260
atcgttttcc gggacgccgg ctggatgatc ctccagcgcg gggatctcat gctggagttc     1320
ttcgcccacc ccaacttgtt tattgcagct tataatggtt acaaataaag caatagcatc     1380
acaaatttca caaataaagc atttttttca ctgcattcta gttgtggttt gtccaaactc     1440
atcaatgtat cttatcatgt ctgt                                            1464
```

<210> 344
<211> 43
<212> **PRT**
<213> Artificial Sequence

<220>
<223> **pGLEX41_HC-GGC-I4(SV40ter)-B7-B7(20)-KCFCK**

<400> 344

```
Leu Ser Pro Gly Gly Gly Gly Ser Pro Pro Glu Asp Pro Pro Asp Ser
1               5                   10                  15

Lys Asn Thr Leu Val Leu Phe Gly Ala Gly Phe Gly Ala Thr Val Val
            20                  25                  30

Val Ile Val Val Ile Ile Lys Cys Phe Cys Lys
        35                  40
```

<210> 345
<211> 9
<212> **DNA**
<213> Artificial Sequence

<220>
<223> Splice donor consensus

<400> 345
maggtragt 9

<210> 346

<211> 12
<212> DNA
<213> Artificial Sequence

<220>
<223> Splice acceptor consensus

<220>
<221> misc_feature
<222> (7)..(7)
<223> poly (Y) tract comprising at least 15 nucleotides and an abundance of pyrimidine (C or T) bases

<220>
<221> misc_feature
<222> (8)..(8)
<223> n is a, c, g, or t

<400> 346
ctrayynnca gg 12

<210> 347
<211> 6
<212> DNA
<213> Artificial Sequence

<220>
<223> Branch Point Consensus

<400> 347
ctrayy 6

## Claims

1. An expression construct comprising in a 5' to 3' direction:

    a promoter;
    a first exon encoding a polypeptide of interest;
    a splice donor site, an intron and a splice acceptor site, wherein a first stop codon is located between the splice donor site and the splice acceptor site within said intron;
    a second exon encoding a non-immunoglobulin transmembrane region;
    a second stop codon; and
    a poly(A) site,

    wherein upon entry into a host cell, transcription of said first and second exons results in the expression of the polypeptide of interest and wherein a proportion of said polypeptide of interest is secreted in the extracellular environment and a proportion is displayed on the host cell membrane, **characterized in that** said splice acceptor site of said intron comprises a poly(Y) tract and wherein the Y content of the poly(Y) tract is modified by altering the number of pyrimidine bases therein.

2. The expression construct according to claim 1, wherein said transmembrane region comprises between 17 and 29 residues, preferably 19 and 26 residues and most preferably between 21 and 24residues.

3. An expression construct according to claim 1 or 2, wherein the non-immunoglobulin transmembrane region is selected from the group consisting of the transmembrane region of human platelet-derived growth factor receptor (PDGFR), human asialoglycoprotein receptor, human and murine B7-1, human ICAM-1, human erbb1, human erbb2, human erbb3, human erbb4, human fibroblast growth factor receptors such as FGFR 1,FGFR2,FGFR3, FGFR4, human VEGFR-1, human VEGFR-2, human erythropoietin receptor, human PRL-R, prolactin receptor, human EphAl, Ephrin type-A receptor 1, human insulin, IGF-1 receptors, human receptor-like protein tyrosine phosphatases, human neuropilin, human major histocompatibility complex class II (alpha and beta chains), human integrins (alpha and beta families), human Syndecans, human Myelin protein, human cadherins, human synaptobrevin-2, human glycophorin-A, human Bnip3, human APP, amyloid precursor protein, human T-cell receptor alpha and beta, CD3 gamma, CD3 delta, CD3 zeta, and CD3 epsilon.

**4.** An expression construct according to claim 1, wherein the non-immunoglobulin transmembrane region is the murine B7-1 transmembrane region (SEQ ID NO: 173), ACLV1 (SEQ ID NO: 174), ANTR2 (SEQ ID NO: 175), CD4 (SEQ ID NO: 176), PTPRM (SEQ ID NO: 177), TNR5 (SEQ ID NO: 178), ITB1 (SEQ ID NO: 179), IGF1R (SEQ ID NO: 181), 1B07 (SEQ ID NO: 180), TRMB (SEQ ID NO: 182), IL4RA (SEQ ID NO: 183), LRP6 (SEQ ID NO: 184), GpA (SEQ ID NO: 185), PTCRA (SEQ ID NO: 186).

**5.** An expression construct according to any one of the preceding claims, wherein said intron comprises a poly(A) site.

**6.** An expression construct according to any one of the preceding claims comprising at least one branch point region, wherein the sequence of said at least one branch point region is modified with respect to the branch point region consensus sequence CTRAYY (SEQ ID NO: 347).

**7.** An expression construct according to any one of the preceding claims, wherein the consensus sequence of the splice donor site of the intron is modified.

**8.** An expression construct according to any one of the preceding claims, wherein the polypeptide of interest comprises an antibody heavy chain or fragment thereof.

**9.** A polynucleotide encoding an expression construct according to any one of the preceding claims.

**10.** A cloning or expression vector comprising one or more polynucleotides according to claim 9.

**11.** A host cell comprising one or more cloning or expression vectors according to claim 10.

**12.** A host cell according to claim 11 comprising an expression vector comprising a polynucleotide encoding the expression construct of claim 8 for the expression of an antibody heavy chain and an expression vector comprising a polynucleotide encoding an expression construct for the expression of an antibody light chain.

**13.** A host cell according to claim 11 comprising an expression vector comprising a polynucleotide encoding the expression construct of claim 8 for the expression of an antibody heavy chain, an expression vector comprising a polynucleotide encoding the expression construct of claim 7 for the expression of a scFv-Fc and an expression vector comprising a polynucleotide encoding an expression construct for the expression of an antibody light chain.

**14.** A method of producing a polypeptide comprising culturing the host cell(s) of any one of claims 11 to 13 in a culture and isolating the polypeptide expressed from the culture.

**15.** A method of selecting a host cell(s) expressing a polypeptide of interest comprising:

(i) transfecting a host cell(s) with an expression construct of claim 1;
(ii) culturing the host cell(s) under conditions suitable to express the polypeptide of interest;
(iii) detecting cell membrane expression of the polypeptide of interest; and
(iv) selecting the host cell(s) displaying the polypeptide of interest on the surface of the cell membrane at the desired expression level.

**16.** A method of selecting a host cell(s) expressing a heteromultimeric polypeptide of interest comprising:

(i) co-transfecting a host cell(s) with at least an expression construct of claim 1 encoding an a first polypeptide of interest and an expression construct of claim 1 encoding a second polypeptide of interest;
(ii) culturing the host cell(s) under conditions suitable to express the heteromultimeric polypeptide of interest;
(iii) detecting cell membrane expression of the heteromultimeric polypeptide of interest; and
(iv) selecting the host cell(s) displaying the desired heteromultimeric polypeptide of interest on the surface of the cell membrane at the desired expression level.

**17.** A method of selecting a host cell(s) expressing a bispecific antibody comprising:

(i) co-transfecting a host cell(s) with an expression construct of claim 1 encoding an antibody heavy chain, an expression construct of claim 1 encoding a scFv-Fc and an expression construct encoding an antibody light chain;
(ii) culturing the host cell(s) under conditions suitable to express the bispecific antibody;
(iii) detecting cell membrane expression of the bispecific antibody; and
(iv) selecting the host cell(s) displaying the desired bispecific antibody on the surface of the cell membrane at the desired expression level.

**Patentansprüche**

**1.** Expressionskonstrukt, umfassend in einer 5'-nach-3'-Richtung:

einen Promotor;
ein erstes Exon, das für ein Polypeptid von Interesse codiert;
eine Spleiß-Donorstelle, ein Intron und eine Spleiß-Akzeptorstelle, wobei sich ein erstes Stoppcodon zwischen der Spleiß-Donorstelle und der Spleiß-Akzeptorstelle innerhalb des Introns befindet;
ein zweites Exon, das für eine Nicht-Immunglobulin-Transmembranregion codiert;
ein zweites Stoppcodon; und
eine Poly(A)-Stelle,
wobei bei einem Eintritt in eine Wirtszelle eine Transkription des ersten und des zweiten Exons zu der Expression des Polypeptids von Interesse führt und wobei ein Anteil des Polypeptids von Interesse in die extrazelluläre Umgebung sezerniert wird und ein Anteil auf der Wirtszellmembran präsentiert wird, **dadurch gekennzeichnet, dass** die Spleiß-Akzeptorstelle des Introns einen Poly(Y)-Abschnitt umfasst und wobei der Y-Gehalt des Poly(Y)-Abschnitts durch ein Verändern der Anzahl der Pyrimidinbasen darin modifiziert wird.

**2.** Expressionskonstrukt nach Anspruch 1, wobei die Transmembranregion zwischen 17 und 29 Reste, vorzugsweise 19 und 26 Reste und am meisten bevorzugt zwischen 21 und 24 Reste umfasst.

**3.** Expressionskonstrukt nach Anspruch 1 oder 2, wobei die Nicht-Immunglobulin-Transmembranregion aus der Gruppe ausgewählt ist, bestehend aus der Transmembranregion des humanen Thrombozyten-Wachstumsfaktorrezeptors (platelet-derived growth factor receptor - PDGFR), humanen Asialoglycoprotein-Rezeptors, humanen und murinen B7-1, humanen ICAM-1, humanen erbb1, humanen erbb2, humanen erbb3, humanen erbb4, humanen Fibroblasten-Wachstumsfaktorrezeptoren wie etwa FGFR1, FGFR2, FGFR3, FGFR4, humanen VEGFR-1, humanen VEGFR-2, humanen Erythropoietinrezeptors, humanen PRL-R, Prolaktinrezeptors, humanen EphAI, Ephrin Typ-A-Rezeptors 1, humanen Insulins, IGF-1-Rezeptoren, humanen rezeptorähnlichen Proteintyrosinphosphatasen, humanen Neuropilins, humanen Haupthistokompatibilitätskomplex Klasse II (Alpha- und Betaketten), humanen Integrinen (Alpha- und Betafamilien), humanen Syndecans, humanen Myelin-Proteins, humanen Cadherinen, humanen Synaptobrevin-2, humanen Glykophorin-A, humanen Bnip3, humanen APP, Amyloid-Vorläuferproteins, humanen T-Zell-Rezeptors Alpha und Beta, CD3-Gamma, CD3-Delta, CD3-Zeta, und CD3-Epsilon.

**4.** Expressionskonstrukt nach Anspruch 1, wobei die Nicht-Immunglobulin-Transmembranregion die murine B7-1-Transmembranregion (SEQ ID NO: 173), ACLV1 (SEQ ID NO: 174), ANTR2 (SEQ ID NO: 175), CD4 (SEQ ID NO: 176), PTPRM (SEQ ID NO: 177), TNR5 (SEQ ID NO: 178), ITB1 (SEQ ID NO: 179), IGF1R (SEQ ID NO: 181), 1B07 (SEQ ID NO: 180), TRMB (SEQ ID NO: 182), IL4RA (SEQ ID NO: 183), LRP6 (SEQ ID NO: 184), GpA (SEQ ID NO: 185), PTCRA (SEQ ID NO: 186) ist.

**5.** Expressionskonstrukt nach einem der vorstehenden Ansprüche, wobei das Intron eine Poly(A)-Stelle umfasst.

**6.** Expressionskonstrukt nach einem der vorstehenden Ansprüche, umfassend mindestens eine Verzweigungspunktregion, wobei die Sequenz der mindestens einen Verzweigungspunktregion hinsichtlich der Konsensussequenz CTRAYY (SEQ ID NO: 347) der Verzweigungspunktregion modifiziert ist.

**7.** Expressionskonstrukt nach einem der vorstehenden Ansprüche, wobei die Konsensussequenz der Spleiß-Donorstelle des Introns modifiziert ist.

**8.** Expressionskonstrukt nach einem der vorstehenden Ansprüche, wobei das Polypeptid von Interesse eine schwere Kette eines Antikörpers oder ein Fragment davon umfasst.

**9.** Polynukleotid, das für ein Expressionskonstrukt nach einem der vorstehenden Ansprüche codiert.

**10.** Klonierungs- oder Expressionsvektor, umfassend ein oder mehrere Polynukleotide nach Anspruch 9.

**11.** Wirtszelle, umfassend einen oder mehrere Klonierungs- oder Expressionsvektoren nach Anspruch 10.

**12.** Wirtszelle nach Anspruch 11, umfassend einen Expressionsvektor, umfassend ein Polynukleotid, das für das Expressionskonstrukt nach Anspruch 8 für die Expression einer schweren Kette eines Antikörpers codiert, und einen Expressionsvektor, umfassend ein Polynukleotid, das für ein Expressionskonstrukt für die Expression einer leichten Kette eines Antikörpers codiert.

**13.** Wirtszelle nach Anspruch 11, umfassend einen Expressionsvektor, umfassend ein Polynukleotid, das für das Expressionskonstrukt nach Anspruch 8 für die Expression einer schweren Kette eines Antikörpers codiert, einen Expressionsvektor, umfassend ein Polynukleotid, das für das Expressionskonstrukt nach Anspruch 7 für die Expression eines scFv-Fc codiert, und einen Expressionsvektor, umfassend ein Polynukleotid, das für ein Expressionskonstrukt für die Expression einer leichten Kette eines Antikörpers codiert.

**14.** Verfahren zum Herstellen eines Polypeptids, umfassend ein Kultivieren der Wirtszelle(n) nach einem der Ansprüche 11 bis 13 in einer Kultur und ein Isolieren des exprimierten Polypeptids aus der Kultur.

**15.** Verfahren zum Auswählen einer oder mehrerer Wirtszellen, die ein Polypeptid von Interesse exprimieren, umfassend:

(i) Transfizieren einer oder mehrerer Wirtszellen mit einem Expressionskonstrukt nach Anspruch 1;
(ii) Kultivieren der Wirtszelle(n) unter Bedingungen, die geeignet sind, um das Polypeptid von Interesse zu exprimieren;
(iii) Detektieren der Zellmembranexpression des Polypeptids von Interesse; und
(iv) Auswählen der Wirtszelle(n), die das Polypeptid von Interesse auf der Oberfläche der Zellmembran in der gewünschten Expressionsstärke präsentiert/- en.

**16.** Verfahren zum Auswählen einer oder mehrerer Wirtszellen, die ein heteromultimeres Polypeptid von Interesse exprimieren, umfassend:

(i) Cotransfizieren einer oder mehrerer Wirtszellen mit mindestens einem Expressionskonstrukt nach Anspruch 1, das für ein erstes Polypeptid von Interesse codiert, und einem Expressionskonstrukt nach Anspruch 1, das für ein zweites Polypeptid von Interesse codiert;
(ii) Kultivieren der Wirtszelle(n) unter Bedingungen, die geeignet sind, um das heteromultimere Polypeptid von Interesse zu exprimieren;
(iii) Detektieren der Zellmembranexpression des heteromultimeren Polypeptids von Interesse; und
(iv) Auswählen der Wirtszelle(n), die das gewünschte heteromultimere Polypeptid von Interesse auf der Oberfläche der Zellmembran in der gewünschten Expressionsstärke präsentiert/-en.

**17.** Verfahren zum Auswählen einer oder mehrerer Wirtszellen, die einen bispezifischen Antikörper exprimieren, umfassend:

(i) Cotransfizieren einer oder mehrerer Wirtszellen mit einem Expressionskonstrukt nach Anspruch 1, das für eine schwere Kette eines Antikörpers codiert, einem Expressionskonstrukt nach Anspruch 1, das für ein scFv-Fc codiert, und einem Expressionskonstrukt, das für eine leichte Kette eines Antikörpers codiert;
(ii) Kultivieren der Wirtszelle(n) unter Bedingungen, die geeignet sind, um den bispezifischen Antikörper zu exprimieren;
(iii) Detektieren der Zellmembranexpression des bispezifischen Antikörpers; und
(iv) Auswählen der Wirtszelle(n), die den gewünschten bispezifischen Antikörper auf der Oberfläche der Zellmembran in der gewünschten Expressionsstärke präsentiert/-en.

## Revendications

1. Construction d'expression comprenant dans une direction de 5' à 3' :

   un promoteur ;
   un premier exon codant pour un polypeptide d'intérêt ;
   un site donneur d'épissage, un intron et un site accepteur d'épissage, un premier codon d'arrêt étant situé entre le site donneur d'épissage et le site accepteur d'épissage à l'intérieur dudit intron ;
   un second exon codant pour une région transmembranaire non immunoglobuline ;
   un second codon d'arrêt ; et
   un site poly(A),
   dans laquelle lors de l'entrée dans une cellule hôte, la transcription desdits premier et second exons aboutit à l'expression du polypeptide d'intérêt et dans laquelle une proportion dudit polypeptide d'intérêt est sécrétée dans l'environnement extracellulaire et une proportion est affichée sur la membrane de la cellule hôte, **caractérisée en ce que** ledit site accepteur d'épissage dudit intron comprend un tractus poly(Y) et dans laquelle la teneur en Y du tractus poly(Y) est modifiée en modifiant le nombre de bases pyrimidiques qu'il contient.

2. Construction d'expression selon la revendication 1, dans laquelle ladite région transmembranaire comprend entre 17 et 29 résidus, de préférence entre 19 et 26 résidus et le plus préférablement entre 21 et 24 résidus.

3. Construction d'expression selon la revendication 1 ou 2, dans laquelle la région transmembranaire non immunoglobuline est choisie dans le groupe constitué de la région transmembranaire du récepteur du facteur de croissance dérivé des plaquettes (PDGFR) humaines, du récepteur de l'asialoglycoprotéine humaine, du B7-1 humain et murin, de l'ICAM-1 humain, de l'erbb1 humain, de l'erbb2 humain, de l'erbb3 humain, de l'erbb4 humain, des récepteurs du facteur de croissance des fibroblastes humains tels que un FGFR1, un FGFR2, un FGFR3, un FGFR4, un VEGFR-1 humain, un VEGFR-2 humain, un récepteur de l'érythropoïétine humaine, un PRL-R humain, un récepteur de la prolactine, l'EphA1 humain, un récepteur 1 de l'éphrine de type A, une insuline humaine, des récepteurs de l'IGF-1, des protéines tyrosine phosphatases humaines de type récepteur, une neuropiline humaine, un complexe majeur d'histocompatibilité humain de classe II (chaînes alpha et bêta), des intégrines humaines (familles alpha et bêta), des syndécanes humains, une protéine de myéline humaine, des cadhérines humaines, une synaptobrévine-2 humaine, une glycophorine-A humaine, un Bnip3 humain, une APP humaine, une protéine précurseur de l'amyloïde, un récepteur alpha et bêta des lymphocytes T humains, un CD3 gamma, un CD3 delta, un CD3 zêta, un CD3 epsilon.

4. Construction d'expression selon la revendication 1, dans laquelle la région transmembranaire non immunoglobuline est la région transmembranaire murine B7-1 (SEQ ID NO : 173), ACLV1 (SEQ ID NO : 174), ANTR2 (SEQ ID NO : 175), CD4 (SEQ ID NO : 176), PTPRM (SEQ ID NO : 177), TNR5 (SEQ ID NO : 178), ITB1 (SEQ ID NO : 179), IGF1R (SEQ ID NO : 181), 1B07 (SEQ ID NO : 180), TRMB (SEQ ID NO : 182), IL4RA (SEQ ID NO : 183), LRP6 (SEQ ID NO : 184), GpA (SEQ ID NO : 185), PTCRA (SEQ ID NO : 186).

5. Construction d'expression selon l'une quelconque des revendications précédentes, dans laquelle ledit intron comprend un site poly(A).

6. Construction d'expression selon l'une quelconque des revendications précédentes comprenant au moins une région de point de branchement, dans laquelle la séquence de ladite au moins une région de point de branchement est modifiée par rapport à la séquence fondamentale de région de point de branchement CTRAYY (SEQ ID NO : 347).

7. Construction d'expression selon l'une quelconque des revendications précédentes, dans laquelle la séquence fondamentale du site donneur d'épissage de l'intron est modifiée.

8. Construction d'expression selon l'une quelconque des revendications précédentes, dans laquelle le polypeptide d'intérêt comprend une chaîne lourde d'anticorps ou un fragment de celle-ci.

9. Polynucléotide codant pour une construction d'expression selon l'une quelconque des revendications précédentes.

10. Vecteur de clonage ou d'expression comprenant un ou plusieurs polynucléotides selon la revendication 9.

11. Cellule hôte comprenant un ou plusieurs vecteurs de clonage ou d'expression selon la revendication 10.

12. Cellule hôte selon la revendication 11, comprenant un vecteur d'expression comprenant un polynucléotide codant pour la construction d'expression de la revendication 8 pour l'expression d'une chaîne lourde d'anticorps et un vecteur d'expression comprenant un polynucléotide codant pour une construction d'expression pour l'expression d'une chaîne légère d'anticorps.

13. Cellule hôte selon la revendication 11 comprenant un vecteur d'expression comprenant un polynucléotide codant pour la construction d'expression de la revendication 8 pour l'expression d'une chaîne lourde d'anticorps, un vecteur d'expression comprenant un polynucléotide codant pour la construction d'expression de la revendication 7 pour l'expression d'un scFv-Fc et un vecteur d'expression comprenant un polynucléotide codant pour une construction d'expression pour l'expression d'une chaîne légère d'anticorps.

14. Procédé de production d'un polypeptide comprenant la culture de la ou des cellules hôtes selon l'une quelconque des revendications 11 à 13 dans une culture et l'isolement du polypeptide exprimé à partir de la culture.

15. Procédé de sélection d'une ou des cellules hôtes exprimant un polypeptide d'intérêt comprenant :

(i) la transfection d'une ou des cellules hôtes avec une construction d'expression selon la revendication 1 ;
(ii) la culture de la ou des cellules hôtes dans des conditions appropriées pour exprimer le polypeptide d'intérêt ;
(iii) la détection de l'expression membranaire cellulaire du polypeptide d'intérêt ; et
(iv) la sélection de la ou des cellules hôtes présentant le polypeptide d'intérêt à la surface de la membrane cellulaire au niveau d'expression souhaité.

16. Procédé de sélection d'une ou plusieurs cellules hôtes exprimant un polypeptide hétéromultimérique d'intérêt comprenant :

(i) la co-transfection d'une ou de plusieurs cellules hôtes avec au moins une construction d'expression selon la revendication 1 codant pour un premier polypeptide d'intérêt et une construction d'expression selon la revendication 1 codant pour un second polypeptide d'intérêt ;
(ii) la culture de la ou des cellules hôtes dans des conditions appropriées pour exprimer le polypeptide hétéromultimérique d'intérêt ;
(iii) la détection de l'expression membranaire cellulaire du polypeptide hétéromultimérique d'intérêt ; et
(iv) la sélection de la ou des cellules hôtes présentant le polypeptide hétéromultimérique d'intérêt souhaité sur la surface de la membrane cellulaire au niveau d'expression souhaité.

17. Procédé de sélection d'une ou des cellules hôtes exprimant un anticorps bispécifique comprenant :

(i) la co-transfection d'une ou des cellules hôtes avec une construction d'expression selon la revendication 1 codant pour une chaîne lourde d'anticorps, une construction d'expression selon la revendication 1 codant pour un scFv-Fc et une construction d'expression codant pour une chaîne légère d'anticorps ;
(ii) la culture de la ou des cellules hôtes dans des conditions appropriées pour exprimer l'anticorps bispécifique ;
(iii) la détection de l'expression membranaire cellulaire de l'anticorps bispécifique ; et
(iv) la sélection de la ou des cellules hôtes présentant l'anticorps bispécifique souhaité sur la surface de la membrane cellulaire au niveau d'expression souhaité.

Figure 1

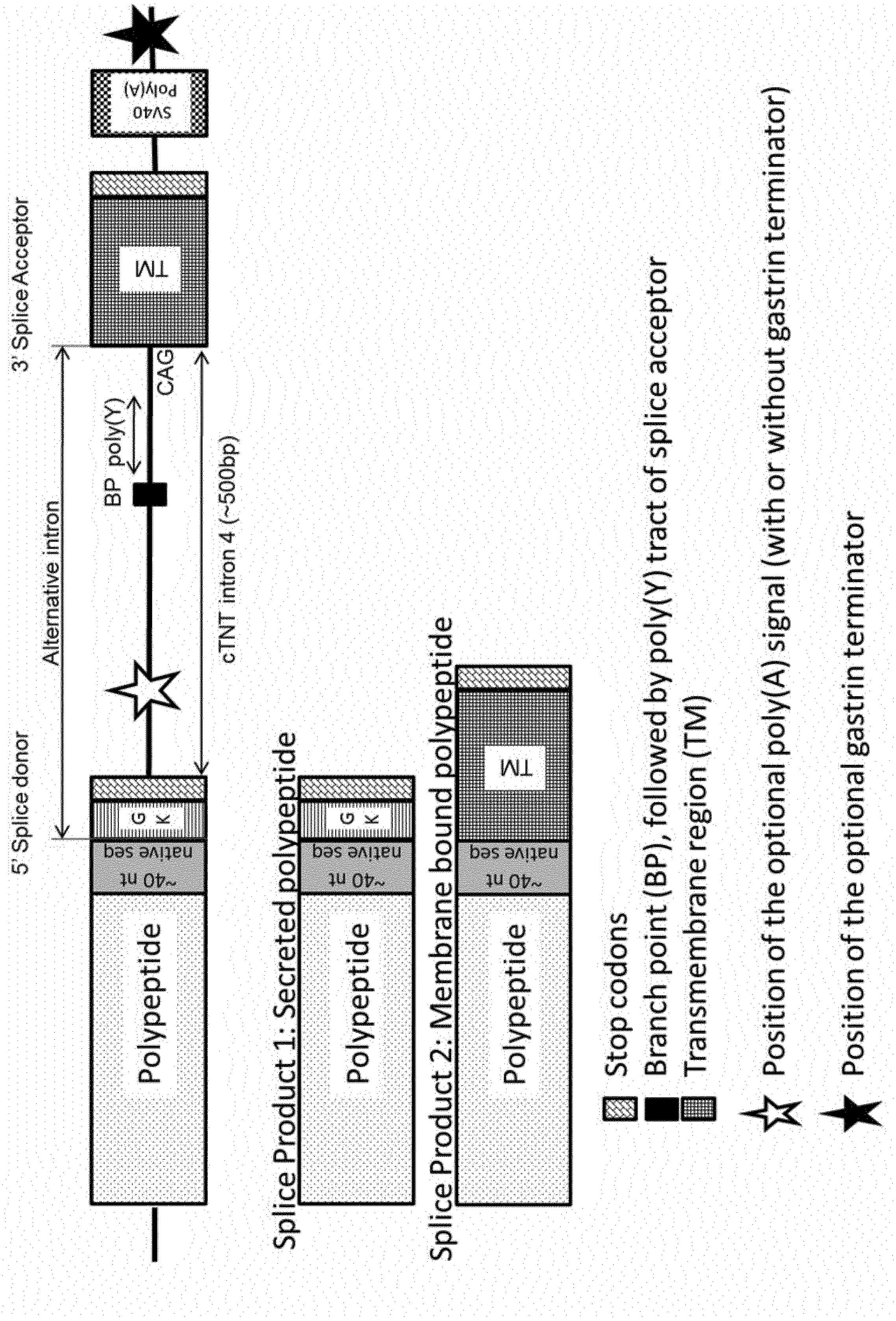
5' Splice donor
Alternative intron
3' Splice Acceptor
Polypeptide
~40 nt
native seq
G
K
BP
poly(Y)
CAG
TM
SV40
Poly(A)
cTNT intron 4 (~500bp)
Splice Product 1: Secreted polypeptide
Polypeptide
~40 nt
native seq
G
K
Splice Product 2: Membrane bound polypeptide
Polypeptide
~40 nt
native seq
TM
Stop codons
Branch point (BP), followed by poly(Y) tract of splice acceptor
Transmembrane region (TM)
Position of the optional poly(A) signal (with or without gastrin terminator)
Position of the optional gastrin terminator

Figure 2

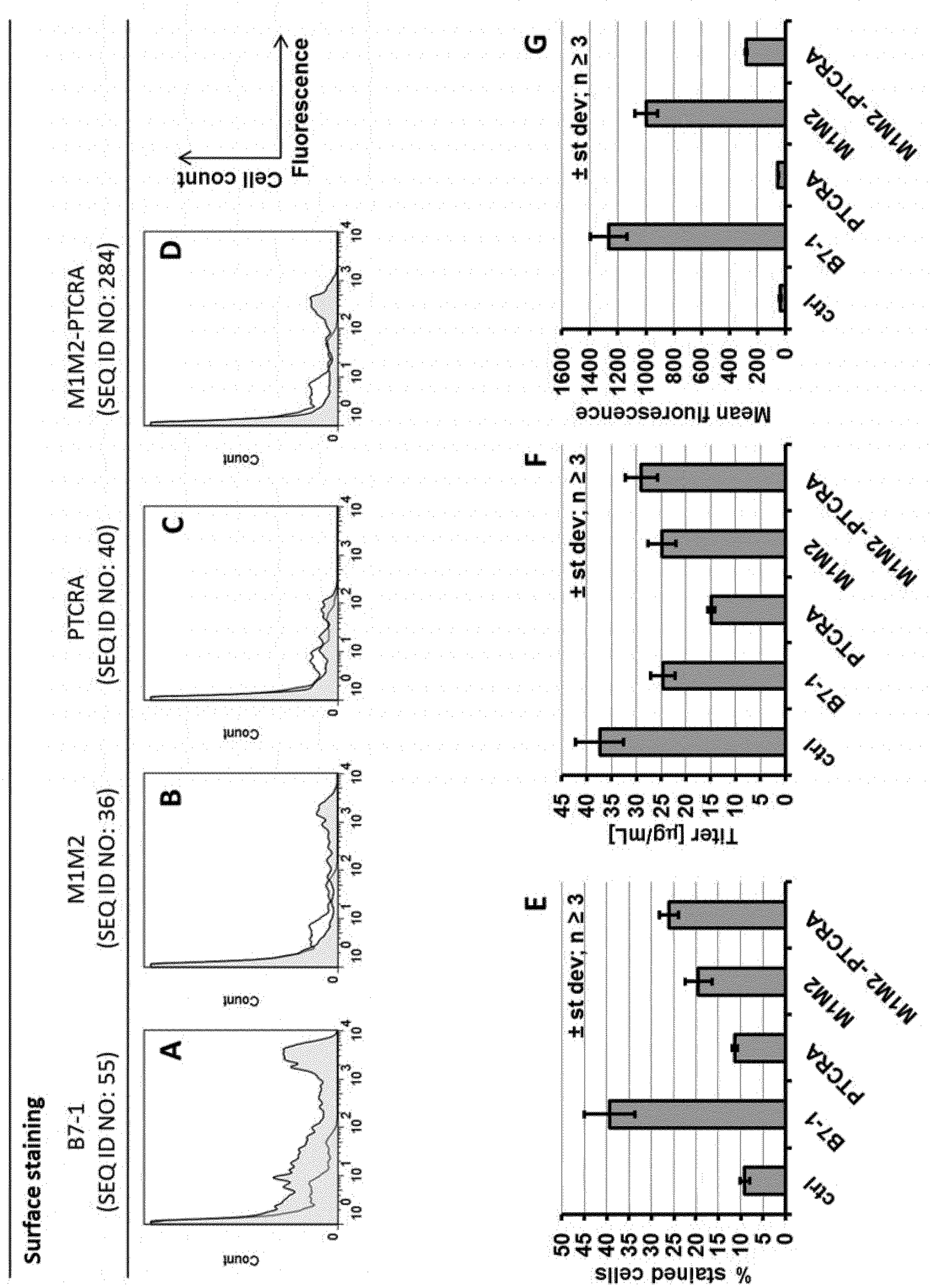
Surface staining
B7-1
(SEQ ID NO: 55)
M1M2
(SEQ ID NO: 36)
PTCRA
(SEQ ID NO: 40)
M1M2-PTCRA
(SEQ ID NO: 284)
A
B
C
D
Count
Cell count
Fluorescence
E
F
G
± st dev; n ≥ 3
% stained cells
Titer [µg/mL]
Mean fluorescence
ctrl
B7-1
PTCRA
M1M2
M1M2-PTCRA

Figure 3

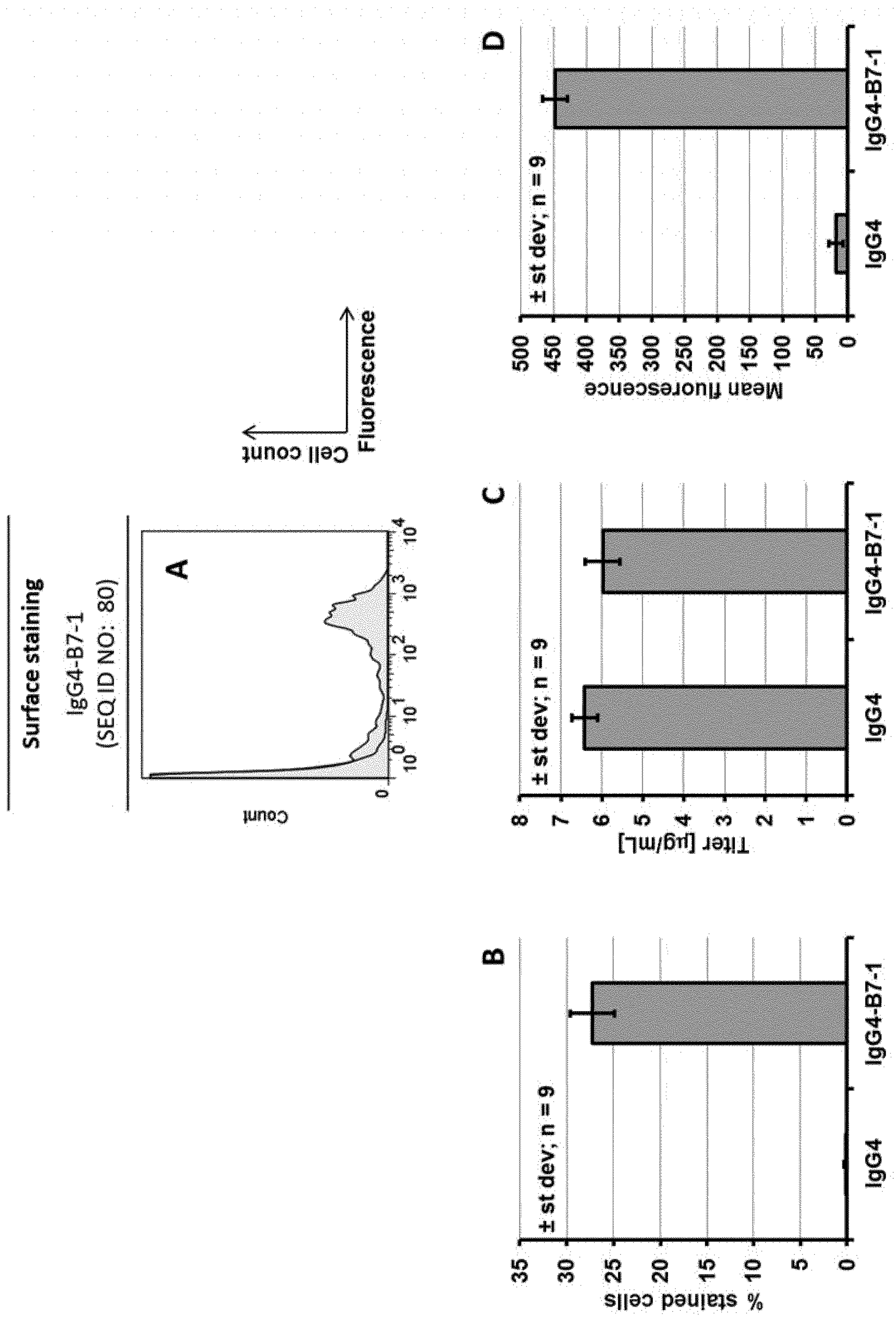
Surface staining
IgG4-B7-1
(SEQ ID NO: 80)
A
Count
Cell count
Fluorescence
B
% stained cells
± st dev; n = 9
IgG4
IgG4-B7-1
C
Titer [µg/mL]
± st dev; n = 9
IgG4
IgG4-B7-1
D
Mean fluorescence
± st dev; n = 9
IgG4
IgG4-B7-1

Figure 4

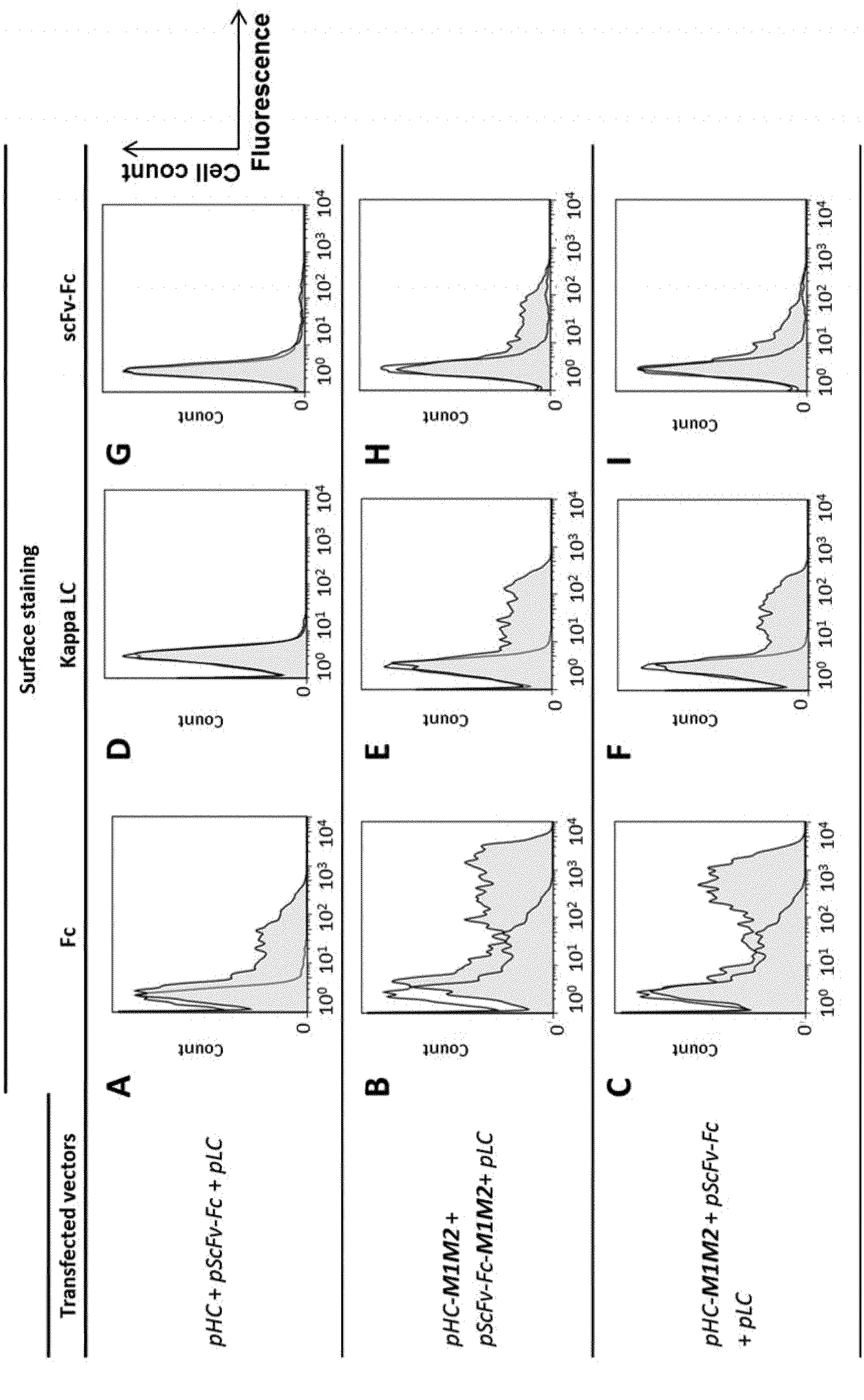
Transfected vectors
Surface staining
Fc
Kappa LC
scFv-Fc
A
B
C
D
E
F
G
H
I
pHC + pScFv-Fc + pLC
pHC-M1M2 +
pScFv-Fc-M1M2+ pLC
pHC-M1M2 + pScFv-Fc
+ pLC
Count
Cell count
Fluorescence

Figure 4 (contd)

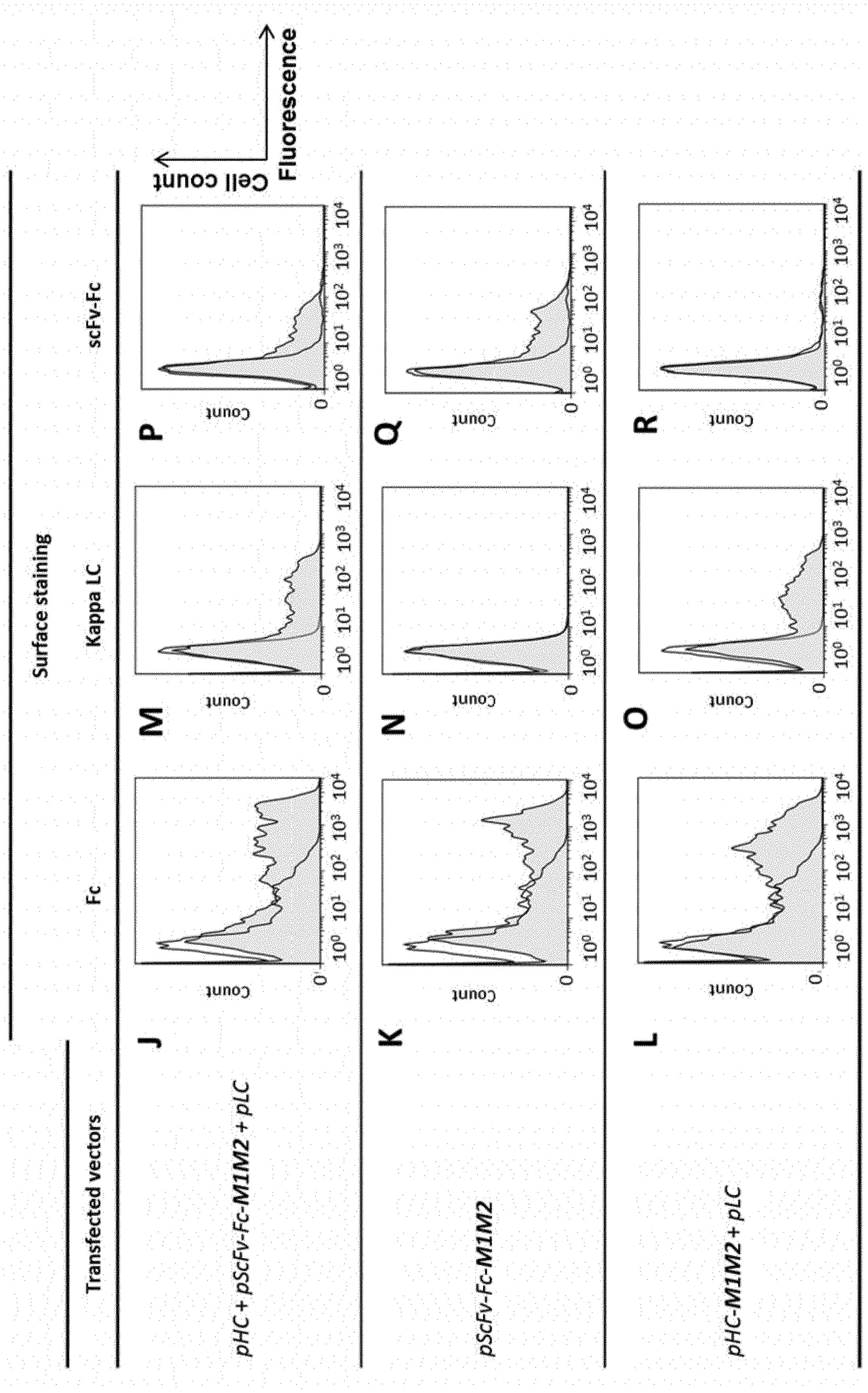

Figure 5

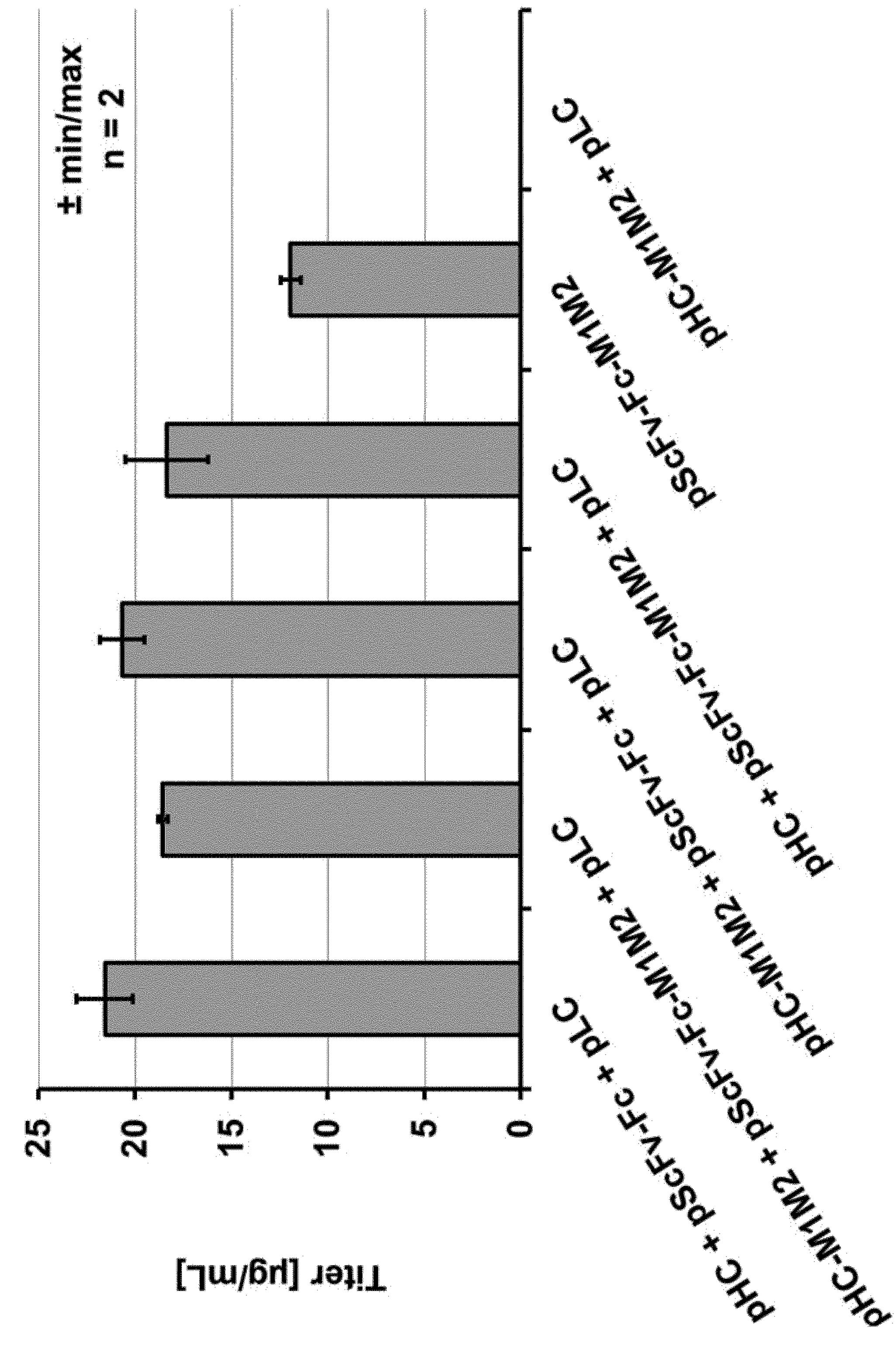
Titer [µg/mL]
0
5
10
15
20
25
± min/max
n = 2
pHC + pScFv-Fc + pLC
pHC-M1M2 + pScFv-Fc-M1M2 + pLC
pHC-M1M2 + pScFv-Fc + pLC
pHC + pScFv-Fc-M1M2 + pLC
pScFv-Fc-M1M2
pHC-M1M2 + pLC

Figure 6

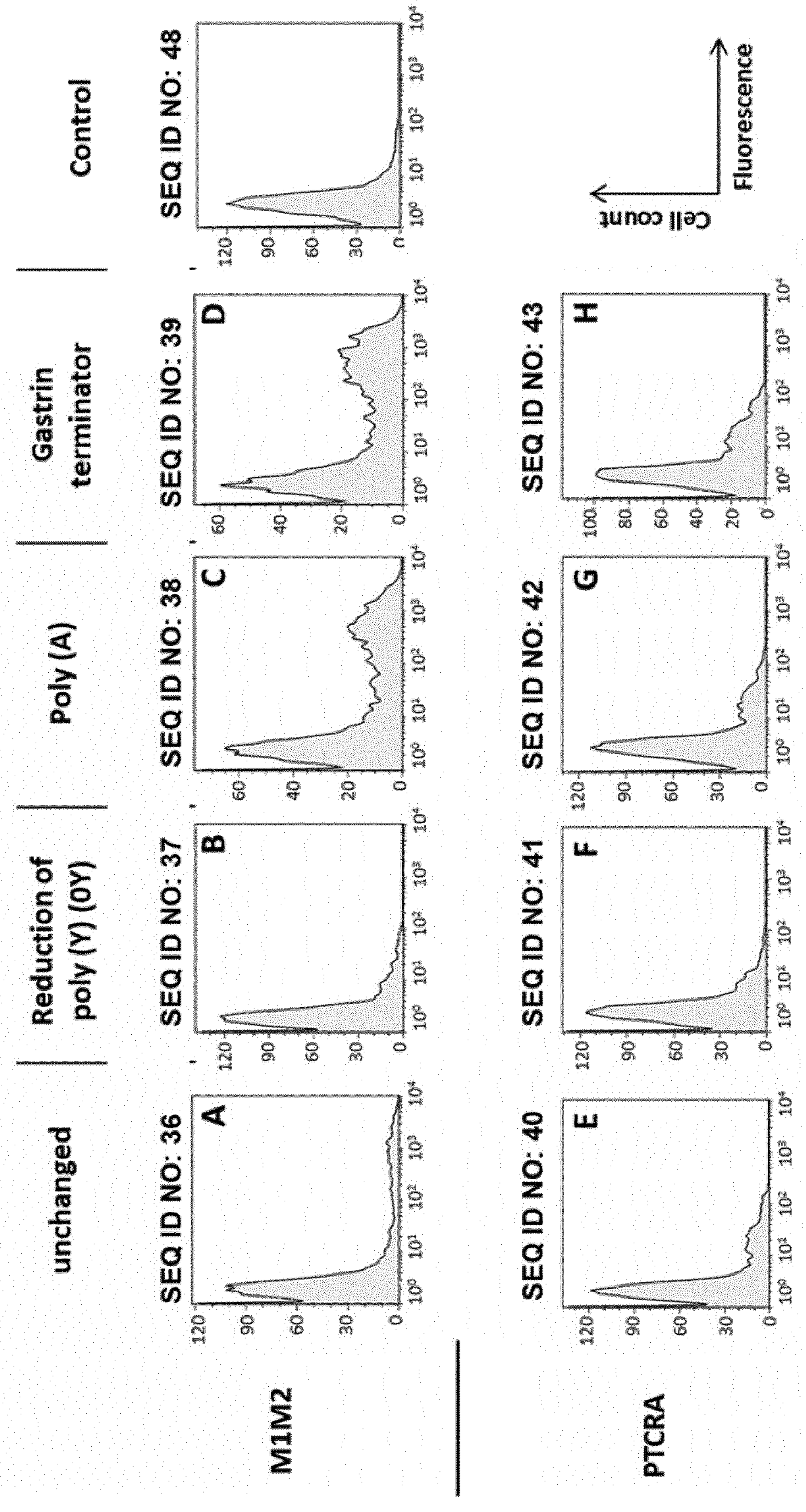
unchanged
Reduction of poly (Y) (0Y)
Poly (A)
Gastrin terminator
Control
M1M2
PTCRA
SEQ ID NO: 36
A
SEQ ID NO: 37
B
SEQ ID NO: 38
C
SEQ ID NO: 39
D
SEQ ID NO: 48
SEQ ID NO: 40
E
SEQ ID NO: 41
F
SEQ ID NO: 42
G
SEQ ID NO: 43
H
Cell count
Fluorescence

Figure 6 (contd)

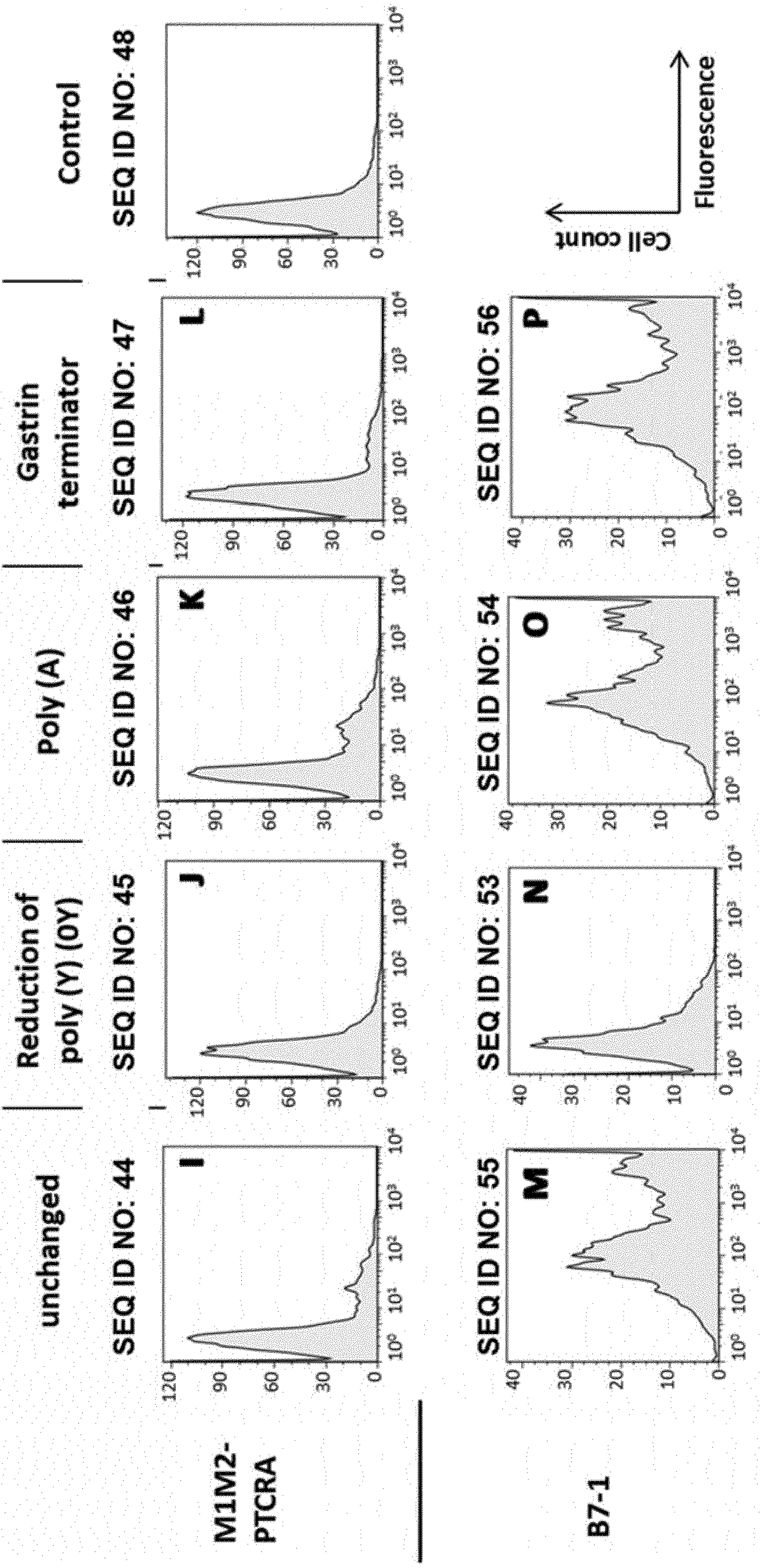

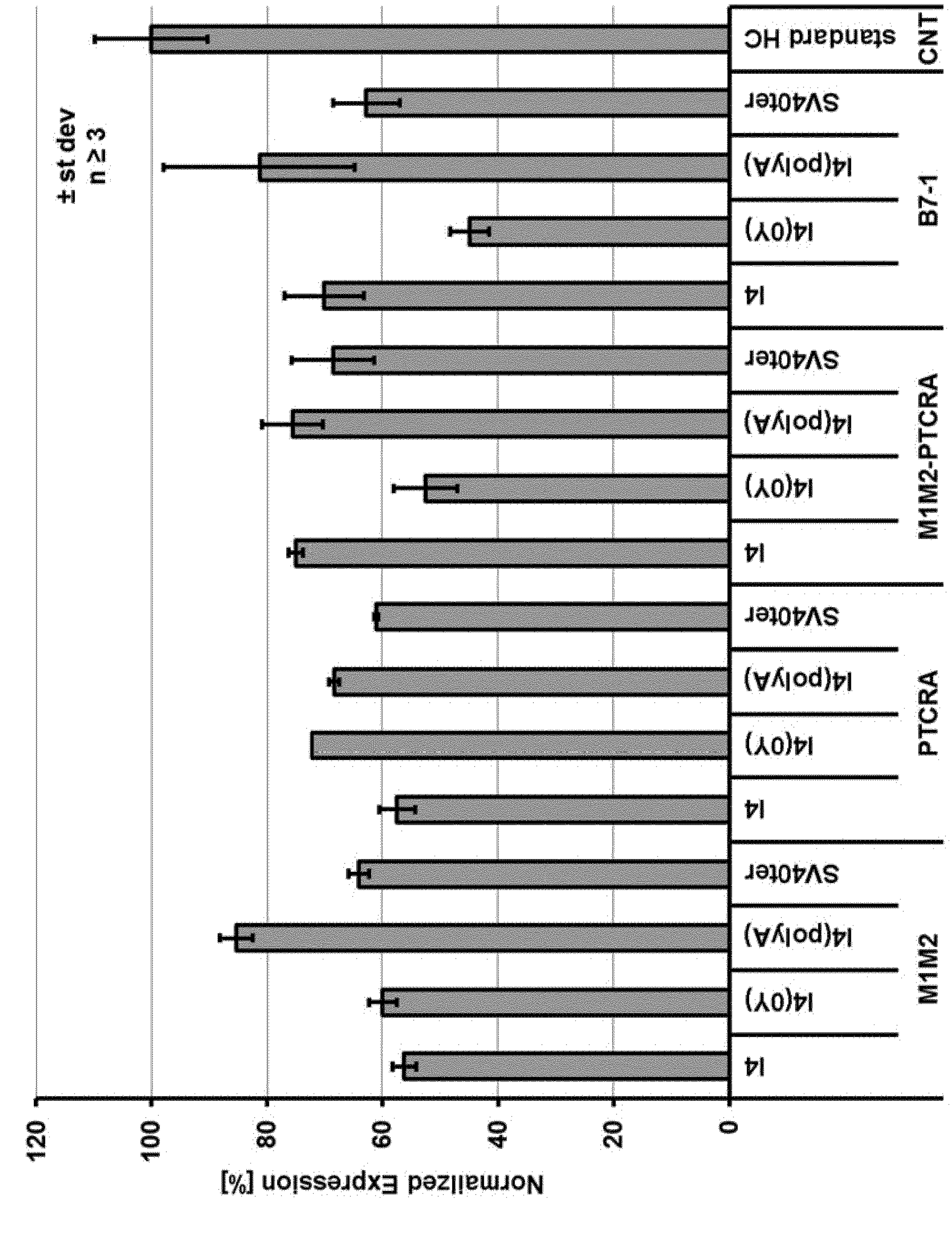
± st dev
n ≥ 3
Normalized Expression [%]
0
20
40
60
80
100
120
I4
I4(0Y)
I4(polyA)
SV40ter
M1M2
PTCRA
M1M2-PTCRA
B7-1
standard HC
CNT

Figure 7

Figure 8

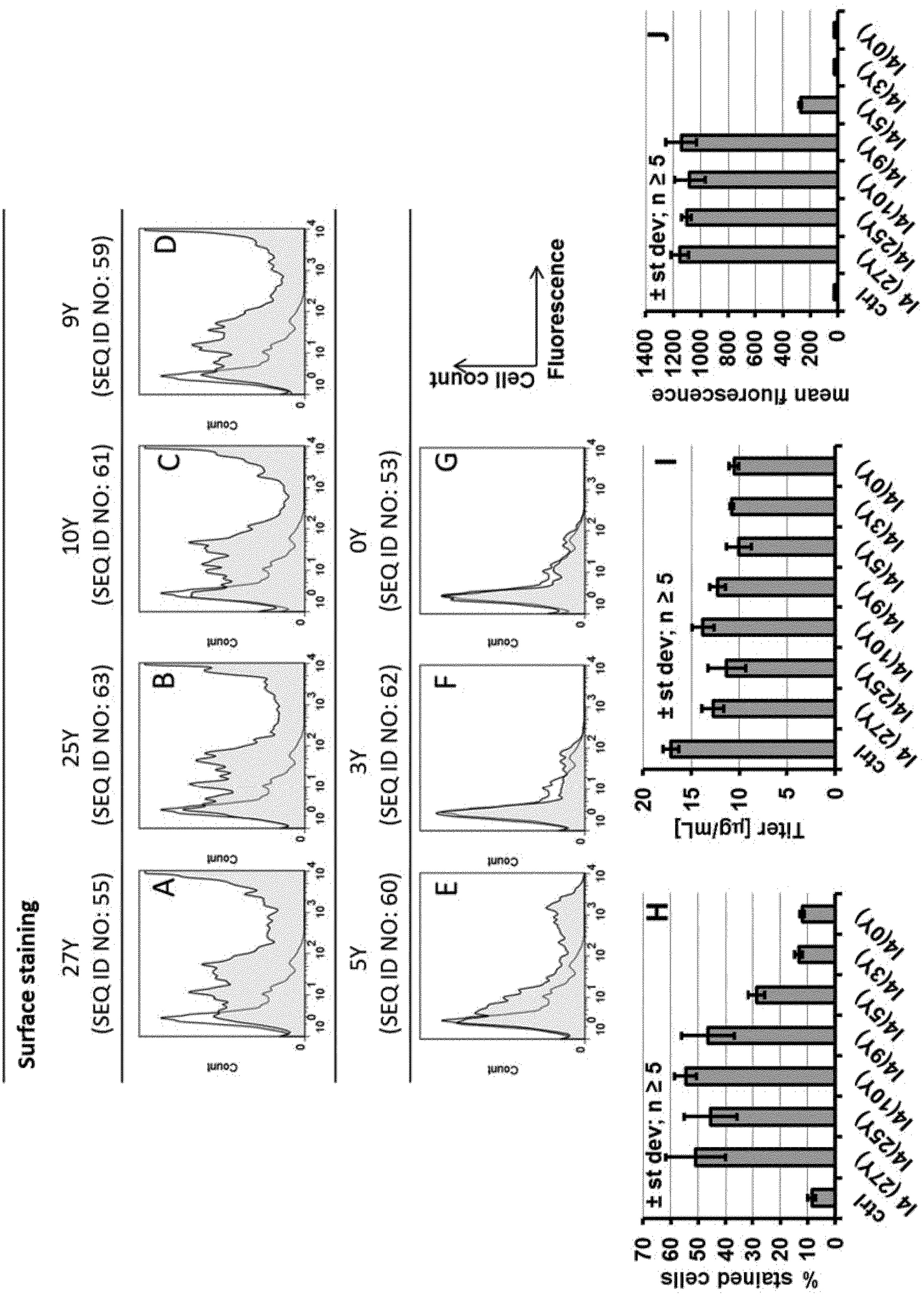
Surface staining
27Y
(SEQ ID NO: 55)
25Y
(SEQ ID NO: 63)
10Y
(SEQ ID NO: 61)
9Y
(SEQ ID NO: 59)
5Y
(SEQ ID NO: 60)
3Y
(SEQ ID NO: 62)
0Y
(SEQ ID NO: 53)
A
B
C
D
E
F
G
Count
Cell count
Fluorescence
H
I
J
± st dev; n ≥ 5
% stained cells
Titer [µg/mL]
mean fluorescence
ctrl
l4 (27Y)
l4(25Y)
l4(10Y)
l4(9Y)
l4(5Y)
l4(3Y)
l4(0Y)

Figure 9

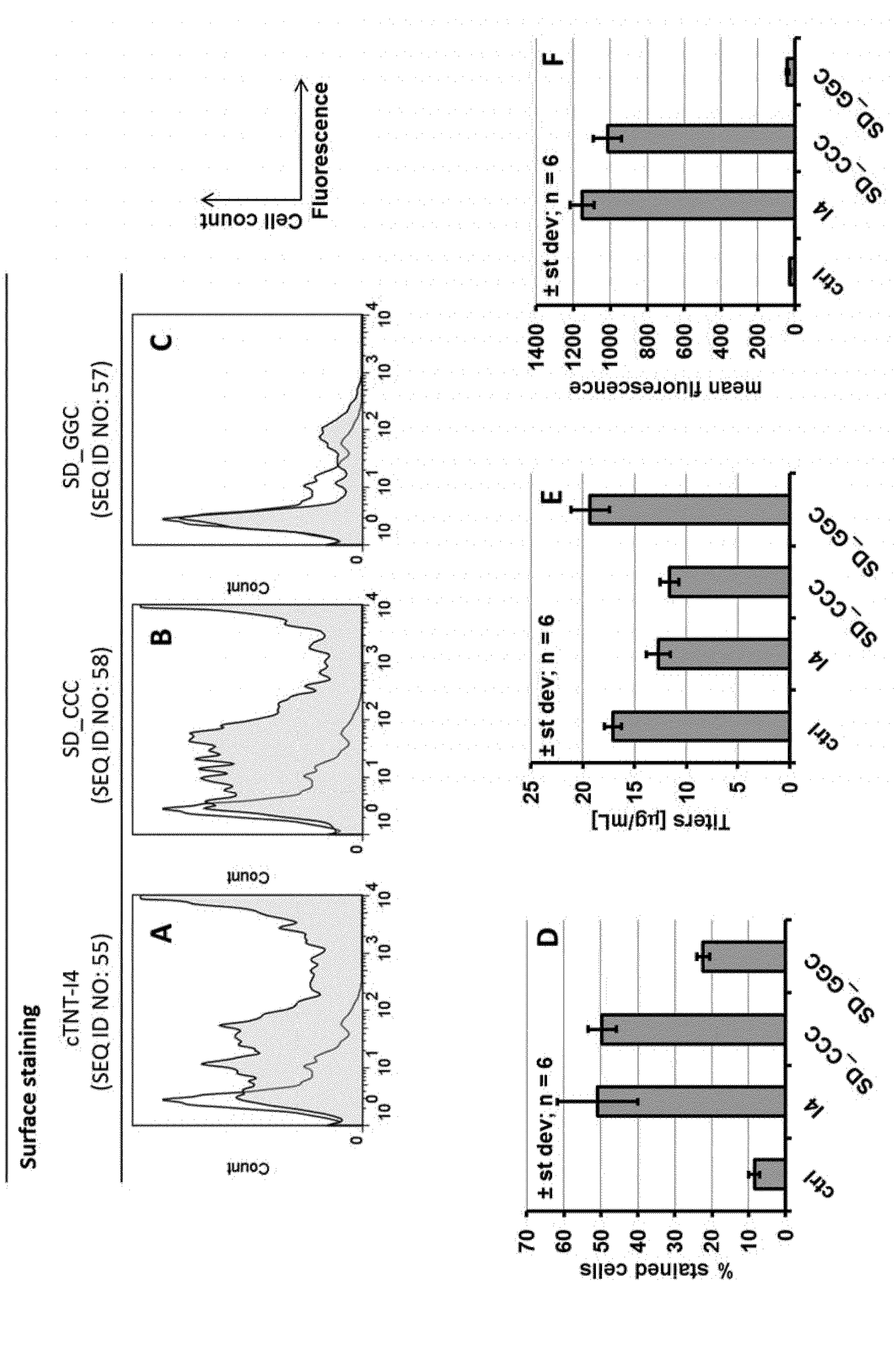
Surface staining
cTNT-I4
(SEQ ID NO: 55)
SD_CCC
(SEQ ID NO: 58)
SD_GGC
(SEQ ID NO: 57)
A
B
C
Count
Cell count
Fluorescence
D
E
F
± st dev; n = 6
% stained cells
Titers [µg/mL]
mean fluorescence
ctrl
I4
SD_CCC
SD_GGC

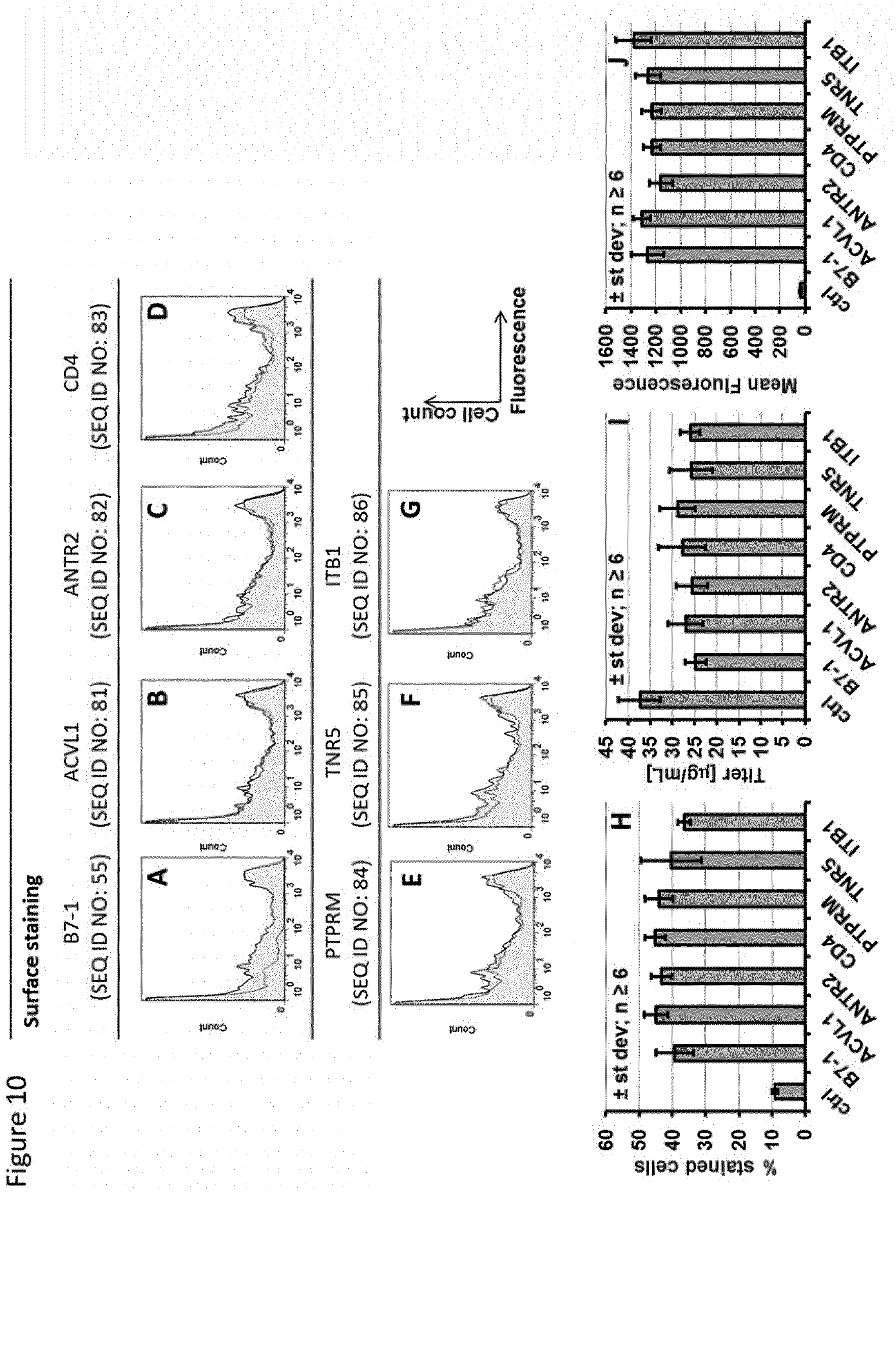
Figure 10
Surface staining
B7-1
(SEQ ID NO: 55)
ACVL1
(SEQ ID NO: 81)
ANTR2
(SEQ ID NO: 82)
CD4
(SEQ ID NO: 83)
PTPRM
(SEQ ID NO: 84)
TNR5
(SEQ ID NO: 85)
ITB1
(SEQ ID NO: 86)
A
B
C
D
E
F
G
Cell count
Fluorescence
H
I
J
± st dev; n ≥ 6
% stained cells
Titer [µg/mL]
Mean Fluorescence
ctrl
B7-1
ACVL1
ANTR2
CD4
PTPRM
TNR5
ITB1

Figure 11

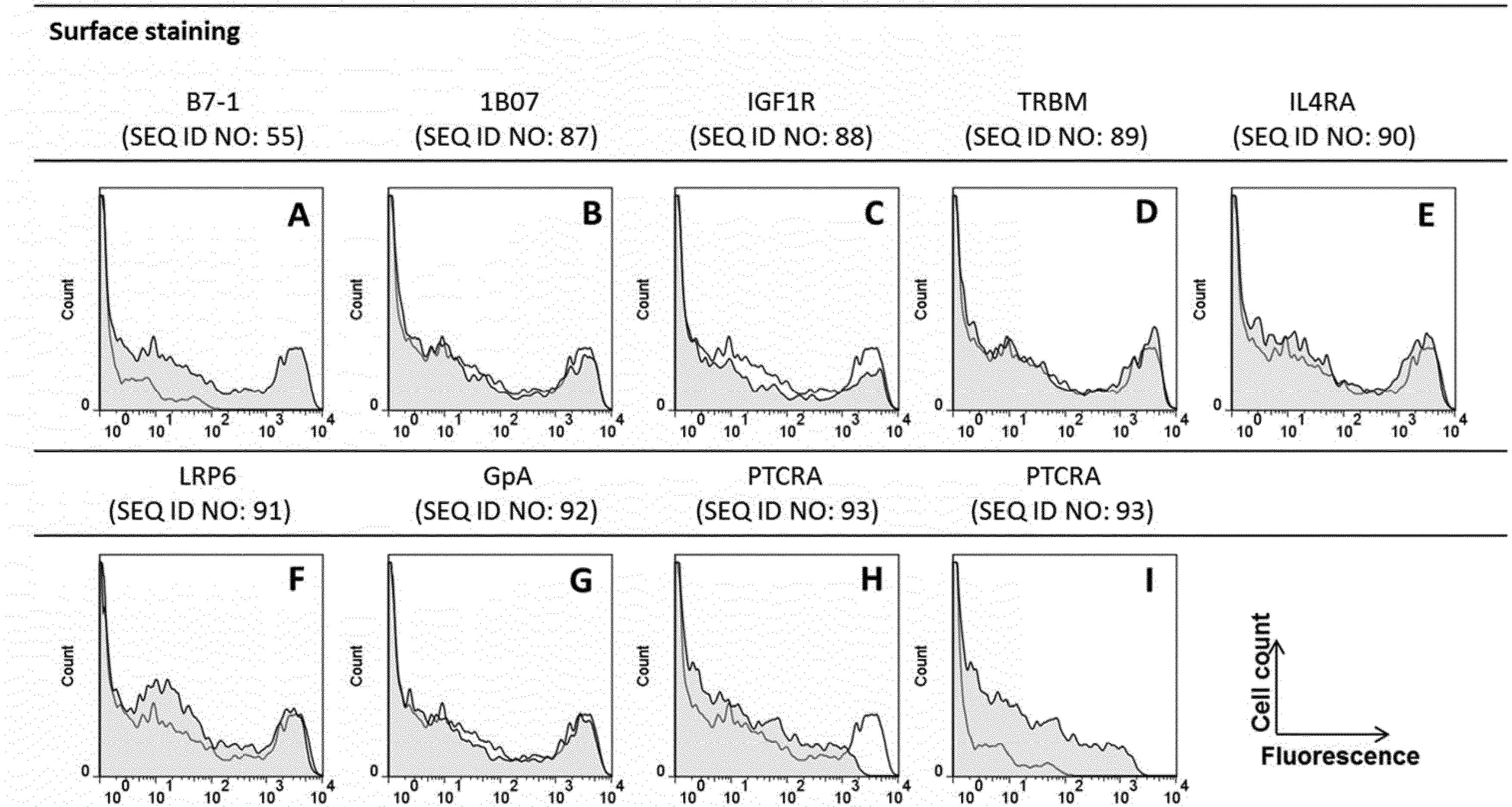
Surface staining
B7-1
(SEQ ID NO: 55)
1B07
(SEQ ID NO: 87)
IGF1R
(SEQ ID NO: 88)
TRBM
(SEQ ID NO: 89)
IL4RA
(SEQ ID NO: 90)
A
B
C
D
E
LRP6
(SEQ ID NO: 91)
GpA
(SEQ ID NO: 92)
PTCRA
(SEQ ID NO: 93)
PTCRA
(SEQ ID NO: 93)
F
G
H
I
Count
0
$10^0$ $10^1$ $10^2$ $10^3$ $10^4$
Cell count
Fluorescence

Figure 11 (contd)

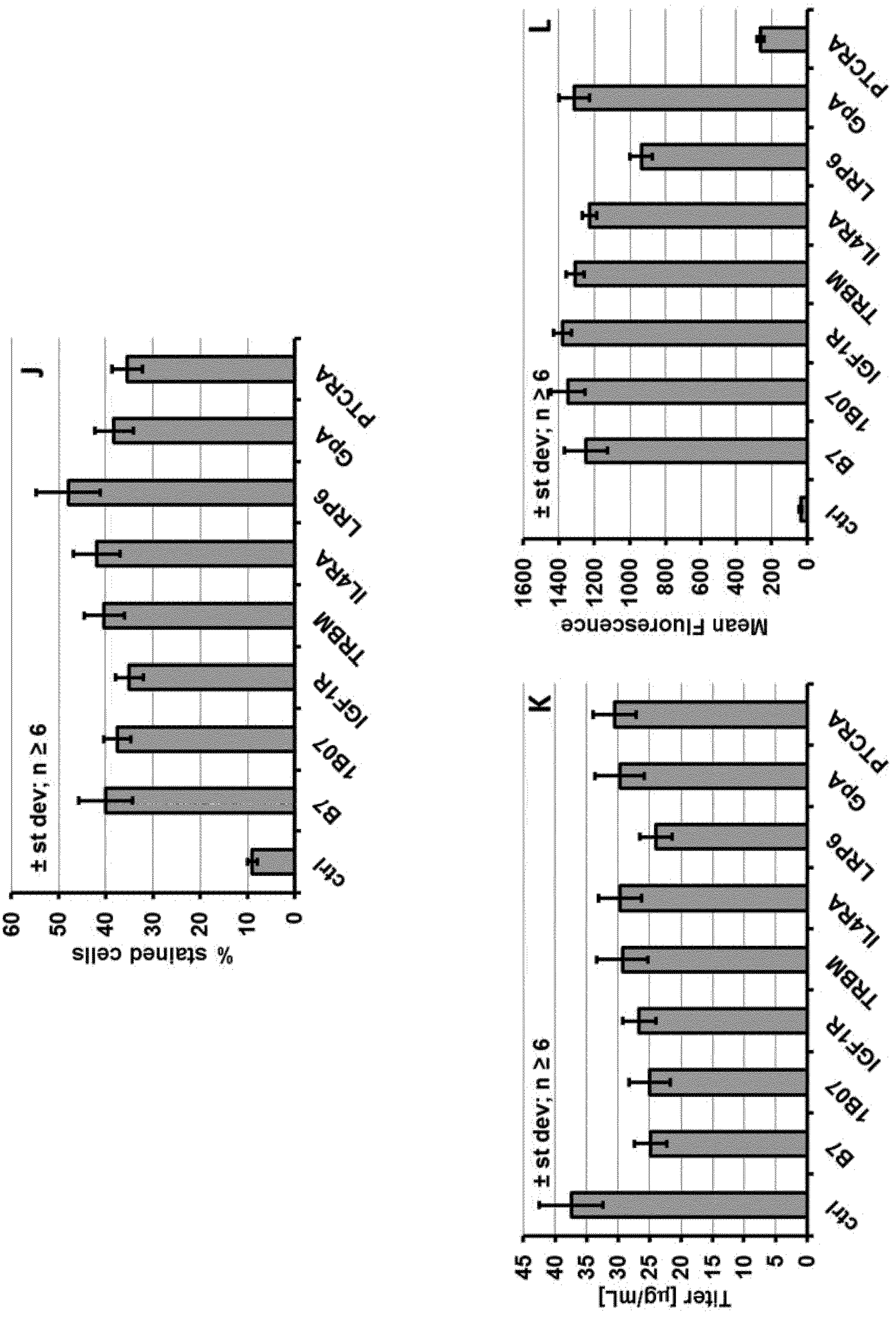

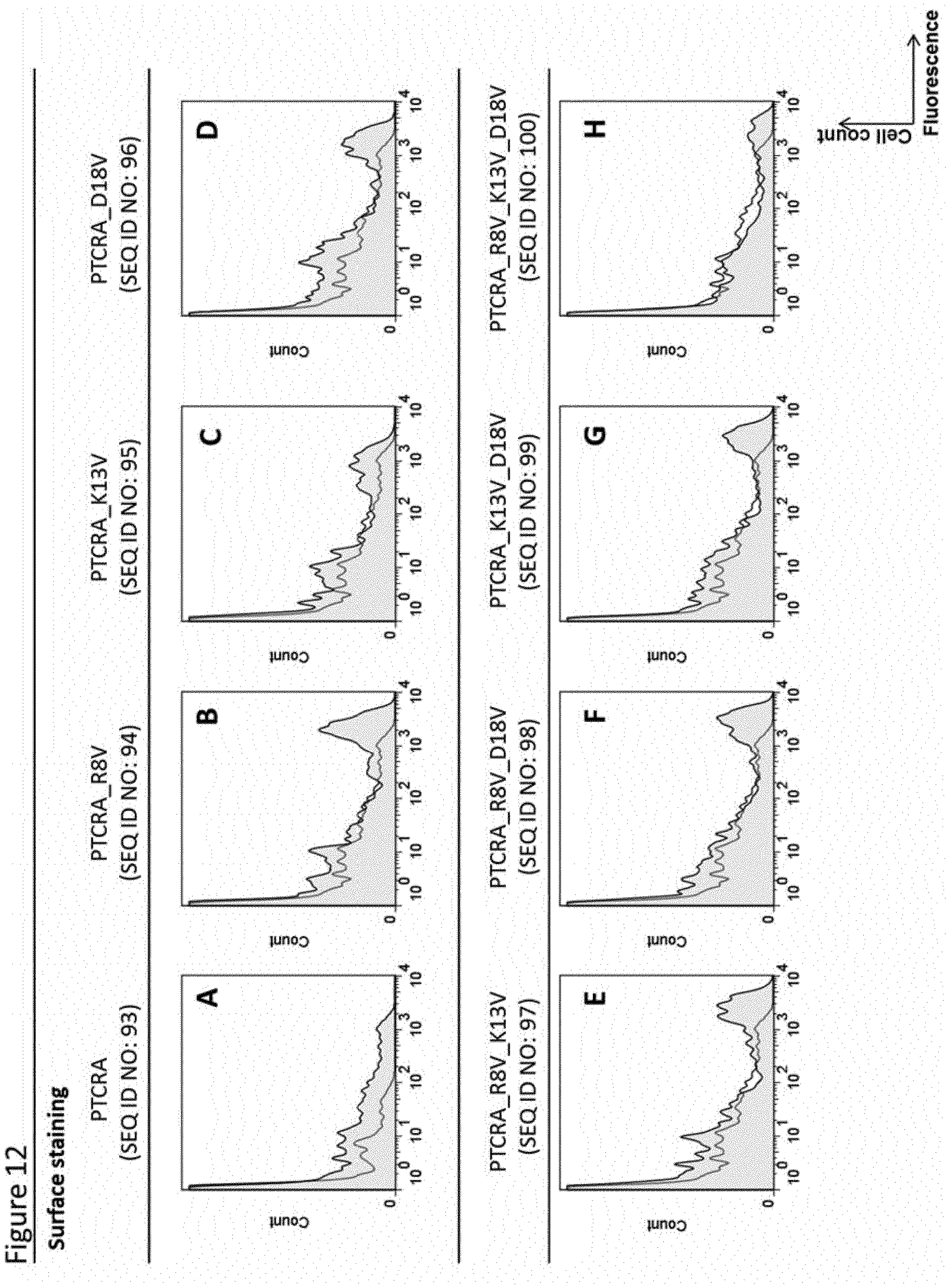
Figure 12
Surface staining
PTCRA
(SEQ ID NO: 93)
PTCRA_R8V
(SEQ ID NO: 94)
PTCRA_K13V
(SEQ ID NO: 95)
PTCRA_D18V
(SEQ ID NO: 96)
PTCRA_R8V_K13V
(SEQ ID NO: 97)
PTCRA_R8V_D18V
(SEQ ID NO: 98)
PTCRA_K13V_D18V
(SEQ ID NO: 99)
PTCRA_R8V_K13V_D18V
(SEQ ID NO: 100)
A
B
C
D
E
F
G
H
Count
0
$10^0$
$10^1$
$10^2$
$10^3$
$10^4$
Cell count
Fluorescence

Figure 12 (contd)

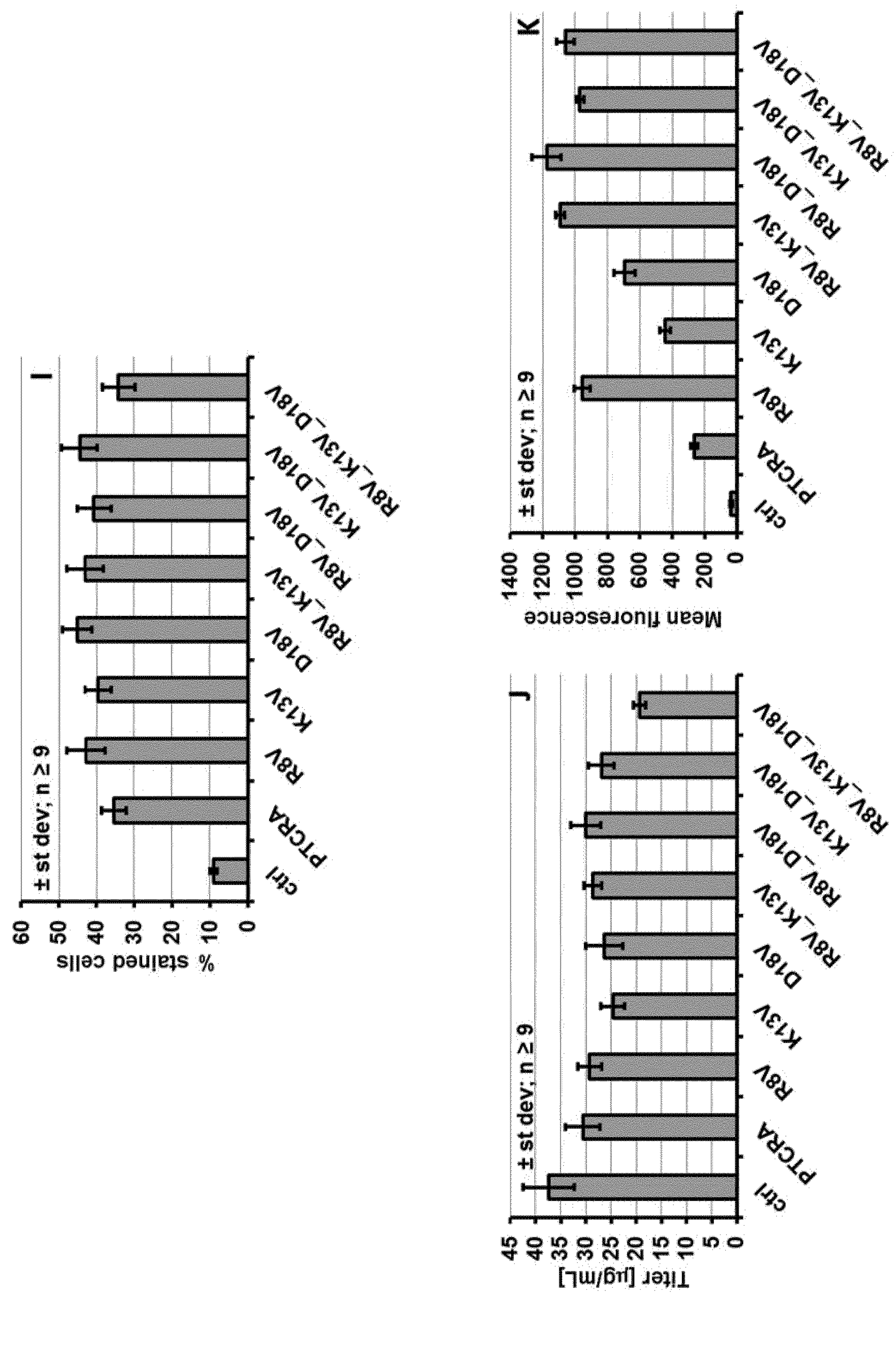

Figure 13

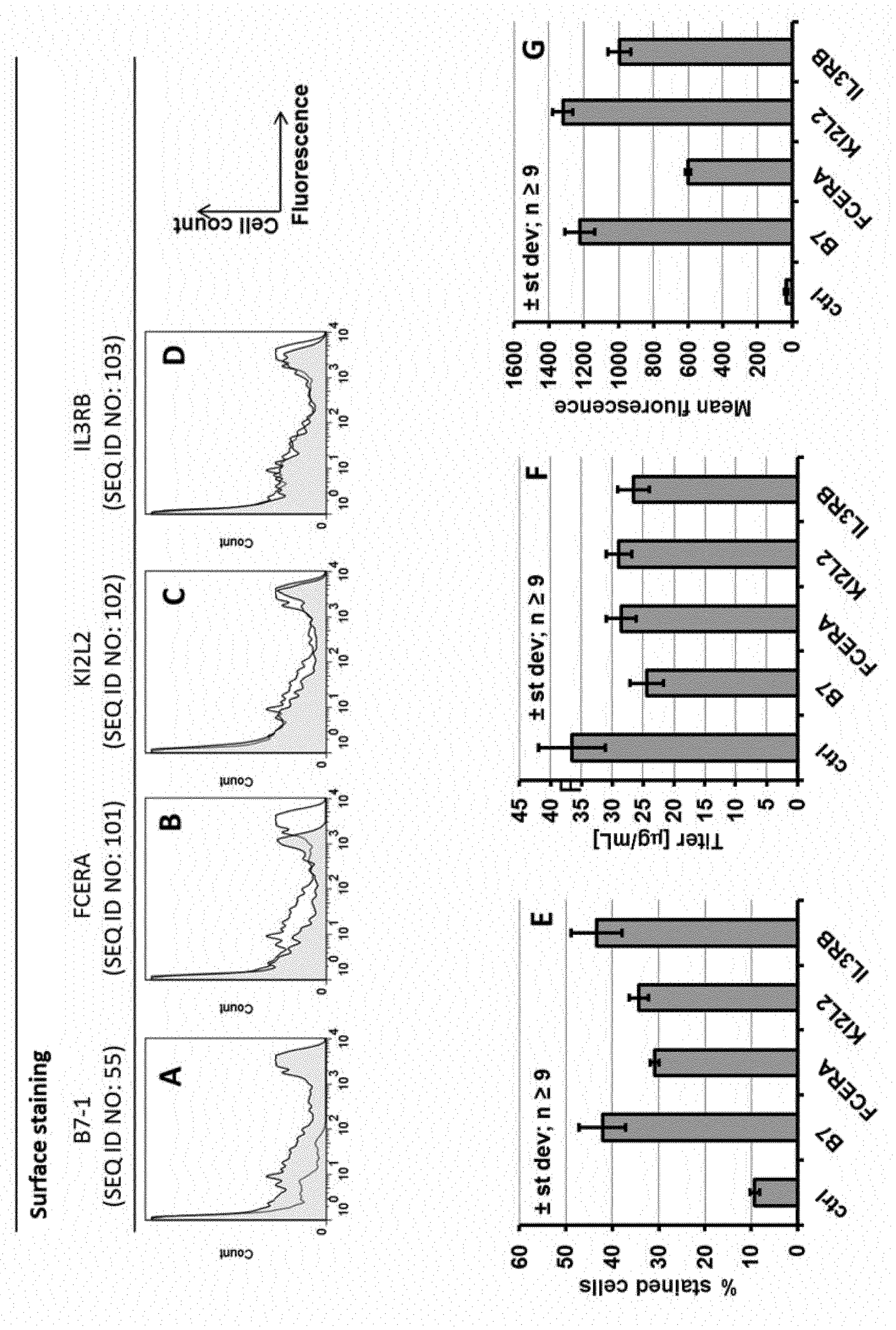
Surface staining
B7-1
(SEQ ID NO: 55)
FCERA
(SEQ ID NO: 101)
KI2L2
(SEQ ID NO: 102)
IL3RB
(SEQ ID NO: 103)
A
B
C
D
Count
Cell count
Fluorescence
E
F
G
± st dev; n ≥ 9
% stained cells
Titer [µg/mL]
Mean fluorescence
ctrl
B7
FCERA
KI2L2
IL3RB

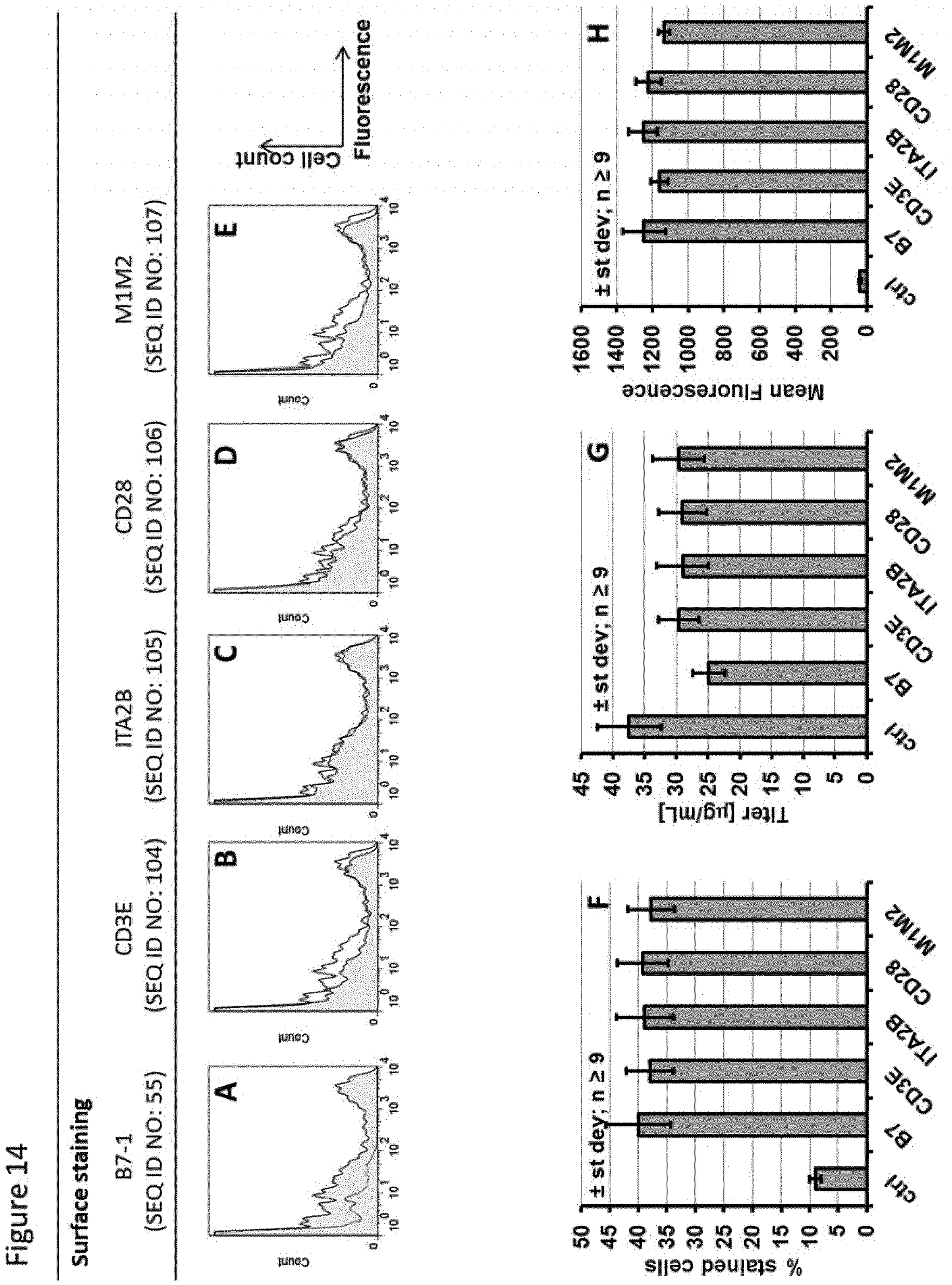

Figure 14
Surface staining
B7-1
(SEQ ID NO: 55)
CD3E
(SEQ ID NO: 104)
ITA2B
(SEQ ID NO: 105)
CD28
(SEQ ID NO: 106)
M1M2
(SEQ ID NO: 107)
A
B
C
D
E
Count
Cell count
Fluorescence
F
% stained cells
± st dev; n ≥ 9
G
Titer [µg/mL]
± st dev; n ≥ 9
H
Mean Fluorescence
± st dev; n ≥ 9
ctrl
B7
CD3E
ITA2B
CD28
M1M2

Figure 15

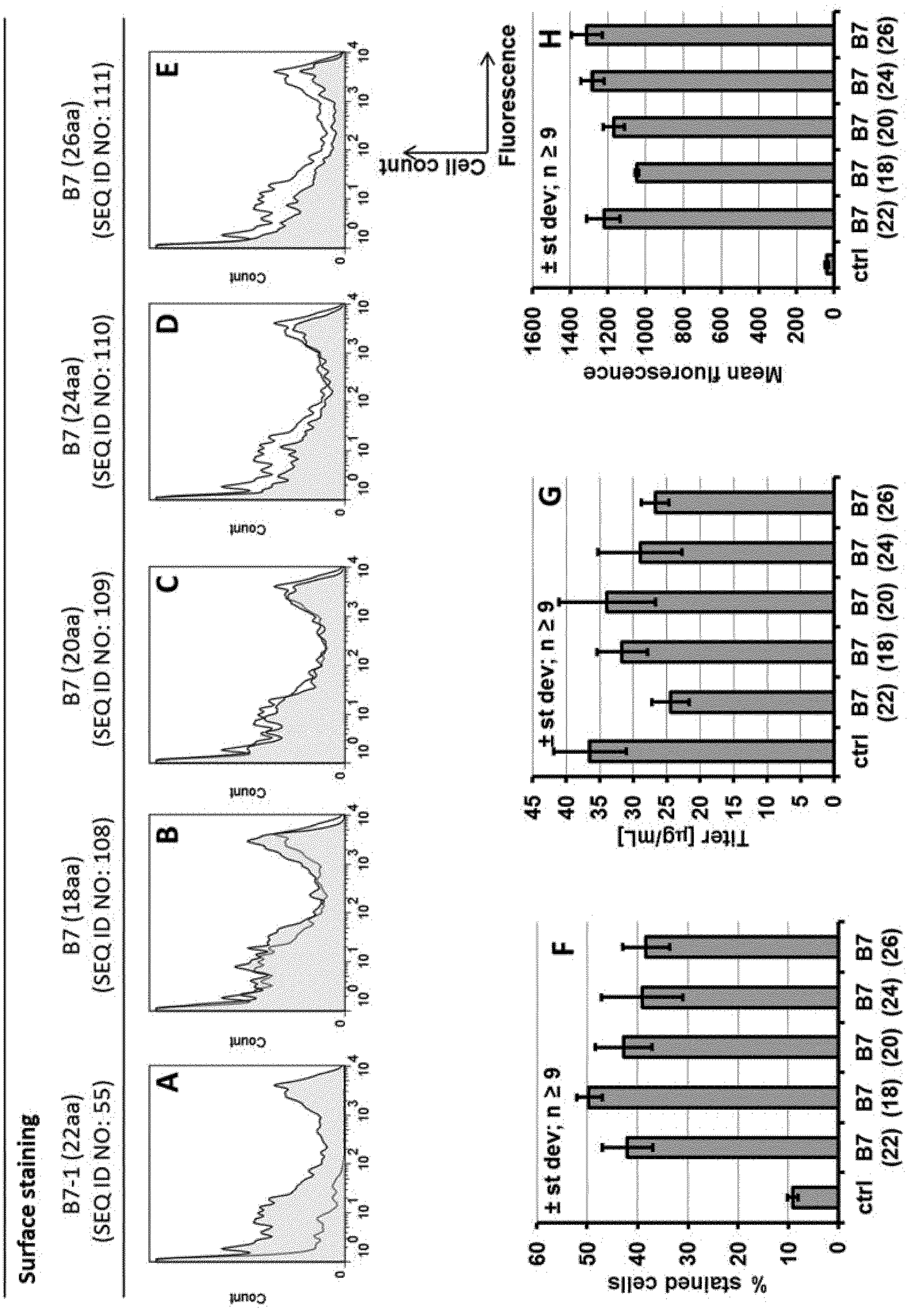
Surface staining
B7-1 (22aa)
(SEQ ID NO: 55)
B7 (18aa)
(SEQ ID NO: 108)
B7 (20aa)
(SEQ ID NO: 109)
B7 (24aa)
(SEQ ID NO: 110)
B7 (26aa)
(SEQ ID NO: 111)
A
B
C
D
E
Count
Cell count
Fluorescence
F
G
H
± st dev; n ≥ 9
% stained cells
Titer [µg/mL]
Mean fluorescence
ctrl
B7 (22)
B7 (18)
B7 (20)
B7 (24)
B7 (26)

Figure 16

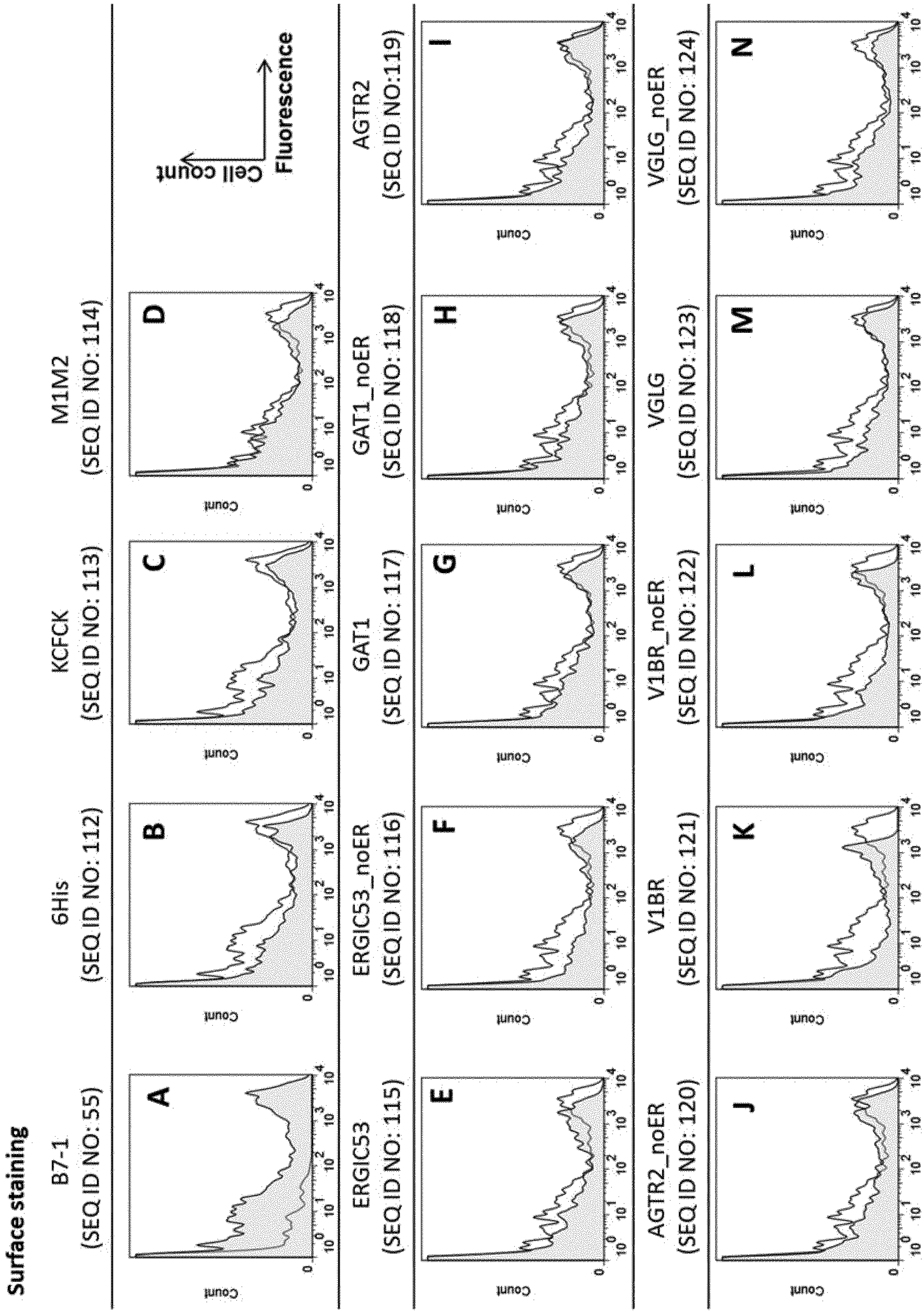
Surface staining
B7-1
(SEQ ID NO: 55)
6His
(SEQ ID NO: 112)
KCFCK
(SEQ ID NO: 113)
M1M2
(SEQ ID NO: 114)
ERGIC53
(SEQ ID NO: 115)
ERGIC53_noER
(SEQ ID NO: 116)
GAT1
(SEQ ID NO: 117)
GAT1_noER
(SEQ ID NO: 118)
AGTR2
(SEQ ID NO:119)
AGTR2_noER
(SEQ ID NO: 120)
V1BR
(SEQ ID NO: 121)
V1BR_noER
(SEQ ID NO: 122)
VGLG
(SEQ ID NO: 123)
VGLG_noER
(SEQ ID NO: 124)
A
B
C
D
E
F
G
H
I
J
K
L
M
N
Cell count
Fluorescence
Count

Figure 16 (contd)

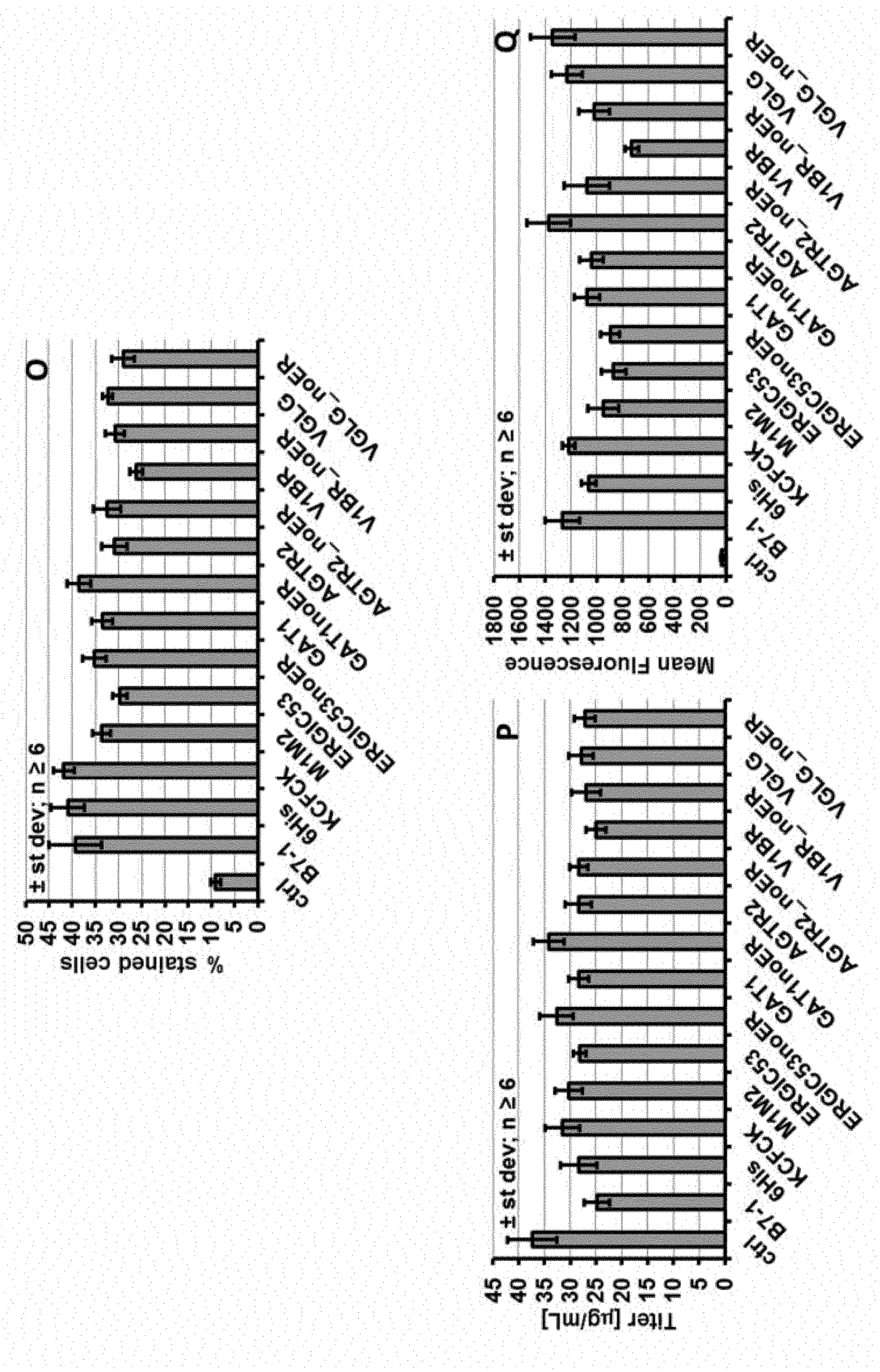

Figure 17

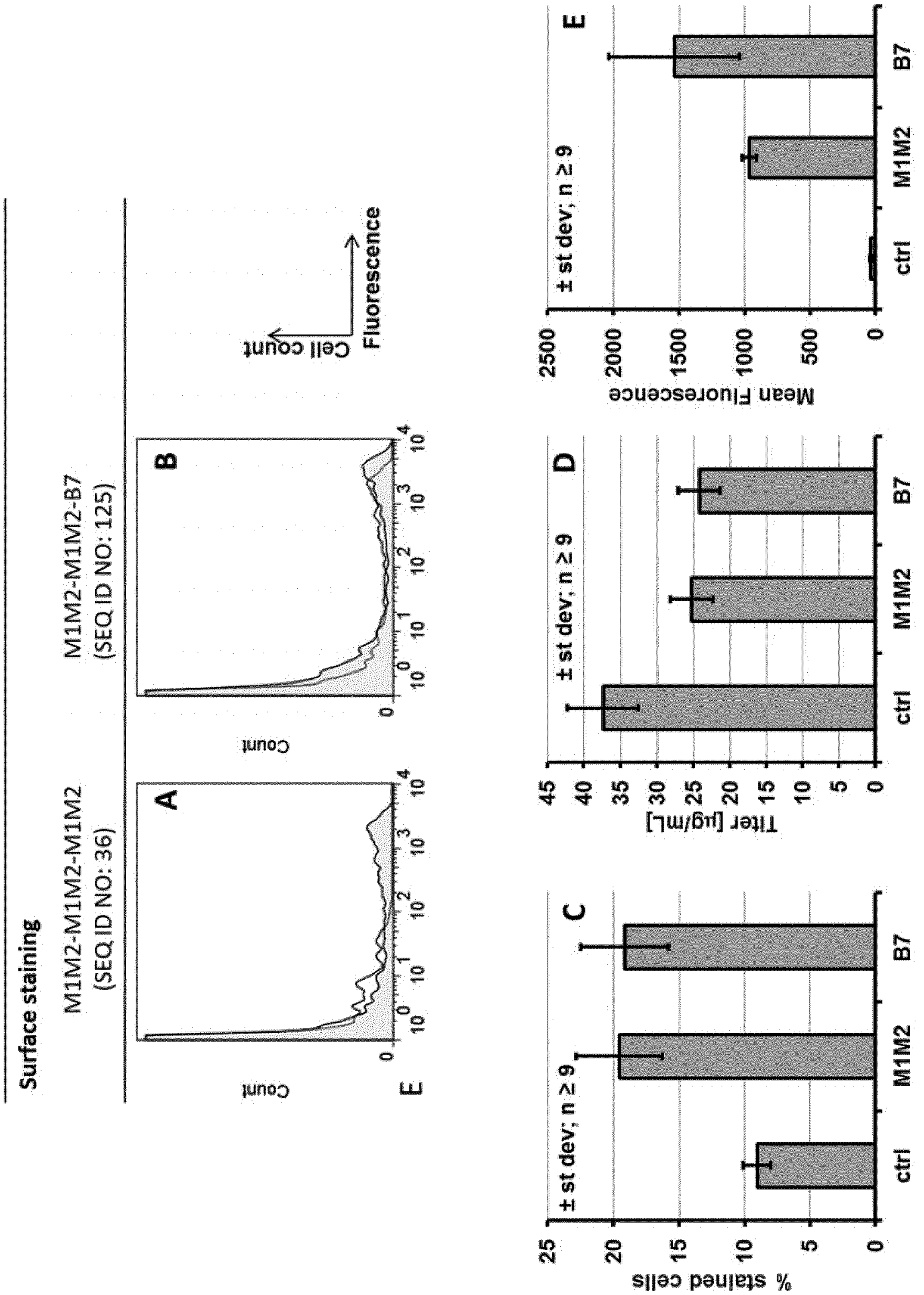
Surface staining
M1M2-M1M2-M1M2
(SEQ ID NO: 36)
M1M2-M1M2-B7
(SEQ ID NO: 125)
A
B
Count
Cell count
Fluorescence
C
D
E
± st dev; n ≥ 9
% stained cells
Titer [µg/mL]
Mean Fluorescence
ctrl
M1M2
B7

Figure 18

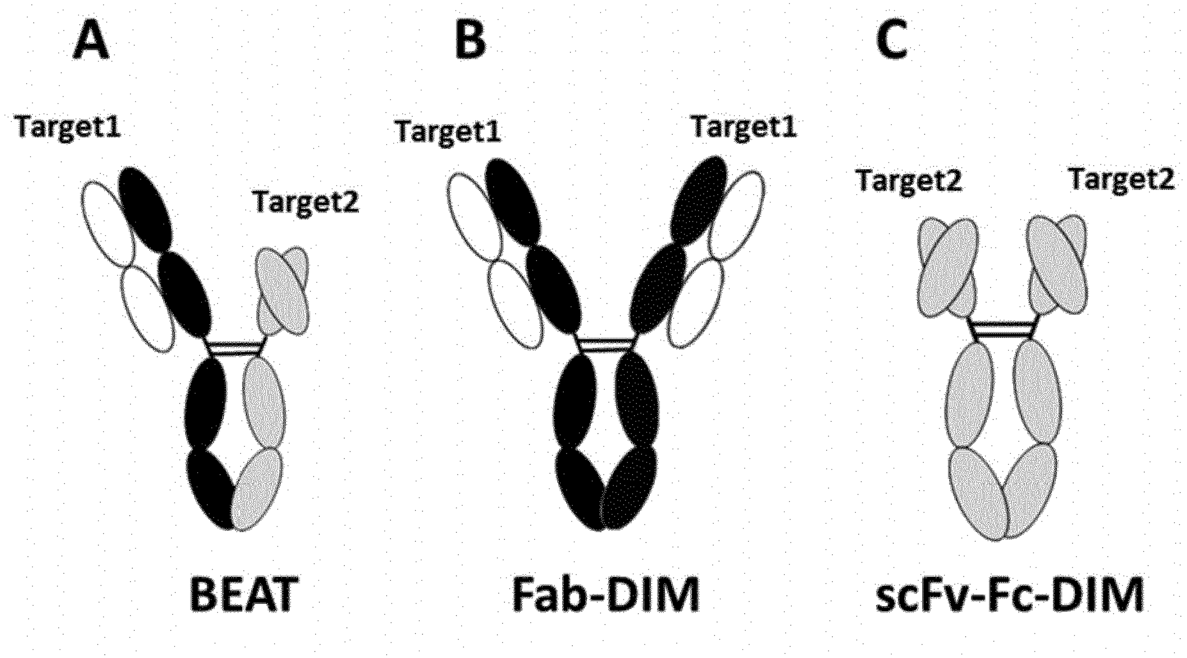
A
B
C
Target1
Target2
Target1
Target1
Target2
Target2
BEAT
Fab-DIM
scFv-Fc-DIM

Figure 19

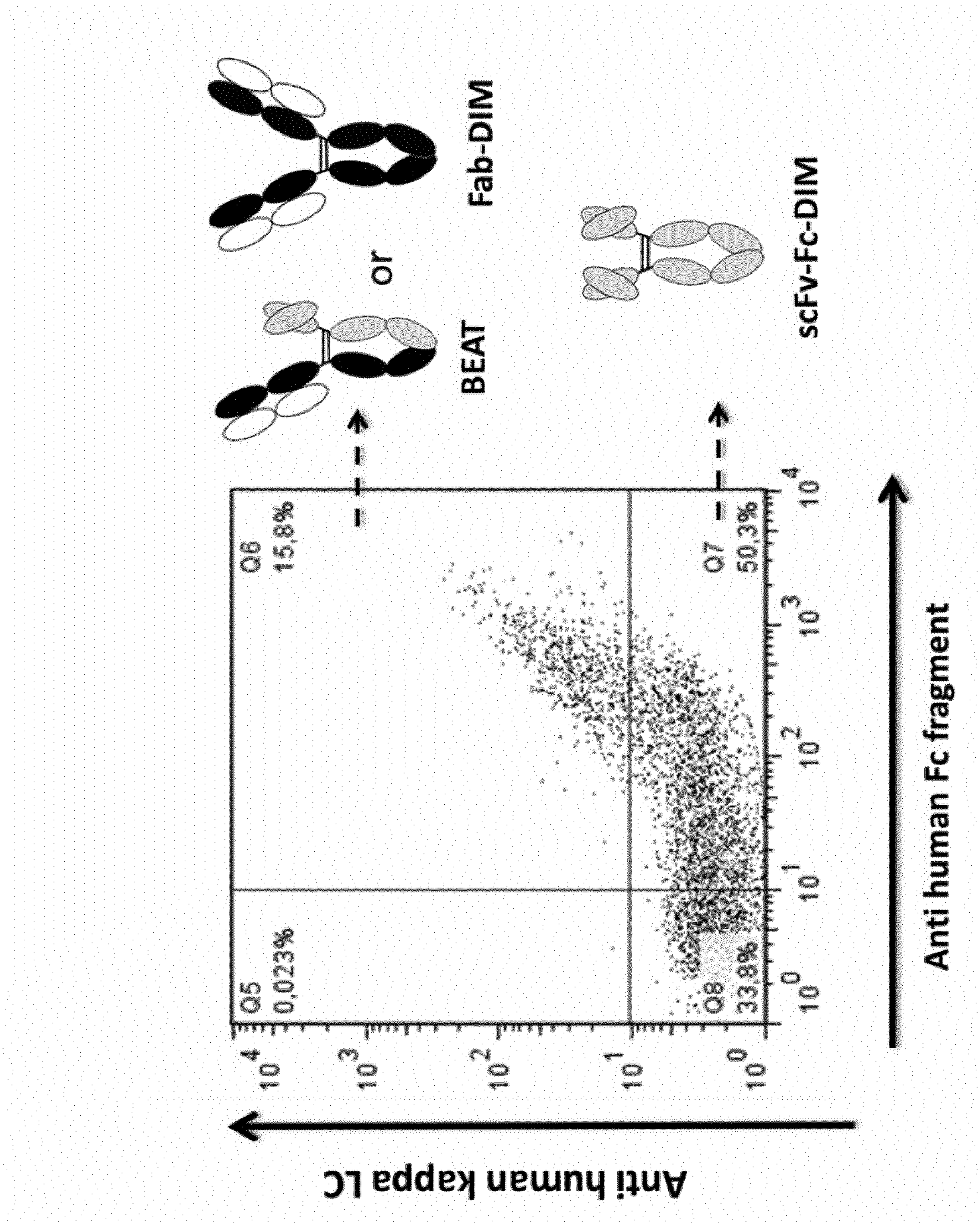
Anti human kappa LC
Anti human Fc fragment
Q5
0,023%
Q6
15,8%
Q7
50,3%
Q8
33,8%
BEAT
or
Fab-DIM
scFv-Fc-DIM

Figure 20

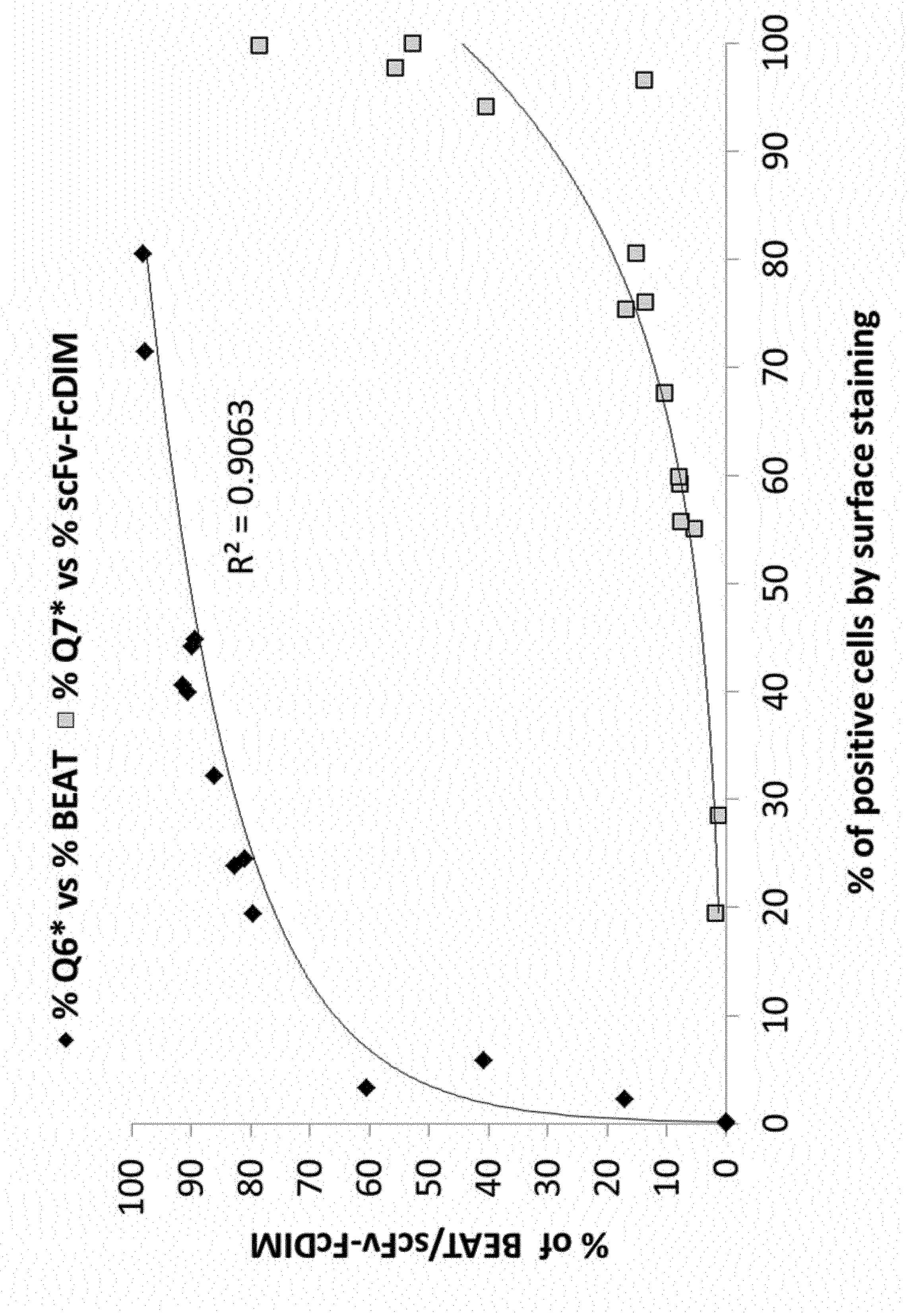
% Q6* vs % BEAT
% Q7* vs % scFv-FcDIM
$R^2$ = 0.9063
% of BEAT/scFv-FcDIM
% of positive cells by surface staining
0
10
20
30
40
50
60
70
80
90
100

Figure 21

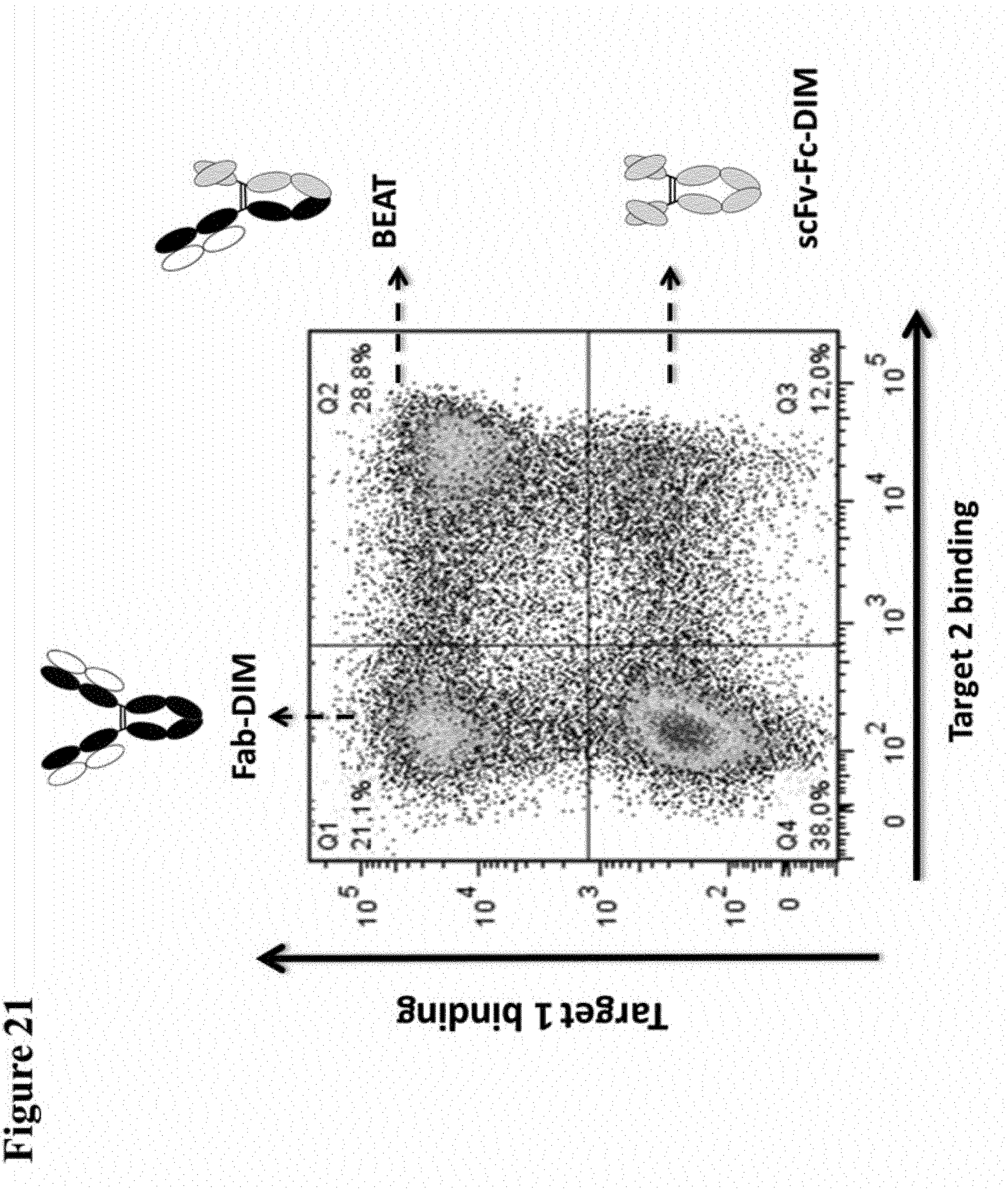
Target 1 binding
Target 2 binding
Q1
21.1%
Q2
28.8%
Q3
12.0%
Q4
38.0%
Fab-DIM
BEAT
scFv-Fc-DIM
0
$10^2$
$10^3$
$10^4$
$10^5$

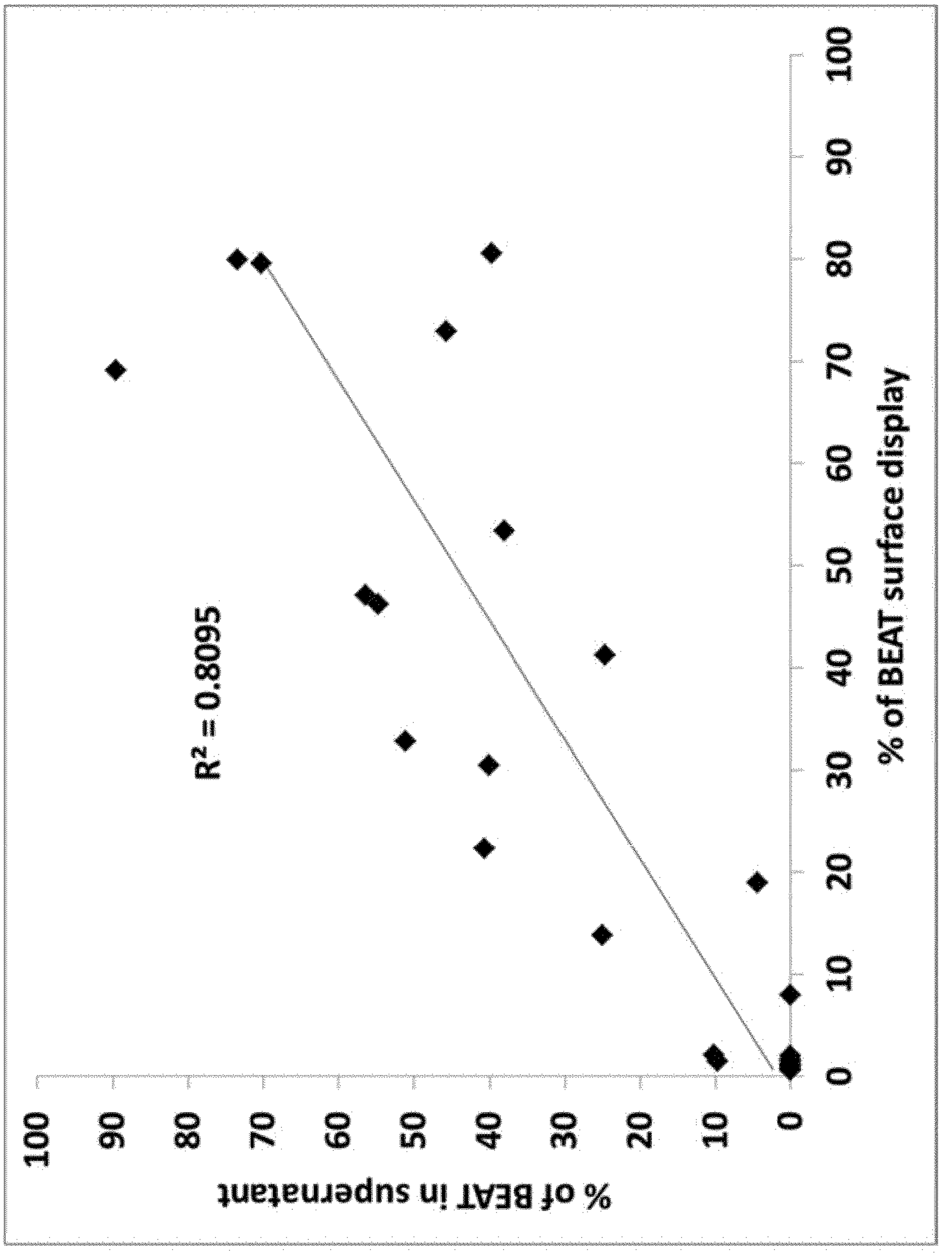
% of BEAT in supernatant
% of BEAT surface display
$R^2 = 0.8095$
0
10
20
30
40
50
60
70
80
90
100

Figure 22

Figure 23

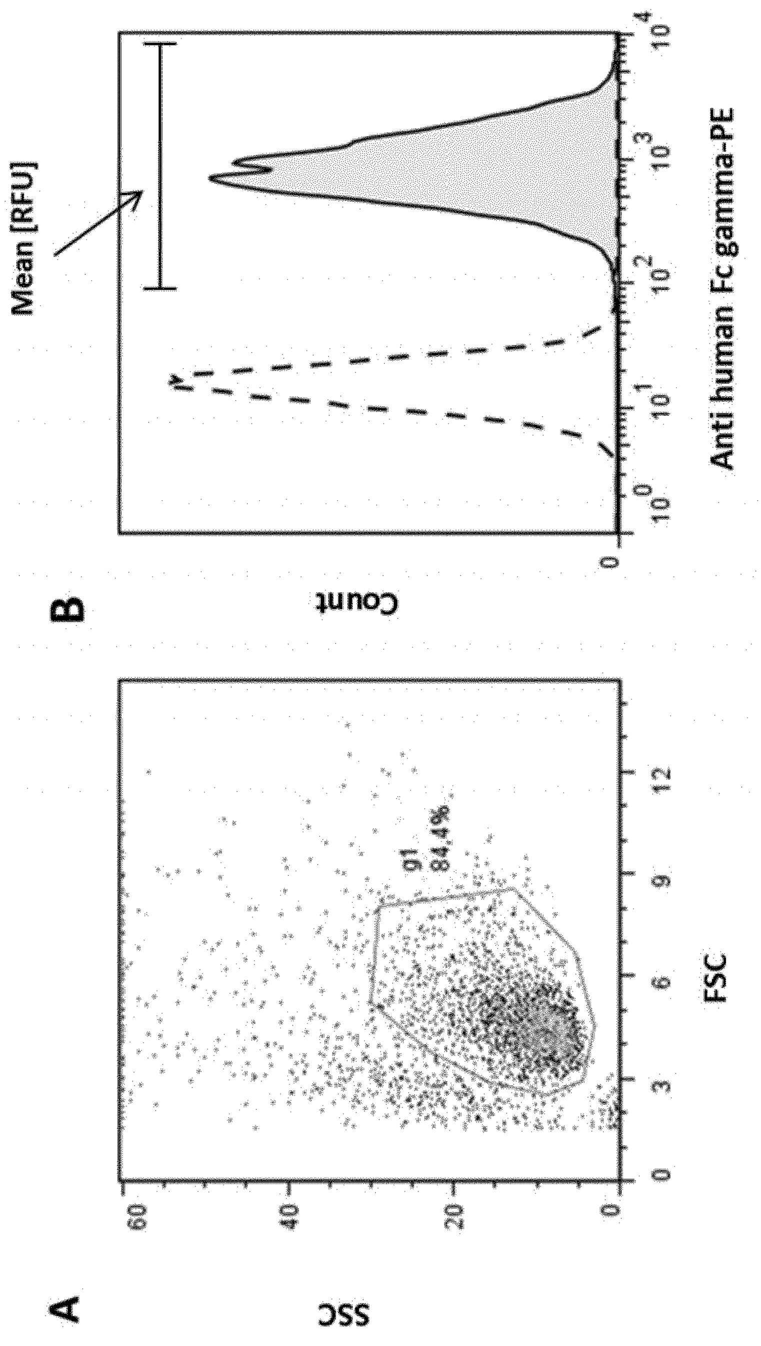

Figure 24

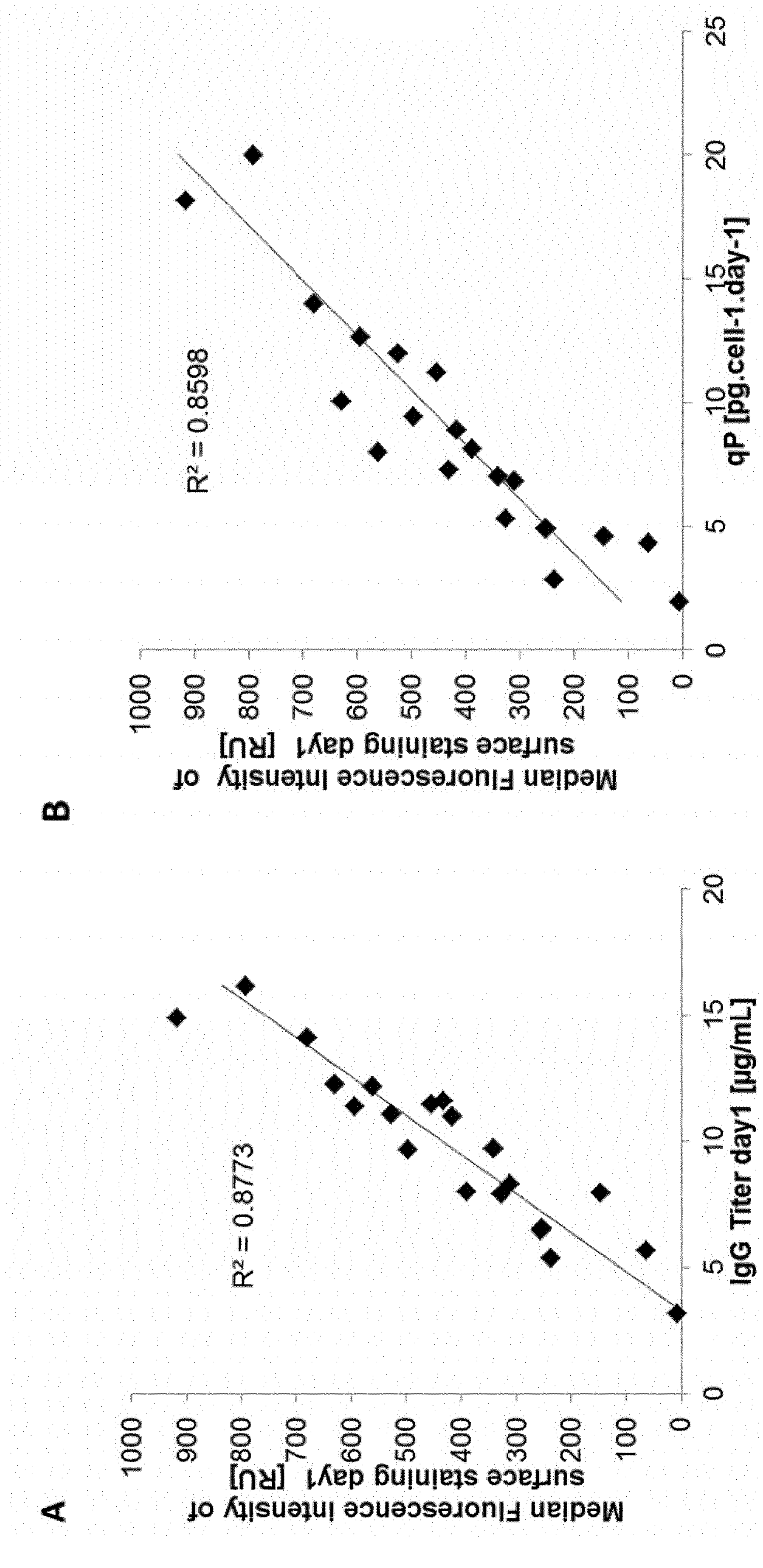
A
Median Fluorescence Intensity of surface staining day1 [RU]
1000
900
800
700
600
500
400
300
200
100
0
0
5
10
15
20
IgG Titer day1 [µg/mL]
R² = 0.8773
B
Median Fluorescence Intensity of surface staining day1 [RU]
1000
900
800
700
600
500
400
300
200
100
0
0
5
10
15
20
25
qP [pg.cell-1.day-1]
R² = 0.8598

Figure 25

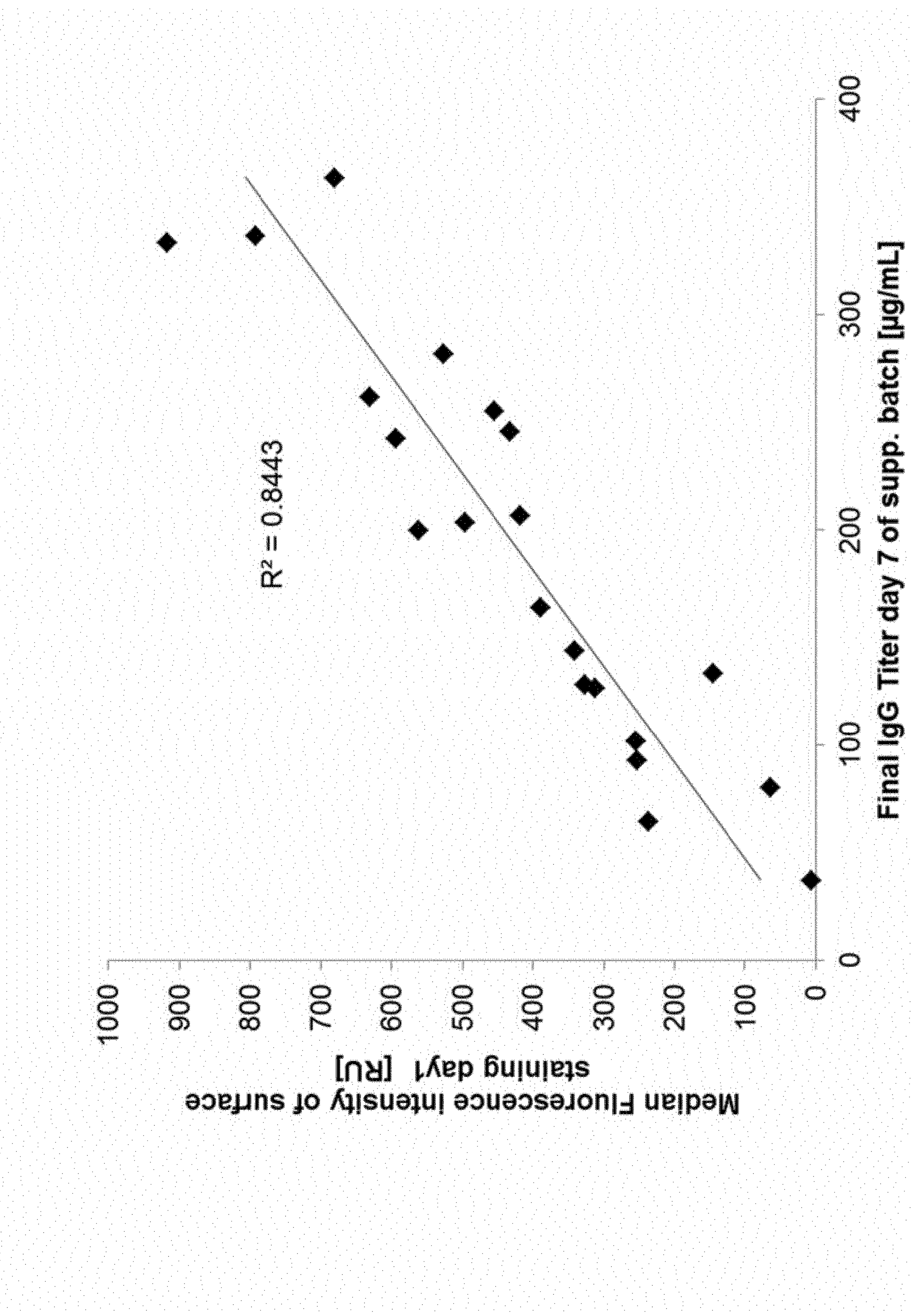

Median Fluorescence intensity of surface staining day1 [RU]
0
100
200
300
400
500
600
700
800
900
1000
R² = 0.8443
0
100
200
300
400
Final IgG Titer day 7 of supp. batch [µg/mL]

Figure 26

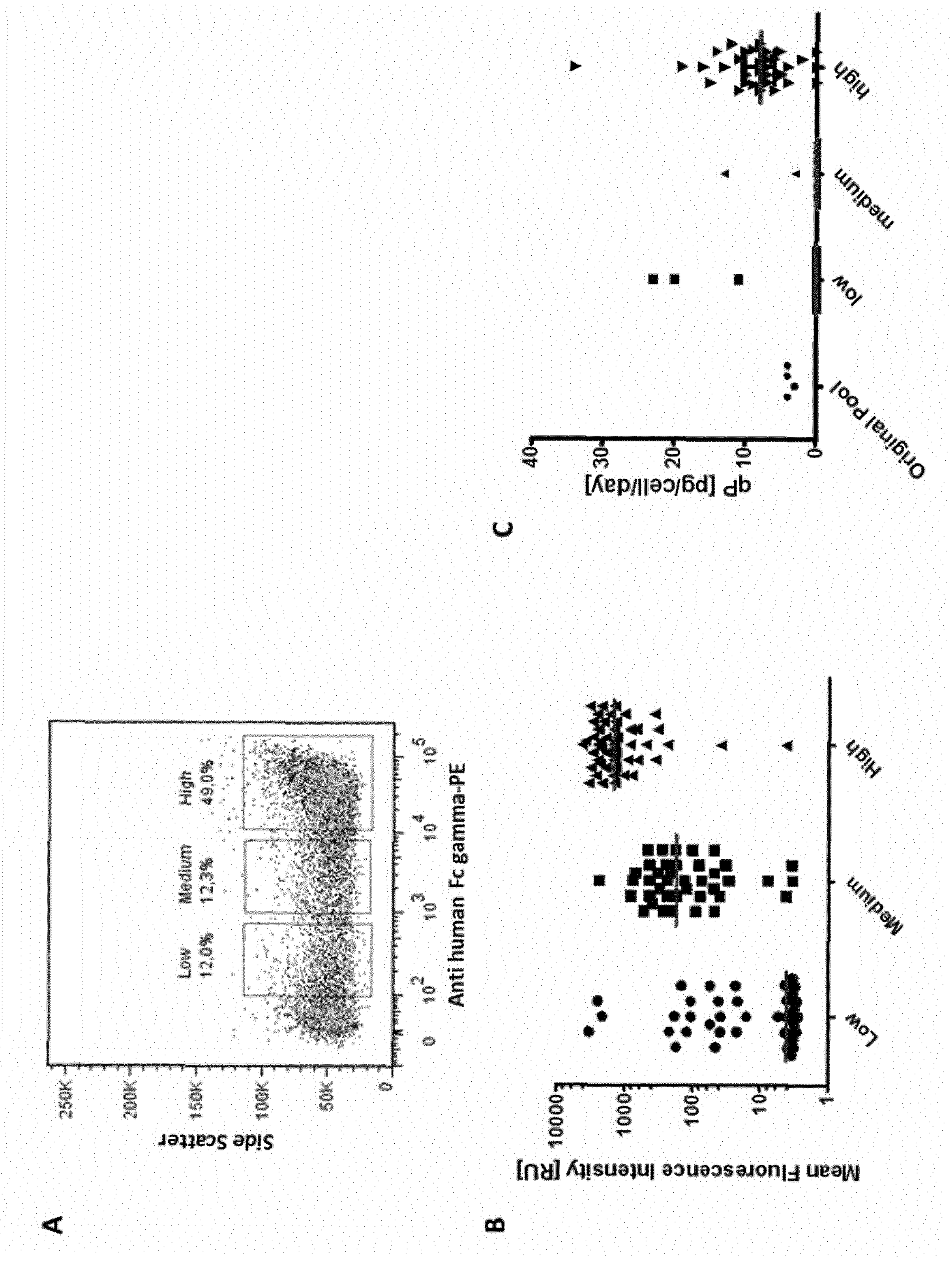
A
Side Scatter
250K
200K
150K
100K
50K
0
Low
12,0%
Medium
12,3%
High
49,0%
0
$10^2$
$10^3$
$10^4$
$10^5$
Anti human Fc gamma-PE
B
Mean Fluorescence Intensity [RU]
10000
1000
100
10
1
Low
Medium
High
C
qP [pg/cell/day]
40
30
20
10
0
Original Pool
low
medium
high

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 20019151605 A
- US 5024939 A
- WO 2005089285 A
- WO 2007131774 A
- US 9209965 W
- WO 9413804 A
- US 5807706 A
- WO 2007110205 A
- WO 2007147901 A
- WO 2009089004 A
- WO 2012131555 A

### Non-patent literature cited in the description


- **COOPER TA ; ORDAHL CP.** *J Biol Chem,* 1985, vol. 260 (20), 11140-8
- **ORENGO JP et al.** *Nucleic Acids Res,* 2006, vol. 34 (22), e148
- **MAJOR JG et al.** *Mol. Immunol.,* 1996, vol. 33, 179-87
- **COWLING VH.** *Biochem J,* 2010, vol. 425, 295-302
- **BOTHWELL et al.** *Cell,* 1981, vol. 24, 625-637
- **ALBERTS et al.** Molecular Biology of the Cell. Garland Publishing, 1995
- **ALBERTS et al.** Molecular Biology of the Cell. Garland Science, 2002
- **JIA J et al.** *Curr Opin Genet Dev,* 2013, vol. 23 (1), 29-34
- **EDELMAN GM et al.** *Proc Natl Acad Sci USA,* 1969, vol. 63 (1), 78-85
- **WARD ES et al.** *Nature,* 1989, vol. 341, 544-546
- **BIRD RE et al.** *Science,* 1988, vol. 242, 423-426
- **HUSTON JS et al.** *Proc. Natl. Acad. Sci. USA,* 1988, vol. 85, 5879-83
- **TOMLINSON I ; HOLLINGER P.** *Methods Enzymol.,* 2000, vol. 326, 461-79
- **HOLLIGER P et al.** *Proc Natl Acad Sci USA,* 1993, vol. 90, 6444-48
- **COLOMA MJ ; MORRISON SL.** *Nature Biotech,* 1997, vol. 15 (2), 159-163
- **MERCHANT AM et al.** *Nat Biotechnol,* 1998, vol. 16 (7), 677-81
- **GRAHAM FL et al.** *J. Gen. Virol.,* 1977, vol. 36, 59-74
- **PUCK TT et al.** *J. Exp. Med.,* 1958, vol. 108, 945-955
- **URLAUB G ; CHASIN LA.** *PNAS,* 1980, vol. 77 (7), 4216-4220
- **SIMONSEN CC ; LEVINSON AD.** *PNAS,* 1983, vol. 80 (9), 2495-2499
- **COOPER ; ORDAHL.** *J Biol Chem,* 1985, vol. 260 (20), 11140-8
- **CHOU W-C et al.** *Biotechnol Bioeng,* 1999, vol. 65, 160-9
- **LIAO K-W et al.** *Biotechnol Bioeng,* 2001, vol. 73, 313-23
- **GALLI G1 ; GUISE J ; TUCKER PW ; NEVINS JR.** Poly(A) site choice rather than splice site choice governs the regulated production of IgM heavy-chain RNAs. *Proc Natl Acad Sci U S A.,* 1988, vol. 85 (8), 2439-43
- **LIN, Y. ; CHEN, B. ; WEI-CHENG LU, W. ; CHIEN-I SU, C. ; PRIJOVICH,Z. ; CHUNG, W. ; WU, P. ; CHEN, K. ; LEE, I. ; JUAN, T.** The B7-1 Cytoplasmic Tail Enhances Intracellular Transport and Mammalian Cell Surface Display of Chimeric Proteins in the Absence of a Linear ER Export Motif. *PLoS One,* 20 September 2013, vol. 8 (9
- **BAEZA-DELGADO, C1 ; MARTI-RENOM, MA ; MINGARRO, I.** Structure-based statistical analysis of transmembrane helices. *Eur Biophys J,* 2012, vol. 42 (2-3), 199-207
- **WHITE, SH ; WIMLEY, WC.** Membrane protein folding and stability: physical principles. *Annu Rev Biophys Biomol Struct.,* 1999, vol. 28, 319-65